# EUROPEAN PATENT SPECIFICATION

(11) **EP 1 831 198 B1**
(45) Date of publication and mention of the grant of the patent: **08.04.2009**
(21) Application number: 05820952.9
(22) Date of filing: 22.12.2005
(51) Int. Cl.: C07D 401/12, C07D 401/14, C07D 213/75, A61K 31/44, A61K 31/4425, A61P 35/00

(54) **PYRIDINE CARBOXAMIDE DERIVATIVES FOR USE AS ANTICANCER AGENTS**
PYRIDINCARBONSÄUREAMIDDERIVATE ZUR VERWENDUNG ALS ANTIKREBSMITTEL
DÉRIVÉS DE PYRIDINECARBOXAMIDE EMPLOYÉS EN TANT QU'AGENTS ANTICANCÉREUX

(30) Priority: 22.12.2004 US 639234 P
(43) Date of publication of application: 12.09.2007
(73) Proprietor: AstraZeneca AB, 151 85 Södertälje (SE)
(72) Inventor: ALMEIDA, Lynsie, AstraZeneca R & D Boston, Waltham, Massachusetts 02451 (US); AQUILA, Brian, AstraZeneca R & D Boston, Waltham, Massachusetts 02451 (US); COOK, Don, AstraZeneca R & D Boston, Waltham, Massachusetts 02451 (US); COWEN, Scott, AstraZeneca R & D Boston, Waltham, Massachusetts 02451 (US); DAKIN, Les, AstraZeneca R & D Boston, Waltham, Massachusetts 02451 (US); EZHUTHACHAN, Jayachandran, AstraZeneca R & D Boston, Waltham, Massachusetts 02451 (US); IOANNIDIS, Stephanos, AstraZeneca R & D Boston, Waltham, Massachusetts 02451 (US); LEE, John W, AstraZeneca R & D Boston, Waltham, Massachusetts 02451 (US); LEE, Stephen, AstraZeneca R & D Boston, Waltham, Massachusetts 02451 (US); LYNE, Paul Dermot, AstraZeneca R & D Boston, Waltham, Massachusetts 02451 (US); PONTZ, Timothy, AstraZeneca R & D Boston, Waltham, Massachusetts 02451 (US); SCOTT, David, AstraZeneca R & D Boston, Waltham, Massachusetts 02451 (US); SU, Mei, AstraZeneca R & D Boston, Waltham, Massachusetts 02451 (US); ZHENG, Xiaolan, AstraZeneca R & D Boston, Waltham, Massachusetts 02451 (US)
(74) Representative: Bryant, Tracey
(86) International application number: PCT/GB2005/004986
(87) International publication number: WO 2006/067446

(56) References cited:
- WO-A-00/55120
- US-A1- 2004 087 626

## Description

The invention relates to chemical compounds, or pharmaceutically acceptable salts thereof, which possess B-Raf inhibitory activity and are accordingly useful for their anti-cancer activity and thus in methods of treatment of the human or animal body. The invention also relates to processes for the manufacture of said chemical compounds, to pharmaceutical compositions containing them and to their use in the manufacture of medicaments of use in the production of an anti-cancer effect in a warm-blooded animal such as man.

The classical Ras, Raf, MAP protein kinase/extracellular signal -regulated kinase kinase (MEK), extracellular signal -regulated kinase (ERK) pathway plays a central role in the regulation of a variety of cellular functions dependent upon cellular context, including cellular proliferation, differentiation, survival, immortalization and angiogenesis (reviewed in Peyssonnaux and Eychene, Biology of the Cell, 2001, 93, 3-62). In this pathway, Rat family members are recruited to the plasma membrane upon binding to guanosine triphosphate (GTP) loaded Ras resulting in the phosphorylation and activation of Raf proteins. Activated Rafs then phosphorylate and activate MEKs, which in turn phosphorylate and activate ERKs. Upon activation, ERKs translocate from the cytoplasm to the nucleus resulting in the phosphorylation and regulation of activity of transcription factors such as Elk-1 and Myc.

The Ras/Raf/MEK/ERK pathway has been reported to contribute to the tumorigenic phenotype by inducing immortalisation, growth factor-independent growth, insensitivity to growth-inhibitory signals, ability to invade and metastasis, stimulating angiogenesis and inhibition of apoptosis (reviewed in Kolch et al., Exp.Rev. Mol. Med., 2002, 25 April, http://www.expertreviews.org/02004386h.htm). In fact, ERK phosphorylation is enhanced in approximately 30% of all human tumours (Hoshino et al., Oncogene, 1999,18, 813-822). This may be a result of overexpression and/or mutation of key members of the pathway.

Three Raf serine/threonine protein kinase isoforms have been reported Kaf-1 /c-Raf, B-Raf and A-Raf (reviewed in Mercer and Pritchard, Biochim. Biophys. Acta, 2003, 1653, 25-40), the genes for which are thought to have arisen from gene duplication. All three Raf genes are expressed in most tissues with high-level expression of B-Raf in neuronal tissue and A-Raf in urogenital tissue. The highly homologous Raf family members have overlapping but distinct biochemical activities and biological functions (Hagemann and Rapp, Expt. Cell Res. 1999, 253, 34-46). Expression of all three Raf genes is required for normal murine development however both c-Raf and B-Raf are required to complete gestation. B-Raf-/- mice die at E12.5 due to vascular haemorrhaging caused by increased apoptosis of endothelial cells (Wojnowski et al., Nature Genet., 1997, 16, 293-297). B-Raf is reportedly the major isoform involved in cell proliferation and the primary target of oncogenic Ras. Activating somatic missense mutations have been identified exclusively for B-Raf, occurring with a frequency of 66% in malignant cutaneous melanomas (Davies et al., Nature, 2002, 417, 949-954) and also present in a wide range of human cancers, including but not limited to papillary thyroid tumours (Cohen et al., J. Natl. Cancer Inst., 2003, 95, 625-627), cholangiocarcinomas (Tannapfel et al., Gut, 2003, 52, 706-712), colon and ovarian cancers (Davies et al., Nature, 2002, 417, 949-954). The most frequent mutation in B-Raf (80%) is a glutamic acid for valine substitution at position 600. These mutations increase the basal kinase activity of B-Raf and are thought to uncouple Raf/MEK/ERK signalling from upstream proliferation drives including Ras and growth factor receptor activation resulting in constitutive activation of ERK. Mutated B-Raf proteins are transforming in NIH3T3 cells (Davies et al., Nature, 2002, 417, 949-954) and melanocytes (Wellbrock et al., Cancer Res., 2004, 64, 2338-2342) and have also been shown to be essential for melanoma cell viability and transformation (Hingorani et al., Cancer Res., 2003, 63, 5198-5202). As a key driver of the Raf/MEK/ERK signalling cascade, B-Raf represents a likely point of intervention in tumours dependent on this pathway. US-A-2004/087626 also disclose compounds for the inhibition of Raf kinase.

AstraZeneca application WO 00/55120 discloses certain amide derivatives which are inhibitors of the production of cytokines such as TNF, in particular of TNFα, and various interleukins, in particular IL-1. The present inventors have surprisingly found that certain other, novel, amide derivatives are potent B-Raf inhibitors and are accordingly expected to be useful in the treatment of neoplastic disease.

Accordingly, the present invention provides a compound of formula **(I)**: 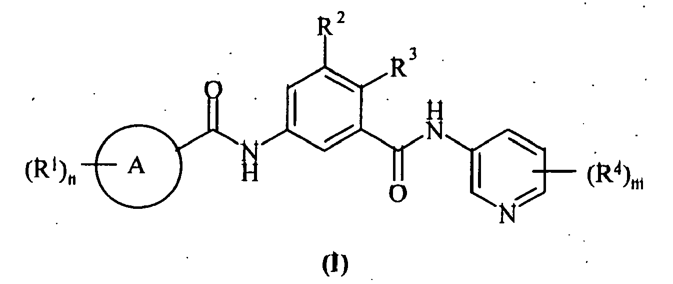 wherein:
**Ring A** is phenyl, or a monocyclic 5 or 6 membered fully-unsaturated heterocyclic ring; wherein said phenyl or heterocyclic ring may be optionally fused to a five or six membered carbocyclyl or heterocyclyl forming a bicyclic ring; and wherein if said heterocyclyl or heterocyclic ring contains an -NH- moiety that nitrogen may be optionally substituted by a group selected from R⁵;
**R¹** is a substituent on carbon and is selected from halo, nitro, cyano, hydroxy, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N,N*-(C₁₋₆alkyl)₂amino, C₁₋₆alkanoylamino, *N*-(C₁₋₆alkyl)carbamoyl, *N,N-*(C₁₋₆alkyl)₂carbamoyl, C₁₋₆alkylS(O)ₐ wherein a is 0 to 2, C₁₋₆alkoxycarbonyl, C₁₋₆alkoxycarbonylamino, *N*-(C₁₋₆alkyl)sulphamoyl, *N,N-*(C₁₋₆alkyl)₂sulphamoyl, *N*-(C₁₋₆alkyl)-N(C₁₋₆alkoxy)sulphamoyl, *N,N*'-(C₁₋₆alkyl)₂ureido, *N*',*N*'-(C₁₋₆alkyl)₂ureido, *N*-(C₁₋₆alkyl)-*N*',*N*'-(C₁₋₆alkyl)₂ureido, C₁₋₆alkylsulphonylamino, carbocyclyl-R⁶- or heterocyclyl-R⁷-; wherein R¹ may be optionally substituted on carbon by one or more R⁸; and wherein if said heterocyclyl contains an -NH- moiety that nitrogen may be optionally substituted by a group selected from R⁹;
**n** is selected from 0-4; wherein the values of R¹ may be the same or different;
**R²** is selected from hydrogen, halo, nitro, cyano, hydroxy, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N,N*-(C₁₋₆alkyl)₂amino, C₁₋₆alkanoylamino, *N*-(C₁₋₆alkyl)carbamoyl, *N,N*-(C₁₋₆alkyl)₂carbamoyl, C₁₋₆alkylS(O)ₐ wherein a is 0 to 2, C₁₋₆alkoxycarbonyl, *N*-(C₁₋₆alkyl)sulphamoyl, *N,N*-(C₁₋₆alkyl)₂sulphamoyl, C₁₋₆alkylsulphonylamino, carbocyclyl-R¹⁰- or heterocyclyl-R¹¹-; wherein R² may be optionally substituted on carbon by one or more R¹²; and wherein if said heterocyclyl contains an -NH- moiety that nitrogen may be optionally substituted by a group selected from R¹³;
**R³** is selected from halo, hydroxy, cyano, methyl, methoxy or hydroxymethyl;
**R⁴** is selected from halo, nitro, cyano, hydroxy, trifluoromethoxy, amino, carboxy, carbamoyl, mercapto, sulphamoyl, ureido, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N,N*-(C₁₋₆alkyl)₂amino, C₁₋₆alkanoylamino, *N*-(C₁₋₆alkyl)carbamoyl, *N,N*-(C₁₋₆alkyl)₂carbamoyl, C₁₋₆alkylS(O)ₐ wherein a is 0 to 2, C₁₋₆alkoxycarbonyl, *N*-(C₁₋₆alkyl)sulphamoyl, *N,N*-(C₁₋₆alkyl)₂sulphamoyl, C₁₋₆alkylsulphonylamino, carbocyclyl-R¹⁴- or heterocyclyl-R¹⁵-; wherein R⁴ may be optionally substituted on carbon by one or more R¹⁶; and wherein if said heterocyclyl contains an -NH- moiety that nitrogen may be optionally substituted by a group selected from R¹⁷;
**m** is selected from 0-4; wherein the values of R⁴ may be the same or different;
**R⁸** and **R¹²** are independently selected from halo, nitro, cyano, hydroxy, trifluoromethoxy, amino, carboxy, carbamoyl, mercapto, sulphamoyl, C₁₋₆alkyl, C₂₋₆alkenyl, C₂₋₆alkynyl,C₁₋₆alkoxy, C₁₋₆alkanoyl, C₁₋₆alkanoyloxy, *N*-(C₁₋₆alkyl)amino, *N,N-*(C₁₋₆alkyl)₂amino, *N*-(d₁₋₆alkyl)-*N*-(C₁₋₆alkoxy)amino, C₁₋₆alkanoylamino, *N*-(C₁₋₆alkyl)carbamoyl, *N,N*-(C₁₋₆alkyl)₂carbamoyl, C₁₋₆alkylS(O)ₐ wherein a is 0 to 2, C₁₋₆alkoxycarbonyl, *N*-(C₁₋₆alkyl)sulphamoyl, *N,N-*(C₁₋₆alkyl)₂sulphamoyl, C₁₋₆alkylsulphonylamino, carbocyclyl-R¹⁸- or heterocyclyl-R¹⁹-; wherein R⁸ and R¹² independently of each other may be optionally substituted on carbon by one or more R²⁰; and wherein if said heterocyclyl contains an -NH- moiety that nitrogen may be optionally substituted by a group selected from R²¹;
**R¹⁶** is selected from halo, nitro, cyano, hydroxy, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N,N*-(C₁₋₆alkyl)₂amino, C₁₋₆alkanoylamino, *N*-(C₁₋₆alkyl)carbamoyl, *N,N*-(C₁₋₆alkyl)₂carbamoyl, C₁₋₆alkylS(O)ₐ wherein a is 0 to 2, C₁₋₆alkoxycarbonyl, *N*-(C₁₋₆alkyl)sulphamoyl, *N,N*-(C₁₋₆alkyl)₂sulphamoyl, C₁₋₆alkylsulphonylamino, carbocyclyl-R²²- or heterocyclyl-R²³-; wherein R¹⁶ may be optionally substituted on carbon by one or more R²⁴; and wherein if said heterocyclyl contains an -NH- moiety that nitrogen may be optionally substituted by a group selected from R²⁵;
**R⁶, R⁷, R¹⁰, R¹¹, R¹⁴, R¹⁵, R¹⁸, R¹⁹, R²²** and **R²³** are independently selected from a direct bond, -O-, -N(R²⁶)-, -C(O)-, -N(R²⁷)C(O)-, -C(O)N(R²⁸)-, -S(O)ₛ-, -SO₂N(R²⁹)- or -N(R³⁰)SO₂-; wherein **R²⁶, R²⁷, R²⁸, R²⁹** and **R³⁰** are independently selected from hydrogen or C₁₋₆alkyl and s is 0-2;
**R⁵, R⁹, R¹³, R¹⁷, R²¹** and **R²⁵** are independently selected from C₁₋₆alkyl, C₁₋₆alkanoyl, C₁₋₆alkylsulphonyl, C₁₋₆alkoxycarbonyl, carbamoyl, *N*-(C₁₋₆alkyl)carbamoyl, *N,N*-(C₁₋₆alkyl)carbamoyl, benzyl, benzyloxycarbonyl, benzoyl and phenylsulphonyl;
**R²⁰** and **R²⁴** are independently selected from halo, nitro, cyano, hydroxy, trifluoromethoxy, trifluoromethyl, amino, carboxy, carbamoyl, mercapto, sulphamoyl, methyl, ethyl, methoxy, ethoxy, acetyl, acetoxy, methylamino, ethylamino, dimethylamino, diethylamino, *N*-methyl-*N*-ethylamino, acetylamino, *N-*methylcarbamoyl, *N-*ethylcarbamoyl, *N*,*N-*dimethylcarbamoyl, *N,N*-diethylcarbamoyl, *N*-methyl-*N*-ethylcarbamoyl, phenyl, methylthio, ethylthio, methylsulphinyl, ethylsulphinyl, mesyl, ethylsulphonyl, methoxycarbonyl, ethoxycarbonyl, *N-*methylsulphamoyl, *N*-ethylsulphamoyl, *N,N*-dimethylsulphamoyl, *N,N*-diethylsulphamoyl or *N*-methyl-*N*-ethylsulphamoyl;
or a pharmaceutically acceptable salt thereof;
with the proviso that said compound is not:
*N*-[4-chloro-3-({[6-(4-methylpiperazin-1-yl)pyridin-3-yl]amino}carbonyl)phenyl]-2-morpholin-4-ylisonicotinamide;
*N*-[4-chloro-3-({[6-(4-ethylpiperazin-1-yl)pyridin-3-yl]amino}carbonyl)phenyl]-2-morpholin-4-ylisonicotinamide;
*N*-{4-chloro-3-[({6-[[3-(dimethylamino)propyl](methyl)amino]pyridin-3-yl}amino)carbonyl] -phenyl}-2-morpholin-4-ylisonicotinamide;
*N*-{4-chloro-3-[({6-[[2-(dimethylamino)ethyl](methyl)amino]pyridin-3-yl}amino)carbonyl] phenyl}-2-morpholin-4-ylisonicotinamide; or
*N*-[4-chloro-3-({[6-(4-methyl-1,4-diazepan-1-yl)pyridin-3-yl]amino}carbonyl)phenyl]-2-morpholin-4-ylisonicotinamide.

According to a further feature of the present invention there is provided a compound of formula **(I)** wherein:
**Ring A** is carbocyclyl or heterocyclyl; wherein if said heterocyclyl contains an -NH-moiety that nitrogen may be optionally substituted by a group selected from R⁵;
**R¹** is a substituent on carbon and is selected from halo, nitro, cyano, hydroxy, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N,N*-(C₁₋₆alkyl)₂amino, C₁₋₆alkanoylamino, *N*-(C₁₋₆alkyl)carbamoyl, *N,N*-(C₁₋₆alkyl)₂carbamoyl, C₁₋₆alkylS(O)ₐ wherein a is 0 to 2, C₁₋₆alkoxycarbonyl, *N*-(C₁₋₆alkyl)sulphamoyl, *N,N*-(C₁₋₆alkyl)₂sulphamoyl, C₁₋₆alkylsulphonylamino, carbocyclyl-R⁶- or heterocyclyl-R⁷-; wherein R¹ may be optionally substituted on carbon by one or more R⁸; and wherein if said heterocyclyl contains an -NH- moiety that nitrogen may be optionally substituted by a group selected from R⁹;
**n** is selected from 0-4; wherein the values of R¹ may be the same ot different;
**R²** is selected from hydrogen, halo, nitro, cyano, hydroxy, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N,N*-(C₁₋₆alkyl)₂amino, C₁₋₆alkanoylamino, *N*-(C₁₋₆alkyl)carbamoyl, *N,N*-(C₁₋₆alkyl)₂carbamoyl, C₁₋₆alkylS(O)ₐ wherein a is 0 to 2, C₁₋₆alkoxycarbonyl, *N*-(C₁₋₆alkyl)sulphamoyl, *N,N-*(C₁₋₆alkyl)₂Sulphamoyl, C₁₋₆alkylsulphonylamino, carbocyclyl-R¹⁰- or heterocyclyl-R¹¹-; wherein R² may be optionally substituted on carbon by one or more R¹²; and wherein if said heterocyclyl contains an -NH- moiety that nitrogen may be optionally substituted by a group selected from **R¹³**;
**R³** is selected from halo, hydroxy, cyano, methyl, methoxy or hydroxymethyl;
**R⁴ is** selected from halo, nitro, cyano, hydroxy, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N,N-*(C₁₋₆alkyl)₂amino, C₁₋₆alkanoylamino, *N*-(C₁₋₆alkyl)carbamoyl, *N,N*-(C₁₋₆alkyl)₂carbamoyl, C₁₋₆alkylS(O)ₐ wherein a is 0 to 2, C₁₋₆alkoxycarbonyl, *N*-(C₁₋₆alkyl)sulphamoyl, *N,N*-(C₁₋₆alkyl)₂sulphamoyl, C₁₋₆alkylsulphonylamino, carbocyclyl-R¹⁴- or heterocyclyl-R¹⁵-; wherein R⁴ may be optionally substituted on carbon by one or more R¹⁶; and wherein if said heterocyclyl contains an -NH- moiety that nitrogen may be optionally substituted by a group selected from R¹⁷;
**m** is selected from 0-4; wherein the values of R⁴ may be the same or different;
**R⁸** and **R¹²** are independently selected from halo, nitro, cyano, hydroxy, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N,N*-(C₁₋₆alkyl)₂amino, C₁₋₆alkanoylamino, *N*-(C₁₋₆alkyl)carbamoyl, *N,N*-(C₁₋₆alkyl)₂carbamoyl, C₁₋₆alkylS(O)ₐ wherein a is 0 to 2, C₁₋₆alkoxycarbonyl, *N-*(C₁₋₆alkyl)sulphamoyl, *N,N*(C₁₋₆alkyl)₂sulphamoyl, C₁₋₆alkylsulphonylamino, carbocyclyl-R¹⁸- or heterocyclyl-R¹⁹-; wherein R⁸ and R¹² independently of each other may be optionally substituted on carbon by one or more R²⁰; and wherein if said heterocyclyl contains an -NH- moiety that nitrogen may be optionally substituted by a group selected from R²¹;
**R¹⁶** is selected from halo, nitro, cyano, hydroxy, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N,N*-(C₁₋₆alkyl)₂amino, C₁₋₆alkanoylamino, *N*-(C₁₋₆alkyl)carbamoyl, *N,N*-(C₁₋₆alkyl)₂carbamoyl,C₁₋₆alkylS(O)ₐ wherein a is 0 to 2, C₁₋₆alkoxycarbonyl, *N*-(C₁₋₆alkyl)sulphamoyl, *N,N*-(C₁₋₆alkyl)₂Sulphamoyl, C₁₋₆alkylsulphonylamino, carbocyclyl-R²²- or heterocyclyl-R²³-; wherein R¹⁶ may be optionally substituted on carbon by one or more R²⁴; and wherein if said heterocyclyl contains an -NH- moiety that nitrogen may be optionally substituted by a group selected from R²⁵;
**R⁶, R⁷, R¹⁰, R¹¹, R¹⁴, R¹⁵, R¹⁸, R¹⁹, R²²** and **R²³** are independently selected from a direct bond, -O-, -N(R²⁶)-, -C(O)-, -N(R²⁷)C(O)-, -C(O)N(R²⁸)-, -S(O)ₛ-, -SO₂N(R²⁹)- or -N(R³⁰)SO₂-; wherein **R²⁶, R²⁷, R²⁸, R²⁹** and **R³⁰** are independently selected from hydrogen or C₁₋₆alkyl and s is 0-2;
**R⁵, R⁹, R¹³, R¹⁷, R²¹** and **R²⁵** are independently selected from C₁₋₆alkyl, C₁₋₆alkanoyl, C₁₋₆alkylsulphonyl, C₁₋₆alkoxycarbonyl, carbamoyl, *N*-(C₁₋₆alkyl)carbamoyl, *N,N*-(C₁₋₆alkyl)carbamoyl, benzyl, benzyloxycarbonyl, benzoyl and phenylsulphonyl;
**R²⁰** and **R²⁴** are independently selected from halo, nitro, cyano, hydroxy, trifluoromethoxy, trifluoromethyl, amino, carboxy, carbamoyl, mercapto, sulphamoyl, methyl, ethyl, methoxy, ethoxy, acetyl, acetoxy, methylamino, ethylamino, dimethylamino, diethylamino, *N*-methyl-*N-*ethylamino, acetylamino, *N*-methylcarbamoyl, *N*-ethylcarbamoyl, *N*,*N-*dimethylcarbamoyl, *N,N*-diethylcarbamoyl, *N-*methyl-*N-*ethylcarbamoyl, methylthio, ethylthio, methylsulphinyl, ethylsulphinyl, mesyl, ethylsulphonyl, methoxycarbonyl, ethoxycarbonyl, *N-*methylsulphamoyl, *N*-ethylsulphamoyl, *N,N*-dimethylsulphamoyl, *N,N*-diethylsulphamoyl or *N*-methyl-*N*-ethylsulphamoyl;
or a pharmaceutically acceptable salt thereof;
with the proviso that said compound is not:
*N*-[4-chloro-3-({[6-(4-methylpiperazin-1-yl)pyridin-3-yl]amino}carbonyl)phenyl]-2-morpholin-4-ylisonicotinamide;
*N*-[4-chloro-3-({[6-(4-ethylpiperazin-1-yl)pyridin-3-yl]amino}carbonyl)phenyl]-2-morpholin-4-ylisonicotinamide;
*N*-{4-chloro-3-[({6-[[3-(dimethylamino)propyl](methyl)amino]pyridin-3-yl}amino)carbonyl] phenyl}-2-morpholin-4-ylisonicotinamide;
*N*-{4-chloro-3-[({6-[[2-(dimethylamino)ethyl](methyl)amino]pyridin-3-yl})amino)carbonyl] phenyl}-2-morpholin-4-ylisonicotinamide; or
*N*-[4-chloro-3-({[6-(4-methyl-1,4-diazepan-1-yl)pyridin-3-yl]amino}carbonyl)phenyl]-2-morpholin-4-ylisonicotinamide.

In this specification the term "alkyl" includes both straight and branched chain alkyl groups. References to individual alkyl groups such as "propyl" are specific for the straight chain version only and references to individual branched chain alkyl groups such as 'isopropyl' are specific for the branched chain version only. For example, "C₁₋₆alkyl" includes C₁₋₄alkyl, C₁₋₃alkyl, propyl, isopropyl and *t*-butyl. A similar convention applies to other radicals, for example "phenylC₁₋₆alkyl" includes phenylC₁₋₄alkyl, benzyl, 1-phenylethyl and 2-phenylethyl. The term "halo" refers to fluoro, chloro, bromo and iodo.

Where optional substituents are chosen from "one or more" groups it is to be understood that this definition includes all substituents being chosen from one of the specified groups or the substituents being chosen from two or more of the specified groups.

Ring A may be a "monocyclic 5 or 6 membered fully unsaturated heterocyclic ring". A "monocyclic 5 or 6 membered fully unsaturated heterocyclic ring" is fully unsaturated, monocyclic ring containing 5 or 6 atoms of which at least one atom is chosen from nitrogen, sulphur or oxygen, which may, unless otherwise specified, be carbon or nitrogen linked, wherein a -CH₂- group can optionally be replaced by a -C(O)- and a ring sulphur atom may be optionally oxidised to form the S-oxides. Examples of a "monocyclic 5 or 6 membered fully unsaturated heterocyclic ring" are pyridyl, pyrazolyl, thienyl, isoxazolyl, furanyl, 1 ,3-thiazolyl, pyrimidinyl and pyrrolyl.

Ring A may also be phenyl or a monocyclic 5 or 6 membered fully unsaturated heterocyclic ring; "wherein said phenyl or heterocyclic ring may be optionally fused to a five or six membered carbocyclyl or heterocyclyl forming a bicyclic ring". Here said bicyclic ring is a bicyclic ring containing 8, 9 or 10 atoms of which at least one atom is chosen from nitrogen, sulphur or oxygen, which may, unless otherwise specified, be carbon or nitrogen linked, wherein a -CH₂- group can optionally be replaced by a -C(O)- and a ring sulphur atom may be optionally oxidised to form the S-oxides. Example of such a bicyclic ring include indolyl, 2,3-dihydrobenzofuranyl, imidazo[1 ,2-a]pyridinyl, benzimidazolyl and 2-oxoindolinyl.

A "heterocyclyl" is a saturated, partially saturated or unsaturated, mono or bicyclic ring containing 4-12 atoms of which at least one atom is chosen from nitrogen, sulphur or oxygen, which may, unless otherwise specified, be carbon or nitrogen linked, wherein a -CH₂-group can optionally be replaced by a -C(O)- and a ring sulphur atom may be optionally oxidised to form the S-oxides. Examples and suitable values of the term "heterocyclyl" are morpholino, piperidyl, pyridyl, pyranyl, pyrrolyl, pyrazolyl, isothiazolyl, indolyl, quinolyl, thienyl, 1,3-benzodioxolyl, thiadiazolyl, piperazinyl, thiazolidinyl, pyrrolidinyl, thiomorpholino, pyrrolinyl, homopiperazinyl, 3,5-dioxapiperidinyl, tetrahydropyranyl, imidazolyl, pyrimidyl, pyrazinyl, pyridazinyl, isoxazolyl, *N*-methylpyrrolyl, 4-pyridine, 1-isoquinolone, 2-pyrrolidone, 4-thiazolidone, pyridine-*N*-oxide and quinoline-*N*-oxide. A particular example of the term "heterocyclyl" is pyrazolyl. In one aspect of the invention a "heterocyclyl" is a saturated, partially saturated or unsaturated, monocyclic ring containing 5 or 6 atoms of which at least one atom is chosen from nitrogen, sulphur or oxygen, it may, unless otherwise specified, be carbon or nitrogen linked, a -CH₂- group can optionally be replaced by a -C(O)-and a ring sulphur atom may be optionally oxidised to form the S-oxides.

A "carbocyclyl" is a saturated, partially saturated or unsaturated, mono or bicyclic carbon ring that contains 3-12 atoms; wherein a -CH₂- group can optionally be replaced by a -C(O)-. Particularly "carbocyclyl" is a monocyclic ring containing 5 or 6 atoms or a bicyclic ring containing 9 or 10 atoms. Suitable values for "carbocyclyl" include cyclopropyl, cyclobutyl, 1-oxocyclopentyl, cyclopentyl, cyclopentenyl, cyclohexyl, cyclohexenyl, phenyl, naphthyl, tetralinyl, indanyl or 1-oxoindanyl. A particular example of "carbocyclyl" is phenyl.

An example of "C₁₋₆alkanoyloxy" is acetoxy. Examples of "C₁₋₆alkoxycarbonyl" include methoxycarbonyl, ethoxycarbonyl, *n*- and *t*-butoxycarbonyl. Examples of "C₁₋₆alkoxy" include methoxy, ethoxy and propoxy. Examples of "C₁₋₆alkanoylamino" include formamido, acetamido and propionylamino. Examples of "C₁₋₆alkylS(O)ₐ wherein a is 0 to 2" include methylthio, ethylthio, methylsulphinyl, ethylsulphinyl, mesyl and ethylsulphonyl. Examples of "C₁₋₆alkanoyl" include propionyl and acetyl. Examples of "*N*-(C₁₋₆alkyl)amino" include methylamino and ethylamino. Examples of "*N,N-*(C₁₋₆alkyl)₂amino" include di-*N*-methylamino, di-(*N*-ethyl)amino and *N-*ethyl-*N*-methylamino. Examples of "C₂₋₆alkenyl" are vinyl, allyl and 1-propenyl. Examples of "C₂₋₆alkynyl" are ethynyl, 1-propynyl and 2-propynyl Examples of "*N*-(C₁₋₆alkyl)sulphamoyl" are *N*-(methyl)sulphamoyl and *N*-(ethyl)sulphamoyl. Examples of "*N*-(C₁₋₆alkyl)₂sulphamoyl" are *N,N*-(dimethyl)sulphamoyl and *N*-(methyl)-N-(ethyl)sulphamoyl. Examples of "*N-*(C₁₋₆alkyl)carbamoyl" are *N*-(C₁₋₄alkyl)carbamoyl, methylaminocarbonyl and ethylaminocarbonyl. Examples of "*N,N*-(C₁₋₆alkyl)₂carbamoyl" are *N,N*-(C₁₋₄alkyl)₂carbamoyl, dimethylaminocarbonyl and methylethylaminocarbonyl. Examples of "C₁₋₆alkylsulphonyl" are mesyl, ethylsulphonyl, and isopropylsulphonyl. Examples of "C₁₋₆alkylsulphonylamino" are mesylamino, ethylsulphonylamino and isopropylsulphonylamino. Examples of "C₁₋₆alkoxycarbonylamino" are methoxycarbonylamino and *t*-butoxycarbonylamino. Examples of "*N*-(C₁₋₆alkyl)-*N*-(C₁₋₆alkoxy)sulphamoyl" are *N*-(methyl)-*N*-(methoxy)sulphamoyl and *N*-(ethyl)-*N*-(propoxy)sulphamoyl. Example of "*N,N*'-(C₁₋₆alkyl)₂ureido" are *N,N*'-dimethylureido and *N*-methyl-*N'*-propylureido. Examples of "*N',N'*-(C₁₋₆alkyl)₂ureido" are *N',N*'-diethylureido and *N'*-methyl-*N'*-propylureido. Example of *"N*-(C₁₋₆alkyl)-*N,N'*-(C₁₋₆alkyl)₂ureido" are *N*-(methyl)-*N'*-ethyl-*N'*-isopropylureido and *N*-ethyl-*N,N'*-diethylureido. Examples of *"N*-(C₁₋₆alkyl)-*N*-(C₁₋₆alkoxy)amino" are *N*-(methyl)-*N-*(propoxy)amino and *N*-methyl-*N*-methoxyamino.

A suitable pharmaceutically acceptable salt of a compound of the invention is, for example, an acid-addition salt of a compound of the invention which is sufficiently basic, for example, an acid-addition salt with, for example, an inorganic or organic acid, for example hydrochloric, hydrobromic, sulphuric, phosphoric, trifluoroacetic, citric or maleic acid. In addition a suitable pharmaceutically acceptable salt of a compound of the invention which is sufficiently acidic is an alkali metal salt, for example a sodium or potassium salt, an alkaline earth metal salt, for example a calcium or magnesium salt, an ammonium salt or a salt with an organic base which affords a physiologically-acceptable caution, for example a salt with methylamine, dimethylamine, trimethylamine, piperidine, morpholine or tris-(2-hydroxyethyl)amine.

Some compounds of the formula **(I)** may have chiral centres and/or geometric isomeric centres (E- and Z- isomers), and it is to be understood that the invention encompasses all such optical, diastereoisomers and geometric isomers that possess B-Raf inhibitory activity. The invention further relates to any and all tautomeric forms of the compounds of the formula **(I)** that possess B-Raf inhibitory activity.

It is also to be understood that certain compounds of the formula **(I)** can exist in solvated as well as unsolvated forms such as, for example, hydrated forms. It is to be understood that the invention encompasses all such solvated forms which possess B-Raf inhibitory activity.

Particular values of variable groups are as follows. Such values may be used where appropriate with any of the definitions, claims or embodiments defined hereinbefore or hereinafter.

Ring A is a monocyclic 5 or 6 membered fully-unsaturated heterocyclic ring; wherein if heterocyclic ring contains an -NH- moiety that nitrogen may be optionally substituted by a group selected from R⁵.

Ring A is phenyl fused to a five or six membered carbocyclyl or heterocyclyl forming a bicyclic ring; wherein if said heterocyclyl contains an -NH- moiety that nitrogen may be optionally substituted by a group selected from R⁵.

Ring A is a heterocyclic ring fused to a five or six membered carbocyclyl or heterocyclyl forming a bicyclic ring; wherein if said heterocyclyl or heterocyclic ring contains an -NH- moiety that nitrogen may be optionally substituted by a groups selected from R⁵.

Ring A is carbocyclyl.

Ring A is phenyl.

Ring A is heterocyclyl; wherein if said heterocyclyl contains an -NH- moiety that nitrogen may be optionally substituted by a group selected from R⁵.

Ring A is pyridyl.

Ring A is pyrazolyl; wherein said pyrazolyl may be optionally substituted on nitrogen by a group selected from R⁵.

Ring A is thienyl.

Ring A is imidazo[1,2-a]pyridinyl.

Ring A is indolyl.

Ring A is 2,3-dihydrobenzofuranyl.

Ring A is isoxazolyl.

Ring A is benzimidazolyl.

Ring A is 2-oxoindolinyl.

Ring A is furanyl.

Ring A is 1,3-thiazolyl.

Ring A is pyrimidinyl.

Ring A is pyrrolyl.

Ring A is phenyl, pyridyl, pyrazolyl, thienyl, indolyl, 2,3-dihydrobenzofuranyl, imidazo[1,2-a]pyridinyl, isoxazolyl, benzimidazolyl, 2-oxoindolinyl, furanyl, 1,3-thiazolyl, pyrimidinyl and pyrrolyl; wherein said pyrazolyl, indolyl, pyrrolyl may be optionally substituted on nitrogen by a group selected from R⁵; wherein

R⁵ is selected from C₁₋₆alkyl.

Ring A is phenyl, pyridyl, pyrazolyl, thienyl, indolyl, 2,3-dihydrobenzofuranyl and imidazo[1,2-a]pyridinyl; wherein said pyrazolyl may be optionally substituted on nitrogen by a group selected from R⁵; wherein

R⁵ is C₁₋₆alkyl.

Ring A is phenyl, pyrid-2-yl, pyrid-3-yl,pyrid-4-yl, 1-methylpyrazol-5-yl, 1-*t-*butylpyrazol-5-yl, thien-2-yl, thien-3-yl, indol-2-yl, 1-methylindol-2-yl, indol-4-yl, indol-5-yl, indol-6-yl, indol-7-yl, 2,3-dihydrobenzofuran-7-yl, imidazo[1,2-a]pyridin-2-yl, isoxazol-3-yl, pyrrol-2-yl, benzimidazol-6-yl, 1-methyl-2-oxoindolin-5-yl, furan-2-yl, 1,3-thiazol-5-yl, pyrimidin-4-yl and 1-methylpyrrol-2-yl.

Ring A is phenyl, pyrid-2-yl, pyrid-3-yl, pyrid-4-yl, 1-methylpyrazol-5-yl, thien-2-yl, thien-3-yl, indol-5-yl, indol-6-yl, 2,3-dihydrobenzofuran-7-yl or imidazo[1,2-a]pyridinyl.

Ring A is not pyridyl.

Ring A is not pyrid-4-yl.

R¹ is a substituent on carbon and is selected from halo, cyano, hydroxy, sulphamoyl, C₁₋₆alkyl, C₂₋₆alkynyl, C₁₋₆alkoxy, *N,N-*(C₁₋₆alkyl)₂amino,- C₁₋₆alkylS(O)ₐ wherein a is 0 to 2, C₁₋₆alkoxycarbonylamino, *N*-(C₁₋₆alkyl)sulphamoyl, *N,N*-(C₁₋₆alkyl)₂sulphamoyml, *N*-(C₁₋₆alkyl)-N-(C₁₋₆alkoxy)sulphamoyl, *N',N*'-(C₁₋₆alkyl)₂ureido, C₁₋₆alkylsulphonylamino, carbocyclyl-R⁶- or heterocyclyl-R⁷ -; wherein R¹ may be optionally substituted on carbon by one or more R⁸;

R⁸ is selected from halo, cyano, hydroxy, C₁₋₆alkyl, C₁₋₆alkoxy, *N,N*-(C₁₋₆alkyl)₂amino, *N,N*-(C₁₋₆alkyl)₂carbamoyl, C₁₋₆atkylS(O)ₐ wherein a is 0 to 2, *N,N*-(C₁₋₆alkyl)₂sulphamoyl, *N*-(C₁₋₆alkyl)-*N*-(C₁₋₆alkoxy)amino carbocyclyl-R¹⁸- or heterocyclyl-R¹⁹ -; wherein R⁸ may be optionally substituted on carbon by one or more R²⁰; and wherein if said heterocyclyl contains an -NH- moiety that nitrogen may be optionally substituted by a group selected from R²¹;

R⁶, R⁷, R¹⁸ and R¹⁹ are independently selected from a direct bond, -O-, -S(O)ₛ- or -N(R³⁰)SO₂-; wherein R³⁰ is selected from hydrogen and s is 2;

R²¹ is selected from C₁₋₆alkyl;

R²⁰ is selected from cyano or hydroxy.

R¹ is a substituent on carbon and is selected from halo, cyano, C₁₋₆alkyl, C₂₋₆alkynyl, C₁₋₆alkoxy, *N,N-*(C₁₋₆alkyl)₂amino, C₁₋₆alkylS(O)ₐ wherein a is 0 to 2, *N,N*-(C₁₋₆alkyl)₂sulphamoyl, C₁₋₆alkylsulphonylamino, carbocyclyl-R⁶- or heterocyclyl-R⁷-; wherein R¹ may be optionally substituted on carbon by one or more R⁸;

R⁸ is selected from halo, cyano, hydroxy, *N,N*-(C₁₋₆alkyl)₂amino or heterocyclyl-R⁹-; wherein if said heterocyclyl contains an -NH- moiety that nitrogen may be optionally substituted by a group selected from R²¹;

R⁶, R⁷ and R¹⁹ are independently selected from a direct bond, -S(O)ₛ-or -N(R³⁰)SO₂-; wherein R³⁰ is hydrogen and s is 0-2;

R²¹ is C₁₋₆alkyl.

R¹ is a substituent on carbon and is selected from fluoro, chloro, bromo, iodo, cyano, hydroxy, sulphamoyl, methyl, ethyl, isopropyl, sec-butyl, i-butyl, 2-methylbut-2-yl, 3-methylbut-2-yl, 1,1-dimethylptop-2-yn-1-yl, 1,1-dimethylbut-2-yn-1-yl, 3,3-dimethylbut-1-yn-1-yl, 3-methylbut-1-yn-1-yl, methoxy, ethoxy, propoxy, isopropoxy, isobutoxy, *N,N*-dimethylamino, *N,N*-diethylamino, methylthio, mesyl, *t*-butoxycarbonylamino, *N*-methylsulphamoyl, *N*-methyl-*N*-propylsulphamoyl, *N,N*-dimethylsulphamoyl, *N*-(methyl)-*N*-(methoxy)sulphamoyl, *N',N'*-dimethylureido, mesylamino, cyclopropyl-R⁶-, phenyl-R⁶-, morpholino-R⁷-, imidazolyl-R⁷ 1,3-thiazolyl-R⁷-,pyridyl-R⁷-, piperidinyl-R⁷- or azetidinyl-R⁷-; wherein R¹ may be optionally substituted on carbon by one or more R⁸;

R⁸ is selected from fluoro, cyano, hydroxy, methyl, methoxy,*N,N*-dimethylamino, *N,N*-dimethylcarbamoyl, methylthio, mesyl, *N,N*-dimethylsulphamoyl, *N*-(methyl)-*N*-(methoxy)sulphamoyl, cyclopropyl-R¹⁸-,piperazinyl-R¹⁹-, pyrrolyl-R¹⁹- or tetrahydrofuryl-R¹⁹-; wherein R⁸ may be optionally substituted on carbon by one or more R²⁰; and wherein said piperazinyl may be optionally substituted on nitrogen by a group selected from R²¹;

R⁶, R⁷, R¹⁸ and R¹⁹ are independently selected from a direct bond, -O-, -S(O)ₛ- or -N(R³⁰)SO₂-; wherein R³⁰ is selected from hydrogen and s is 2;

R²¹ is selected from methyl or ethyl;

R²⁰ is selected from cyano or hydroxy.

R¹ is a substituent on carbon and is selected from fluoro, chloro, bromo, iodo, cyan, methyl, isopropyl, t-butyl, 3-methylbut-1-yn-1-yl, 3,3-dimethylbut-1-yn-1-yl, methoxy, propoxy, isopropoxy, isobutoxy, dimethylamino, methylthio, mesyl, *N,N*-dimethylsulphamoyl, mesylamino, cyclopropyl-R⁶- or azetidin-1-yl-R⁷-; wherein R¹ may be optionally substituted on carbon by one or more R⁸;

R⁸ is selected from fluoro, cyano, hydroxy, dimethylamine or piperazin-1-yl-R¹⁹-; wherein said piperazinyl may be optionally substituted on nitrogen by a group selected from R²¹;

R⁶, R⁷ and R¹⁹ are independently selected from a direct bond, -S(O)ₛ- or -N(R³⁰)SO₂-; wherein R³⁰ is hydrogen and s is 2;

R²¹ is methyl or ethyl.

R¹ is a substituent on carbon and is selected from fluoro, chloro, bromo, iodo, cyano, hydroxy, sulphamoyl, methyl, trifluoromethyl, 1-cyano-1-methylethyl, methoxy, ethoxy, propoxy, isopropoxy, isobutoxy, *N,N*-dimethylamino, difluoromethylthio, *N,N*-dimethylsulphamoyl, *t*-butyl, mesyl, cyclopropylaminosulphonyl, azetidin-1-ylsulphonyl, tetrahydrofuran-2-ylmethylaminosulphonyl, *N*-methyl-*N*-(2,3-dihydroxypropyl)sulphamoyl, mesylamino, morpholinosulphonyl, 1-methylpiperazin-4-ylmethyl, 1-ethylpiperazin-4-ylmethyl, 3,3-dimethylbut-1-yn-1-yl, morpholino, *N,N*-dimethylaminomethyl, 3-methyl-3-hydroxybut-1-yn-1-yl, methylthiomethyl, mesylmethyl, *N*-(methyl)-*N*-(methoxy)sulphamoyl, 2-hydroxymethylpiperidin-1-ylsulphonyl, 3-hydroxymethylpiperidin-1-ylsulphonyl, 4-hydroxymethylpiperidin-1-ylsulphonyl, 1,1-difluoroethyl, piperidin-1-yl, *N,N*-diethylamino, *N',N'*-dimethylureido, cyclopropyl, *t*-butoxycarbonylamino, pyrid-2-yl, phenoxy, 2-methoxy-1,1-dimethylethyl, mesylmethyl, 1,3-thiazol-2-yl, 2-methyl-1,3-thiazol-5-yl, 1-methylcyclopropyl,1,1-dimethylprop-2-yn-1-yl, 1-(*N,N*-dimethylsulphamoyl)-1-methylethyl, 1,1-dimethylbut-2-yn-1-yl, *N*-(methyl)-*N*-(methoxy)aminomethyl, 1-(*N,N-*dimethylcarbamoyl)-1-methylethyl, 4-methylimidazol-1-yl, 1-(cyclopropyl)-1-methylethyl, 2-methyl-3,4-dihydroxybut-2-yl, 2-methylbut-2-yl, 1-hydroxy-1-cyclopropylethyl, 1-cyanoethyl, 2-cyano-3-methylbut-2-yl, 2-cyanobut-2-yl, 1-hydroxy-2-cyanoprop-2-yl and 2-cyanopyrrol-1-ylmethyl.

R¹ is a substituent on carbon and is selected from fluoro, chloro, bronco, iodo, cyano,; methyl, t-butyl, trifluoromethyl, dimethylaminomethyl, 1-methyl-1-cyanoethyl, 4-methylpiperazin-1-ylmethyl, 4-ethylpiperazin-1-ylmethyl, 3-hydroxy-3-methylbut-1-yn-1-yl, 3,3-dimethylbut-1-yn-1-yl, methoxy, propoxy, isopropoxy, isobutoxy, dimethylamino, difluoromethylthio, *N,N-*dimethylsulphamoyl, mesyl, cyclopropylaminosulphonyl, azetidin-1-ylsulphonyl or mesylamino.

R¹ is a substituent on carbon and is selected from 1-methyl-1-cyanoethyl.

R¹ is a substituent on carbon and is selected from trifluoromethyl.

n is selected from 0-2; wherein the values of R¹ may be the same or different.

n is 0.

n is 1.

n is 2; wherein the values of R¹ may be the same or different.

R² is hydrogen.

R³ is selected from halo, methyl or methoxy.

R³ is selected from halo or methyl.

R³ is selected from fluoro, chloro, methyl or methoxy.

R³ is selected from fluoro, chloro or methyl.

R³ is fluoro.

R³ is chloro.

R³ is methyl.

R³ is methoxy.

R³ is not chloro.

R⁴ is selected from halo, cyano, hydroxy, amino, carbamoyl, ureido, C₁₋₆alkyl, C₂₋₆alkynyl, C₁₋₆alkoxy, C₁₋₆alkanoyl, *N*-(C₁₋₆alkyl)amino, *N,N*-(C₁₋₆alkyl)₂amino, C₁₋₆alkanoylamino, *N*-(C₁₋₆alkyl)carbamoyl, C₁₋₆alkoxycarbonyl, *N,N-*(C₁₋₆alkyl)₂sulphamoyl, C₁₋₆alkylsulphonylamino, carbocyclyl-R¹⁴- or heterocyclyl-R¹⁵ -;' wherein R⁴ may be optionally substituted on carbon by one or more R¹⁶; and wherein if said heterocyclyl contains an -NH- moiety that nitrogen may be optionally substituted by a group selected from R¹⁷;

R¹⁶ is selected from halo, hydroxy, amino,C₁₋₆alkoxy,*N*-(C₁₋₆alkyl)amino, *N,N*-(C₁₋₆alkyl)₂amino, carbocyclyl-R²²- or heterocyclyl-R²³-; wherein R¹⁶ may be optionally substituted on carbon by one or more R²⁴; and wherein if said heterocyclyl contains an -NH-moiety that nitrogen may be optionally substituted by a group selected from R²⁵;

R¹⁴, R¹⁵,R²² and R²³ are independently selected from a direct bond, -N(R²⁶)- or -C(O)N(R²⁸)-; wherein R²⁶ and R²⁸ are hydrogen;

R¹⁷and R²⁵ are independently selected from C₁₋₆alkyl and C₁₋₆alkoxycarbonyl;

R²⁴ is methyl or phenyl.

R⁴ is selected from halo, cyano, amino, C₁₋₆alkyl, C₁₋₆alkoxy,*N*-(C₁₋₆alkyl)amino, C₁₋₆alkanoylamino, *N*-(C₁₋₆alkyl)carbamoyl or heterocyclyl-R¹⁵-; wherein

R¹⁵ is a direct bond.

R⁴ is selected from fluoro, chloro, bromo, cyano, hydroxy, amino, carbamoyl, ureido, methyl, ethyl, propyl, prop-1-ynyl, methoxy, ethoxy, propoxy, isopropoxy, acetyl, methylamino, ethylamino, propylamino, isopropylamino, butylamino, dimethylamino, *N-*methyl-*N*-ethylamino, *N*-methyl-*N*-propylamino, formylamino, acetylamino, propanoylamino, 2,2-dimethylpropanoylamino, *N*-methylcarbamoyl, methoxycarbonyl, *N,N*-dimethylsulphamoyl, mesylamino, cyclopropyl-R¹⁴-, cyclobutyl-R¹⁴-, piperazinyl-R¹⁵-, pyrrolyl-R¹⁵-, pyrrolidinyl-R¹⁵-, pyrazolyl-R¹⁵-or morpholino-R¹⁵-; wherein R⁴ may be optionally substituted on carbon by one or more R¹⁶; and wherein said piperazinyl or pyrrolidinyl may be optionally substituted on nitrogen by a group selected from R¹⁷;

R¹⁶ is selected from fluoro, hydroxy, amino, methoxy, methylamino, *N,N*-dimethylamino, cyclopropyl-R²²-, 1,3-dioxolanyl-R²³-, imidazolyl-R²³-, morpholino-R²³-, piperazihyl-R²³-, piperidinyl-R²³- or pyrrolidinyl-R²³-; wherein R¹⁶ may be optionally substituted on carbon by one or more R²⁴; and wherein said piperazinyl or pyrrolidinyl may be optionally substituted on nitrogen by a group selected from R²⁵;

R¹⁴, R¹⁵, R²² and R²³ are independently selected from a direct bond, -N(R²⁶)- or -C(O)N(R²⁸)-; wherein R²⁶ and R²⁸ are hydrogen;

R¹⁷ and R²⁵ are independently selected from methyl and t-butoxycarbonyl;

R²⁴ is methyl or phenyl.

R⁴ is selected from fluoro, chloro, bromo, cyano, amino" methyl, methoxy, methylamino, acetylamino, *N*-methylcarbamoyl or morpholino.

R⁴ is selected from fluoro, chloro, bromo, cyano, hydroxy, amino, carbamoyl, ureido, methyl, ethyl, methoxy, methylamino, isopropylamino, morpholino, 2-(dimethylamino)ethylamino,2-(hydroxy)ethylamino,2-(amino)ethylamino,3-(pyrrolidin-1-yl)propylamino, *N*-methylcarbamoyl, acetylamino, 2-hydroxyacetylamino, trifluoromethyl, mesylamino, 2,2-dimethylpropanoylamino, 3-methoxypropanoylamino, cyclobutylcarbonylamino, cyclopropylamino, 2,3-dihydroxypropylamino, 1,3-dihydroxyprop-2-ylamino, 1-methylpiperazin-4-yl, 1-methylpiperazin-4-ylmethyl, acetyl, *N*-methyl-N-(3-dimethylaminopropyl)amino, *N*-methyl-*N*-(2-methoxyethyl)amino, dimethylamino, hydroxymethyl, 1,2-dihydroxyethyl, pyrazol-5-ylamino, 3-aminoprop-1-yn-1-yl, 3-hydroxyptop-1-yn-1-y1, 3-methylaminoprop-1-yn-1-yl, 3-dimethylaminoprop-1-yn-1-yl, 4-aminobutylamino, pyrrolidin-2-ylamino, 3-methylaminopropyl, 3-dimethylaminopropyl, 3-hydroxypropyl, 3-dimethylaminopropylamino, aminomethyl, piperazin-1-yl, 1-methylpiperazin-4-yl, 2,2-dimethyl-1,3-dioxolan-4-ylmethylamino, pyrrolidin-3-ylmethylamino, piperidin-4-ylmethylamino, imidazol-2-ylmethylamino, methoxymethyl, *N,N*-dimethylsulphamoyl, formylamino, morpholinomethyl, aminomethyl,2-(dimethylamino)ethylamino, pyrrol-1-yl, pyrrol-2-yl, pyrrolidin-2-yl, imidazol-4-yl, cyclobutylamino, *N*-methyl-*N*-(2-dimethylaminoethyl)amino, 2-dimethylaminoethoxy, dimethylaminomethyl, cyclopropylaminomethyl, piperidin-1-ylmethyl, methylaminomethyl, pyrrolidin-2-ylmethoxy, 3-dimethylaminopropoxy, methoxycarbonyl, 1-(*t-*butoxycarbonyl)pyrrolidin-2-ylmethylamino, 1-(*t*-butoxycarbonyl)pyrrolidin-2-ylmethoxy; 2-phenoxyacetylamino and 1-(*t*-butoxycarbonyl)pyrrolidin-2-yl.

m is selected from 0-2; wherein the values of R⁴ may be the same or different.

m is 0.

m is 1.

m is 2; wherein the values of R⁴ may be the same or different.

Therefor in a further aspect of the invention there is provided a compound of formula **(I)** (as depicted above) wherein:
Ring A is phenyl, pyridyl, pyrazolyl, thienyl, indolyl, 2,3-dihydrobenzofuranyl, imidazo[1,2-a]pyridinyl, isoxazolyl, benzimidazolyl, 2-oxoindolinyl, furanyl, 1,3-thiazolyl, pyrimidinyl and pyrrolyl; wherein said pyrazolyl, indolyl, pyrrolyl may be optionally substituted on nitrogen by a group selected from R⁵;
R¹ is a substituent on carbon and is selected from halo, cyano, hydroxy, sulphamoyl, C₁₋₆-akyl, C₂₋₆alkynyl, C₁₋₆alkoxy, *N,N*-(C₁₋₆alkyl)₂amino, C₁₋₆alkylS(O)ₐ wherein a is 0 to 2, C₁₋₆alkoxycarbonylamino, *N*-(C₁₋₆alkyl)sulphamoyl, *N,N*-(C₁₋₆alkyl)₂sulphamoyl, *N*-(C₁₋₆alkyl)-*N*-(C₁₋₆alkoxy)sulphamoyl, *N'*,*N*'-(C₁₋₆alkyl)₂ureido, C₁₋₆alkylsulphonylamino, carbocyclyl-R⁶- or heterocyclyl-R⁷-; wherein R¹ may be optionally substituted on carbon by one or mote R⁸;
n is selected from 0-2; wherein the values of R¹ may be the same or different;
R² is hydrogen;
R³ is selected from halo, methyl or methoxy;
R⁴ is selected from halo, cyano, hydroxy, amino, carbamoyl, ureido, C₁₋₆alkyl, C₂₋₆alkynyl, C₁₋₆alkoxy, C₁₋₆alkanoyl, *N*-(C₁₋₆alkyl)amino, *N,N*-(C₁₋₆alkyl)₂amino, C₁₋₆alkanoylamino, *N*-(C₁₋₆alkyl)carbamoyl, C₁₋₆alkoxycarbonyl, *N,N-*(C₁₋₆alkyl)₂sulphamoyl, C_{I}-₆alkylsulphonylaiₕiho, carbocyclyl-R¹⁴- or heterocyclyl-R¹⁵-; wherein R⁴ may be optionally substituted on carbon by one or more R¹⁶; and wherein if said heterocyclyl contains an -NH- moiety that nitrogen may be optionally substituted by a group selected from R¹⁷;
R⁵ is selected from C₁₋₆alkyl;
m is selected from 0-2; wherein the values of R⁴ may be the same or different;
R⁶, R⁷, R¹⁸ and R¹⁹ are independently selected from a direct bond, -O-, -S(O)ₛ- or -N(R³⁰)SO₂-; wherein R³⁰ is selected from hydrogen and s is 2;
R⁸ is selected from halo, cyano, hydroxy, C₁₋₆alkyl, C₁₋₆alkoxy, *N,N*-(C₁₋₆alkyl)₂amino, *N,N*-(C₁₋₆alkyl)₂carbamoyl, C₁₋₆alkylS(O)ₐ wherein a is 0 to 2, *N,N*-(C₁₋₆alkyl)₂sulphamoyl, *N-*(C₁₋₆alkyl)-*N*-(C₁₋₆alkoxy)amino, carbocyclyl-R¹⁸- or heterocyclyl-R¹⁹-; wherein R⁸ may be optionally substituted on carbon by one or more R²⁰; and wherein if said heterocyclyl contains an -NH- moiety that nitrogen may be optionally substituted by a group selected from R²¹;
R¹⁴, R¹⁵, R²² and R²³ are independently selected from a direct bond, -N(R²⁶)- or -C(O)N(R²⁸)-; wherein R²⁶ and R²⁸ are hydrogen;
R¹⁶ is selected from halo, hydroxy, amino, C₁₋₆alkoxy, *N*-(C₁₋₆alkyl)amino, *N,N*-(C₁₋₆alkyl)₂amino, carbocyclyl-R²²- or heterocyclyl-R²³ ; wherein R¹⁶ may be optionally substituted on carbon by one or more R²⁴ ; and wherein if said heterocyclyl contains an -NH-moiety that-nitrogen may be optionally substituted by a group selected from R²⁵;
R¹⁷ and R²⁵ are independently selected from C₁₋₆alkyl and C₁₋₆alkoxycarbonyl;
R²¹ is selected from C₁₋₆alkyl;
R²⁰ is selected from cyano or hydroxy;
R²⁴ is methyl or phenyl;
or a pharmaceutically acceptable salt thereof;
with the proviso that said compound is not:
*N*-[4-chloro-3-({[6-(4-methylpiperazin-1-yl)pyridin-3-yl]amino}carbonyl)phenyl]-2-morpholin-4-ylisonicotinamide;
*N*-[4-chloro-3-({[6-(4-ethylpiperazin-1-yl)pyridin-3-yl]amino}carbonyl)phenyl]-2-morpholin-4-ylisonicotinamide;
*N*-{4-chloro-3-[({6-[[3-(dimethylamino)propyl](methyl)amino]pyridin-3-yl}amino)carbonyl] phenyl}-2-morpholin-4-ylisonicotinamide;
*N*-{4-chloro-3-[({6-[[2-(dimethylamino)ethyl](methyl)amino]pyridin-3-yl}amino)carbonyl] phenyl}-2-morpholin-4-ylisonicotinamide; or
*N*-[4-chloro-3-({[6-(4-methyl-1,4-diazepan-1-yl)pyridin-3-yl]amino]carbonyl)phenyl]-2-morpholin-4-ylisonicotinamide.

Therefore in a further aspect of the invention there is provided a compound of formula **(I)** (as depicted above) wherein:
Ring A is phenyl, pyridyl, pyrazolyl, thienyl, indolyl, 2,3-dihydrobenzofuranyl and imidazo[1,2-a]pyridinyl; wherein said pyrazolyl may be optionally substituted on nitrogen by a group selected from R⁵;
R¹ is a substituent on carbon and is selected from halo, cyano, C₁₋₆alkyl, C₂₋₆alkynyl, C₁₋₆alkoxy, *N,N*-(C₁₋₆alkyl)₂amino, C₁₋₆alkylS(O)ₐ wherein a is 0 to 2, *N,N-*(C₁₋₆alkyl)₂sulphamoyl, C₁₋₆alkylsulphonylamino, carbocyclyl-R⁶- or heterocyclyl-R⁷-; wherein R¹ may be optionally substituted on carbon by one or more R⁸;
n is selected from 0-2; wherein the values of R¹ may be the same or different;
R² is hydrogen;
R³ is selected from halo or methyl;
R⁴ is selected from halo, cyano, amino, C₁₋₆alkyl, C₁₋₆alkoxy, *N*-(C₁₋₆alkyl)amino, C₁₋₆alkanoylamino, *N*-(C₁₋₆alkyl)carbamoyl or heterocyclyl-R¹⁵-;
m is selected from 0-2; wherein the values of R⁴ may be the same or different;
R⁵ is C₁₋₆alkyl;
R⁶, R⁷ and R¹⁹ are independently selected, from a direct bond, -S(O)ₛ- or -N(R³⁰)SO₂-; wherein R³⁰ is hydrogen and s is 0-2;
R⁸ is selected from halo, cyano, hydroxy, *N,N-*(C₁₋₆alkyl)₂amino or heterocyclyl-R¹⁹-; wherein if said heterocyclyl contains an -NH- moiety that nitrogen may be optionally substituted by a group selected from R²¹;
R¹⁵ is a direct bond;
R²¹ is C₁₋₆alkyl;
or a pharmaceutically acceptable salt thereof;
with the proviso that said compound is not:
*N*-{4-chloro-3-[({6-[[3-(dimethylamino)propyl](methyl)amino]pyridin-3-yl}amino)carbonyl] phenyl}-2-morpholin-4-ylisonicotinamide; or
*N*-{4-chloro-3-[{6-[[2-(dimethylamino)ethyl](methyl)amino]pyridin-3-yl}amino)carbonyl] phenyl}-2-morpholin-4-ylisonicotinamide.

Therefore in a further aspect of the invention there is provided a compound of formula **(I)** (as depicted above) wherein
Ring A is phenyl, pyrid-2-yl, pyrid-3-yl, pyrid-4-yl, 1-methylpyrazol-S-yl, 1-*t-*butylpyrazol-5-yl, thien-2-yl, thien-3-yl, indol-2-yl, 1-methylindol-2-yl, indol-4-yl, indol-5-yl, indol-6-yl, indol-7-yl, 2,3-dihydrobenzofuran-7-yl, imidazo[1,2-a]pyridin-2-yl, isoxazol-3-yl, pyrrol-2-yl, benzimidazol-6-yl, 1-methyl-2-oxoindolin-5-yl, furan-2-yl, 1,3-thiazol-5-yl, pyrimidin-4-yl and 1-methylpyrrol-2-yl;
R¹ is a substituent on carbon and is selected from fluoro, chloro, bromo, iodo, cyano, hydroxy, sulphamoyl, methyl, trifluoromethyl, 1-cyano-1-methylethyl, methoxy, methoxy, propoxy, isopropoxy, isobutoxy, *N,N*-dimethylamino, difluoromethylthio, *N,N*-dimethylsulphamoyl, *t*-butyl, mesyl, cyclopropylaminosulphonyl, azetidin-1-ylsulphonyl, tetrahydrofuran-2-ylmethylaminosulphonyl, *N*-methyl-*N*-(2,3-dihydroxypropyl)sulphamoyl, mesylamino, morpholinosulphonyl, 1-methylpiperazin-4-ylmethyl, 1-ethylpiperazin-d-ylmethyl, 3,3-dimethylbut-1-yn-1-yl, morpholino, *N,N*-dimethylaminomethyl, 3-methyl-3-hydroxybut-1-yn-1-yl, methylthiomethyl, mesylmethyl, *N*-(methyl)-*N*-(methoxy)sulphamoyl, 2-hydroxymethylpiperidin-1-ylsulphonyl, 3-hydroxymethylpiperidin-1-ylsulphonyl, 4-hydroxymethylpiperidin-1-ylsulphonyl, 1,1-difluoroethyl, piperidin-1-yl, *N,N*-diethylamino, *N,N'*-dimethylureido, cyclopropyl, *t*-butoxycarbonylamino, pyrid-2-yl, phenoxy, 2-methoxy-1,1-dimethylethyl, mesylmethyl, 1,3-thiazol-2-yl, 2-methyl-1,3-thiazol-5-yl,1-methylcyclopropyl, 1,1-dimethylprop-2-yn-1-yl, 1-(*N,N*-dimethylsulphamoyl)-1-methylethyl, 1,1-dimethylbut-2-yn-1-yl, *N*-(methyl)-*N*-(methoxy)aminomethyl, 1-(*N,N*-dimethylcarbamoyl)-1-methylethyl, 4-methylimidazol-1-yl, 1-(cyclopropyl)-1-methylethyl, 2-methyl-3,4-dihydroxybut-2-yl, 2-methylbut-2-yl, 1-hydroxy-1-cyclopropylethyl, 1-cyanoethyl, 2-cyano-3-methylbut-2-yl, 2-cyanobut-2-yl, 1-hydroxy-2-cyanoprop-2-yl and 2-cyanopyrrol-1-ylmethyl;
n is selected from 0-2; wherein the values of R¹ may be the same or different;
R² is hydrogen;
R³ is selected from fluoro, chloro, methyl or methoxy;
R⁴ is selected from fluoro, chloro, bromo, cyano, hydroxy, amino, carbamoyl, ureido, methyl, ethyl, methoxy, methylamino, isopropylamino, morpholino, 2-(dimethylamino)ethylampino, 2-(hydroxy)ethylamino, 2-(amino)ethylamino, 3-(pyrrolidin-1-yl)propylamino, *N*-methylcarbamoyl, acetylamino, 2-hydroxyacetylamino, trifluoromethyl, mesylamino, 2,2-dimethylpropanoylamino, 3-methoxypropanoylamino, cyclobutylcarbonylamino, cyclopropylamino, 2,3-dihydroxypropylamino, 1,3-dihydroxyprop-2-ylamino, 1-methylpiperazin-4-yl, 1-methylpiperazin-4-ylmethyl, acetyl, *N*-methyl-*N*-(3-dimethylaminopropyl)amino, *N*-methyl-*N*-(2-methoxyethyl)amino, dimethylamino, hydroxymethyl, 1,2-dihydroxyethyl, pyrazol-5-ylamino, 3-aminoprop-1-yn-1-yl, 3-hydroxyprop-1-yn-1-yl, 3-methylaminoprop-1-yn-1-yl, 3-dimethylaminoprop-1-yn-1-yl, 4-aminobutylamino, pyrrolidin-2-ylamino, 3-methylaminopropyl, 3-dimethylaminopropyl, 3-hydroxypropyl, 3-dimethylaminopropylamino, aminomethyl, piperazin-1-yl, 1-methylpiperazin-4-yl, 2,2-dimethyl-1,3-dioxolan-4-ylmethylamino, pyrrolidin-3-ylmethylamino, piperidin-4-ylmethylamino, imidazol-2-ylmethylamino, methoxymethyl, *N,N*-dimethylsulphamoyl, formylamino, morpholinomethyl, aminomethyl, 2-(dimethylamino)ethylamino, pyrrol-1-yl, pyrrol-2-yl, pyrrolidin-2-yl, imidazol-4-yl, cyclobutylamino, *N*-methyl-*N*-(2-dimethylaminoethyl)amino, 2-dimethylaminoethoxy, dimethylaminomethyl, cyclopropylaminomethyl, piperidin-1-ylmethyl, methylaminomethyl, pyrrolidin-2-ylmethoxy, 3-dimethylaminopropoxy, methoxycarbonyl, 1-(*t-*butoxycarbonyl)pyrrolidin-2-ylmethylamino, 1-(*t*-butoxycarbonyl)pyrrolidin-2-ylmethoxy, 2-phenoxyacetylamino and 1-(*t*-butoxycarbonyl)pyrrolidin-2-yl;
m is selected from 0-2; wherein the values of R⁴ may be the same or different;
or a pharmaceutically acceptable salt thereof;
with the proviso that said compound is not:
*N*-[4-chloro-3-({[6-(4-methylpiperazin-1-yl)pyridin-3-yl]amino}carbonyl)phenyl]-2-morpholin-4-ylisonicotinamide;
*N-*{4-chloro-3-[({6-[[3-(dimethylamino)propyl](methyl)amino]pyridin-3-yl}amino)carbonyl] phenyl}-2-morpholin-4-ylisonicotinamide; or
*N*-{4-chloro-3-[({6-[[2-(dimethylamino)ethyl](methyl)amino]pyridin-3-yl}amino)carbonyl] phenyl}-2-morpholin-4-ylisonicotinamide.

Therefore in a further aspect of the invention there is provided a compound of formula **(I)** (as depicted above) wherein:
Ring A is phenyl, pyrid-2-yl, pyrid-3-yl, pyrid-4-yl, 1-methylpyrazol-5-yl, thien-2-yl, thien-3-yl, indol-5-yl, indol-6-yl, 2,3-dihydrobenzofuran-7-yl or imidazo[1,2-a]pyridinyl;
R¹ is a substituent on carbon and is selected from fluoro, chloro, bromo, iodo, cyano, methyl, t-butyl, trifluoromethyl, dimethylaminomethyl, 1-methyl-1-cyanoethyl, 4-methylpiperazin-1-ylmethyl, 4-ethylpiperazin-1-ylmethyl, 3-hydroxy-3-methylbut-1-yn-1-yl, 3,3-dimethylbut-1-yn-1-yl, methoxy, propoxy, isopropoxy, isobutoxy, dimethylamino, difluoromethylthio, *N,N*-dimethylsulphamoyl, mesyl, cyclopropylaminosulphonyl, azetidin-1-ylsulphonyl or mesylamino;
n is selected from 0-2; wherein the values of R¹ may be the same or different;
R² is hydrogen;
R³ is selected from fluoro, chloro or methyl;
R⁴ is selected from fluoro, chloro, bromo, cyano, amino, methyl, methoxy, methylamino, acetylamino, *N*-methylcarbamoyl or morpholino;
m is selected from 0-2; wherein the values of R⁴ may be the same or different.
or a pharmaceutically acceptable salt thereof.

In another aspect of the invention, preferred compounds of the invention are any one of the Examples or a pharmaceutically acceptable salt thereof.

In another aspect of the invention, preferred compounds of the invention are any one of:
*N*-(6-amino-5-chloropyridin-3-yl)-5-{[3-(1-cyano-1-methylethyl)benzoyl]amino}-2-methylbenzamide;
*N*-(6-amino-5-chloropyridin-3-yl)-5-{[3-(1-cyano-1-methylethyl)-5-fluorobenzoyl]amino}-2-methylbenzamide;
5-{[3-(1-cyano-1-methylethyl)benzoyl]amino}-*N*-(5-methoxypyridin-3-yl)-2-methyl benzamide;
*N*-(6-amino-5-chloropyridin-3-yl)-2-methyl-5-{[3-(trifluoromethyl)benzoyl]amino} benzamide;
5-{[3-(1-cyano-1-methylethyl)benzoyl]amino}-*N*-(5,6-dimethylpyridin-3-yl)-2-methylbenzamide;
*N*-(3-{[(6-amino-5-chloropyridin-3-yl)amino]carbonyl}-4-methylphenyl)-2-(1-cyano-1-methylethyl)isonicotinamide;
*N*-(6-amino-5-chloropyridin-3-yl)-2-chloro-5-{[3-(trifluoromethyl)benzoyl]amino} benzamide;
*N*-(6-acetylamino-pyridin-3-yl)-5-[3-(cyano-dimethyl-methyl)-benzoylamino]-2-methylbenzamide;
*N*-[6-(acetylamino)pyridin-3-yl]-2-chloro-5-{[3-(trifluoromethyl)benzoyl]amino}benzamide; and
*N*-(6-amino-5-methylpyridin-3-yl)-2-chloro-5-{[3-(1-cyano-1-methylethyl)benzoyl]amino} benzamide;
or a pharmaceutically acceptable salt thereof.

Another aspect of the present invention provides a process for preparing a compound of formula **(I**) or a pharmaceutically acceptable salt thereof which process (wherein variable groups are, unless otherwise specified, as defined in formula **(I))** comprises of:
*Process a)* reacting an amine of the formula **(II)** 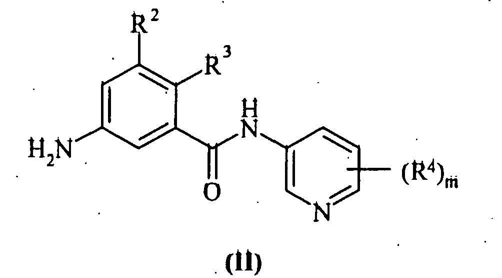 with an acid of formula **(III)**: 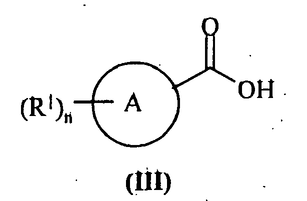 or ah activated acid derivative thereof;
*Process b)* reacting an acid of formula **(IV):** 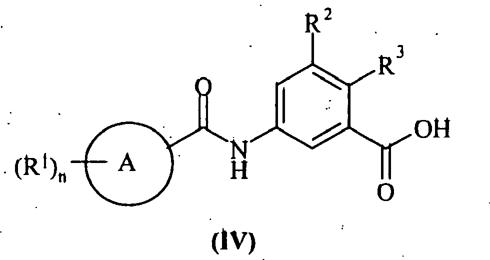 with an amine of formula **(V):** 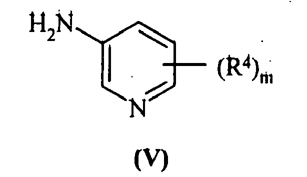
or an activated acid derivative thereof;
and thereafter if necessary:
i) converting a compound of the formula **(I)** into another compound of the formula **(I);**
ii) removing any protecting groups;
iii) forming a pharmaceutically acceptable salt.

Specific reaction conditions for the above reactions are as follows. *Process a)* and *Process b)* Amines and acids may be coupled together in the presence of a suitable coupling reagent. Standard peptide coupling reagents known in the art can be employed as suitable coupling reagents, or for example carbonyldiimidazole and dicyclohexyl-carbodiimide, optionally in the presence of a catalyst such as dimethylaminopyridine or 4-pyrrolidinopyridine, optionally in the presence of a base for Example triethylamine, pyridine, or 2,6-di-*alkyl*-pyridines such as 2,6-lutidine or 2,6-di-*tert-*butylpyridine. Suitable solvents include dimethylacetamide, dichloromethane, benzene, tetrahydrofuran and dimethylformamide. The coupling reaction may conveniently be performed at a temperature in the range of -40 to 40°C.

Suitable activated acid derivatives include acid halides, for Example acid chlorides, and active esters, for Example pentafluorophenyl esters. The reaction of these types of compounds with amines is well known in the art, for Examples they may be reacted in the presence of a base, such as those described above, and in a suitable solvent, such as those described above. The reaction may conveniently be performed at a temperature in the range of -40 to 40 °C.

Amines of formula **(II)** may be prepared according to *Scheme 1:*

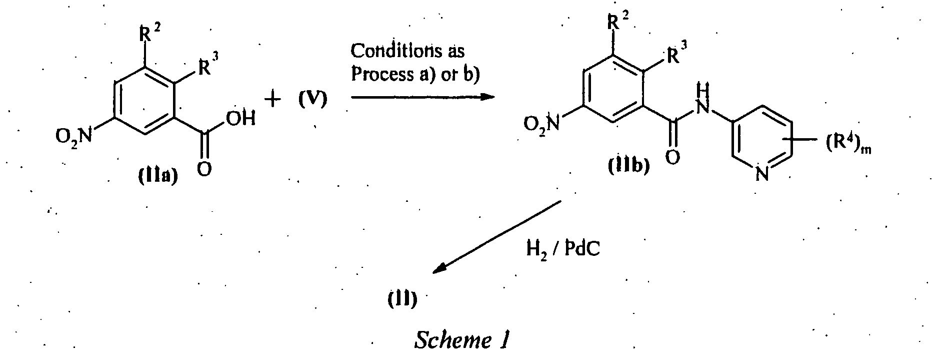

Acids of formula (IV) may be prepared according to *Scheme 2:*

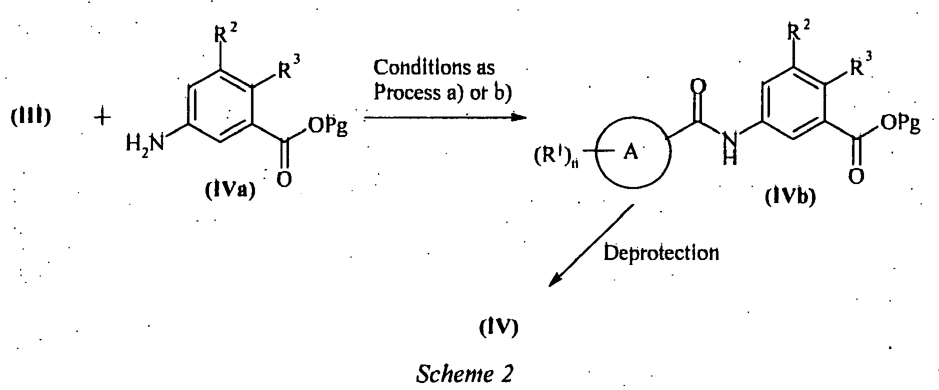

Wherein Pg is an acid protecting group, for example such as those described herein below.

Compounds of formula **(III), (V), (IIa)** and **(IVa)** are commercially available compounds, or they are known in the literature or they may be prepared by standard processes known in the art.

It will be appreciated that certain of the various ring substituents in the compounds of the present invention may be introduced by standard aromatic substitution reactions or generated by conventional functional group modifications either prior to or immediately following the processes mentioned above, and as such are included in the process aspect of the invention. Such reactions and modifications include, for example, introduction of a substituent by means of an aromatic substitution reaction, reduction of substituents, alkylation of substituents and oxidation of substituents. The reagents and reaction conditions for such procedures are well known in the chemical art. Particular examples of aromatic substitution reactions include the introduction of a nitro group using concentrated nitric acid, the introduction of an acyl group using, for example, an acyl halide and Lewis acid (such as aluminium trichloride) under Friedel Crafts conditions; the introduction of an alkyl group using an alkyl halide and Lewis acid (such as aluminium trichloride) under Friedel Crafts conditions; and the introduction of a halogeno group. Particular examples of modifications include the reduction of a nitro group to an amino group by for examples, catalytic hydrogenation with a nickel catalyst or treatment with iron in the presence of hydrochloric acid with heaving; oxidation of alkylthio to alkylsulphinyl or alkylsulphonyl.

It will also be appreciated that in some of the reactions mentioned herein it may be necessary/desirable to protect any sensitive groups in the compounds. The instances where protection is necessary or desirable and suitable methods for protection are known to those skilled in the art. Conventional protecting groups may be used in accordance with standard practice (for illustration see T.W. Green, Protective Groups in Organic Synthesis, John Wiley and Sons, 1991). Thus, if reactants include groups such as amino, carboxy or hydroxy it may be desirable to protect the group in some of the reactions mentioned herein.

A suitable protecting group for an amino or alkylamino group is, for example, an acyl group, for example an alkanoyl group such as acetyl, an alkoxycarbonyl group, for example a methoxycarbonyl, ethoxycarbonyl or *t*-butoxycarbonyl group, an arylmethoxycarbonyl group, for example benzyloxycarbonyl, or an aroyl group, for example benzoyl. The deprotection conditions for the above protecting groups necessarily vary with the choice of protecting group. Thus, for example, an acyl group such as an alkanoyl or alkoxycarbonyl group or an aroyl group may be removed for example, by hydrolysis with a suitable base such as an alkali metal hydroxide, for example lithium or sodium hydroxide. Alternatively an acyl group such as a *t*-butoxycatbonyl group may be removed, for example, by treatment with a suitable acid as hydrochloric, sulphuric or phosphoric acid or trifluoroacetic acid and an arylmethoxycarbonyl group such as a benzyloxycarbonyl group may be removed, for example, by hydrogenation over a catalyst such as palladium-on-carbon, or by treatment with a Lewis acid for example boron tris(trifluoroacetate). A suitable alternative protecting group for a primary amino group is, for example, a phthaloyl group which may be removed by treatment with an alkylamine, for example dimethylaminopropylamine, or with hydrazine.

A suitable protecting group for a hydroxy group is, for example, an acyl group, for example an alkanoyl group such as acetyl, an aroyl group, for example benzoyl, or an arylmethyl group, for example benzyl. The deprotection conditions for the above protecting groups will necessarily vary with the choice of protecting group. Thus, for examples, an acyl group such as an alkanoyl or an aroyl group may be removed, for example, by hydrolysis with a suitable base such as an alkali metal hydroxide, for example lithium or sodium hydroxide. Alternatively an arylmethyl group such as a benzyl group may be removed, for example, by hydrogenation over a catalyst such as palladium-on-carbon.

A suitable protecting group for a carboy group is, for example, an esterifying group, for example a methyl or an ethyl group which may be removed, for example, by hydrolysis with a base such as sodium hydroxide, or for example a *t-*butyl group which may be removed, for example, by treatment with an acid, for example an organic acid such as trifluoroacetic acid, or for example a benzyl group which may be removed, for examples, by hydrogenation over a catalyst such as palladium-on-carbon.

The protecting groups may be removed at any convenient stage in the synthesis using conventional techniques well known in the chemical art.

As stated hereinbefore the compounds defined in the present invention possess anti-cancer activity which is believed to arise from the B-Raf inhibitory activity of the compounds. These properties may be assessed, for example, using the procedure set out below.

### B-Raf in vitro ELISA assay

Activity of human recombinant, purified wild type His-B-Raf protein kinase was determined in vitro using an enzyme-linked immunosorbent assay (ELISA) assay format, which measures phosphorylation of the B-Raf substrate, human recombinant, purified His-derived (detagged) MEK1. The reaction utilized 2.5 nM B-Raf, 0.15 µM MEK1 and 10 µM adenosine triphosphate (ATP) in 40 mM *N*-(2-hydroxyethyl)piperazine-N'-(2-ethanesulfonic acid hemisodium salt (HEPES), 5 mM 1,4-dithio-DL-threitol (DTT), 10 mM MgCl₂, 1 mM ethylenediaminetetraacetic acid (EDTA) and 0.2 M NaCl (1x HEPES buffer), with or without compound at various concentrations, in a total reaction volume of 25µl in 384 well plates. B-Raf and compound were preincubated in 1x HEPES buffer for 1 hour at 25°C. Reactions were initiated with addition of MEK 1 and ATP in 1x HEPES buffer and incubated at 25 °C for 50 minutes and reactions stopped by addition of 10 µl 175 mM EDTA (final concentration 50 mM) in 1x HEPES buffer. 5 µl of the assay mix was then diluted 1:20 into 50 mM EDTA in 1x HEPES buffer, transferred to 384 well black high protein binding plates and incubated overnight at 4 °C. Plates were washed in tris buffered saline containing 0.1 % Tween20 (TBST), blocked with 50 µl Superblock (Pierce) for 1 hour at 25 °C , washed in TBST, incubated with 50 µl rabbit polyclonal anti-phospho-MEK antibody (Cell Signaling) diluted 1:1000 in TBS for 2 hours at 25 °C, washed with TBST, incubated with 50 µl goat anti-rabbit horseradish peroxidase -linked antibody (Cell Signaling) diluted 1:2000 in TBS for 1 hour at 25 °C and washed with TBST. 50 µl of fluorogenic peroxidase substrate (Quantablu Pierce) was added and following incubation for 45-60 minutes, 50 µl QuantabluSTOP (Pierce) was added. Blue fluorescent product was detected at excitation 325 and emission 420 using a TECAN Ultra plate reader. Data was graphed and IC₅₀s calculated using Excel Fit (Microsoft).

When tested in the above *in vitro* assay, the compounds of the present invention exhibited activity less than 30 µM. For example the following results were obtained:

| **Example No** | **IC₅₀ (µM)** |
|---|---|
| Example 44 | 0.057 |
| Example 5 | 0.505 |
| Example 9 | 1.08 |

According to a further aspect of the invention there is provided a pharmaceutical composition which comprises a compound of the formula (I), or a pharmaceutically acceptable salt thereof, as defined hereinbefore, in association with a pharmaceutically-acceptable diluent or carrier.

The composition may be in a form suitable for oral administration, for example as a tablet or capsule, for parenteral injection (including intravenous, subcutaneous, intramuscular, intravascular or infusion) as a sterile solution, suspension or emulsion, for topical administration as an ointment or cream or for rectal administration as a suppository.

In general the above compositions may be prepared in a conventional manner using conventional excipients.

The compound of formula **(I)** will normally be administered to a warm-blooded animal at a unit dose within the range 1-1000 mg/kg, and this normally provides a therapeutically-effective dose. Preferably a daily dose in the range of 10-100 mg/kg is employed. However the daily dose will necessarily be varied depending upon the host treated, the particular route of administration, and the severity of the illness being treated. Accordingly the optimum dosage may be determined by the practitioner who is treating any particular patient.

According to a further aspect of the present invention there is provided a compound of the formula **(I)**, or a pharmaceutically acceptable salt thereof, as defined hereinbefore for use in a method of treatment of the human or animal body by therapy.

We have found that the compounds defined in the present invention, or a pharmaceutically acceptable salt thereof, are effective anti-cancer agents which property is believed to arise from their B-Raf inhibitory properties. Accordingly the compounds of the present invention are expected to be useful in the treatment of diseases or medical conditions mediated alone or in part by B-Raf, i.e. the compounds may be used to produce a B-Raf inhibitory effect in a warm-blooded animal in need of such treatment.

Thus the compound of the present invention provide a method for treating cancer characterised by inhibition of B-Raf, i.e. the compounds may be used to produce an anti-cancer effect mediated alone or in part by the inhibition of B-Raf.

. Such a compound of the invention is expected to possess a wide range of anti-cancer properties as activating mutations in B-Raf have been observed in many human cancers, including but not limited to, melanoma, papillary thyroid tumors, cholangiocarcinomas, colon, ovarian and lung cancers. Thus it is expected that a compound of the invention will possess anti-cancer activity against these cancers. It is in addition expected that a compound of the present invention will possess activity against a range of leukaemias, lymphoid malignancies and solid tumours such as carcinomas and sarcomas in tissues such as the liver, kidney, bladder, prostate, breast and pancreas. In particular such compounds of the invention are expected to slow advantageously the growth of primary and recurrent solid tumours of, for example, the skin, colon, thyroid, lungs and ovaries. More particularly such compounds of the invention, or a pharmaceutically acceptable salt thereof, are expected to inhibit the growth of those primary and recurrent solid tumours which are associated with B-Raf, especially those tumours which are significantly dependent on B-Raf for their growth and spread, including for example, certain tumours of the skin, colon, thyroid, lungs and ovaries. Particularly the compounds of the present invention are useful in the treatment of melanomas.

Thus according to this aspect of the invention there is provided a compound of the formula **(I)**, or a pharmaceutically acceptable salt thereof, as defined hereinbefore for use as a medicament.

According to a further aspect of the invention there is provided the Use of a compound of the formula **(I)**, or a pharmaceutically acceptable salt thereof, as defined hereinbefore in the manufacture of a medicament for use in the production of a B-Raf inhibitory effect in a warm-blooded animal such as man.

According to this aspect of the invention there is provided the use of a compound of the formula **(I)**, or a pharmaceutically acceptable salt thereof, as defined hereinbefore in the manufacture of a medicament for use in the production of an anti-cancer effect in a warm-blooded animal such as man.

According to a further feature of the invention, there is provided the use of a compound of the formula **(I)**, or a pharmaceutically acceptable salt thereof, as defined herein before in the manufacture of a medicament for use in the treatment of melanoma, papillary thyroid tumours, cholangiocarcinomas, colon cancer, ovarian cancer, lung cancer, leukaemias, lymphoid malignancies, carcinomas and sarcomas in the liver, kidney, bladder, prostrate, breast and pancreas, and primary and recurrent solid tumours of the skin, colon, thyroid, lungs and ovaries.

According to a further feature of this aspect of the invention there is provided a method for producing a B-Raf inhibitory effect in a warm-blooded animal, such as man, in need of such treatment which comprises administering to said animal an effective amount of a compound of formula **(I)**, or a pharmaceutically acceptable salt thereof, as defined above.

According to a further feature of this aspect of the invention there is provided as method for producing an anti-cancer effect in a warm-blooded animal, such as man, in need of such treatment which comprises administering to said animal an effective amount of a compound of formula **(I)**, or a pharmaceutically acceptable salt thereof, as defined above.

According to an additional feature of this aspect of the invention there is provided a method of treating melanoma, papillary thyroid tumours, cholangiocarcinomas, colon cancer, ovarian cancer, lung cancer, leukaemias, lymphoid malignancies, carcinomas and sarcomas in the liver, kidney, bladder, prostate, breast and pancreas, and primary and recurrent solid tumours of the skin, colon, thyroid, lungs and ovaries, in a warm-blooded animal, such as man, in need of such treatment which comprises administering to said animal an effective amount of a compound of formula **(I)** or a pharmaceutically acceptable salt thereof as defined herein before.

According to a further aspect of the invention there is provided the use of a compound of the formula **(I)**, or a pharmaceutical acceptable salt thereof, as defined hereinbefore for use in the production of a B-Raf inhibitory effect in a warm-blooded animal such as man.

. According to this aspect of the invention there is provided the use of a compound of the formula **(I)**, or a pharmaceutically acceptable salt thereof, as defined hereinbefore for use in the production of an anti-cancer effect in a warm-blooded animal such as man.

According to a further feature of the invention, there is provided the use of a compound of the formula **(I),** or a pharmaceutically acceptable salt thereof, as defined herein before for use in the treatment of melanoma, papillary thyroid tumours, cholangiocarcinomas, colon cancer, ovarian cancer, lung cancer, leukaemias, lymphoid malignancies, carcinomas and sarcomas in the liver, kidney, bladder, prostate, breast and pancreas, and primary and recurrent solid tumours of the skin, colon, thyroid, lungs and ovaries.

In a further aspect of the invention there is provided a pharmaceutical composition which comprises a compound of the formula **(I)**, or a pharmaceutically acceptable salt thereof, as defined herein before in association with a pharmaceutically-acceptable diluent or carrier for use in the production of a B-Raf inhibitory effect in a warm-blooded animal such as man.

In a further aspect of the invention there is provided a pharmaceutical composition which comprises a compound of the formula **(I)**, or a pharmaceutically acceptable salt thereof, as defined herein before in association with a pharmaceutically-acceptable diluent or carrier for use in the production of an anti-cancer effect in a warm-blooded animal such as man.

In a further aspect of the invention there is provided a pharmaceutical composition which comprises a compound of the formula **(I)**, or a pharmaceutically acceptable salt thereof, as defined herein before in association with a pharmaceutically-acceptable diluent or carrier for use in the treatment of melanoma, papillary thyroid tumours, cholangiocarcinomas, colon cancer, ovarian cancer, lung cancer, leukaemias, lymphoid malignancies, carcinomas and sarcomas in the liver, kidney, bladder, prostate, breast and pancreas, and primary and recurrent solid tumours of the skin, colon, thyroid, lungs and ovaries in a warm-blooded animal such as man.

The B-Raf inhibitory treatment defined hereinbefore may be applied as a sole therapy or may involve, in addition to the compound of the invention, conventional surgery or radiotherapy or chemotherapy. Such chemotherapy may include one or more of the following categories of anti-tumour agents :-
(i) antiproliferative/antineoplastic drugs and combinations thereof, as used in medical oncology, such as alkylating agents (for example cis-platin, carboplatin, cyclophosphamide, nitrogen mustard, melphalan, chlorambucil, busulphan and nitrosoureas); antimetabolites (for example antifolates such as fluoropyrimidines like 5-fluorouracil and tegafur, raltitrexed, methotrexate, cytosine arabinoside and hydroxyurea; antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, amsacrine, topotecan and camptothecin);
(ii) cytostatic agents such as antioestrogens (for examples tamoxifen, toremifene, raloxifene, droloxifene and iodoxyfene), oestrogen receptor down regulators (for example fulvestrant), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride;
(iii) Agents which inhibit cancer cell invasion (for example metalloproteinase inhibitors like marimastat and inhibitors of urokinase plasminogen activator receptor function);
(iv) inhibitors of growth factor function, for example such inhibitors include growth factor antibodies, growth factor receptor antibodies (for example the anti-erbb2 antibody trastuzumab [Herceptin^{™}] and the anti-erbb1 antibody cetuximab [C225]), farnesyl transferase inhibitors, MEK inhibitors, tyrosine kinase inhibitors and serine/threonine kinase inhibitors, for example inhibitors of the epidermal growth factor family (for example EGFR family tyrosine kinase inhibitors such as *N*-(3-chloro-4-fluorophenyl)-7-methoxy-6-(3-morpholinopropoxy)quinazolin-4-amine (gefitinib, AZD1839), *N*-(3-ethynylphenyl)-6,7-bis(2-methyxyethoxy)quinazolin-4-amine (erlotinib, OS1-774) and 6-acrylamido-*N*-(3-chloro-4-fluorophenyl)-7-(3-morpholinopropoxy)quinazolin-4-amine (Cl 1033)), for example inhibitors of the platelet-derived growth factor family and for example inhibitors of the hepatocyte growth factor family;
(v) antiangiogenic agents such as those which inhibit the effects of vascular endothelial growth factor, (for example the anti-vascular endothelial cell growth factor antibody bevacizumab [Avastin^{™}], compounds such as those disclosed in International Patent Applications WO 97/22596, WO 97/30035, WO 97/32856 and WO 98/13354) and compounds that work by other mechanisms (for example linomide, inhibitors of integrin αvβ3 function and angiostatin);
(vi) vascular damaging agents such as Combretastatin A4 and compounds disclosed in International Patent Applications WO 99/02166, WO00/40529, WO 00/41669, WO01/92224, WO02/04434 and WO02/08213;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy;
(ix) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies;
(x) Cell cycle inhibitors including for example CDK inhibitiors (eg flavopiridol) and other inhibitors of cell cycle checkpoints (eg checkpoint kinase); inhibitors of aurora kinase and other kinases involved in mitosis and cytokinesis regulation (eg mitotic kinesins); and histone deacetylase inhibitors; and
(xi) endothelin antagonists, including endothelin A antagonists, endothelin B antagonists and endothelin A and B antagonists; for example ZD4054 and ZD1611 (WO 96 40681), atrasentan and YM598.

Such conjoint treatment may be achieved by way of the simultaneous, sequential or separate dosing of the individual components of the treatment. Such combination products employ the compounds of this invention within the dosage range described hereinbefore and the other pharmaceutically-active agent within its approved dosage range.

In addition to their use in therapeutic medicine, the compounds of formula **(I)** and their pharmaceutically acceptable salts are also useful as pharmacological tools in the development and standardisation of in vitro and *in vivo* test systems for the evaluation of the effects of inhibitors of B-Raf in laboratory animals such as cats, dogs, rabbits, monkeys, rats and mice, as part of the search for new therapeutic agents.

In the above other pharmaceutical composition, process, method, Use and medicament manufacture features, the alternative and preferred embodiments of the compounds of the invention described herein also apply.

### Examples

The invention will now be illustrated by the following non limiting examples in which, unless stated otherwise:
(i) temperatures are given in degrees Celsius (°C); operations were carried out at room or ambient temperature, that is, at a temperature in the range of 18-25°C;
(ii) organic solutions were dried over anhydrous sodium sulphate; evaporation of solvent was carried out using a rotary evaporator under reduced pressure (600-4000 Pascals; 4.5-30 mmHg) with a bath temperature of up to 60 °C;
(iii) in general, the course of reactions was followed by TLC and reaction times are given for illustration only;
(iv) final products had satisfactory proton nuclear magnetic resonance (NMR) spectra and/or mass spectral data;
(v) yields are given for illustration only and are not necessarily those which can be obtained by diligent process development; preparations were repeated if more material was required;
(vii) when given, NMR data is in the form of delta values for major diagnostic protons, given in parts per million (ppm) relative to tetramethylsilane (TMS) as an internal standard, determined at 400 MHz using perdeuterio dimethyl sulphoxide (DMSO-d₆) as solvent unless otherwise indicated;
(vii) chemical symbols have their usual meanings; SI units and symbols are used;
(viii) solvent ratios are given in volume:volume (v/v) terms; and
(ix) mass spectra were run with an electron energy of 70 electron volts in the chemical ionization (CI) mode using a direct exposure probe; where indicated ionization was effected by electron impact (EI), fast atom bombardment (FAB) or electrospray (ESP); values for m/z are given; generally, only ions which indicate the parent mass are reported; and unless otherwise stated, the mass ion quoted is (MH)⁺;
(x) where a synthesis is described as being analogous to that described in a previous example the amounts used are the millimolar ratio equivalents to those used in the previous example;
(xi) the following abbreviations have been used:
   - HATU: *O*-(7-Azabenzotriazol-1-yl)-*N,N,N',N*'-tetramethyluronium hexafluorophosphate;
   - THF: tetrahydrofuran;
   - DMF: *N,N*-dimethylformamide;
   - EtOAc: ethyl acetate;
   - DIEA: *N*, *N*-diisopropylethylamine;
   - DCM: dichloromethane;
   - DMSO: dimethylsulphoxide;
   - MeCN: acetonitrile;
   - NBS: *N*-bromosuccinimide; and
   - MeoH: methanol;
(xii) "ISCO" refers to normal phase flash column chromatography using 12 g and 40 g prepacked silica gel cartridges used according to the manufacturers instruction obtained from ISCO, Inc, 4700 superior street Lincoln, NE, USA.; and
(xiii) "Gilson HPLC" refers to a YMC-AQC18 reverse phase HPLC Column with dimension 20 mm/100 and 50 mm/250 in water/MeCN) with 0.1% TFA as mobile phase, obtained
(xiv) Parr Hydrogenator or Parr shaker type hydrogenators are systems for treating chemicals with hydrogen in the presence of a catalyst at pressures up to 5 atmospheres (60 psig) and temperatures to 80 °C.

### Example 1

### 5-[(3-Fluorobenzoyl)amino]-2-methyl-N-pyridin-3-ylbenzamide

A solution of 5-amino-2-methyl-*N*-pyridin-3-ylbenzamide (Method 68; 60 mg, 0.264 mmol), 3-fluorobenzoic acid (41 mg, 0.290 mmol) and DIEA (115 µL, 0.66 mmol) in DMF (1.5 ml) was treated with HATU (120 mg, 0.3168 mmol). The reaction mixture was shaken overnight at 25 °C. Water (10 ml) was added slowly to precipitate the product. The resulting precipitate was washed with water (10 ml), isolated and dried overnight in a vacuum oven at 70 °C to give the title compound 61.2 mg, (66%) as a solid. NMR (300 MHz): 10.59 (s, 1H), 10.43 (s, 1H), 8.87 (s, 1H), 8.30 (d, 1H), 8.18 (d, 1H), 7.92 (s, 1H), 7.75-7.84 (m, 3H), 7.55-7.63 (m, 1H), 7.37-7.48 (m, 2H), 7.31 (d, 1H), 2.35 (s, 3H); *m*/*z* 349.

### Examples 2-120

The following compounds were prepared by the procedure in Examples 1 using 5-amino-2-methyl-*N*-pyridin-3-ylbenzamide (Method 68) or 5-amino-2-chloro-*N-*(5-fluoropyridin-3-yl)benzamide for (Method 79) Examples 55 and the appropriate SM. In some cases, further purification was required (supercritical fluid, Gilson reverse phase preparatory HPLC or column chromatography utilizing an ISCO system).

| **Ex.** | **Compound** | **NMR** | **m/z** | **SM** |
|---|---|---|---|---|
| **2** | 4-Chloro-*N-*{4-methyl-3-[(pyridin-3-ylamino)carbonyl]phenyl}pyridine-2-carboxamide | 10.83 (s, 1H), 10.62 (s, 1H), 8.90 (s, 1H), 8.72 (d, 1H), 8.30 (d, 1H), 8.14-8.21 (m, 2H), 8.09 (s, 1H), 7.83=7.95 (m, 2H), 7.29-7.41 (m, 2H), 2.35 (s, 3H) | 367 | 4-Chloropyridine-2-carboxylic acid |
| **3** | 2-Methyl-5-{[4-methyl-3-(trifluoromethyl) benzoyl]amino}-*N-*pyridin-3-ylbenzamide | 10.59 (s, 1H), 10.52 (s, 1H), 8.88 (s, 1H), 8.25-8.32 (m, 2H), 8:17 (d, 2H), 7.83-7.94 (m, 2H), 7.63 (d, 1H), 7.30-7.41 (m, 2H), 2.52 (s, 3H), 2.35 (s, 3H) | 414 | 4-Methyl-3-(trifluoromethyl )benzoic acid |
| **4** | *N-*{4-Methyl-3-[(pyridin-3-ylamino)carbonyl]phenyl}-1H-indole-6-carboxamide | 11.48 (s, 1H), 10.58 (s, 1H), 10.28 (s, 1H), 8.89 (s, 1H), 8.31 (d, 1H), 8.19 (d, 1H), 8.08 (s, 1H), 7.98 (s, 1H), 7.87 (d, 1H), 7.65 (s, 2H), 7.55 (t, 1H), 7.39 (dd, 1H), 7.29 (d, 1H), 6.52 (s, 1H), 2.35 (s, 3H) | 371 | 1H-Indole-6-carboxylic acid |
| **5** | 5-[(3-Cyanobenzoyl)amino]-2-methyl-*N-*pyridin-3-ylbenzamide | 10.58 (d, 2H), 8.88 (s, 1H), 8.42 (s, 1H), 8.24-8.32 (m, 2H), 8.18 (d, 1H), 8.07 (d, 1H), 7.91 (s, 1H), 7.73-7.85 (m, 2H), 7.30-7.42 (m, 2H), 2.36 (s, 3H) | 357 | 3-Cyanobenzoic acid |
| **6** | 1,3-Dimethyl-*N-*{4-methyl-3-[(pyridin-3-ylamino)carbonyl]phenyl}-1H-pyrazole-5-carboxamide | 10.55 (s, 1H), 10.05 (s, 1H), 8.88 (s, 1H), 8.30 (d, 1H), 8.18 (d, 1H), 8.00 (s, 1H), 7.83 (d, 1H), 7.39 (dd, 1H), 7.24 (d, 1H), 6.55 (s, 1H), 3.82 (s, 3H),2.31 (d, 6H) | 350 | 1,3-Dimethyl-1H-pyrazole-5-carboxylic acid |
| **7** | 5-[(4-Fluoro-3-methylbenzoyl) amino]-2-methyl-*N*-pyridin-3-ylbenzamide | 10.59 (s, 1H), 10.35 (s, 1H), 8.89 (s, 1H), 8.30 (d, 1H), 8.18 (d; 1H), 7.81-7.95 (m, 4H), 7.26-7.41 (m, 3H), 2.33 (d, 6H) | 364 | 4-Fluoro-3-methylbenzoic acid |
| **8** | 5-[(4-Methoxy-3-methylbenzoyl) amino]-2-methyl-*N-*pyridin-3-ylbenzamide | 10.57 (s, 1H), 10.14 (s, 1H), 8.88 (s, 1H), 8.30 (d, 1H), 8.18 (d, 1H), 7.91 (s, 1H), 7.80-7.88 (m, 3H), 7.39 (dd, 1H), 7.28 (d, 1H), 7.06 (d, 1H), 3.79-3.88 (m, 3H), 2.34 (s, 3H), 2.21 (s, 3H) | 376 | 4-Methoxy-3-methylbenzoic acid |
| **9** | *N*-{4-Methyl-3-[(pyridin-3-ylamino)carbonyl]phenyl}-2,3-dihydro-1-benzofuran-7-carboxamide | 10.58 (s, 1H), 9.84 (s, 1H), 8.89 (s, 1H), 8.31 (d, 1H), 8.18 (d, 1H), 7.86 (s, 1H), 7.77 (d, 1H), 7.60 (d,1H), 7.37-7.46 (m, 2H), 7.29 (d, 1H), 6.98 (t, 1H), 4.74 (t, 2H), 3.27 (t, 2H), 2.34 (s, 3H) | 374 | 2,3-Dihydro-1-benzofuran-7-carboxylic acid |
| **10** | *N*-{4-Methyl-3-[(pyridin-3-ylamino)carbonyl]phenyl}-1H-indole-5-carboxamide | 11.45 (s, 1H), 10.59 (s, 1H), 10.25 (s, 1H), 8.90 (s, 1H), 8.26-8.32 (m, 2H), 8.20 (d, 1H), 8.00 (s, 1H), 7.87 (d, 1H), 7.75 (d, 1H), 7.37-7.50 (m, 3H), 7.28 (d, 1H), 6.58 (s, 1H), 2.35 (s,3H) | 371 | 1H-Indole-5-carboxylic acid |
| **11** | 8-Methyl-*N-*{4-methyl-3-[(pyridin-3-ylamino)carbonyl]phenyl}imidazo[1,2-a]pyridine-2-carboxamide | 10.58 (s, 1H), 10.14 (s, 1H), 8.89 (s, 1H), 8.44-8.52 (m, 2H), 8.31 (d, 1H), 8.19 (d, 1H), 8.07 (s, 1H), 7.94 (d, 1H), 7.39 (dd, 1H), 7.30 (d, 1H), 7.17 (d, 1H), 6.91 (t, 1H), 2.57 (s, 3H), 2.35 (s, 3H) | 386 | 8-Methyl-imidazo[1,2-a]pyridine-2-carboxylic acid |
| **12** | 5-Methyl-*N*-{4-methyl-3-[(pyridin-3-ylamino)carbonyl] phenyl} nicotinamide | 10.53 (s, 1H), 10.46 (s, 1H), 8.86, (d, 1H), 8.23 (d, 1H), 8.55, (s, 1H), 8.24 (d, 1H), 8.12 (d, 1H), 8.06 (s, 1H), 7.85 (d, 1H), 7.75 (m, 1H), 7.33 (m, 1H), 7.25 (m, 1H), 2.33 (s, 3H), 2.30 (s, 3H) | 346 | 5-Methylnicotinic acid |
| **13** | 6-Methyl-*N*-{4-methyl-3-[(pyridin- 3-ylamino)carbonyl]phenyl}pyridine-2-carboxamide | 10.46 (s, 1H), 10.39 (s, 1H), 8.74 (d, 1H), 8.14 (d, 1H), 8.02 (s, 1H), 7.90 (d, 1H), 7.7-7.8 (m, 3H), 7.37 (m, 1H), 7.23 (m, 1H), 7.15 (d, 1H), 2.46 (s, 3H), 2.20 (s, 3H) | 346 | 6-Methylpyridine-2-carboxylic acid |
| **14** | 5-{[4-Methoxy-3-(trifluoromethyl)benzoyl]amino}-2-methyl-*N*-pyridin-3-ylbenzamide | 10.65 (s, 1H), 10.59 (s, 1H), 9.00 (s, 1H), 8.39 (s, 3H), 8.26 (s, 1H), 8.02 (s, 1H), 7.93.(s, 1H), 7.49 (s, 2H), 7.37 (s, 1H), 4.06 (s, 3H), 2.43 (s, 3H) | 429 | 4-Methoxy-3-(trifluoromethyl )-benzoic acid |
| **15** | 2-Methyl-5-{[2-methyl-5-(trifluoromethyl)benzoyl]amino}-*N*-pyridin-3-ylbenzamide | 10.61 (s, 2H), 8.89 (s, 1H), 8.32 (d, 1H), 8.19 (d, 1H), 7.88 (s, 1H), 7.77 (m, 3H), 7.58 (d, 1H), 7.40 (m, 1H), 7.32 (d, 1H), 3.39 (m, 1H), 2.47 (s, 3H), 2.36(s, 3H) | 413 | 2-Methyl-5-(trifluoromethyl )-benzoic acid |
| **16** | 2-Chloro-*N*-{4-methyl-3-[(pyridin-3-ylamino)carbonyl]phenyl}-5-(trifluoromethyl) benzamide | 10.73 (s, 1H), 10.57 (s, 1H), 8.83 (s, 1H), 8.26 (d, 1H), 8.13 (d, 1H), 7.97 (s, 1H), 7.74-7.89 (m, 3H), 7.68 (d; 1H), 7.24-7.39 (m, 2H), 3.29 (s, 4H), 2.45 (s, 3H), 2.31 (s, 3H) | 434 | 2-Chloro-5-(trifluoromethyl )-benzoic acid |
| **17** | 2-Fluoro-*N*-{4-methyl-3-[(pyridin-3-ylamino)carbonyl]phenyl)-5-(trifluoromethyl) benzamide | 10.95 (s, 1H), 10.84 (s, 1H), 9.10 (s, 1H), 8.53 (d, 1H), 8.41 (d, 1H), 8.28 (d, 1H), 8.23 (dd,1H), 8.06 (s, 1H), 7.97 (d, 1H), 7.85 (t, 1H), 7.63 (dd, 1H), 7.55 (d, 1H), 2.58 (s, 3H) | 417 | 2-Fluoro-5-(trifluoromethyl )-benzoic acid |
| **18** | 5-{[3-Fluoro-5-(trifluoromethyl) benzoyl]amino}-2-methyl-*N*-pyridin-3-ylbenzamide | 10.60 (s, 2H), 10.58 (s, 2H), 8.85 (s, 1H), 8.27 (d, 1H), 8.14 (s, 2H), 8.09 (d, 1H), 7.93 (d, 1H), 7.85 (s, 1H), 7.80 (d, 1H), 7.37 (dd, 1H), 7.29 (d, 1H), 2.31 (s, 3H), | 417 | 3-Fluoro-5-(trifluoromethyl )-benzoic acid |
| **19** | 5-{[4-Fluoro-3-(trifluoromethyl) benzoyl]amino}-2-methyl-*N*-pyridin-3-ylbenzamide | 10.53 (s, 2H), 8.80 (s, 1H), 8.21-8.36 (m, 314), 8.10 (d, 1H), 7.81 (s, 1 H), 7.76 (d, 1H), 7.64 (t, 1H), 7.32 (dd, 1H), 7.24 (d, 1H), 2.28 (s, 3H) | 417 | 4-Fluoro-3-(trifluoromethyl )-benzoic acid |
| **20** | 1-Methyl-*N*-{4-methyl-3-[(pyridin-3-ylamino)carbonyl]phenyl}-3-(trifluoromethyl)-1H-pyrazole-5-carboxamide | 10.61 (s, 1H), 10.54 (s, 1H), 8.88 (s, 1H), 8.32 (s, 1H), 8.18 (s, 1H), 7.88 (s, 1H), 7.78 (s, 1H), 7.58 (s, 1H), 7.37 (s, 2H), 4.18 (s, 3H), 2.36 (s, 3H) | 403 | 1-Methyl-3-(trifluoromethyl)-1H-pyrazole-5-carboxylic acid |
| **21** | 2-Methyl-5-({3-[(methylsulfonyl) amino]benzoyl} amino)-*N*-pyridin-3-ylbenzamide | 10.61 (s, 1H), 10.43 (s, 1H), 10.02 (s, 1H), 8.91 (s, 1H), 8.32 (d, 1H), 8.20 (d, 1H), 7.90-7.99 (m, 1H), 7.70-7.85 (m, 3H), 7.38-7.54 (m, 3H), 7.32 (d, 1H), 3.05 (s, 3H), 2.37 (s, 3H) | 424 | 3-[(Methylsulfonyl)amino]benzoic acid |
| **22** | 5-(1-Cyano-1-methylethyl)-*N*-{4-methyl-3-[(pyridin-3-ylamino)carbonyl]phenyl}thiophene-3-carboxamide | 11.09 (s, 1H), 10.29 (s, 1H), 9.21 (s, 1H), 8.55 (d, 1M), 8.47 (d, 1H), 8.37 (s, 1H), 7.97 (d, 1H), 7.84 (m, 2H), 7.72 (d, 1H), 7.33 (d, 1H), 2.36 (s, 3H), 1.79 (s, 6H) | 405 | Method 110 |
| **23** | 5-(1-Cyano-1-methylethyl)-*N*-{4-methyl-3-[(pyridin-3-ylamino)carbonyl]phenyl}thiophene-2-carboxamide | 11.01 (s, 1H), 10.46 (s, 1H), 9.16 (s, 1H), 8.51 (d, 1H), 8.46 (d, 1H), 7.96 (dd, 2H), 7.78 (dd, 2H), 7.34-7.30 (m, 2H), 2.36 (s, 3H), 1.78 (s, 6H) | 404 | Method 109 |
| **24** | 5-{[4-Chloro-3-(1-cyano-1-methylethyl)benzoyl]amino}-2-methyl-*N*-pyridin-3-ylbenzamide | 11.04 (s, 1H), 10.58 (s, 1H), 9.17 (d, 1H), 8.53 (d, 1H), 8.45 (d, 1H), 8.03-7.94 (m, 3H), 7.82-7.73 (m, 3H), 7.35 (d, 1H), 2.3.7 (s, 3H), 1.86 (s, 6H) | 433 | Method 26 |
| **25** | 2-(1-Cyano-1-methylethyl)-*N*-{4-methyl-3-[(pyridin-3-ylamino)carbonyl]phenyl}isonicotinamide | 11.21 (s, 1H), 10.81 (s, 1H), 9.28 (s, 1H), 8.81 (d, 1H), 8.62-8.53 (m, 2H), 8.03 (d, 2H), 7.99-7.82 (m, 3H), 7.37 (d, 1H), 2.39 (s, 3H), 1.76 (s, 6H) | 400 | Method 112 |
| **26** | 5-[(3-tert-Butylbenzoyl)amino]-2-methyl-*N*-pyridin-3-ylbenzamide | 10.97 (s, 1H), 10.36 (s, 1H), 9.15 (s, 1H), 8.50 (d, 1H), 8.42 (d, 1H), 8.00 (d, 1H), 7.94.(s, 1H), 7.84-7.73 (m, 3H), 7.62 (d, 1H), 7.45 (t, 1H), 7.32 (d, 1H), 2.37 (s, 3H), 1.33 (s, 9H) | 388 | Method 113 |
| **27** | 5-{[3-(Azetidin-1-ylsulfonyl)benzoyl]amino}-2-methyl-*N*-pyridin-3-ylbenzamide | 10.66 (s, 1H), 10.58 (s, 1H), 8.87 (d, 1H), 8.35 (m, 2H), 8.30 (dd, 1H), 8.17 (m, 1H), 8.02 (m, 1H), 7.88 (m, 3H), 7.39 (dd, 1H), 7.33 (d, 1H), 3.71 (t, 2H), 2.68 (s, 3H), 2.36 (bs, 2H), 2.00 (m, 2H) | 451 | Method 121 |
| **28** | 2-Methyl-5-{[3-(methylsulfonyl) benzoyl]amino}-*N*-pyridin-3-ylbenzamide | 11.19 (s, 1H), 10.80 (s, 1H), 9.25 (s, 1H), 8.56 (m, 3H), 8.31 (m, 1H), 8.13 (m, 1H), 8.03 (d, 1H), 7.86 (m, 3H), 7.35 (d, 1H), 2.49 (s, 3H), 2.38 (s, 3H) | 409 | 3-(Methylsulfonyl)-benzoic acid |
| **29** | 5-({3-[(Cyclopropylamino)sulfonyl]benzoyl}amino)-2-methyl-*N*-pyridin-3-ylbenzamide | 11.16 (s, 1H), 10.74 (s, 1H), 9.24 (s, 1H), 8.58 (d, 1H), 8.53 (d, 1H), 8.42 (bs, 1H), 8.27 (d, 1H), 8.08 (d, 1H), 8.03 (d, 1H), 8.01 (d, 1H), 7.86 (m, 2H), 7.79 (t, 1H), 7.35 (d, 1H), 2.38 (s, 3H), 2.12 (m, 1H), 0.47 (m, 2H), 0.37 (m, 2H) | 450 | Method 122 |
| **30** | 5-{[4-[(Dimethylamino)methyl]-3-(trifluoromethyl)benzoyl]amino}-2-methyl-*N*-pyridin-3-ylbenzamide | 11.25 (s, 1H), 10.82 (s, 1H), 9.20-9.35 (m, 1H), 8.52-8.65 (m, 2H), 8.37-8.48 (m, 2H), 8.05 (s, 1H), 7.83-7.98 (m, 2H), 7.31-7.45 (m, 2H), 4.58 (s, 2H), 2.73-2.85 (m, 6H), 2.40 (s, 3H) | 456 | Method 23 |
| **3**1 | 5-{[4-[(4-Ethylpiperazin-1-yl)methyl]-3-(trifluoromethyl) benzoyl]amino}-2-methyl-*N*-pyridin-3-ylbenzamide | 11.33 (s, 1H), 10.78 (s, 1H), 9.32 (s, 1H), 8.58-8.72 (m, 2H), 8.29-8.43 (m, 2H), 8.06 (s, 2H), 8.00 (dd, 1H), 7.88 (d, 1H), 7.37 (d, 1H), 3.99 (s, 2H), 3.43-3.58 (m, 3H), 3.12 (d, 5H), 2.85 (s, 1H), 2.40 (s, 3H) | 525 | Method 22 |
| **32** | 5-({3-(1-Cyano-1-methylethyl)-5-[(dimethylamino)methyl]benzoyl} amino)-2-methyl-*N*-pyridin-3-ylbenzamide | 11.12 (s, 1H), 10.70 (s, 1H), 9.20 (s, 1 H), 8.45-8.60 (m, 2H), 8.18 (d, 2H), 8.04 (d, 2H), 7.78-7.92 (m, 2H), 7.37 (d, 1H), 4.43 (s, 2H), 2.74 (s, 6H), 2.32-2.44 (m, 3H), 1.79 (s, 6H) | 455 | Method 28 |
| **33** | 5-{[3-(3-Hydroxy-3-methylbut-1-yn-1-yl)benzoyl]amino}-2-methyl-*N*-pyridin-3-ylbenzamide | 10.38 (s, 1H), 10.25 (s, 1H), 8.68 (s, 1H), 8.11 (d, 1H), 7.98 (d, 1H), 7.80 (s, 1H), 7.73 (s, 2H), 7.64 (d, 1H), 7.29-7.42 (m, 2H), 7.19 (dd, 1H), 7.11 (d, 1H), 2.16 (s, 3H), 1.28 (s, 6H) | 413 | Method 25 |
| **34** | 5-{[3-(3,3-Dimethylbut-1-yn-1-yl)benzoyl]amino}-2-methyl-*N*-pyridin-3-ylbenzamide | 11.07 (s, 1H), 10.47 (s, 1H), 9.20 (s, 1H), 8.55 (d, 1H), 8.47 (d, 1H), 8.02 (d, 1H), 7.95 (s, 1H), 7.89 (d, 1H), 7.84-7.80 (m, 2H), 7.58-7.47 (m, 2H), 7.33 (d, 1H), 2.36 (s, 3H), 1.30 (s, 9H) | 411 | Method 24 |
| **3**5 | 5-({3-(1-Cyano-1-methylethyl)-5-[(4-methylpiperazin-1-yl)methyl]benzoyl}amino)-2-methyl-*N*-pyridin-3-ylbenzamide | 10.92 (s, 1H), 10.60 (s, 1H), 9.09 (s, 1 H), 8.34-8.49 (m, 2H), 8.09 (s, 2H), 7.99 (s, 1H), 7.89 (d, 2H), 7.67 (dd, 1H), 7.35 (d, 1H), 4.15 (br. s, 2H), 3.2- 3.6 (br. m, 8H), 2.78 (s, 3H), 2.39 (s, 3H), 1.69-1.82 (m, 6H) | 510 | Method 27 |
| **36** | 5-[(3,5-Dimethylbenzoyl)amino]-2-methyl-*N*-pyridin-3-ylbenzamide | 10.58 (s, 1H), 10.26 (s, 1H), 8.87 (s, 1H), 8.30 (d, 1H), 8.18 (d, 1H), 7.91 (s, 1H), 7.83 (d, 1H), 7.56 (s, 2H), 7.39 (dd, 1H), 7.29 (d, 1H), 7.22 (s, 1H), 2.34 (s, 9H) | 359 | 3,5-Dimethylbenzoic acid |
| **37** | 5-[(3,5-Difluorobenzoyl)amino]-2-methyl-*N*-pyridin-3-ylbenzamide | 10.60 (s, 1H), 10.47 (s, 1H), 8.88 (s, 1H), 8.31 (d, 1H), 8.18 (d, 1H), 7.90 (s, 1H), 7.81 (d, 1H), 7.69 (d, 2H), 7.55 (s, 1H), 7.30-7.42 (m, 2H), 2.36 (s, 3H) | 367 | 3,5-Difluorobenzoic acid |
| **38** | 5-[(3-Iodobenzoyl)amino]-2-methyl-*N*-pyridin-3-ylbenzamide | 10.59 (s, 1H), 10.42 (s, 1H), 8.88 (s, 1H), 8.31 (s, 2H), 8.18 (d, 1H), 7.89-7.98 (m, 3H), 7.83. (d, 1H), 7.29-7.41 (m, 3H), 2.35 (s, 3H) | 457 | 3-Iodobenzoic acid |
| **39** | 5-[(3,5-Dimethoxybenzoyl)amino]-2-methyl-*N*-pyridin-3-ylbenzamide | 10.58 (s, 1H), 10.28 (s, 1H), 8.88 (s, 1H), 8.30 (d, 1H), 8.17 (d, 1H), 7.90 (s, 1H), 7.83 (d, 1H), 7.39 (dd, 1H), 7.30 (d, 1H), 7.10 (s, 2H), 6.71 (s, 1H), 3.80-3.83 (m, 6H), 2.35 (s, 3H) | 391 | 3,5-Dimethoxybenzoic acid |
| **40** | 2-Methyl-5-[(3-methylbenzoyl) amino]-*N*-pyridin-3-ylbenzamide | 10.58 (s, 1H), 10.31 (s, 1H), 8.88 (s, 1H), 8.30 (d, 1), 8.18 (d, 1H), 7.92 (s, 1H), 7.83 (d, 1H), 7.73-7.79 (m, 2H), 7.36-7.43 (m,3H), 7.29 (d, 1H), 2.39 (s, 3H), 2.34 (s, 3H) | 345 . | 3-Methylbenzoic acid |
| **41** | 5-[(3-isopropoxybenzoyl)amino]-2-methyl-*N-*pyridin-3-ylbenzamide | 10.58 (s, 1H), 10.29 (s, 1H), 8.87 (s, 1H), 8.30 (d, 1H), 8.18 (d, 1H), 7.93 (s, 1H), 7.83 (d, 1H), 7.37-7.50 (m, 4H), 7.29 (d, 1H), 7.14 (d, 1H), 4.67-4.74 (m, 1H), 2.35 (s, 3H), 1.28 (d, 6H) | 389 | 3-Isopropoxybenzoic acid |
| **42** | 5-{[3-(Dimethylamino)benzoyl]amino}-2- methyl-*N*-pyridin-3-ylbenzamide | 10.57 (s, 1H), 10.23 (s, 1H), 8.87 (s, 1H), 8.30 (d, 1H), 8.18 (d, 1H), 7.91 (s, 1H), 7.83 (d, 1H), 7.27-7.41 (m, 3H), 7.21-7.24 (m, 2H), 6.92 (d, 1H), 2.95 (s, 6H), 2.34 (s, 3H) | 374 | 3-(Dimethylamino)-benzoic acid |
| **43** | 5-({3-[(Difluoromethyl)thio]penzoyl}amino)-2-methyl-*N*-pyridin-3-ylbenzamide | 10.29 (s, 1 H); 10.23 (s, 1 H), 8.88 (s, 1H), 8.31 (d, 1H), 8.10-8.18 (m, 2H), 8.07 (d, 1H), 7.89 (s, 1H), 7.75-7.84 (m, 2H), 7.62 (t, 1H), 7.34-7.38 (m, 1H), 7.30 (d, 1H); 3.77 (d, 1H), 2.38 (s, 3H) | 413 | 3-[(Difluoromethyl}-thio]benzoic acid |
| **44** | 5-[(3-Chlorobenzoyl)amino]-2-methyl-*N-*pyridin-3-ylbenzamide | 10.29 (s, 1H), 10.22 (s, 1H), 8.88 (s, 1H), 8.30 (s, 11-1),8.14 (d, 1H), 8.02 (s, 1H), 7.93 (d, 1H), 7.89 (s, 1H), 7.80 (d, 1H), 7.61-7.65 (m, 1H), 7.55 (t, 1H), 7.36 (dd, 1H), 7.29 (d, 1H), 2.38 (s, 3H) | 365 | 3-Chlorobenzoic acid |
| **45** | 2-Methyl-5-[(3-ptopoxybenzoyl) amino]-*N-*pyridin-3-ylbenzamide | 10.58 (s, 1H), 10.31 (s, 1H), 8.88(s, 1H), 8.31 (d, 1H), 8.18 (d, 114), 7.93 (s, 1H), 7.84 (d, 1H), 7.48-7.55 (m, 2H), 7.37-7.46 (m, 2H), 7.30.(d, 1H), 7.15 (d, 1H), 4.00 (t, 2H), 2.35 (s, 3H), 1.75 (q, 2H), 0.99 (t, 3H) | 389 | 3-Propoxybenzoic acid |
| **46** | 5-[(3-Methoxybenzoyl)amino]-2-methyl-*N*-pyridin-3-ylbenzamide | 10.28 (s, 1H), 10.07 (s, 1H), 8.88 (s, 1H), 8.30 (d, 1H), 8.14 (d, 1H), 7.91 (s, 1H), 7.81 (d, 1H), 7.56 (d, 1H), 7.51 (s, 1H), 7.43 (t, 1H), 7.36 (dd, 1H), 7.28 (d, 1H), 7.14 (d, 1H), 3.85 (s, 3H), 2.38 (s, 3H) | 361 | 3-Methoxybenzoic acid |
| **47** | 5-[(3-Bromobenzoyl)amino]-2-methyl-*N-*pyridin-3-ylbenzamide | 10.29 (s, 1H), 10.22 (s, 1H), 8.88 (s, 1H), 8.31 (d, 1H), 8.12-8.22 (m, 2H), 7.97 (d, 1H), 7.89 (s, 1H), 7.75-7.83 (m, 2H), 7.49 (t, 1H), 7.34-7.39 (m, 1H), 7.29 (d, 1H), 2.38 (s, 3H) | 410 | 3-Bromobenzoic acid |
| **48** | 5-[(3-Isobutoxybenzoyl)amino]-2-methyl-*N-*pyridin-3-ylbenzamide | 10.57 (s, 1H), 10.30 (s, 1H), 8.87 (s, 1H), 8.30 (d, 1H), 8.17 (d,1H), 7.91 (s, 1H), 7.83 (d, 1H), 7.48-7.54 (m, 2H), 7.36-7.45 (m,2H), 7.29 (d, 1H), 7.15 (d, 1H), 3.81 (d, 2H), 2.34 (s, 3H), 1.96-2.10 (m, 1H), 0.94-1.00 (m, 6H) | 403 | 3-Isobutoxybenzoic acid |
| **49** | 5-{[3-(1-Cyano-1-methylethyl) benzoyl]amino}-2-methyl-*N*-pyridin-3-ylbenzamide | 10.59 (s, 1H), 10.43 (s, 1H), 8.88 (s, 1H), 8.30 (d, 1H), 8.18 (d, 1H), 8.05 (s, 1H), 7.88-7.97 (m, 2H), 7.84 (d, 1H), 7.75 (d, 1H), 7.60 (t, 1H), 7.39 (dd, 1H), 7.32 (d, 1H), 2.36 (s, 3H), 1.71-1.76 (m, 6H) | 398 | 3-(1-Cyano-1-methylethyl)-benzoic acid |
| **50** | 2-Methyl-*N-*pyridin-3-yl-5-{[3-(trifluoromethyl) benzoyl]amino}benzamide | 10.44 (s, 1H), 10.42 (s, 1H), 8.72 (d J=2.2Hz; 1H), 8.10-8.15 (m, 3H), 8.04 (d J=8.3Hz, 1H), 7.61-7.83 (m, 4H), 7.15-7.25 (m, 2H), 2.18 (s, 1H) | 400 | 3-Trifluorobenzoicacid |
| **51** | *N*-{4-Methyl-3-[(pyridin-3-ylamino)carbonyl]phenyl}-5-piperidin-1-ylnicotinamide | 10.58 (s, 1H), 10.40 (s, 1H), 8.87 (d, 1H), 8.46 (s, 2H), 8.30 (d, 1H), 8.17 (d, 1H), 7.90 (d, 1H), 7.82 (dd, 1H), 7.70 (s, 1H), 7.40 (dd, 1H), 7.32 (d, 1H), 3.30-3.26 (m, 4H), 2.35 (s, 3H), 1.69-1.59 (m, 6H) | 416 | Method 30 |
| **52** | *N-*{4-Methyl-3-[(pyridin-3-ylamino)carbonyl]phenyl}-5-morpholin-4-ylnicotinamide | 11.00.(s, 11-1), 10.65 (s, 1H), 9.14 (s, 1H), 8.60 (s, 1H), 8.54-8.50 (m, 2H), 8.32 (d, 1H), 8.00-7.97 (m, 2H), 7.83-7.77 (m, 2H), 7.36 (d, 1H), 3.77 (t, 4H), 3.33 (t, 4H), 2.37 (s, 3H) | 418 | Method 31 |
| **53** | 5-(Diethylamino)-*N-*{4-methyl-3-[(pyridin-3-ylamino)carbonyl] phenyl}nicotinamide | 11. 18 (s, 11-1), 11.05 (s, 1H), 9.23 (s, 1H), 8.59-8.5 (m, 3H), 8.32 (d, 1H), 8.07 (s, 1H), 8.03 (s, 114), 7.90-7.85 (m, 2H), 7.36 (d, 1H), 3:53 (q, 4H), 2.38 (s, 3H), 1.14 (t, 6H) | 404 | Method 32 |
| **54** | 5-(1-Cyano-1-methylethyl)-*N-*{4-methyl-3-[(pyridin-3-ylamino)carbonyl] phenyl}nicotinamide | 11.23 (s, 1H); 10.78 (s, 1H), 9.29 (d, 1H), 9.12 (d, 1H), 8.96 (d, 1H), 8.62 (d, 114), 8.56 (d, 1H), 8.44 (t, 1H), 8.03 (d, 1H), 7.95 (dd, 1H), 7.84 (dd, 1H), 7.37 (d, 1H), 2.39 (s, 3H), 1.80 (s, 6H) | 400 | Method 33 |
| **55** | 2-Chloro-5-[(3.-cyclopropylbenzoyl) amino]-*N*-(5-fluoropyridin-3-yl)benzamide | 11.06 (s, 1 H), 10.45 (s, 1H), 8.68 (s, 1H), 8.36 (d, 1H), 8.16 (dt, 1H), 8.05 (d, 1H), 7.95 (dd, 1H), 7.71 (d, 1H), 7.55 - 7.65 (m, 2H), 7.40 (t, 1H), 7.27 - 7.36 (m, 1H), 2.01 (ddd, 1H), 0.95 - 1.04 (m, 2H), 0.76 (ddd, 2H) | 410 | Method 34 |
| **56** | 5-[(3-Cyclopropyl-5-fluorobenzoyl) amino]-2-methyl-*N*-pyridin-3-ylbenzamide | 11.15 (s, 1H), 10.44 (s, 1H), 9.25 (s, 1H), 8.58 (d, 1H), 8.52 (d, 1H), 8.01 (d, 1H), 7.78 - 7.93 (m, 2H), 7.46 - 7.56 (m, 2H), 7.34 (d, 1H), 7.16 (d, 1H), 2.37 (s, 3H), 2.04 (td, 1H), 0.95 - 1.07 (m, 2H), 0.81 (dt, 2H) | 391 | Method 226 |
| **57** | 5-Chloro-*N-*{4-methyl-3-[(pyridin-3-ylamino)carbonyl] phenyl}-1*H*-indole-2-carboxamide | 11.95 (s, 1H), 10.60 (s, 1H), 10.40 (s, 1H), 8.88 (s, 1H), 8.31 (d, 1H), 8.17 (d, 1H), 7.93 (s, 1H), 7.87 (d, 1H), 7.78 (s, 1H), 7.45 (d, 1H), 7.42 (s, 1H), 7.39 (m, 1H), 7.32 (d, 1H), 7.24 (d, 1H), 2.36 (s, 3H) | 405 | 5-chloro-1*H*-indole-2-carboxylic acid |
| **58** | 4,6-Difluoro-*N-*{4-methyl-3-[(pyridin-3-ylamino)carbonyl] phenyl}-1*H*-indole-2-carboxamide | 12.20 (s, 1H), 10.62 (s, 1H), 10.39 (s, 1H), 8.89 (s, 1H), 8.32 (d, 1H), 8.18 (d, 1H), 7.93 (s, 1H), 7.86 (d, 1H), 7.56 (s, 1H), 7.40 (m, 1H), 7.34 (d, 1H), 7.08 (d, 1H), 6.95 (m, 1H), 2.37 (s, 3H) | 40.7 | 4,6-difluoro-1*H*-indole-2-carboxylic acid |
| **59** | 2-Chloro-*N*-{4-methyl-3-[(pyridin-3-ylamino)carbonyl] phenyl} isonicotinamide | 10.17 (s, 1H), 9.98 (s, 1H), 9.56 (s, 1H), 8.80 (s, 1H), 8.19 (s, 1H), 8.11 (s, 1H), 7.80 (s, 1H), 7.72 (s, 1H), 7.14 (s, 2H), 6.99 (s, 1H), 3.66 (s, 3H), 2.15 (s, 3H) | 367 | 2-chloroisonicotinic acid |
| **60** | 5-{[4-Chloro-3-(trifluoromethyl) benzoyl]amino}-2-methyl-*N-*pyridin-3-ylbenzamide | 10.65 (s, 2H), 8.92 (s, 1H), 8.40 (s, 1H), 8.34 (s, 1H), 8.24 (s, 2H), 7.92 (s, 2H), 7.83 (s, 1H), 7.44 (s, 1H), 7.33 (s, 1H), 2.37 (s, 3H) | 434 | 4-chloro-3-(trifluoromethyl )benzoic acid |
| **61** | 5-Methoxy-*N-*14-methyl-3-[(pyridin-3-ylamino)catbonyl]-phenyl}-1*H*-indole-2-carboxamide | 11.58 (s, 1H), 10.59 (s, 1H), 10.26 (s, 1H), 8.90 (s, 1H), 8:31 (s, 1H), 8.19 (s, 1H), 7.91 (s, 2H), 7.35 (s, 3H), 7.13 (s, 1H), 6.87 (s, 1H), 3.78 (s, 3H), 2.34 (s, 3H) | 401 | 5-methoxy-1H indole-2-carboxylic acid |
| **62** | *N*-{4-Methyl-3-[(pyridin-3-ylamino) carbonyl]phenyl}-1*H-*indole-2-carboxamide | 11.75 (s, 1H), 10.60 (s, 1H), 10.32 (s, 1H), 8.89 (s, 1H), 8.31 (s, 1H), 8.19 (s, 1H), 7.95 (s, 1H), 7.87 (s, 1H), 7.67 (s, 1H), 7.43 (s, 3H), 7.32 (s, 1H), 7.21 (s, 1H), 7.05 (s, 1H), 2.36 (s, 3H) | 371 | 1*H*-indole-2-carboxylic acid |
| **63** | 5-Chloro-2-methoxy-*N*-{4-methyl-3-[(pyridin-3-ylamino)carbonyl]phenyl}benzamide | 10.59 (s, 1H), 10.30 (s, 1H), 8.89 (s, 1H), 8.33 (s, 1H), 8.19 (s, 1H), 7.88 (s, 1H), 7.76 (s, 1H), 7.58 (s, 2H), 7.40 (s, 1H), 7.31 (s, 1H), 7.22 (s, 1H), 3.91 (s, 3H), 2.35 (s, 3H) | 396 | 5-chloro,-2-methoxybenzoic acid |
| **64** | 5-Methyl-*N*-{4-methyl-3-[(pyridin-3-ylamino)carbonyl] pheriyl}-1*H*-indole-2-carboxamide | 11.60 (s, 1H), 10.59 (s, 1H), 10.26 (s, 1H), 8.89 (s, 1H), 8.31 (s, 1H), 8.19 (s, 1H), 7.94 (s, 1H), 7.87 (s, 1H), 7.44 (s, 1H), 7.35 (s, 3H), 7.05 (s, 1H), 3.31 (s, 3H), 2.36 (s, 3H) | 385 | 5-methyl-1*H*-indole-2-carboxylic acid |
| **65** | 5-Methyl-*N-*{4-methyl-3-[(pyridin-3-ylammo)carbonyl]phenyl}isoxazole-3-carboxamide | 10.75 (s, 1H), 10.57 (s, 1H), 8.87 (s, 1 H), 8.31 (s, 1H), 8.18 (s, 1H), 7.95 (s, 1H), 7.81 (s, 1H), 7.39 (s, 1H), 7.30 (s, 1H), 6.66 (s, 1H), 3.30 (s, 3H), 2.34 (s, 3H) | 337 | 5-methylisoxazole -3-carboxylic acid |
| **66** | 2-Chloro-6-methyl-*N*-{4-methyl-3-[(pyridin-3-ylamino) carbonyl]phenyl}isonicotinamide | 10.76 (s, 2H), 8.97 (s, 1H), 8.37 (s, 1H), 8.27 (s, 1H), 7.95 (s, 1H), 7.82 (s, 3H), 7.52 (s, 1H), 7.33 (s, 1H), 2.55 (s, 3H), 2.36 (s, 3H) | 381 | 2-chloro-6-methylisonicotinic acid |
| **67** | *N-*{4-Methyl-3-[(pyridin-3-ylamino)carbonyl]phenyl}-2-morpholin-4-ylisonicotinamide | 10.59 (s, 1H), 10.44 (s, 1H), 8.90 (s, 1H), 8.32 (s, 2H), 8.19 (s, 1H), 7.91 (s, 1H), 7.83 (s, 1H), 7.41 (s, 1H), 7.34 (s, 1H), 7.27 (s, 1H), 7.15 (s, 1H), 3.73 (s, 4H), 3.54 (s, 4H), 2.36 (s, 3H) | 416 | 2-morpholin-4-ylisonicotinic acid |
| **68** | 1-*tert*-Butyl-3-methyl-*N-*{4-methyl-3-[(pyridin-3-ylamino)carbonyl] phenyl}-1*H-*pyrazole-5-carboxamide | 10.66 (s, 1H), 10.56 (s, 1H), 8.86 (s, 1H), 8.31 (s, 1H), 8.17 (s, 1H), 7.84 (s, 1H), 7.70 (s, 1H), 7.40 (s, 1H), 7.28 (s, 1H), 6.38 (s, 1H), 2.33 (s, 3H), 2.18 (s, 3H), 1.57 (s, 9H) | 392 | 1-*tert*-butyl-3-methyl-1*H-*pyrazole-5-carboxylic acid |
| **69** | 5-[(3-Cyclopropylbenzoyl)amino]-2-methyl-*N-*pyridin-3-ylbenzamide | 10.57 (s, 1H), 10.29 (s, 1H), 8.88 (s, 1H), 8.31 (s, 1H), 8.18 (s, 1H), 7.93 (s, 1H), 7.84 (s, 1H), 7.71 (s, 1H), 7.63 (s, 1H), 7.40 (s, 2H), 7.30 (s, 2H), 4.09 (s, 1H), 2.35 (s, 3H), 1.00 (s, 2H), 0.76 (s, 2H) | 372 | Method 34 |
| **70** | *N-*{4-Methyl-3-[(pyridin-3-ylamino) carbonyl]phenyl}-1*H-*indole-7-carboxamide | 11.29 (s, 1H), 10.62 (s, 1H), 10.40 (s, 1H), 8.91 (s, 1H), 8.33 (s, 1H), 8.19 (s, 1H), 8.11 (s, 1H), 7.84 (s, 3H), 7.36 (s, 3H), 7.12 (s, 1H), 6.55 (s, 1H), 2.37 (s, 3H) | 371 | 1*H-*indole-7-carboxylic acid |
| **71** | *N-*(4-Methyl-3-[(pyridin-3-ylamino) carbonyl]phenyl}-1*H-*indole-4-carboxamide | 11.38 (s, 1H), 10.59 (s, 1H), 10.29 (s, 1H), 8.91 (s, 1H), 8.32 (s, 1H), 8.20 (s, 1H), 8.02 (s, 1H), 7.85 (s, 1 H), 7.59 (s, 2H), 7.48 (s, 1H), 7.40 (s, 1H), 7.30 (s, 1H), 7.21 (s, 1H), 6.86 (s, 1H), 2.36 (s, 3H) | 371 | 1*H-*indole-4-carboxylic acid |
| **72** | *tert*-Butyl {3-[({4-methyl-3-[(pyridin-3-ylamino)carbonyl]phenyl}amino) carbonyl]phenyl} carbamate | 10.62 (s, 1H), 10.35 (s, 1H), 9.58 (s, 1H), 8.90 (s, 1H), 8.33 (s, 1H), 8.20 (s, 1H), 8.04 (s, 1H), 7.93 (s, 1H), 7.81 (s, 1H), 7.56 (s, 2H), 7.41 (s, 2H), 7.29 (s, 1H), 2.35 (s, 3H), 1.49 (s, 9H) | 446 | 3-[(*tert-*butoxycarbonyl )amino]benzoic acid |
| **73** | 2,2-bimethyl-*N-*{4-methyl-3-[(pyridin-3-ylamino)carbonyl]phenyl}-2,3-dihydro-1-benzofuran-7-carboxamide | 10.60 (s, 1H), 9.78 (s, 1H), 8.89 (s, 1H), 8.31 (s, 1H), 8.19 (s, 1H), 7.87 (s, 1H), 7.68 (s, 2H), 7.36 (s, 3H), 6.97 (s, 1H), 3.10 (s, 2H), 2.35 (s, 3H), 1.53 (s, 6H) | 402. | 2,2-dimethyl-2,3-dihydro-1-benzofuran-7-carboxylic acid |
| **74** | 5-Bromo-*N-*{4-methy]-3-[(pyridin-3-ylamino)carbonyl]phenyl}-2-furamide | 10.59 (s, 1H), 10.42 (s, 1H), 8.88 (s, 1H), 8.31 (s, 1H), 8.17 (s, 1H), 7.84 (s, 2H), 7.47 (s, 1H), 7.39 (s, 1H), 7.29 (s, 1H), 6.83 (s, 1H), 2.34 (s, 3H) | 401 | 5-bromo-2-furoic acid |
| **75** | 2,4-Dimethyl-*N-*14-methyl-3-[(pyridin-3-ylamino)carbonyl]phenyl}-1,3-thiazole-5-carboxamide | 10.57 (s, 1H), 10.18 (s, 1H), 8.88 (s, 1H), 8.30 (s, 1H), 8.18 (s, 1H), 7.80 (s, 1H), 7.70 (s, 1H), 7.39 (s, 1H), 7.28 (s, 1H), 2.65 (s, 3H), 2.53 (s, 3H), 2.33 (s, 3H) | 367 | 2,4-dimethyl-1,3-thiazole-5-carboxylic acid |
| **76** | *N*-{4-Methyl-3-[(pyridin-3-ylamino)carbonyl]phenyl}-5-pyridin-2-ylthiophene-2-carboxamide | 10.59 (s, 1H), 10.39 (s, 1H), 8.88 (s, 1H), 8.57 (s, 1H), 8.31 (s, 1H), 8.19 (s, 1H), 8.01 (s, 2H), 7.85 (s, 4H), 7.36 (s, 3H), 2.35 (s, 3H) | 414 | 5-pyridin-2-ylthiophene-2-carboxylic acid |
| **77** | 1-Methyl-*N*-{4-methyl-3-[(pyridin-3-ylamino)carbonyl] phenyl}-1*H*-indole-2-carboxamide | 10.61 (s, 1H), 10.43 (s, 1H), 8.90 (s, 1H), 8.32 (s, 1H), 8.20 (s, 1H), 7.97 (s, 1H), 7.80 (s, 1 H), 7.71 (s, 1H), 7.58 (s, 1H), 7.35 (s, 4H), 7.15 (s, 1H), 4.01 (s, 3H), 2.35 (s, 3H) | 385 | 1-methyl-1*H*-indole-2-carboxylic acid |
| **78** | 2-Methyl-*N*-pyridin-3-yl-5-{[2-(trifluoromethyl) benzoyl]amino}benzamide | 10.66 (s, 1H), 10.59 (s, 1H), 8.87 (s, 1H), 8.30 (s, 1H), 8.17 (s, 1H), 7.82 (s, 3H), 7.70 (s, 3H), 7.38 (s, 1H), 7.30 (s, 1H), 2.34 (s, 3H) | 400 | 2-(trifluoromethyl ) benzoic acid |
| **79** | 2-Methyl-5-[(3-phenoxybenzoyl) amino]-*N*-pyridin-3- ylbenzamide | 10.57 (s, 1H), 10.39 (s, 1H), 8.89 (s, 1H), 8.31 (s, 1H), 8.19 (s, 1H), 7.92 (s, 1H), 7.79 (s, 2H), 7.57 (s, 2H), 7.41 (s, 3H), 7.30 (s, 1H), 7.20 (s, 2H), 7.07 (s, 2H), 2.35 (s, 3H) | 424 | 3-phenoxybenzoic acid |
| **80** | 5-({3-(1-Cyano-1-methylethyl)-5-[(methylthio)methyl]benzoyl}amino)-2-methyl-*N*-pyridin-3-ylbenzamide | 10.93 (s, 1H), 10.49 (s, 1H), 9.14 (s, 1H), 8.49 (d, 1H), 8.36 (d, 1H), 7.92 - 8.03 (m, 2H), 7.80-7.92 (m, 2H), 7.64 - 7.75 (m, 2H), 7.38 (d, 1H), 3.84 (s, 2H), 2.38(s, 3H), 2.01 (s, 3H), 1.77 (s, 6H) | 458 | Method 37 |
| **81** | {3-(1-Cyano-1-methylethyl)-5-[({4-methyl-3-[(pyridin-3-ylamino)carbony] phenyl}amino) carbonyl]phenyl}methanesulfonic acid | 10.94 (8, 1H), 10.43 (s, 1H), 9.13 (s, 1H), 8.44 (dd, 2H), 7.99 (s, 1H), 7.88 - 7.95 (m, 2H), 7.84 (dd, 1H), 7.68 - 7.76 (m, 1H), 7.65 (s, 1H), 7.34 (d, 1H), 3.81 (s, 2H), 2.39 (s, 3H), 1.75 (s, 6H) | 492 | Method 38 |
| **82** | 5-[(3-{1-[(Dimethylamino) sulfonyl]-1-methylethyl}benzoyl)amino]-2-methyl-*N*-pyridin-3-ylbenzamide | 10.85 (s, 1H), 10.44 (s, 1H), 9.06 (s, 1H), 8.45. (d, 1H), 8.35 (d, 1H), 8.15 (s, 1H), 7.96 - 8.01 (m, 2H), 7.80 - 7.88 (m, 2H), 7.65 (dd, 1H), 7.57 (t, 1 H), 7.33 (d, 1H), 2.60 (s, 6H), 2.37 (s, 3H), 1.79 (s, 6H) | 480 | Method 40 |
| **83** | 5-{[3-(1,1-Dimethylprop-2-yn-1-yl)benzoyl]amino}-2-methyl-*N*-pyridin-3-ylbenzamide | 10.85 (s, 1H), 10.38 (s, 1H), 9.07 (s, 1H), 8.45 (d, 1H), 8.36 (d, 1H), 8.08 (s, 1H), 7.96 (s, 1H), 7.74 - 7.86 (m, 3H), 7.65 (q, 1H), 7.50 (t, 1H), 7.32 (d, 1H), 2.35 (s, 3H), 1.57 (s, 6H) | 397 | Method 41 |
| **84** | 5-{[3-(1,1-Dimethylbut-2-yn-1-yl)benzoyl]amino}-2-methyl-*N*-pyridin-3-ylbenzamide | 10.59 (s, 1H), 10.38 (s, 1H), 8.88 (s, 1H), 8.30 (d, 1H), 8.18 (d, 1H), 8.08 (s, 1H), 7.92 (s, 1H), 7.80-7.87 (m, 2H), 7.77 (d, 1H), 7.50 (t, 1H), 7.40 (dd, 1H), 7.31 (d, 1H), 2.36 (s, 3H), 1.85 (s, 3H), 1.55 (s, 6H) | 411 | Method 42 |
| **85** | 5-[(3-{[(Dimethylamino)carbonyl]amino}benzoyl)amino]-2-methyl-*N*-pyridin-3-ylbenzamide | 10.45 (s, 1H), 9.90 (s, 1H), 8.66 (s, 1H), 8.08 (s, 1H), 8.02 (d, 1H), 7.93 (d, 1H), 7.56 (d, 2H), 7.36 (dd, 1H), 7.20-7.28 (m, 2H), 7.07 (d, 1H), 6.84 - 6.97 (m, 2H), 2.05 (s, 7H), 1.90 (s, 3H) | 417 | Method 45 |
| **86** | 5-{[3-(1,1-Difluoroethyl)benzoyl]amino}-2-methyl-*N*-pyridin-3-ylbenzamide | 10.92 (s, 1H), 10.50 (s, 1H), 9.11 (s, 1H), 8.48 (d, 2H), 8.37 (d, 1H), 8.12 (s, 2H), 8.06 (d, 1H), 7.96 (s, 1H), 7.79 (dd, 3H), 7.60 - 7.72 (m, 2H), 7.32 (d, 1H), 2.35 (s, 3H), 1.98 (t, 3H) | 395 | Method 47 |
| **8**7 | 2-Methyl-5-{[3-(1-methylcyclopropyl) benzoyl]amino}-*N-*pyridin-3-ylbenzamide | 10.85 (s, 1H), 10.36 (s, 1H), 9.08 (s, 1H), 8.44 (d, 1H), 8.34 (d, 1H), 7.98 (s, 1H), 7.72-7.85 (m,3H), 7.64 (q, 1H), 7.44 (s, 2H), 7.33 (d, 1H), 2.37 (s, 3H), 1.43 (s, 3H), 0.89 - 0.95 (m, 2H), 0.78-0.87 (m, 2H) | 385 | Method 49 |
| **88** | 5-[(3-(1-Cyano-1-methylethyl)-5-{[methoxy(methyl)amino]methyl}benzoyl)amino]-2-methyl-*N*-pyridin-3-ylbenzamide | 10.61 (s, 1H), 10.44 (s, 1H), 8.90 (s, 1H), 8.31 (d, 1H), 8.19 (d, 1H), 7.83 - 7.99 (m, 4H), 7.76 (s, 1H), 7.40 (dd, 1H), 7.33 (d, 1H), 3.88 (s, 2H), 3.27 (s, 3H), 2.62 (s,3H), 2.38 (s, 3H), 1.76 (s, 6H) | 471 | Method 50 |
| **89** | 5-({3-(1-Cyano-1-methylethyl)-5[(methylsulfonyl)methyl]benzoyl}amino)-2-methyl-*N*-pyridin-3-ylbenzamide | 10.93 (s, 1H), 10.52 (s, 1H), 9.13 (s, 1H), 8.48 (s, 114), 8.39 (d, 1H), 8.07 (s, 1H), 7.95 (d, 2H), 7.78 - 7.89 (m, 2H), 7.64 - 7.77 (m, 1H), 7.37 (d, 1 H), 4.67 (s, 2H), 3.00 (s, 3H, 2.39 (s, 3H), 1.78 (s, 6H) | 490 | Method 175 |
| **90** | 2-Methyl-*N*-pyridin-3-yl-5-{[3-(1,3-thiazol-2-yl)benzoyl]amino}benzamide | 11.03 (s, 1H), 10.62 (s, 1H), 9.20 (s, 1H), 8.49 - 8.56 (m, 2H), 8.45 (d, 1H), 8.16 (d, 1H), 8.02 - 8.08 (m, 2H), 7.99 (d, 1H), 7.76 - 7.88 (m, 3H), 7.68 (t, 1H), 7.35 (d, 1H), 2.37 (s, 3H) | 414 | Method 51 |
| **91** | 5-[(3-Fluoro-5-methylbenzoyl) amino]-2-methyl-*N*-pyridin-3-ylbenzamide | 10.56 (s, 1H), 10.35 (s, 1H), 8.88 (s, 1H), 8.30 d, 1H), 8.17 (d, 1H), 7.91 (s, 1H), 7.82 (dd, 1H), 7.65 (s, 1H), 7.56 (d, 1H), 7.40 (dd, 1H), 7.31 (d, 1H), 7.28 (d, 1H), 2.41 (s,3H), 2.36 (s, 3H) | 364 | 3-fluoro-5-methylbenzoic acid |
| **92** | *N*-{4-Methyl-3-[(pyridin-3-ylamino)carbonyl]phenyl}-2-(trifluoromethyl)-1*H*-benzimidazole-6-carboxamide | 9.72 (s, 1H), 9.62 (s, 1H), 8.78 (d, 1H), 8.85 (s, 1H), 8.83 (s, 1H), 8.27 (dd, 1H), 8.00 - 8.03 (m,2H), 7.75 (dd, 2H), &.27 (dd, 1H), 7.22 (d, 1H), 2.42 (s, 3H) | 440 | 2-(trifluoromethyl )-1*H*-benzimidazole-6-carboxylic acid |
| **93** | 1-Methyl-*N*-{4-methyl-3-[(pyridin-3-ylamino)carbonyl] phenyl)-1*H*-pyrrole-2-carboxamide | 10.55 (s, 1H), 9.83 s, 114), 8.87 (d, 1H), 8.29 (dd, 1H), 8.17 (dd,1H), 7.87 (d, 1H), 7.75 (dd, 1H), 7.38 (dd, 1H), 7.25 (d, 1H), 7.04 (dd, 1H), 7.00 (s, 1H), 6.09 (dd, 1 H0 3.86 (s, 3H), 2.83 (s,3H) | 335 | 1-methyl-1*H* pyrrole-2-carboxylic acid |
| **9**4 | *N*-{4-Methyl-3-[(pyridin-3-ylamino) carbonyl]phenyl}-1*H*-pyrrole-2-carboxamide | 8.86 (d,1H), 8.30 (s, 1H), 8.26 (d, 1H), 7.89 (d, 1H), 7.59 (dd, 1H), 7.45 (dd, 1H), 7.28 (d, 1H), 7.01 (dd, 1H), 7.96-7.99 (m, 1H), 3.25 (s, 3H). | 321 | 1*H*-pyrrole-2-carboxylic acid |
| **95** | 5-[(4-Bromo-3-methylbenzoyl) amino]-2-methy]-*N*-pyridin-3-ylbenzamide | 9.64 (s,2H), 8.94 (d, 1H), 8.80 - 8.83 (m, 2H), 7.99 (d, 1H), 7.94 (s, 1H), 7.79 (dd, 1H), 7.68 - 7.73 (m, 2H), 7.86 (dd, 1H), 7.27 (d, 1H), 2.45 (s, 3H), 2.42 (ss, 3H) | 424 | 3-methyl-4-bromobenzoic acid |
| **96** | 5-[(3,5-Dichlorobenzoyl)amino]-2-methyl-*N*-pyridin-3-ylbenzamide | 9.77 (s, 1H), 9.67 (s, 1H), 8.95 (s, 1H), 8.28 - 8.34 (m, 2H), 7.99 (d, 1H), 7.96 (d, 2H), 7.79 (dd, 1H), 7.71 (t, 1H), 7.37 (dd, 1H), 7.28 (d, 1H), 2.42 (s, 3H) | 400 | 3,5-dichlorobenzoic acid |
| **97** | 5-[(4-Methoxybenzoyl)amino]-2-methyl-*N*-pyridin-3-ylbenzamide | 9.63 (s, 1H), 9.46 (s, 1H), 8.95 (d, 1H), 8.80 - 8.83 (m, 2H), 7.97 - 8.01 (m,3H), 7.80 (dd, 1H), 7.37 (dd, 1H), 7.25 (d 1H), 7.03 (s, 2H), 3.87 (s, 3H), 2.41 (s, 3H) | 362 | 4-methoxybenzoic acid |
| **98** | 2,5-Dichloro-*N*-{4-methyl-3-[(pyridin-3-ylamino)carbonyl]phenyl}benzamide | 9.63 (s, 1H), 9.46 (s, 1H), 8.95 (d, 1H), 8.30 - 8.33 (m; 2H), 7.95 - 8.02 (m, 3H), 7.80 (dd, 1H), 7.86 (dd, 1H), 7.25 (dd, 1H), 7.00 - 7.03 (m, 2H), 4.12 (dd, 2H), 2.41 (s, 3H), 1.88 (t, 3H) | 400 | 2,5-dichlorobenzoic acid |
| **99** | 5-[(2,5-Dimethylbenzoyl)amino]-2-methyl-*N*-pyridin-3-ylbenzamide | 9.83 (s, 1H), 8.71 (s, 1H), 8.48 (d, 1H), 8.21 (s, 1H), 8.17 (d, 1H), 7.90 (s, 1H), 7.50 (d, 1H), 7.85 (dd, 1H), 7.17 (s, 1H), 7.02 - 7.09 (m, 3H), 2.37 (s, 3H), 2.32 S, 3H), 2.24 (s, 3H) | 360 | 2,5-dimethylbenzoic acid |
| **100** | 5-Chloro-2-fluoro-*N*-{4-methyl-3-[(pyridin-3-ylamino)carbonyl]phenyl}benzamide | 9.67 (s, 1H), 9.57 (s, 1H), 8.94 (s, 1 H), 8.80 - 8.83 (m,2H), 7.96 (s, 1H), 7.75-7.81 (m, 2H), 7.58 - 7.63 (m, 1H), 7.29 - 7.37 (m, 3H), 2.43 (s, 3H) | 384 | 2-fluoro-5-chlorobenzoic acid |
| **101** | 5-[(3,4-Dimethylbenzoyl)amino]-2-methyl-*N*-pyridin-3-ylbenzamide | 10.81 (s, 1H), 9.65 (s, 1H), 9.10 (d, 1H), 8.83 (s, 1H), 8.19 (s, 1H), 8.11 (s, 1H), 7.64-7.70 (m, 3H), 7.52 (d, 1H), 7.01 (d, 1H), 6.70 (d, 1H), 2.25 (s, 3H), 2.17 (s, 3H), 2.12 (s, 3H) | 360 | 3,4-dimethylbenzoicacid |
| **102** | 5-[(4-Ethoxybenzoyl)amino]-2-methyl-*N*-pyridin-3-ylbenzamide | 9.74 (s, 2H), 8.94 (s, 1H), 8.28 - 8.32 (m, 2H), 7.93 - 7.97 (m, 2H), 7.73 (d, 1H), 7.64 (s, 1H), 7.52 (s, 2H), 7.86 (dd, 1H), 7.30 (d, 1H), 2.42 (s,3H) | 376 | 4-ethoxybenzoic acid |
| **103** | 4-Methoxy-2-methyl-*N*-{4-methyl-3-[(pyridin-3-ylamino)carbonyl]phenyl}benzamide | 10.56 (s, 1H), 10.26 (s, 1H), 8.87 (s, 1H), 8.30 (d, 1H), 8.17 (d, 1H), 7.90 (s, 1H), 7.71 (d, 1H), 7.36 - 7.45 (m, 2H), 7.26 (d, 1H), 6.83 - 6.86 (s, 3H), 2.38 (s, 3H), 2.33 (s, 3H) | 376 | 2-methyl-4-methoxybenzoic acid |
| **104** | 5-[(3,4-Dimethoxybenzoyl)amino-2-methyl-*N*-pyridin-3-ylbenzamide | 10.56 (s, 1H), 10.16 (s, 1H), 8.87 (s, 1H), 8.30 (d, 1H), 8.16 (d, 1H), 7.89 (d, 1H), 7.83 (dd, 1H), 7.62 (dd, 1H), 7.53 (d, 1H), 7.38 (dd, 1H), 7.28 (d, 1H), 7.07 (d, 1H), 3.83 (s, 6H), 2.34 (s, 3H) | 392 | 3,4-dimethoxybenzoic acid |
| **105** | 5-[(3-Chloro-5-fluorobenzoyl) amino]-2-methy]-*N*-pyridin-3-ylbenzamide | 10.59 (s, 1H), 10.50 (s, 1H), 8.87 (d, 1H), 8.30 (dd, 1H), 8.17 (d, 1H), 7.89 - 7.91 (m,2H), 7.75 - 7.83 (m, 2H), 7.71 (ddd, 1H), 7.38 (dd, 1H), 7.80 (d, 1H), 2.36 (s, 3H) | 384 | 3-fluoro-5-chlorobenzoic acid |
| **106** | 2-Methyl-5-[(4-propoxybenzoyl) amino]-*N*-pyridin-3-ylbenzamide | 10.57 (s, 1H), 10.19 (s, 1H), 8.88 (d, 1H), 8.80 (dd, 1H), 8.18 (d, 1H), 7.97 (s, 1H), 7.92 - 7.94 (m, 2H), 7.83 (dd, 1H), 7.39 (dd, 1H), 7.28 (d, 1H), 7.06 (d, 2H), 4.01 (t, 2H), 2.35 (s, 3H), 1.75 (m, 2H), 0.99 (t, 3H) | 390 | 4-propoxybenzoic acid |
| **107** | 5-[(3,4-Dichlorobenzoyl)amino]-2-methy]-*N*-pyridin-3-ylbenzamide | 10.58 (s, 1H), 10.46 (s, 1H), 8.88 (s, 1H), 8.80 (d, 1H), 8.17 (d, 1H), 8.02 (t, 1H), 7.91 - 7.93 (m, 1H), 7.82 (dd, 1H), 7.67 (d, 1H), 7.55 - 7.60 (m, 1H), 7.40 (dd, 1H), 7.32 (d, 1H), 2.83 (s, 3H) | 400 | 3,4-dichlorobenzoic acid |
| **108** | 5-{[3-(Aminosulfonyl)benzoyl]amino}-2-methyl-*N*-pyridin-3-ylbenzamide | 10.83 (s, 1H), 10.63 (s, 1H), 9.04 (s, 1H), 8.38 - 8.44 (m, 2H), 8.32 (d, 1H), 8.18 (d, 1H), 8.02 (d, 1H), 7.96 (s, 1H), 7.83 (d, 1H), 7.75 (t, 1H), 7.62 (dd, 1H), 7.50 (s, 2H), 7.34 (d, 1H), 2.36 (s, 3H) | 411 | Method 130 |
| **109** | 5-[(3-{[4-(Hydroxymethyl)piperidin-1-yl]sulfonyl}benzoyl) amino]-2-methyl-*N*pyridin-3-ylbenzamide | 10.86 (s, 1H), 10.64 (s, 1H), 9.07 (s, 1H), 8.45 (d, 1H), 8.32 -8.40 (m, 2H), 8.23 (d, 1H), 8.04 (d, 1H), 7.97 (s, 1H), 7.79 (ddd, 2H), 7.65 (dd, 1H), 7.34 (d, 1H), 3.84 - 3.98 (m, 1H), 3.63 - 3.72.(m, 1H), 3.50 - 3.57 (m, 1H), 3.37 (dd, 1H), 2.94 - 3.07 (m, 1H), 2.36 - 2.42 (m, 2H), 1.72 (d, 1H), 1.34 - 1.49 (m, 3H), 1.05 - 1.21 (m, 2H) | 509 | Method 131 |
| **110** | 5-[(3-{[3-(Hydroxymethyl)piperidin-1-yl]sulfonyl}benzoyl) amino]-2-methyl-*N*-pyridin-3-ylbenzamide | 11.07 (s, 1H), 10.71 (s, 1H), 9.19 (s, 1H), 8.41 - 8.56 (m, 2H), 8.21 - 8.34 (m, 2H), 8.00 (s, 1H), 7.89 7.96 (m, 1H), 7.82 (t, 3H), 7.35 (d, 1H), 3.68 (d, 1H), 3.56 (d, 1H), 3.30 (dd, 1H), 3.01 - 3.17 (m, 3H), 2.34 - 2:42 (m, 3H), 2.23 (t, 1H), 1.98 (t, 1H), 1.60 - 1.72 (m, 1H), 1.44 - 1.59 (m, 2H), 1..18 (t, 2H), 0.87 (m, 1H) | 509 | Method 123 |
| **111** | 5-[(3-{[2-(Hydroxymethyl)piperidin-1-yl]sulfonyl}benzoyl) amino]-2-methyl-*N*-pyridine-3-ylbenzamide | 11.23 (s, 1H), 10.74 (s, 1H), 9.27 (s, 1H), 8.51 - 8.63 (m, 2H), 8.23 - 8.35 (m, 2H), 8.02 (s, 1H), 7.89-7.96 (m, 2H), 7.76 - 7.89 (m, 2H), 7.35 (d, 1H), 3.68 (d, 2H), 3.18 (d, 2H), 3.00 - 3.09 (m, 1H), 2.38 (s, 3H), 2.23 (t, 2H), 1.70 (d, 2H), 1.33 (s, 1H), 1.10 - 1.22 (m, 3H) | 509 | Method 124 |
| **112** | 5-[(3-{[Methoxy(methyl)amino]sulfonyl}benzoyl)amino]-2-methyl-*N*-pyridin-3-ylbenzamide | 10.80 (s, 1H), 10.69 (s, 1H), 9.02 (s, 1H), 8.36 - 8.43 (m, 3H), 8.30 (d, 1H), 8.02 - 8.06 (m, 1H), 7.94 (s, 1H), 7.81 - 7.90 (m, 2H), 7.59 (dd, 1H), 7.34 (d, 1H), 3.74 (s, 3H), 2.77 (s, 3H), 2.37 (s, 3H) | 455 | Method 125 |
| **113** | 5-[(3-{[(2,3-Dihydroxypropyl) (methyl)amino]sulfonyl}benzoyl)amino]-2-methyl-*N*-pyridin-3-ylbenzamide | 10.60 (m, 2H), 8.88 (d, 1H), 8.29 - 8.35 (m, 2H), 8.28 (d, 1H), 8.18 (d, 1H), 7.97 (d, 1H), 7.91(d, 1H), 7.78 - 7.89 (m, 2H), 7.39 (dd, 1H), 7.33 (d, 1H), 4.88 (d, 1H), 4.63 (t, 1H), 3.58 - 3.68 (m, 1H), 3.30 (m, 5.12, 2H), 3.12 (dd, 1H), 2.85 (dd, 1H), 2.78 (s, 3H), 2.36 (s, 3H) | 536 | Method 126 |
| **114** | 2-Methyl-*N*-pyridin-3-yl-5-[(3-{[(tetrahydrofuran-2-y]methyl)amino] sulfonyl}benzoyl)amino]benzamide | 11.19 (s, 1H), 10.75 (s, 1H), 9.23 (m, 1H), 8.50 - 8.60 (m, 2H), 8.41 (t, 1H), 8.24 (d, 1H), 8.03 (d, 1H), 7.92 - 8.00 (m, 2H), 7.85 - 7.90 (m, 2H), 7.75 (t, 1H), 7.34 (d, 1H), 3.79 (m, 1H), 3.64 (m, 1H), 3.50 - 3.58 (m, 1H), 2.79 (t, 2H), 2.37 (s, 2H), 1.78 - 1.85 (m, 1H), 1.69 -1.77 (m, 2H), 1.47 - 1.58 (m, 1H) | 495 | Method 127 |
| **115** | 2-Methyl-5-{[3-(morpholin-4-ylsulfonyl)benzoyl] amino}-*N*-pyridin-3-ylbenzamide | 11.19 (s, 1H), 10.75 (s, 1H), 9.26 (d, 1 H), 8.51 - 8.62 (m, 2H), 8.33 - 8.40 (m, 1H), 8.29 (t, 1H), 8.02 (d, 1H), 7.89 - 7.97 (m, 2H), 7.83 - 7.87 (m, 2H), 7.36 (d, 1H), 3.60 - 3.66 (m, 4H), 2.87 - 2.94 (m, 4H), 2.39 (s, 3H) | 481 | Method 128 |
| **116** | *N*-{4-Methyl-3-[(pyridin-3-ylamino)carbonyl]phenyl}-2-(trifluoromethyl)pyrimidine-4-carboxamide | 10.79 (s, 1H), 10.59 (s, 1H), 9.35 (d, 1H), 8.88 (d, 1H), 8.37 (d, 1H), 8.31 (dd, 1H), 8.18 (d, 1H), 8.01 (d, 1H), 7.95 (dd, 1H), 7.33 - 7.43 (m, 2H), 2.38 (s, 3H) | 402 | Method 177 |
| **117** | 3-(1-Cyano-1-methylethyl)-2-fluoro-*N*-(4-methyl-3-[(pyridin-3-ylamino)carbonyl] phenyl}benzamide | 10.91 (s, 1H), 10.63 (s, 1H), 9.11 (m, 1H), 8.48 (m, 1H), 8.39 (d, 1H), 7.90 (s, 1H), 7.72 (s, 2H), 7.64 (m, 2H), 7.36 (m, 2H), 2.36 (s, 3H), 1.77 (s, 6H) | 417 | Method 114 |
| **118** | 5-{[3-(2-Methoxy-1,1-dimethylethyl)benzoyl]amino}-2-methyl-*N*-pyridin-3-ylbenzamide | 10.56 (s, 1H), 10.30 (s, 1H), 8.88 (s, 1H), 8.26 - 8.36 (m, 1H), 8.18 (d, 1H), 7.91 (m, 2H), 7.84 (dd, 1H), 7.79 (d, 1H), 7.59 (d, 1H), 7.36 - 7.48 (m, 2H), 7.30 (d, 1H), 3.41 (s, 2H), 3.21 (s, 3H), 2.36 (s, 3H), 1.28 (s, 6H) | 418 | Method 178 |
| **119** | 5-(1-Cyano-1-methylethyl)-2-fluoro-*N*-{4-methyl-3-[(pyridin-3-ylamino)carbonyl] phenyl}benzamide | 11.14 (s, 1H), 10.67 (s, 1H), 9.23 (m, 1H), 8.57 (d, 1H), 8.51 (d, 1H), 7.96 (m, 1H), 7.87 (m, 1H), 7.74 (m, 3H), 7.43 (t, 1H), 7.34 (d, 1H), 2.37 (s, 3H), 1.72 (s, 6H) | 417 | Method 53 |
| **120** | 5-[(3-Chloro-4-fluorobenzoyl) amino]-2-methyl-*N*-pyridin-3-ylbenzamide | 10.59 (s, 1H), 10.46 (s, 1H), 8.88 (d, 1H), 8.82 (dd, 1H), 8.17 - 8.24 (m, 2H), 7.99 - 8.04 (m, 1H), 7.90 (s, 1H), 7.82 (dd, 1H), 7.61 (t, 1H), 7.40 (dd, 1H), 7.32 (d, 1H), 2.36 (s 3H) | 384 | 3-chloro-4-fluorobenzoic acid |

### Example 121

### 2-Chloro-5-[(3-fluorobenzoyl)amino]-N-pyridin-3-ylbenzamide

A solution of 5-amino-2-chloro-*N*-pyridin-3-ylbenzamide (Method 76; 65 mg, 0.264 mmol), 3-fluorobenzoic acid (41 mg, 0.290 mmol), and DIEA (115 µl, 0.66 mmol) in DMF (1.5 ml) was treated with HATU (120 mg, 0.3168 mmol). The reaction mixture was shaken overnight at 25 °C. Water (10 ml) was added slowly to precipitate the product. The resulting precipitate was washed with water (10 ml), isolated and dried overnight in a vacuum oven at 70 °C to give the title compound 46.9 mg, (48%) as a solid. NMR (300 MHz): 10.81 (s, 1H), 10.59 (s, 1H), 8.85 (s, 1H), 8.33 (d, 1H), 8.16 (d, 1H), 8.03 (s, 1H), 7.93 (dd, 1H), 7.74-7.84 (m,2H), 7.54-7.65 (m, 2H), 7.37-7.51 (m, 2H); *m*/*z* 369.

### Examples 122-157

The following compounds were prepared by the procedure in example 121 using 5-amino-2-chloro-*N*-pyridin-3-ylbenzamide (Method 76) and the appropriate SM with the exception of Example 157 which was prepared from 2-chloro-5-{([3-(trifluoromethyl)benzoyl] amino}benzoic acid (Method 224) and *N*-(5-aminopyridin-2-yl)acetamide. In some cases, further purification was required (supercritical fluid, Gilson reverse phase preparatory HPLC or column chromatography utilizing an ISCO system).

| **Ex.** | **Compound** | **NMR** | **m/z** | **SM** |
|---|---|---|---|---|
| **122** | 2-Chloro-5-[(3,5-dimethylbenzoyl) amino]-*N*-pyridin-3-ylbenzamide | 10.81 (s, 1H), 10.44 (s, 1H), 8.86 (s, 1H), 8.33 (s, 1H), 8.16 (d, 1H),8.04 (s, 1H), 7.90-7.99 (m, 1H), 7.52-7.60 (m, 3H), 7.41 (dd, 1H), 7.23 (s, 1H), 2.30-2.37 (m, 6H) | 379 | 3,5-Dimethylbenzoic acid |
| **123** | 2-Chloro-5-[(3,5-difluorobenzoyl) amino]-*N*-pyridin-3-ylbenzamide | 10.82 (s, 1H), 10.63 (s, 1H), 8.85 (s, 1H), 8.33 (s, 1H), 8.16 (d, 1H), 8.01 (s, 1H), 7.87-7.95 (m, 1H), 7.69 (d, 2H), 7.51-7.63 (m, 214), 7.41 (dd, 1H) | 387 | 3,5-Difluorobenzoic acid |
| **124** | 2-chloro-5-[(3-iodobenzoyl)amino]-*N-*pyridin-3-ylbenzamide | 10.81 (s, 1H), 10.59 (s, 1H), 8.85 (s, 1H), 8.31 (s, 2H), 8.16 (d, 1H), 7.90-8.03 (m, 4H), 7.58 (d, 1H), 7.31-7.45 (m, 2H) | 477 | 3-lodobenzoic acid |
| **125** | 2-Chloro-5-[(3,5-dimethoxybenzoyl) amino]-*N*-pyridin-3-ylbenzamide | 10.80 (s, 1H), 10.44 (s, 1H), 8.85 (s, 1H), 8.33 (s, 1H), 8.16 (d, 1H), 8.02 (s, 1H), 7.91-7.98 (m, 1H), 7.57 (d, 1H), 7.41 (dd, 1H), 7.10 (s, 2H), 6.73 (s, 1H), 3.79-3.86 (m, 6H) | 411 | 3,5-Dimethoxybenzoi acid |
| **126** | 2-Chloro-5-[(3-methylbenzoyl) amino]-*N*-pyridin-3-ylbenzamide | 10.80 (s, 1H), 10.49 (s, 1H), 8.86 (s, 1H), 8.33 (s, 1H), 8.16 (d, 1H), 8.04 (s, 1H), 7.90-7.98 (m, 1H), 7.72-7.79 (m, 2H), 7.57 (d, 1H), 7.36-7.44 (m, 3H), 2.39 (s, 3H) | 365 | 3-Methylbenzoic acid |
| **127** | 2-Chloro-5-[(3-isopropoxybenzoyl) amino]-*N*-pyridin-3-ylbenzamide | 10.81 (s, 1H), 10.46 (s, 1H), 8.86 (s, 1H), 8.33 (s, 1H), 8.16 (d, 1H), 8.05 (s, 1H), 7.90-7.98 (m, 1H), 7.38-7.52 (m, 5H), 7.15 (d, 1H), 4.61-4.74 (m, 1H), 1.28 (d, 6H) | 409 | 3-Isopropoxybenzoic acid |
| **128** | 2-Chloro-5-{[3-(dimethylamino) benzoyl]amino}-*N*-pyridin-3-ylbenzamide | 10.80 (s, 1H), 10.40 (s, 1H), 8.85 (s, 1H), 8.33 (d, 1H), 8.16 (d, 1H), 8.03 (s, 1H), 7.95 (d, 1H), 7.56 (d, 1H), 7.41 (dd, 1H), 7.32 (t, 1H), 7.19-7.25 (m, 2H), 6.93 (d, 1H), 2.90-2.98 (m, 6H) | 394 | 3-(Dimethylamino)-benzoic acid |
| **129** | 2-Chloro-5-({3-[(difluoromethyl)thio] benzoyl}amino)-*N*pyridin-3-ylbenzamide | 10.81 (s, 1H), 10.65 (s, 1H), 8.85 (s, 1H), 8.33 (d, 1H), 8.17 (s, 2H), 8.08 (d, 1H), 8.03 (s, 1H), 7.91-7.98 (m, 1H), 7.73-7.88 (m, 1H), 7.54-7.69 (m, 2H), 7.31-7.45 (m, 2H) | 433 | 3-[(Difluoromethyl)-thio]benzoic acid |
| **130** | 2-Chloro-5-[(3-chlorobenzoyl)amino] -*N*-pyridin-3-ylbenzamide | 10.81 (s, 1H), 10.61 (s, 1H), 8.85 (s, 1H), 8.33 (d, 1H), 8.16 (d, 1H), 8.02 (s, 2H), 7.88-7.96 (m, 2H), 7.65-7.73 (m, 1H), 7.59 (dd, 2H), 7.41 (dd, 1H) | 386 | 3-Chlorobenzoic acid |
| **131** | 2-Chloro-5-[(3-propoxybenzoyl) amino]-*N*-pyridin-3-ylbenzamide | 10.80 (s, 1H), 10.47 (s, 1H), 8.86 (s, 1H), 8.33 (s, 1H), 8.16 (d, 1H), 8.04 (s, 1H), 7.95 (d, 1H), 7.46-7.55 (m, 3H), 7.39-7.44 (m, 2H), 7.16 (d, 1H), 4.00 (t, 2H), 1.74 (m, 2H), 0.99 (t, 3H) | 409 | 3-Propoxybenzoic acid |
| **132** | 2-Chloro-5-[(3-methoxybenzoyl) amino]-*N*-pyridin-3-ylbenzamide | 10.80 (s, 1H), 10.49 (s, 1H), 8.85 (s, 1H), 8.33 (d, 1H), 8.16 (d, 1H), 8.03 (s, 1H), 7.91-7.98 (m, 1H), 7.52-7.59 (m, 2H), 7.38-7.50 (m, 3H), 7.18 (d, 1H), 3.83 (s, 3H) | 381 | 3-Methoxybenzoic acid |
| **133** | 5-[(3-Bromobenzoyl)amino]-2-chloro-*N*-pyridin-3-ylbenzamide | 10.81 (s, 1H), 10.61 (s, 1H), 8.85 (s, 1H), 8.33 (d, 1H), 8.16 (s, 2H), 8.02 (s, 1H), 7.95 (t, 2H), 7.82 (d, 1H), 7.54 (m, 2H), 7.41 (dd, 1H) | 430 | 3-Bromobenzoic acid |
| **134** | 2-Chloro-5-[(3-isobutoxybenzoyl) amino]-*N*-pyridin-3-ylbenzamide | 10.80 (s, 1H), 10.47 (s, 1H), 8.85 (s, 1H), 8.33 (d, 1H), 8.1_{.}6 (d, 1H), 8.04 (s, 1H), 7.91-7.98 (m, 1H), 7.54 (dd, 3H), 7.38-7.48 (m, 2H), 7.17 (d, 1H), 3.82 (d, 2H), 2.04 (ddd, 1H), 0.99 (d, 6H) | 423 | 3-Isobutoxybenzoic acid |
| **135** | 2-Chloro-*N*-pyridin-3-yl-5-{[3-(trifluoromethyl) benzoyl]amino} benzamide | 10.82 (s, 1H), 10.73 (s, 1H), 8.85 (s, 1H), 8.24-8.38 (m, 3H), 8.17 (d, 1H), 8.02 (s, 1H), 7.98 (t, 2H), 7.80 (t, 1H), 7.60 (d, 1H), 7.41 (dd, 1H) | 419 | 3-(Trifluoromethyl)-benzoic acid |
| **136** | 2-Chloro-5-{[3-(1-cyano-1-methylethyl) benzoyl]amino}-*N*-pyridin-3-ylbenzamide | 10.81 (s, 1H), 10.58 (s, 1H), 8.85 (s, 1H), 8.33 (d, 1H), 8.16 (d, 1H), 8.03 (d, 2H), 7.95 (d, 2H), 7.77 (d, 1H), 7.55-7.66 (m, 2H), 7.41 (dd, 1H), 1.74 (s, 6H) | 418 | 3-(1-Cyano-1-methylethyl)-benzoic acid |
| **137** | 2-Chloro-5-({3-[(dimethylamino) sulfonyl]benzoyl} amino)-*N*-pyridin-3-ylbenzamide | 10.79 (d, 2H), 8.85 (d, 1H), 8.29-8.35 (m, 3H), 8.16 (d, 1H), 8.02 (s, 1H), 7.94-7.99 (m, 2H), 7.84 (t, 1 H), 7.60 (d, 1H), 7.41 (dd, 1H), 2.65 (s, 6H) | 458 | Method 120 |
| **138** | 2-Chloro5-[(3-cyclopropyl-5-fluorobenzoyl)amino] -*N*-pyridin-3-ylbenzamide | 11.33 (s, 1H), 10.65 (s, 1H), 9.16 (d, 1H), 8.56 (d, 1H), 8.45 (d, 1H), 8.06 - 8.16 (m, 1H), 7.95 (dd, 1H), 7.82 (dd, 1H), 7.50 - 7.63 (m, 3H), 7.17 (d, 1H), 2.04 (ddd, 1H), 0.96 - 1.07 (m, 2H), 0.81 (ddd, 2H) | 410 | Method 226 |
| **139** | *N*-{4-Chloro-3-[(pyridin-3-ylamino) carbonyl]phenyl}-2-methyl isonicotinamide | 11:11 (s, 1H), 10.85 (s, 1H), 9.03 (s, 1H), 8.7 (d, 1H), 8.45 (s, 1H), 8.30 (d, 1H), 8.03 (d, 1H), 7.75-7.89 (m, 3H), 7.54 - 7.69 (m, 2H), 2.58 (s, 3H) | 367 | 2-methylisonicotinic acid |
| **140** | *N*-(4-Chloro-3-(pyridin-3-ylamino) carbonyl]phenyl}-2-cyanoisonicotinamide | 11.10 (s, 1H), 10.95 (s, 1H), 9.03 (s, 1H), 8.98 (d, 1H), 8.52 (s, 1H), 8.47 (d, 1H), 8.31 (d, 1H), 8.19 (dd, 1H), 8.06 (d, 1H), 7.91 (dd, 1H), 7.63 - 7.68 (m, 1H), 7.63 (s, 1H) | 378 | 2-cyanoisonicotinic acid |
| **141** | 2-Chloro-5-[(5-chloro-2-fluoro benzoyl)amino]-*N*-pyridin-3-ylbenzamide | 9.57 (s, 1H), 9.45 (s, 1H), 8.80 (s, 1H), 8.16 - 8.23 (m, 2H), 84 (s, 1H), 7.63 - 7.70 (m, 2H), 7.45 - 7.52 (m, 1H), 7.16 - 7.27 (m, 3H) | 405 | 2-fluoro-5-chlorobenzoic acid |
| **142** | 2-Chloro-5-[(3-fluoro-5-methylbenzoyl) amino]-*N*-pyridin-3-ylbenzamide | 9.77 (s, 2H), 9.92 (d, 1H), 8.35 (d, 1H), 8.30 d, 1H), 8.10 - 8.12 (m, 1H), 7.94 - 7.98 (m, 1H), 7.67 (s, 1H), 7.54 (d 1H), 7.51 (d, 1H), 7.88 (dd, 1H), 7.21 (d, 1H), 2.42 (s, 3H) | 384 | 3-fluoro-5-methylbenzoic acid |
| **143** | *N-*{4-Chloro-3-[(pyridin-3-ylamino) carbonyl]phenyl}-4-methoxy-2-methyl benzamide | 10.74-(s, 1H), 10.38 (s,1H), 9.00 (d, 1H), 8.88 (d, 114), 8.29 - 8.33 (m, 1H), 8.16 (d, 1H), 8.02 (s, 1H), 7.99 (dd, 1H), 7.67 (dd, 1H), 7.45 (dd, 1H), 6.86 - 6.91 (m, 2H), 3.86 (s, 3H), 2.47 (s, 3H) | 396 | 2-methyl-4-methoxybenzoic acid |
| **144** | 2-Chloro-5-{[3-fluoro-5-(trifluoromethyl) benzoyl]amino}-*N-*pyridin-3-ylbenzamide | 10.88 (s, 1H), 10.76 (s, 1H), 9.01 (d, 1H), 8.38 - 8.40 (m, 1H), 8.29 - 8.34 (m, 1H), 8.28 (s, 1H), 8.18 - 8.22 (m, 2H), 8.03 (dd, 1 H0 7.97 - 8.00 (d, 1H), 8.62 (d, 1H), 8.46 (dd, 1H), | 438 | 3-fluoro-5-trifluoromethylbenzoic acid |
| **145** | 2-Chloro-5-[(2,5-dimethylbenzoyl) amino]-*N-*pyridin-3-ylbenzamide | 10.76 (s, 1H), 10.50 (s, 1H), 9.02 (d, 1H), 8.39 (d, 1H), 8.32 (d, 1H), 8.18.(d, 1H), 7.99 - 8.03 (m, 2H), 8.59 (d, 1H), 7.22 (s, 1H), 7.46 (dd, 1H), 2.41 (s, 3H), 2.33 (s, 3H) | 380 | 2,5-dimethylbenzoic acid |
| **146** | 5-[(4-Bromo-3-methylbenzoyl) amino]-2-chloro-*N-*pyridine-3-ylbenzamide | 10.75 (s, 1H), 10.62 (s, 1H), 9.02 (d,1H), 8.40 (dd, 1H), 8.30 -8.34 (m, 1H), 8.20 (d, 1H), 8.03 -8.07 (m, 2H), 8.71 - 8.86 (m, 2H), 7.60 (d, 1H), 7.46 (dd, 1H), 2.47 (s, 3H) | 446 | 3-methyl-4-bromobenzoic acid |
| **147** | 2-Chloro-5-[(3,4-dimethoxybenzoyl) amino]-*N-*pyridin-3-ylbenzamide | 10.72 (s, 1H), 10.41 (s, 1H), 9.00 (d, 1H), 8.88 (dd, 1H), 8.29 - 8.33 (m, 1H), 8.18 (d, 1H), 8.06 (d, 1H), 7.76 (d, 1H), 7.68 (d, 1H), 7.56 (d, 1H), 7.47 (dd, 1H), 7.16 (d, 114), 3.92 (s, 3H), 6.99 (s,3H) | 412 | 3,4-dimethoxybenzoic acid |
| **148** | 2-Chloro-5-[(3-chloro-5-fluoro benzoyl)amino]-*N-*pyridin-3-ylbenzamide | 10.22 (s, 1H), 10.11 (s, 1H), 8.65 (d, 1H), 8.02 - 8.04 (m, 1H), 7.98 (d, 1H), 7.87 (d, 1H), 7.69 - 7.75 (m, 2H), 7.56 (d, 1H), 7.48 (dd, 1H), 7.21 (dd, 1H), 6.99 (d, 1H) | 404 | 3-fluoro-5-chlorobenzoic acid |
| **149** | 2-Chloro-5-[(3,4-dimethylbenzoyl) amino]-*N-*pyridin-3-ylbenzamide | 10.74 (s 1H), 10.45 (s, 1H), 9.02 (s, 1H); 8.39 (dd, 1H), 8.30 - 8.34 (m, 1H), 8.22 (d, 1H); 8.07 (dd, 1H), 7.85 (s, 1H), 7.82 (dd, 1H), 7.46 (dd, 1H), 7.81 (d, 1H), 2.32 (s, 6H) | 380 | 3,4-dimethylbenzoic acid |
| **150** | 2-Chloro-5-[(4-methoxy-3-methyl benzoyl)amino]-*N-*pyridin-3-ylbenzamide | 10.72 (s, 1H), 10.37 (s, 1H), 9.00 (d, 1H), 8.88 (dd; 1H), 8.29 - 8.33 (m, 1H), 8.28 (d, 1H), 8.04 - 8.08 (m, 1H), 7.96 - 7.99 (m, 1H), 7.91 (s, 1H), 7.65 (d, 1H), 7.46 (dd, 1H), 7.42 (d, 1H), 3.94 (s, 3H), 2.23 (s, 3H) | 396 | 3-methyl-4-methoxybenzoic acid |
| **151** | 2-Chloro-5-[(4-methoxybenzoyl) amino]-*N-*pyridin-3-ylbenzamide | 10.73 (s, 1H), 10.43 (s, 1H), 9.03 (d, 1H), 8.39 (dd, 1H), 8.30 -8.34 (m, 1H), 8.21 (d, 1H), 8.04 - 8.11 (m, 3H), 7.56 (d, 1H), 7.46 (dd, 1H), 7.12 (d, 1H), 3.90 (s, 3H) | 382 | 4-methoxybenzoic acid |
| **152** | 2-Chloro-5-[(3,5-dichlorobenzoyl) amino-*N-*pyridin-3-ylbenzamide | 10.75 (s, 1H), 9.00 (d, 1H), 8.39 (dd, 1H), 8.29 - 8.34 (m, 1H), 8.17 (d, 1H), 8.08 (s, 1H), 8.01 - 8.05 (m, 2H), 7.86 - 7.89 (m, 1H), 7.62 (d, 1H), 7.46 (dd, 1H) | 421 | 3,5-dichlorobenzoic acid |
| **153** | 2-Chloro-5-[(4-ethoxybenzoyl)amino] -*N-*pyridin-3ylbenzamide | 10.73 (s, 1H), 10.42 (s, 1H), 9.03 (s, 1H), 8.39 (d, 1H), 8.30 - 8.35 (m, 1H), 8.23 (d; 1H), 8.02 - 8.10 (m, 3H), 7.56 (d, 1H), 7.45 (dd, 1H), 7.09 (d, 2H), 4.15 5 (dd, 2H), 1.39 (t, 3H) | 396 | 4-ethoxybenzoic acid |
| **154** | 2-Chloro-5-{[3-morpholin-4-yl-5-(trifluoromethyl) benzoyl}amino}-*N*-pyridin-3-ylbenzamide | 10.83 (s, 1H), 10.60 (s, 1H), 8.86 (d, 1H), 8.34 (d, 1H), 8.17 (ddd, 1H), 7.94 - 8.00 (m, 2H), 7.71 (s, 1H), 7.66 (s, 1H), 7.58 (d, 1H), 7.40 - 7.45 (m, 2H), 3.75 - 3.78 (m, 4H), 3.27 - 3.31 (m, 4H) | 506 | 3-trifluoromethyl-5-morpholinobenzoic acid |
| **155** | 2-Chloro-5-[(3-cyclopropylbenzoyl) amino]-*N-*pyridin-3-ylbenzamide | 11.24 (s, 1H), 10.51 (d, 1H), 9.14 (s, 1H), 8.54 (s, 1H), 8.35 - 8.48 (m, 1H), 8.06 - 8.16 (m, 1H), 7.93 (dd, 1H), 7.70 (d, 2H), 7.56 - 7.66 (m, 2H), 7.28 - 7.43 (m, 2H), 1.91 - 2.06 (m, 1H), 0.95 - 1.04 (m, 2H), 0.70 - 0.79 (m, 2H) | 393 | Method 34 |
| **156** | 2-Chloro-5-[(3,4-dichlorobenzoyl) amino]-*N-*pyridin-3-ylbenzamide | 10.93 (s, 1H), 10.92 (s, 1H), 9.20 (d, 1H), 8.57 (dd, 1H), 8.61 (dd 1H), 8.47 (d 1H), 8.36 (d 1H), 8.20 - 8.24 (m, 2H), 8.05 (d 1H), 7.78 (d 1H), 7.65 (dd 1H) | 400 | 3,4-dichlorobenzoic acid |
| **157** | *N-*[6-(Acetylamino)pyridin-3-yl]-2-chloro-5-{[3-(trifluoromethyl) benzoyl]amino} benzamide | 1H NMR (300 MHz) 10.72 (d, 2H), 10.51 (s, 1H), 8.67 (s, 1H), 8.17 - 8.34 (m, 3H), 7.92 - 8.10 (m, 5H), 7.76 - 7.86 (m, 1H), 7.60 (d, 1H), 2.09 (s, 3H) | 477 | Method 224 and *N-*(5-aminopyridin-2-yl)acetamide |

### Example 158

### N-(6-Acetylpyridin-3-yl)-2-chloro-5-{[3-(trifluoromethyl)benzoyl]amino)}benzamide

To a solution of *N-*(6-bromopyridin-3-yl)-2-chloro-5-{[3-(trifluoromethyl) benzoyl]amino}benzamide (Example 215; 100 mg) in NMP (3 ml) were added tributyl[(1*E*)-2-methoxyprop-1-en-1-yl]stannane (0.4 ml), Pd(PPh₃)₄ (50 mg) under argon. The resulting mixture was heated to 80°C for 10 hours. The mixture was partitioned between H₂O and EtOAc, and the organic layer was washed with brine, saturated aqueous NaF solution and dried with MgSO₄ Evaporation of the solvent gave a solid, which was dissolved in 2 N HCl and stirred for 3 hours at room temperature. Extraction of the aqueous layer with EtOAc (3x 25 ml) and the organic layer was washed with brine and dried with MgSO₄. Evaporation gave a brown solid, which was purified by Reverse phase HPLC (5-95% MeCN/H₂O,15 min). The title compounds (2 mg) was collected by evaporation. NMR (300 MHz) 11.26 (s, 1H), 10.82 (s, 1H), 9.03 (d, 1H), 8.-6-8.63 (m, 3H), 7.-8 - 8.23 (m, 4H), 7.88 (t, 1H), 7.69 (d, 1H), 2.68 (s, 3H), m/z 461.

### Example 159

### N-{6-[(2-Hydroxyethyl)amino]pyridin-3-yl}-2-methyl-5-{[3-(trifluoromethyl) benzoyl]amino}benzamide

To a solution of *N-*{6-[(2-{[tert-butyl(dimethyl)silyl]oxy}ethyl)amino]pyridine-3-yl}-2-methyl-5-{[3-(trifluoromethyl)benzoyl]amino)benzamide (Method 99; 125 mg) in THF was added tertabutylammonium fluoride (3 ml, 1.0M in THF) and the resulting dark red solution was stirred at room temperature for 2 hours. The mixture was partitioned between H₂O and EtOAc, and the organic layer was washed with brine and dried with MgSO₄. Evaporation of the solvent gave a pale purple oil, which was purified by Reverse phase HPLC (5-95% ACN/ H₂O, 20 min). The title compound (43 mg) was collected by evaporation as a pale purple solid. NMR (300 MHz): 10.59 (s, 2H), 8.47 (s, 1H), 8.23 - 8.35 (m, 2H), 7.88 - 8.03 (m, 3H), 7.75 -7.86 (m, 2H), 7.34 (d, 1H), 7.06 (s, 1H), 3.58 - 3.67 (m, 4H), 2.36 (s, 3H); m/z 459.

### Example 160

### 5-{[3-(1-Cyano-1-methylethyl)benzoyl]amino}-N-[6-(glycoloylamino)-5-methylpyridin-3-yl]-2-methylbenzamide

To a solution of *N-*(6-{[(benzyloxy)acetyl]amino}-5-methylpyridin-3-yl)-5-{[3-(1-cyano-1-methylethyl)benzoyl]amino}-2-methylbenzamide (Example 316; 30 mg) in MeOH (3 ml) were added HCO₂NH₄ and NCO₂H (5 ml, 1:10 v/v) and the resulting mixture was refluxed for 12 hours. The mixture was partitioned between EtOAc and H₂O and the organics were washed with NaCl₍ₛₐₜ₎ and then dried with Na₂SO₄₍ₛ₎. Evaporation of the solvent gave a solid, which was purified by reverse phase HPLC (5-95% ACN/H₂O, 15 min). The title compound (5 mg) was collected by evaporation. NMR (300 MHz): 10.55 (s, 1H), 10.43 (s, 1H), 9.62 (s, 1H), 8.55 (d, 1H), 8.03 - 8.10 (m, 2H), 7.96 (d, 1H), 7.89 (d, 1H), 7.85 (dd, 1H), 7.75 (d, 1H), 7.61 (t, 1H), 7.33 (d, 1H), 4.08 - 4.18 (m, 2H), 2.37 (s, 3H), 2.19 (s, 3H), 1.76 (s, 6H); m/z 486.

### Example 161

### N-[6-(Aminomethyl)pyridin-3-yl]-5-{[3-(1-cyano-1-methylethyl)benzoyl]amino}-2-methylbenzamide

To a solution of 5-{[3-(1-cyano-1-methylethyl)benzoyl]amino}-*N-*(6-cyanopyridin-3-yl)-2-methylbenzamide (Example 321; 100 mg) in THF (3ml) was added slowly LiAlH₄ (3 ml, 1.0M in THF) and the resulting mixture was stirred at ambient temperature for 5 minutes. The mixture was cooled with an ice bath and a saturated solution of tartaric acid was added slowly until evolution of gas ceased. The mixture was filtered to remove the aluminium salts and the filtrate was partitioned between EtOAc and H₂O. The organics were washed with NaCl₍ₛₐₜ₎ and then dried with Na₂SO₄₍ₛ₎. Evaporation of the solvent gave a solid, which was purified by reverse phase HPLC (5-95% ACN/H₂O,15 min). The title compound (50 mg) was collected by evaporation. NMR (300 MHz): 10.72 (s, 1H), 10.52 (s, 1H), 8.96 (s, 1H), 8.33 - 8.41 (bs, 2H), 8.22 (d, 1H), 8.08 (s, 1H), 7.95 - 8.03 (m, 2H), 7.82 (d, 1H), 7.77 (d, 1H), 7.61 (t, 1H), 7.54 (d, 1H), 7.28 - 7.41 (m, 2H), 4.11 - 4.20 (m, 2H), 2.38 (s, 3H), 1.79 (s, 6H); m/z 429.

### Example 162

The following compound was prepared by the procedure of Example 161, using the appropriate starting material.

| **Ex** | **Compound** | **NMR** | **M/z** | **SM** |
|---|---|---|---|---|
| **162** | *N-*[5-(Aminomethyl) pyridin-3-yl]-2-chloro-5-{[3-(1-cyano-1-methyl ethyl)benzoyl]amino} benzamide | 10.99 (s, 1H), 10.60 (s, 1H), 8.71 (s, 1H), 8.42 (s, 1H), 8.17-8.28 (m, 2H), 8.01 8.11 (m, 2H), 7.88 - 8.01 (m, 2H), 7.78 (d, 1H), 7.53 - 7.68 (m, 2H); 4.13 (dd, 2H) 1.74 (s, 6H) | 449 | Example 313 |

### Example 163

### 5-{[3-(1-Cyano-1-methylethyl)benzoyl]amino}-N-[5-(methoxymethyl)pyridin-3-yl]-2-methylbenzamide

To a solution of 5-{[3-(1-cyano-1-methylethyl)benzoyl]amino}-*N-*[5-(hydroxymethyl)pyridin-3-yl]-2-methylbenzamide (Example 299; 24 mg) in THF (2 ml) was added slowly at 0°C NaH (5 mg) and the mixture was stirred at this temperature for 30 minutes. Mel (0.05 ml) was added and the mixture was stirred at room temperatures for 4 hours. The mixture was cooled with an ice bath and H₂O was added until evolution of gas ceased. The mixture was partitioned between EtoAc and H₂O. The organics were washed with NaCl₍ₛₐₜ₎ and then dried with Na₂SO₄₍ₛ₎. Evaporation of the solvent gave a solid, which was purified by reverse phase HPLC (5-95% MeCN/H₂O, 15 min). The title compound (5.7 mg) was collected by evaporation. NMR (300 MHz): 10.89 (s, 1H), 10.41 (s, 1H), 8.45 (s, 1H), 8.30 (s, 1H), 8.20 (d, 1H), 8.05 (s, 1H), 7.90 - 8.00 (m, 2H), 7.86 (d, 1H), 7.76 (d, 1H), 7.65(t, 1H), 7.32 (d, 1H), 4.50 (s, 2H) 3.35(s, 3H) 2.10 (s, 3H), 1.76 (s, 6H); m/z 443.

### Example 164

### 2-Chloro-N-(5-fluoropyridin-3-yl)-5-{[3-fluoro-5-(trifluoromethyl)benzoyl]amino}-benzamide

A solution of 5-amino-2-chloro-*N-*(5-fluoropyridin-3-yl)benzamide (Method 79; 124 mg, 0.466 mmol) and DIEA (405 µL, 2.33 mmol, 5.0 equiv) in THF (2.0 ml) was treated with 3-fluoro-5-(trifluoromethyl)benzoyl chloride (131 mg, 0.582 mmol, 1.25 equiv). The reaction mixture was stirred for 12 h at 25 °C. The reaction was quenched with 10% NaOH and extracted with EtoAc. The organics were dried with NaCl₍ₛₐₜ₎ and then Na₂SO₄₍ₛ₎ and removed under reduced pressure. The residue was purified directly by Gilson reverse phase preparatory HPLC (5-95% MeCN/H₂O) to give 75 mg of product (35%). NMR: 11.11 (s, 1H), 10.80 (s, 1H), 8.68 (s, 1H), 8.37 (s, 1H), 8.18-8.12 (m, 3H), 8.03-7.93 (m, 3H), 7.63 (d, 1H); *mlz* 456.

### Example 165

### 2-Chloro-N-[6-(1,2-dihydroxyethyl)pyridin-3-yl]-5-{[3-trifluoromethyl)benzoyl] amino}benzamide

To a solution of *N-*(6-bromopyridin-3-yl)-2-chloro-5-{[3-(trifluoromethyl)benzoyl] amino}benzamide (Example 215; 100 mg) in NMP (3 ml) were added tributyl vinyl tin (0.4 ml), Pd(PPh₃)₄ (50 mg) under argon. The resulting mixture was heated to 80°C for 10 hours. The mixture was partitioned between H₂O and EtOAc, and the organic layer was washed with brine, saturated aqueous NaF solution and dried with MgSO₄. Evaporation of the solvent gave a solid, which was dissolved in a mixture of acetone/H₂O (1:1 v/v, 2 ml). *N-*methyl morpholine oxide (60 mg) was added followed by a solution of OsO₄ in t-BuOH (0.04 ml, 2.5 w/v) and the resulting mixture was stirred at ambient temperature for 12 hours. A solution of sodium thiosulphate (1N, 10 ml) was added and the resulting solution was stirred for 3 hours at room temperature. The mixture was partitioned between H₂O and EtOAc, and the organics layer was washed with brine and dried with MgSO₄. Evaporation of the solvent gave a solid, which was purified by reverse phase HPLC (5-95% MeCN/H₂O, 15 min). The title compound (11 mg) was collected by evaporation. NMR (300 MHz): 10.54 (s, 1H), 10.34 (s, 1H), 8.00-8.55 (m, 4H), 7.80 - 7.95 (m, 3H), 7.08-7.55 (m, 3H), 3.23-3.51 (m, 3H); m/z 480.

### Example 166

### 2-Fluoro-5-[(3-fluorobenzoyl)amino]-N-pyridin-3-ylbenzamide

A solution of 5-amino-2-fluoro-*N-*pyridin-3-ylbenzamide (Method 75; 30.5 mg, 0.132 mmol), 3-fluorobenzoic acid (20.5 mg, 0.145 mmol) and DIEA (60 µl, 0.35 mmol) in DMF (1.5 ml) was treated with HATU (60 mg, 0.1584 mmol). The reaction mixture was shaken overnight at 25 °C. Water (10 ml) was added slowly to precipitate the product. The resulting precipitate was washed with water (10 ml), isolated and dried overnight in a vacuum oven at 70 °C to give the title compound 32 mg, (68%) as a solid. NMR: 10.65 (s, 1H), 10.52 (s, 1H), 8.86 (s, 1H), 8.33 (d, 1H), 8.15 (d, 1H), 8.09 (d, 1H), 7.93-8.02 (m, 1H), 7.75-7.84 (m, 2H), 7.56-7.66 (m, 1H), 7.37-7.48 (m, 3H); *m*/*z* 353.

### Examples 167-182

The following compounds were prepared by the procedure in Example 166 using 5-amino-2-fluoro-*N-*pyridin-3-ylbenzamide (Method 75) and the appropriate SM. In some cases, further purification was required (supercritical fluid, Gilson reverse phase preparatory HPLC or column chromatography utilizing an ISCO system).

| **Ex.** | **Compound** | **NMR** | **m/z** | **SM** |
|---|---|---|---|---|
| **167** | 5-[(3,5-Dimethylbenzoyl)amino]-2-fluoro-*N-*pyridin-3-ylbenzamide | 10.65 (s, 1H), 10.37 (s, 1H), 8.87 (s, 1H), 8.33 (d, 1H), 8.07-8.18 (m, 2H), 7.93-8.01 (m, 1H), 7.57 (s, 2H), 7.34-7.43 (m, 2H), 7.23 (s, 1H), 2.35 (s, 6H) | 363 | 3,5-Dimethylbenzoic acid |
| **168** | 5-[(3,5-Difluorobenzoyl)amino]-2-fluoro-*N-*pyridin-3-ylbenzamide | 10.66 (s, 1H), 10.57 (s, 1H), 8.86 (s, 1H), 8.33 (d, 1H), 8.15 (d, 1H), 8.08 (d, 1H), 7.97 (d, 1H), 7.69 (d, 2H), 7.55 (t, 1H), 7.36-7.44 (m, 2H) | 371 | 3,5-Difluorobenzoic acid |
| **169** | 2-Fluoro-5-[(3-iodobenzoyl)amino] -*N*-piridin-3-ylbenzamide | 10.65 (s, 1H), 10.52 (s, 1H), 8.86 (s, 1H), 8.32 (s, 2H), 8.15 (d, 1H), 8.07 (d, 1H), 7.97 (d, 3H), 7.32-7.43 (m, 3H) | 461 | 3-Iodobenzoic acid |
| **170** | 5-[(3,5-Dimethoxybenzoyl)amino]-2-fluoro-*N*-pyridin-3-ylbenzamide | 10.65 (s, 1H), 10.38 (s, 1H), 8.86 (s, 1H), 8.33 (d, 1H), 8.15 (d, 1H), 8.08 (d, 1H), 7.94-8.03 (m, 1H), 7.34-7.43 (m, 2H), 7.11 (s, 2H), 6.72 (s, 1H), 3.82 (s, 6H) | 395 | 3,5-Dimethoxybenzoic acid |
| **171** | 2-Fluoro-5-[(3-methylbenzoyl) amino]-*N-*pyridin-3-ylbenzamide | 10.74 (s, 1H), 10.51 (s, 1H), 8.96 (s, 1H), 8.42 (d, 1H), 8.17-8.28 (m, 2H), 8.07 (d, 1H), 7.81-7.89 (m, 2H), 7.44-7.54 (m, 4H), 2.58 (s, 3H) | 349 | 3-Methylbenzoic acid |
| **172** | 2-Fluoro-5-[(3-isopropoxybenzoyl) amino]-*N-*pyridin-3-ylbenzamide | 10.67 (s, 1H), 10.39 (s, 1H), 8.88 (s, 1H), 8.34 (d, 1H), 8.17 (d, 1H), 8.11 (s, 1H), 7.92-8.02 (m, 1H), 7.47-7.53 (m, 2H), 7.38-7.45 (m, 3H), 7.15 (d, 1H), 4.70 (dt, 1H), 1.29 (d, 6H) | 393 | 3-Isopropoxybenzoic acid |
| **173** | 5-{[3-(Dimethylamino) benzoyl]amino}-2-fluoro-*N-*pyridin-3-ylbenzamide | 10.65 (s, 1H), 10.33 (s, 1H), 8.86 (s, 1H), 8.33 (d, 1H), 8.16 (d, 1H), 8.09 (d, 1H), 7.97 (d, 1H), 7.36-7.42 (m, 2H), 7.31 (d, 1H), 7.22-7.24 (m, 2H), 6.93 (d, 1H), 2.96 (s, 6H) | 378 | 3-(Dimethylamino) benzoic acid |
| **174** | 5-({3-[(Difluoromethyl)thio]benzoyl} amino)-2-fluoro-*N-*pyridin-3-ylbenzamide | 10.65 (s, 1H), 10.57 (s, 1H), 8.86 (s, 1H), 8.33 (d, 1H), 8.14-8.18 (m, 2H), 8.08 (m, 2H), 7.96-8.01 (m, 1H), 7.81 (d, 1H), 7.55-7.71 (m, 2H), 7.40 (m, 2H) | 417 | 3-[(Difluoromethyl) thio]benzoic acid |
| **175** | 5-[(3-Chlorobenzoyl)amino]-2-fluoro-*N-*pyridin-3-ylbenzamide | 10.67 (s, 1H), 10.56 (s, 1H), 8.87 (s, 1H), 8.33 (d, 1H), 8.16 (d, 1H), 8.09 (d, 1H), 8.03 (s, 1H), 7.95-8.00 (m, 1H), 7.93 (d, 1H), 7.68 (d, 1H), 7.58 (t, 1H), 7.35-7.44 (m, 2H) | 369 | 3-Chlorobenzoic acid |
| **176** | 2-Fluoro-5-[(3-propoxybenzoyl) amino]-*N-*pyridin-3-ylbenzamide | 10.65 (s, 1H), 10.41 (s, 1H), 8.87 (s, 1H), 8.33 (d, 1H), 8.08-8.18 (m, 2H), 7.94-8.01 (m, 1H), 7.49-7.55 (m, 2H), 7.35-7.46 (m, 3H); 7.16 (d, 1H), 4.00 (t, 2H), 1.71-1.80 (m, 2H), 1.00 (t, 3H) | 393 | 3-Propoxybenzoic acid |
| **177** | 2-Fluoro-5-[(3-methoxybenzoyl) amino]-*N-*pyridin-3-ylbenzamide | 10.65 (s, 1H), 10.43 (s, 1H), 8.86 (s, 1H), 8.33 (d, 1H), 8.16 (d, 1H), 8.09 (s, 1H), 7.98 (d, 1H), 7.57-7.35 (m, 5H), 7.17 (d, 1H), 3.83 (s, 3H) | 365 | 3-Methoxybenzoic acid |
| **178** | 5-[(3-Bromobenzoyl)amino]-2-fluoro-*N-*pyridin-3-ylbenzamide | 10.65 (s, 1H), 10.55 (s, 1H), 8.86 (s, 1H), 8.33 (d, 1H), 8.16 (s, 2H), 8.08 (d, 1H), 7.97 (d, 2H), 7.81 (d, 1H), 7.51 (t, 1H), 7.39 (t, 2H) | 414 | 3-Bromobenzoic acid |
| **179** | 2-Fluoro-5-[(3-isobutoxybenzoyl) amino]-*N-*pyridin-3-ylbenzamide | 10.66 (s, 1H), 10.41 (s, 1H), 8.87 (s, 1H), 8.33 (d, 1H), 8.16 (d, 1H), 8.10 (d, 1H), 7.94-8.02 (m, 1H), 7.49-7.55 (m, 2H), 7.36-7.46 (m, 3H), 7.16 (d, 1H), 3.82 (d, 2H), 1.99-2.08 (m, 1H), 1.00 (d, 6H) | 407 | 3-Isobutoxybenzoic acid |
| **180** | 2-Fluoro-*N-*pyridin-3-yl-5-{[3-(trifluoromethyl) benzoyl]amino}benzamide | 10.67 (s,2H), 8.86 (s, 1H), 8.25-8.35 (m, 3H), 8.16 (d, 1H), 8.09 (d, 1H), 7.99 (d, 2H), 7.80 (t, 1H), 7.36-7.45 (m, 2H) | 403 | 3-(Trifluoromethyl) -benzoic acid |
| **181** | 5-{[3-(1-Cyano-1-methylethyl) benzoyl]amino}-2-fluoro-*N-*pyridin-3-ylbenzamide | 10.64 (s, 1H), 10.57 (s, 1H), 8.78 (5, 1H), 8.19-8.27 (m,3H), 8.07 (d, 1 H), 7.99 (d, 1H), 7.85-7.95 (m, 2H), 7.75 (t, 1H), 7.28-7.36 (m, 2H), 2.56 (s, 6H) | 402 | Method 19 |
| **182** | 5-({3-[(Dimethylamino)sulfonyl]benzoyl}amino)-2-fluoro-*N-*pyridin-3-ylbenzamide | 10.66 (s, 1H), 10.52 (s, 1H), 8.87 (s, 1H), 8.33 (d, 1H), 8.16 (d, 1H), 8.03-8.09 (m, 2H), 7.93-8.03 (m, 2H), 7.76 (d, 1H), 7.61 (t, 1H), 7.40 (t, 2H), 1.75 (s, 6H) | 442 | Method 120 |

### Example 183

### 5-{[3-(1-Cyano-1-methylethyl)benzoyl]amino}-2-methyl-N-(6-methylpyridin-3-yl)benzamide

A solution of 5-[3-(cyano-dimethyl-methyl)-benzoylamino]-2-methyl-benzoic acid (Method 20; 200 mg, 0.620 mmol), 6-methylpyridin-3-amine (65 mg, 0.602 mmol) and DIEA (0.32 ml, 1.86 mmol) in DMF (1.3 ml) was treated with HATU (354 mg, 0.930 mmol). The reaction was stirred at 25 °C for 12 h. The reaction was then quenched with H₂O (5 ml) and extracted with EtOAc (20 ml). The organics were washed with NaCl₍ₛₐₜ₎ (50 ml) and dried with MgSO₄ The solvents were removed under reduced pressure to give a yellow solid (208 mg), which was purified by Gilson reverse phase preparatory HPLC (5-95% MeCN/H₂O,20 min). The solvents were removed under reduced pressure to give a white solid (116mg) of the title compound. NMR: 11.01-11.30 (s, 1H), 10.37-10.68 (s, 1H), 9.01-9.32 (s, 1H), 8.48 (d, 1H), 7.93-8.13 (m, 3H), 7.68-7.88 (m, 3H), 7.59 (t, 1H), 7.28 (d, 1H), 2.56-2.76 (s, 3H), 2.23-2.43 (s,3H), 1.45-1.92 (s, 6H); m/z 413.

### Examples 184-317

The following compounds were prepared by the procedure in example 183 using the appropriate SMs. In some cases, further purification was required (supercritical fluid, Gilson reverse phase preparatory HPLC or column chromatography utilizing an ISCO system).

| **Ex.** | **Compound** | **NMR** | **m/z** | **SM** |
|---|---|---|---|---|
| **184** | 5-{[3-(1-Cyano-1-methylethyl) benzoyl]amino}-*N-*(6-methoxypyridin-3-yl)-2-methylbenzamide | 10.29-10.49 (s, 1H), 9.53-9.71 (s, 1H), 8.20 (d, 1H), 8.02-8.11 (s, 1H), 7.97 (dd, 2H), 7.84-7.92 (s, 2H), 7.83 (d, 1H), 7.75 (d, 1H), 7.60 (t, 1H), 7.29 (d, 1H), 7.03 (d, 1H), 3.82-4.03 (s, 3H), 2.29-2.43 (s, 3H), 1.63-1.84 (s, 6H) | 429 | 6-Methoxy-pyridin-3-amine and Method 20 |
| **185** | 5-{[3-(1-Cyano-1-methylethyl)benzoyl]amino}-2-methyl-*N-*(4-methylpyridin-3-y])benzamide | 10.28-10.64 (s, 2H), 9.00-9.18 (s, 1H), 8.61 (d, 1H), 8.03-8.18 (s, 2H), 7.98 (d, 1H), 7.90 (d, 1H), 7.80-7.87 (s, 1H), 7.72-7.78 (s, 1H), 7.62 (t, 1H), 7.34 (d, 1H), 2.51-2.58 (s, 3H), 2.36-2.47 (s, 3H), 1.72-1.85 (s, 6H) | 413 | 4-Methylpyridin-3-amine and Method 20 |
| **186** | 5-{[3-(1-Cyano-1-methylethyl) benzoyl]amino}-*N-*(4-methoxy pyridin-3-yl)-2-methylbenzamide | 10.48-10.50 (s, 1H), 10.37-10.41 (s, 1H), 9.35-9.37 (s, 1H), 8.75 (s, 1H), 8.06-8.13 (s, 1H), 7.90-8.04 (m, 2H), 7.68-7.90 (m, 3H), 7.62 (t, 1 H), 7.33 (d, 1H), 4.14-4.16 (s, 3H), 2.36-2.45 (s, 3H), 1.74-1.79 (s, 6H) | 429 | 4-Methoxy-pyridin-3-amine and Method 20 |
| **187** | 5-{[3-(1-Cyano-1-methylethyl)benzoyl]amino}-2-methyl-*N-*(6-morpholin-4-ylpyridin-3-yl)benzamide | 10.40-10.45 (s, 1H), 10.31-10.38 (s, 1H), 8.41-8.55 (s, 1H), 8.03-8.07 (s, 1H), 7.84-8.03 (m, 3H), 7.71-7.84 (m, 2H), 7.61 (t, 1H), 7.32 (d, 1H), 7.03 (d, 1H), 3.74 (t, 4H), 3.42 (t, 4H), 2.28-2.43 (s, 3H), 1.71-1.80 (s, 6H) | 484 | Method 84 and Method 20 |
| **188** | 5-{[3-(1-Cyano-1-methylethyl)benzoyl]amino}-2-methyl-*N-*(5-methylpyridin-3-yl)benzamide | 10.36-10.42 (s, 1H), 8.71-8.92 (s, 2H), 8.02-8.07 (s; 1H), 7.95 (d, 1H), 7.82-7.87 (s, 2H), 7.74 (d, 3H), 7.60 (t, 1H), 7.32 (d, 2H), 4.17-4.29 (s, 2H), 1.71-1.79 (s, 6H) | 413 | Method 100 and Method 20 |
| **189** | *N-*(5-Chloropyridin-3-yl)-2-methyl-5-{[3-(trifluoromethyl)benzoyl]amino} benzamide | 10.81-10.87 (s, 1H), 10.61-10.63 (s, 1H), 8.80-8.83 (s, 1H), 8.36-8.41 (s, 2H), 8.22-8.35 (m, 2H), 7.92-8.02 (m, 2H), 7.71-7.90 (m, 2H), 7.35.(d, 1H), 2.35-2.41 (s, 3H) | 434 | 5-Chloropyridin-3-amine and Method 21 |
| **190** | *N-*(5-Methoxypyridin-3-yl)-2-methyl-5-{[3-(trifluoromethyl)benzoyl]amino}benzamide | 10.79-10.83 (s, 1H), 10.59-10.63 (s, 114), 8.63-8.69 (s, 1H), 8.25-8.38 (m, 2H), 8.15-8.22 (s, 1H), 7.91-8.07 (m, 3H), 7.77-7.90 (m, 2H), 7.35 (d, 1H), 3.85-3.92 (s, 3H), 2.35-2.41 (s, 3H) | 430 | Method 118 and Method 21 |
| **191** | *N-*(6-Amino-5-bromopyridin-3-yl)-2-methyl-5-{[3-(trifluoromethyl) benzoyl]amino} benzamide | 10.54-10.63 (s, 1H), 10.32-10.42 (s, 1H), 8.18-8.45 (m, 4H), 8.00 (d, 1H), 7.92-7.94 (s, 1H), 7.76-7.85 (m, 2H), 7.31 (d, 2H), 2.31-2.40 (s, 3H) | 494 | Method 77 and Method 21 |
| **192** | *N-*(5-Bromopyridin-3-yl)-5-{[3-(1-cyano-1-methylethyl)benzoyl]amino}-2-methylbenzamide | 10.76-10.85 (s, 1H), 10.40-10.48 (s, 1H), 8.80-8.88 (s, 1H), 8.48-8.59 (s, 1H), 8.41-8.47 (s, 1H), 8.04-8.09 (s, 1H), 7.89-8.00 (m, 2H), 7.85 (d, 1H), 7.76 (d, 1H), 7.60 (t, 1H), 7.33 (d, 1H), 2.33-2.42 (s, 3H), 1.69-1.81 (s, 6H) | 477 | Method 119 and Method 20 |
| **193** | *N-*(5-Bromopyridin-3-yl)-2-methyl]-5-{[3-(trifluoromethyl)benzoyl]amino} benzamide | 10.77-10.87 (s, 1H), 10.60-10.62 (s, 1H), 8.82-8.86 (s, 1H), 8.53-8.55 (s, 1H), 8.47-8.48 (s, 1 H), 8.24-8.36 (m, 2H), 7.72-8.04 (m, 4H), 7.35 (d, 2H), 2.36-2.39 (s, 3H) | 478 | Method 119 and Method 21 |
| **194** | 2-Methyl-*N-*(5-methylpyridin-3-yl)-5-{[3-(trifluoromethyl)benzoyl]amino} benzamide | 10.99-11.08 (s, 1H), 10.56-10.69 (s, 1H), 8.96-9.08 (s, 1H), 8.43-8.47 (s, 1H), 8.21-8.42 (m, 3H), 7.92-8.09 (m, 2H), 7.70-7.89 (m, 2H), 7.35 (d, 1H), 2.42-2.49 (s, 3H), 2.33-2.41 (s, 3H) | 413 | Method 100 and Method 21 |
| **195** | *N-*(6-Chloro-5-methylpyridin-3-yl)-5-{[3-(1cyano-1-methyl ethyl)benzoyl]amino}-2-methyl benzamide | 10.65-10.70 (s, 1H), 10.41-10.46 (s, 1H), 8.53-8.58 (s, 1H), 8.19-8.26 (s, 1H), 8.02-8.09 (s, 1H), 7.89-8.02 (m, 2H), 7.85 (d, 1H), 7.74 (d, 1H), 7.61 (t, 1H), 7.33 (d, 1H), 2.30-2.42 (m, 6H), 1.67-1.81 (s, 6H) | 446 | Method 85 and Method 20 |
| **196** | 5-[3-(Cyano-dimethyl-methyl)-benzoylamino]-*N*-(6-fluoro-5-methyl-pyridin-3-yl)-2-methyl-benzamide | 10.60 (s, 1H), 10.42 (s, 1H), 8.33 (s, 1H), 8.14-8.27 (m,1H), 8.02-8.11 (m, 1H), 7.96 (d, 1H), 7.89-7.93 (m, 1H), 7.84 (dd, 1H), 7.74-7.80 (m, 1H), 7.61 (t, 1H), 7.33 (d, 1H), 2.33-2.41 (m, 3H), 2.27 (s, 3H), 1.76 (s, 6H) | 431 | 6-Fluoro-5-methylpyridin-3-amine and Method 20 |
| **197** | *N-*(6-Amino-5-bromopyridin-3-yl)-5-{[3-(1-cyano-1-methylethyl)benzoyl]amino}-2-methylbenzamide | 10.26-10.53 (m, 2H), 8.36-8.46 (s, 1H), 8.23-8.33 (s, 1H), 8.04-8.12 (s, 1H), 7.70-7.99 (m, 4H), 7.52-7.69 (s, 1H), 7.22-7.41 (s, 1H), 2.34-2.39 (s, 3H), 1.71-1.81 (s, 6H) | 492 | Method 77 and Method 20 |
| **198** | 5-[3-(Cyano-dimethyl-methyl)-benzoylamino]-*N-*(6-fluoro-pyridin-3-yl)-2-methyl-benzamide | 10.67 (s, 1H), 10.43 (s, 1H), 8.57 (s, 1H), 8.25-8.35 (m, 1H), 8.03-8.13 (m, 1H), 7.89-8.00 (m, 2H), 7.84 (dd, 1H), 7.74-7.79 (m, 1H), 7.61 (t, 1H), 7.33 (d, 1H), 7.22 (dd, 1H), 2.30-2.35 (m, 3H), 1.76 (s, 6H) | 417 | 6-Fluoropyridin-3-amine and Method 20 |
| **199** | 5-{[3-(1-Cyano-1-methylethyl)benzoyl]amino}-2-methyl-*N-*[6-(methylamino) pyridin-3-yl]benzamide | 10.57-10.61 (s,1H), 10.43-10.48 (s, 1H), 8.48-8.56 (s, 1H), 8.04-8.09 (s, 1H), 7.90-8.01 (m, 3H), 7.70-7.85 (m, 2H), 7.61.(t, 1H), 7.33 (d, 1H); 7.04 (d,1H), 2.90-2.97 (s, 3H), 2.36-2.39 (s, 3H), 1.72-1.77 (s, 6H) | 427 | Method 93 and Method 20 |
| **200** | 2-Methyl-*N-*[6-(methylamino)pyridin-3-yl]-5-{[3-(trifluoromethyl) benzoyl]amino} benzamide | 10.48=10.74 (m,2H), 8.61-8.86 (s, 1H), 8.47-8.57 (s, 1H), 8.20-8.39 (m, 2H), 7.91-8.12 (m, 3H), 7.80 (dd, 2H), 7.34 (d, 1H), 7.09 (d, 1H), 2.86-3.01 (s, 3H), 2.32-2.41 (s, 3H) | 428 | Method 93 and Method 21 |
| **201** | *N-*(6- Acetylamino-pyridin-3-yl)-5-[3-(cyano-dimethyl-methyl)-benzoylamino]-2-methyl-benzamide | 10.65 (s, 1H), 10.55(s, 1H),10.43 (s, 1H), 8.73 (d, 1H), 7.89-8.30 (m, 5H), 7.83 (dd, 1H), 7.73-7.79 (m, 1H), 7.61 (t, 3N), 7.32 (d, 1H), 2.37 (s, 3H), 2.04-2.21 (m,3H), 1.56-1.91 (m, 6H) | 455 | *N*-(5-Aminopyridin-2-yl)acetamide and Method 20 |
| **202** | *N-*(6-Aminopyridin-3-yl)-5-{[3-(1-cyano-1-methylethyl)benzoyl]amino}-2-methylbenzamide | 10.60-10.65 (s, 1H), 10.45-10.49 (s, 1H), 8.54-8.61 (s, 1H), 7.91-8.16 (m, 6H), 7.77 (dd, 2H), 7.60 (t, 1H), 7.33 (d, 1H), 7.03 (d, 1H), 2.33-2.39 (s, 3H), 1.69-1.78 (s, 6H) | 413 | Pyridine-2,5-diamine and Method 20 |
| **203** | *N-*Methyl-5-[(2-methyl-5-{[3-(trifluoromethyl)benzoyl]amino}benzoyl)amino]nicotinamide | 10.76-10.83 (s, 1H), 10.59-10.63 (s, 1H), 8.99-9.00 (s, 1H), 8.75 (d, 2H), 8.63-8.66 (s, 1H), 8.16-8.37 (m, 2H), 7.90-8.08 (m, 2H), 7.74-7.90 (m, 2H), 7.35 (d, 1H), 2.71-2.75 (q, 3H), 2.37-2.39 (m, 3H) | 456 | Method 133 and Method 21 |
| **204** | *N-*(6-Amino-5-methylpyridin-3-yl)-5-{[3-(1-cyano-1-methylethyl)benzoyl]amino}-2-methylbenzamide | 10.58-10.60 (s, 1H), 10.43-10.46 (s, 1H), 8.40-8.47 (s, 1H), 8.03-8.10 (m, 1H), 7.86-8.01 (m, 3H), 7.71-7.87 (m, 4H), 7.63 (t, 1H), 7.33 (d,1H), 2.34-2.39 (s, 3H), 2.19-2.23 (s, 3H), 1.72-1.79 (s, 6H) | 427 | Method 94 and Method 20 |
| **205** | *N-*(5-Aminopyridin-3-yl)-5-{[3-(1-cyano-1-methylethyl)benzoyl]amino}-2-methylbenzamide | .10.97-11.07 (s, 1H), 10.50-10.53 (s, 1H), 8.38-8.41 (s, 1H), 8.05.-8.08 (s, 1H), 7.93-8.00 (m, 2.H), 7.73-7.90 (m, 2H), 7.62 (t, 1H), 7.38 (d, 1H), 2.35-2.39 (s, 3H), 1.74-1.79 (s, 6H) | 413 | Method 95 and Method 20 |
| **206** | 2-Methyl-*N-*(6-morpholin-4-ylpyridin-3-yl)-5-{[3-(trifluoromethyl) benzoyl]amino} benzamide | 10.55-10.65 (s, 1H), 10.32-10.45 (s, 1H), 8.37-8.45 (s, 1H), 8.24-8.36 (m, 2H), 7.90-8.09 (m, 3H), 7.76-7.86 (m, 2H), 7.36 (d, 1H), 7.16 (d, 1H), 3.75 (t, 4H), 3.49 (t, 4H), 2.32-2.40 (s, 3H) | 484 | Method 84 and Method 21 |
| **207** | 5-[(5-{[3-(1-Cyano-1-methylethyl)benzoyl]amino}-2-methylbenzoyl) amino]-*N-*methyl nicotinamide | 10.76-10.83 (s, 1H), 10.59-10.63 (s, 1H), 8.99-9.00 (s, 1H), 8.75 (d, 2H); 8.63-8.66 (s, 1H), 8.16-8.37 (m, 2H), 7.90-8.08 (m, 2H), 7.74-7.90 (m, 2H), 7.35 (d, 1N), 2.71-2.75 (q, 3H), 2.37-2.39 (m, 3H) | 456 | Method 133 and Method 20 |
| **208** | *N-*(3-{[(5-Bromopyridin-3-yl)amino]carbonyl}-4-methylphenyl)-2-(1-cyano-1-methyl ethyl) isonicotinamide | 10.80-10.86 (s, 1H), 10.65-10.73 (s, 1H), 8.79-8.87 (s, 2H), 8.50-8.55 (s, 1H), 8.46-8.49 (s, 1H), 8.00-8.05 (s, 1H), 7.80-7.94 (m, 3H), 7.36 (d, 1H), 2.36-2.43 (s, 3H),1.75-1.79 (s, 6H) | 478 | Method 119 and Method 29 |
| **209** | *N-*(5-Chloropyridin-3-yl)-5-{[3-(1-cyano-1-methylethyl)benzoyl]amino}-2-methylbenzamide | 10.80-10.85 (s, 1H), 10.44-10.49 . (s, 1H), 8.78-8.82 (s, 1H), 8.38-8.40 (s, 2H), 8.04-8.10 (s, 1H), 7.90-8.00 (m, 2H), 7.85 (d, 1H), 7.76 (d, 1H), 7.61 (t, 1H), 7.34 (d, 1H), 2.35-2.41 (s,3H), 1.72-1.78 (s, 6H) | 432 | 5-Chloropyridin-3-amine and Method 20 |
| **210** | *N-*[6-(Cyclopropylamino)pyridin-3-yl]-2-methyl-5-{[3-(trifluoromethyl) benzoyl]amino} benzamide | 10.62 (d, 2H), 8.57 (d, 1H), 8.30 (s, 1H), 8.27 (d, 1H), 7.99 - 8:05 (m, 1H), 7.96 (d, 2N), 7.78 - 7.83 (m, 1H), 7.77 (s, 1H), 7.33 (d, 1H), 7.05 (d, 1H), 2.64-2.60 (m, 1H), 2.35 (s, 3H), 0.89 (s, 2H), 0.56 - 0.65 (m, 2H) | 455 | Method 243 and Method 21 |
| **211** | 2-Chloro-*N-*[6-(cyclobutylamino)pyridin-3-yl]-5-{[3-(trifluoromethyl) benzoyl]amino} benzamide | 10.84 (d, 2H), 8.48 (d, 1H), 8.24 - 8.34 (m, 2H), 8.07 (d, 1H), 7.90 -8.02 (m,3H), 7.80 (t, 1H), 7.60 (d, 1H), 7.05 (d, 1H), 4.13 - 4.28 (m, 1H), 2.38 - 2.47 (m, 2H), 1.92 - 2.06 (m, 2H), 1.67 - 1.82 (m, 2H) | 490 | Method 244 and Method 224 |
| **212** | *N*-[6- (Cyclobutylamino)pyridin-3-yl]-2-methyl-5-{[3- (trifluoromethyl)benzoyl]amino} benzamide | 10.43 (s, 2H), 8.31 (d, 1H), 8.12 (s, 1H), 8.09 (d, 1H), 7.74 - 7.84 (m, 3H), 7.61 (t, 2H), 7.14 (d, 1 H), 6.83 (d, 1H), 3.95 - 4.09 (m, 1H); 2.20 - 2.29 (m, 2H), 2.16 (s, 3H), 1.73 - 1.87 (m, 2H), 1.48 - 1.63 (m, 2H) | 469 | Method 244 and Method 21 |
| **213** | 2-Chloro-*N-*[6-(isopropylamino)pyridin-3-yl]-5-{[3-(trifluoromethyl) benzoyl]amino} benzamide | 10.80 (d, 2H), 8.47 (d, 1H), 8.31 (s, 1H), 8.27 (d, 1H), 8.07 (d, 1H), 7.88-8.02 (m,3H), 7.80 (t, 1H), 7.60 (d, 1H), 7.03 (d, 1H), 3.92-3.88 (m, 1H), 1.22 (d, 6H) | 477 | Method 245 and Method 224 |
| **214** | *N-*[6-(Isopropylamino)pyridin-3-yl]-2-methyl-5-{[3-(trifluoromethyl)benzoyl]amino} benzamide | 10.54 (s, 1H), 10.01 (s, 1H), 8.22 - 8.32 (m, 3H), 7.97 (d, 1H), 7.76 - 7.85 (m, 3H), 7.68 (dd, 1H), 7.28 (d, 1H), 6.43 (d, 1H), 6.21 (d, 1H), 3.86 - 4.01 (m, 1H), 2.34 (s, 3H), 1.12 (d, 6H) | 466 | Method 245 and Method 21 |
| **215** | *N-*(6-Bromo pyridin-3-yl)-2-chloro-5-{[3-(trifluoromethyl)benzoyl]amino} benzamide | 10.99 (s, 1H), 10.81 (s, 1H), 8.72 (d, 1H), 8.25 - 8.38 (m, 2H), 8.17 - 8.25 (m, 1H), 8.04 - 8.14 (m, 1H), 7.93 - 8.01 (m, 1H), 7.71 - 7.86 (m, 2H), 7.63 (dd, 2H) | 499 | 6-bromopyridin-3-amine and Method 224 |
| **216** | 2-Chloro-*N-*(5-fluoropyridin-3-yl)-5-{[3-(trifluoromethyl)benzoyl]amino}benzamide | 11.11 (s, 1H), 10.78 (s, 1H), 8.69 (s, 1H), 8.25 - 8.40 (m, 3H), 8.17 (d, 1H), 8.06 (s, 1H), 7.98 (s, 2H), 7.80 (t, 1H), 7.61 (d, 1H) | 439 | Method 252 and Method 224 |
| **217** | *N-*(5-Fluoropyridin-3-yl)-2-Methyl-5-{[3-(trifluoromethyl)benzoyl]amino}benzamide | 10.84 (s, 1H), 10.58 (s, 1H), 8.70. (s, 1H), 8.24 - 8.35 (m, 3.14), 8.15 - 8.21 (m, 1H), 7.91 - 7.99 (m, 2H), 7.76 - 7.89 (m, 2H), 7.34 (d, 1H), 2.36 (s, 3H) | 418 | Method 252 and Method 21 |
| **218** | 2-Chloro-5-[(3,5-dimethylbenzoyl) amino]-*N-*(5-fluoropyridin-3-yl)benzamide | 11.06 (s, 1H), 10.44 (s, 1H), 8.68 (s, 1H), 8.36 (d, 1H), 8.11 - 8.22 (m, 1H), 8.06 (d, 1H), 7.95 (dd, 1 H), 7.54 - 7.61 (m, 3H), 7.24 (s, 1H), 2.35 (s, 6H) | 398 | Method 79 and 3,5-dimethylbenzoic acid |
| **219** | *N-*(5-Bromopyridin-3-yl)-2-chloro-5-[(3,5-dimethylbenzoyl) amino]benzamide | 11.00 (s, 1 H), 10.43 (s, 1H), 8.79 (d, 1H), 8.42 - 8.51 (m, 2H), 8.05 (d, 1H), 7.94 (dd, 1H), 7.51 - 7.61 (m, 3H), 7.23 (s, 1H), 2.35 (s, 6H) | 460 | Method 80 and 3,5-dimethylbenzoic acid |
| **220** | 5-[(3-Cyclopropylbenzoyl)amino]-*N-*(5-methoxypyridin-3-yl)-2-methylbenzamide | 10.69 (s, 1H), 10.28 (s, 1H), 8.59 (s, 1H), 8.14 (s, 1H), 7.94 (d, 2H), 7.81 (dd, 1H), 7.60 - 7.73 (m, 2H), 7.27 - 7.42 (m, 3H), 3.80-3.88 (m, 3H), 2.35 (s, 3H), 1.93 - 2.06 (m, 1H), 0.92.-1.03 (m, 2H); 0.69 -0.79 (m, 2H) | 402 | Method 69 and Method 34 |
| **221** | 2-Chloro-5-[(3-cyclopropyl benzoyl) amino]-*N-*(5-methoxypyridin-3-yl)benzamide | 10.91 (s, 1H), 10.46 (s, 1H), 8.54 (d, 1H), 8.16 (d, 1H), 8.05 (d, 1H), 7.89 - 7.96 (m, 2H), 7.62 - 7.74 (m, 2H), 7.57 (d, 1H), 7.40 (t, 1H), 7.28 - 7.34 (m, 1H), 3.84 - 3.88 (m, 3H), 1.98 - 2.06 (m, 1H), 1.00 (ddd, 2H), 0.72 - 0.83 (m, 2H) | 422 | Method 78 and Method 344 |
| **222** | 5-[(3-Cyclopropyl-5-fluorobenzoyl) amino]-*N-*(5-methoxypyridin-3-yl)-2-methyl benzamide | 10.93 - 11.07 (m, 1H), 10.42 (d, 1 H), 8.69 - 8.82 (m, 1H), 8.32 (dd, 1H), 8.10 (d, 1H), 7.98 (d, 1H), 7.76-7.87 (m, 1H), 7.46-7.56 (m, 2H), 7.33 (d, 1H), 7.16 (d, 1H), 3.91 (s, 3H); 2.36 (s, 3H), 2.04 (ddd, 1H), 0.97-1.07 (m, 2H), 0.76-0.87 (m, 2H) | 420 | Method 69 and Method 226 |
| **223** | *N-*[6*-* (Acetylamino)pyridine-3-yl]-2-methyl-5-{[4-[(4-methylpiperazin-1-yl)methyl]-3-(trifluoromethyl) benzoyl]amino} benzamide hydrochloride | 10.50 (s, 1H), 10.45 (s, 2H), 9.50 (s, br, 1H), 8.70 (s, 1H), 8.30 (m, 2H), 7.85-8.05 (m, 5H), 7.30 (d, 1H), 3.75 (s, 2H), 3.40 (m, 2H), 3.05 (m, 2H), 2.90 (m, 2H), 2.80 (s, 3H), 2.40 (m, 2H), 2.35 (s, 3H), 2.10 (s, 3H) | 568 | Method 35 and Methods 70 |
| **224** | *N-*[6- (Acetylamino)pyridine-3-yl]-5-{[4-[(dimethylamino)methyl]-3-(trifluoromethyl) benzoyl]amino}-2-methylbenzamide hydrochloride | 10.70 (s, 1H), 10.50 (m, 2H), 9.90 (brs, 1H), 8.75 (s, 1H), 8.40 (m, 2H), 7.90-8.10 (m, 5H), 7.40 (m, 1H), 4.60 (s, 2H), 2..90 (s, 6H), 2.42 (s, 3H), 2.19 (s, 3H) | 513 | Method 23 and Method 70 |
| **225** | *N-*[3-({[6- (Acetylamino)pyridine-3-yl]amino}carbonyl)-4-methylphenyl]-2-[(dimethylamino) methyl]-5-(trifluoromethyl) benzamide hydrochloride | 1-1.02 (s, 1H), 10.60 (m, 2H), 9.70 (brs, 1H), 8.78 (s, 1H), 8.00-8.29 (m, 6H), 7.80 (d, 1H), 7.40 (d, 1H), 4.56 (s, 2H), 2.86 (s, 6H), 2.45 (s, 3H), 2.15 (s, 3H) | 513 | Method 36 and Method 70 |
| **226** | N-[3-({[6-(Acetylamino) pyridin-3-yl]amino}carbonyl)-4-methylphenyl]-1,3,3-trimethyl-2-oxoindoline-5-carboxamide | 10.53 (s, 1H), 10.48 (s, 1 H), 10.16 (s, 1H), 8.68 (s, 1H), 8.00, - 8.10 (m, 2H), 7.95-8.00 (m, 2H), 7.89 (s, 1H), 7.84 (d, 1H), 7.29 (d, 1H), 7.15 (d, 1H), 3.18 (s, 3H), 2.34 (s, 3H), 2.07 (s, 3H), 1.31 (s, 6H) | 485 | Method 39 and Method 70 |
| **227** | N-[6-(Acetylamino)pyridin-3-yl]-2-methyl-5-{[3-(4-methyl-1*H-*imidazol-1-yl)-5-(trifluormethyl) benzoyl]amino}benzamide | 10.66 (s, 1H), 10.43 - 10.50 (m, 214),-9.52 (s, 1H), 8.67 (s, 1H), 8.58 (s, 1H), 8.43 (d, 2H), 8.12 (s, 1H), 8.04 (s, 2H), 7.82- 7.88 (m, 2H), 7.36 (d, 1H), 2.38 (s, 3H), 2.33 (s, 3H), 2.07 (s, 3H) | 536 | Method 201 and Method 70 |
| **228** | *N*-[6-(Acetylamino)pyridin-3-yl]-5-{[3-(2,3-dihydroxy-1,1-dimethy]propyl)benzoyl]amino}-2-methylbenzamide | 8.65 (s, 1H), 8.10 (s, 2H), 7.97 (s, 1H), 7.89 (s, 1H), 7.78 (d, 1H), 7.67 (t, 2H), 7.47 (t, 1H), 7.33 (d, 1H), 3.80 (d, 1H), 3.24 (d, 2H), 2.46 (s, 3H), 2.19 (s, 3H), 1.40 (d, 6H) | 490 | Method 43 and Method 70 |
| **229** | *N-*[6-(Acetylamino)pyridin-3-yl]-5-{[3-(1-cyclopropyl-1-methylethyl)benzoyl]amino}-2-methylbenzamide | 10.47 (s, 2H), 10.29 (s, 1H), 8.68 (s, 1H), 8.06 (s, 2H), 8.00. (s, 1H), 7.91 (s, 1H), 7.81 (t, 2H), 7.69 (d, 1H), 7.46 (dd, 1H), 7.30 (d, 1H), 2.36 (s, 3H), 2.09 (s, 3H), 1.21 (s, 6H), 0.99-1.12 (m, 1H), 0.40 (d, 2H), 0.30 (d, 2H) | 470 | Method 44 and Method 70 |
| **230** | *N-*[6-(Acetylamino) pyridin-3-yl]-5-{[3-(1,1-dimethyl propyl)benzoyl]amino}-2-methy]benzamide | 10.56 (s, 1H), 10.50 (s, 1H), 10.32 (s, 1H), 8.71 (s, 1H), 8.00 - 8.13 (m, 2H), 7.86 - 7.94 (m, 2H), 7.81 (dd, 2H), 7.56 (d, 1H), 7.46 (t, 1H), 7.31 (d, 1H), 2.37 (s, 3H), 2.09 (s, 3H), 1.67 (q, 2H), 1.31 (s, 6H), 0.64 (t, 3H) | 458 | Method 167 and Method 70 |
| **231** | *N-*[6-(Acetylamino) pyridin-3-yl]-5-{[3-(1-cyclopropyl-1-hydroxyethyl)benzoyl]amino}-2-methylbenzamide | 8.55 (s, 1H), 8.03 (s, 1H), 7.98 (s, 1H), 7.81 (s, 1H), 7.63 - 7.73 (m, 2H), 7.58 (d, 1H), 7.37 (t, 1H), 7.21 (d, 1H), 2.35 (s, 3H), 2.09 (s, 3H), 1.42 (s, 3H), 1.14 -1.24 (m, 2H), 0.28 - 0.43 (m, 4H) | 472 | Method 46 and Method 70 |
| **232** | *N-*[6-(Acetylamino) pyridin-3-yl]-5-({3-[cyclopropyl (hydroxy) methyl]benzoyl}amino)-2-methylbenzamide | 10.56 (s, 1H), 10.51 (s, 1H), 10.34 (s, 1H), 8.69 (s, 1H), 8.00 - 8.11 (m, 2H), 7.97 (s, 1H), 7.92 (s, 1H), 7.78-7.86 (m, 2H), 7.58 (d, 1H), 7.47 (t, 1H), 7.29 (d, 1H), 4.04 (d, 1H), 2.34 (s, 3H), 2.07 (s, 3H), 1.00-1.13 (m, 1H), 0.34 - 0.49 (m, 4H) | 458 | Method 48 and Method 70 |
| **233** | 5-[(3-Chlorobenzoyl)amino]-*N-*(5-methoxypyridin-3-yl)-2-methylbenzamide | 10.56 (s, 1H), 10.49 (s, 1H), 8.64 (d, 1H), 8.00 - 8.12 (m, 5H), 7.92 (dd, 2.17 Hz, 1H), 7.58 - 7.71 (m, 2H), 7.34 (d, Hz, 1H), 3.92 (s, 3H), 2.43 (s, 3H) | 396 | Method 69 and 3-chlorobenzoic acid |
| **234** | 5-[(3,5- . Dimethylbenzoyl) amino]-*N-*(5-methoxypyridin-3-yl)-2-methylbenzamide | 10.51 (s, 1H), 10.24 (s, 1H), 8.61 (d, 1H), 8.06 (d, 2H), 7.99 (d, 2H), 7.89 (dd, 1H), 7.63 (s, 2H), 7.27 (d, 1H), 7.19 (s, 1H), 3.87 (s, 3H), 2.38 (s, 3H), 2.32 (s, 6H) | 390 | Method 69 and 3,5-dimethylbenzoic acid |
| **235** | 5-[(3,4-Dimethylbenzoyl) amino]-*N*-(5-methoxypyridih-3-yl)-2-methylbenzamide | 10.55 (s, 1H), 8.65 (d, 1H), 8.11 (s, 1H), 8.10 (s, 1H), 8.05 (t, 1H), 7.94 (dd, 1H), 7.86 (s, 1H), 7.82 (d, 1H), 7.81 (dd, 2H), 3.92 (s, 3H), 2.42 (s, 3H), 2.32 (s,6H) | 390 | Method 69 and 3,4-dimethybenzoic acid |
| **236** | 5-[(3,4-Dichlorobenzoyl)amino]-*N*-(5-methoxypyridin-3-yl)-2-methyl benzamide | 10.55 (s, 1H), 10.75 (s, 1H), 8.82 (d, 1H), 8.49 (d, 1H), 8.21-8.80 (m, 4H), 8.10.(dd, 1H), 8.03 (d, 1H), 7.62 (d, 1H), 4.10 (s, 3H), 2.61 (s, 3H) | 430 | Method 69 and 3,4-dichlorobenzoic acid |
| **237** | 5-[(3,5-Dichlorobenzoyl)amino]-*N-*(5-methoxypyridin-3-yl)-2-methylbenzamide | 10.56 (s, 2H), 8.63 (s, 1H), 8.10 (d, 1H), 8.06 - 8.07 (m, 2H), 8.02 - 8.04 (m, 2H), 7.90 (dd, 1H), 7.84 (t, 1H), 7.83 (d, 1H), 3.92 (s, 3H), 2.43 (s, 3H) | 430 | Method 69 and 3,5-dichlorobenzoic acid |
| **238** | 5-[(3-Chloro-5-fluorobenzoyl) amino]-*N-*(5-methoxypyridin-3-yl)-2-methylbenzamide | 10.57 (s 1H), 10.55 (s, 1H), 8.65 (d, 1H), 8.10 (dd, 2H), 8.03 (d, 1H), 7.99 (s, 1H), 7.83 - 7.92 (m, 2H), 7.64 - 7.69 (m, 1H), 7.34 (d, 1H), 7.34 (d, 1H), 3.92 (s, 3H), 2.44 (s, 3H) | 414 | Method 69 and 3-chloro-5-fluorobenzoic acid |
| **239** | 5-[(4-Methoxy-3-methylbenzoyl) amino]-*N-*(5-methoxypyridin-3-yl)-2-methylbenzamide | 10.55 (s 1H), 10.15 (s, 1H), 8.62 (d, 1H), 8.02 - 8.10 (m, 3H), 7.86 (dd, 1H), 7.65 (d, 1H), 7.81 (d, 1 H), 6.82 - 6.90 (m, 2H), 3.91 (s, 3H), 3.86 (s, 3H), 2.45 (s, 3H), 2.41 (s, 3H) | 406 | Method 69 and 3-methyl-4-methoxybenzoic acid |
| **240** | 5-{[3-Fluoro-5-(trifluoromethyl) benzoyl]amino}-*N-*(5-methoxypyridin-3-yl)-2-methylbenzamide | 10.70 (s, 1H), 10.57 (s, 1H), 8.63 (s, 1H), 8.29 (s, 1H), 8.20 (d, 1H), 8.11 (d, 1H), 8.06 (d, 1H), 8.02 - 8.04 (m, 1H), 7.95 (d, 1H), 7.90 (dd, 1H), 7.35 (d, 114), 3.92 (s, 3H), 2.44 (s, 3H) | 448 | Method 69 and 3-methyl-5-trifluoromethylbenzoic acid |
| **241** | *N-*(5-Methoxypyridin-3-yl)-2-methyl-5-[(3-methylbenzoyl)amino]benzamide | 10.55 (s, 1H), 10.33 (s, 1H), 8.65 (s, 1 H), 8.11 (s, 2H), 8.04 (s, 1H), 7.94 (dd, 1H), 7.86 - 7.89 (m, 2H), 7.43 (d, 2H), 7.32 (d, 1H), 3.92 (s, 3H), 2.43 (s, 3H), 2.41 (s, 3H) | 376 | Method 69 and 3-methylbenzoic acid |
| **242** | 2-Methoxy-*N-*pyridin-3-yl-5-{[3-(trifluoromethyl) benzoyl]amino} benzamide | 10.52 (s, 1H), 10.35 (s, 1H), 8.87 (d, 1H), 8.26 - 8.31 (m, 3H), 8.20 (d, 1H), 8.06 (d, 1H), 7.96 - 8.00 (m, 2H), 7.78 (t, 1H), 7.39 (dd, 1H), 7.23 (d, 1H), 3.92 (s, 3H) | 416 | Method 71 and 3-trifluoromethylbenzoic acid |
| **243** | *N-*(5-Aminopyridin-3-yl)-2-chloro-5-{[3-(trifluoromethyl)benzoyl]amino} benzamide | 10.70 (s, 1H), 10.48 (s, 1H), 8.31 (s, 2H), 7.92 - 8.02 (m, 4H), 7.76 - 7.84 (m, 1H), 7.68 (d, 1H), 7.55 - 7.62 (m, 114), 7.49 (t, 1H), 5.40 (s, 2H) | 435 | Method 95 and Method 224 |
| **244** | *N*-(5- Aminopyridin-3-yl)-2-methyl-5-{[3-(trifluoromethyl)benzoyl]amino} benzamide | 10.98 (s, 1H), 10.61 (s, 1H), 8.37 (s, 1H), 8.28 (s, 2H), 7.89 - 8.01 (m, 3H), 7.76-7.89 (m, 4H), 7.35 (d, 1H), 6.59 (s, 1H), 1.98 (s, 3H) | 415 | Method 95 and Method 21 |
| **245** | 2-Chloro-*N-*[6-(cyclopropylamino)pyridin-3-yl]-5-{[3-(trifluoromethyl) benzoyl]amino} benzamide | 10.88 (s, 1H), 10.76 (s, 1H), 8.55 (s, 1H), 8.24 - 8.32 (m, 2H), 8.09 (d, 1H), 7.88-8.02 (m, 3H), 7.81 (t, 1H), 7.61 (d, 1H), 7.06 (d, 1H), 2.60-2.69 (m, 1H), 0.91 (d, 2H), 0.56 - 0.70 (m, 2H) | 476 | Method 243 and Method 224 |
| **246** | *N-*(6-Amino-5-chloropyridin-3-yl)-5-({3-[(2-cyano-1*H*-pyrrol-1-yl)methyl]benzoyl}amino)-2-methylbenzamide | 10.38 (s, 1H), 10.26 (s, 1H), 8.24 (d, 1H), 8.04 (d, 1H), 7.91 (d, 1H), 7.85 (s, 1H), 7.72 - 7.81 (m, 2H), 7.55 (t, 1H), 7.42 (dd, 2H), 7.36 (d, 1H), 7.29 (d, 1H), 7.01 (dd, 1H), 6.28 (dd, 1H), 5.32 (s, 2H), 2.34 (s, 3H) | 487 | Method 81 and Method 228 |
| **247** | 2-Methyl-5-{[3-(trifluoromethyl)b enzoyl]amino}-*N-*[6-(trifluoromethyl)pyridin-3-yl]benzamide | 10.70 (s, 1H), 10.61 (s, 1H), 8.21 - 8.45 (m, 3H), 7.90 - 8.11 (m, 4H), 7.80 (t, 1H), 7.58 (d, 1H), 7.37 - 7.43 (m, 1H) | 468 | 6-(trifluoromethyl) pyridin-3-amine and Method 21 |
| **248** | 5-[(3-Chloro-4-methoxybenzoyl) amino]-2-methoxy-*N-*pyridin-3-ylbenzamide | 10.34 (s, 1H), 10.24 (s, 1H), 8.87 (d, 1H), 8.30 (dd, 1H), 8.20 (d, 1H), 8.10 (d, 1H), 8.04 (d, 1H), 7.96 (ddd, 2H), 7.39 (dd, 1H), 7.29 (d, 1H), 7.20 (d, 1H), 3.94 (s, 3H), 3.91 (s, 3H) | 412 | 3-chloro-4-methoxybenzoic acid and Method 71 |
| **249** | 2-Chloro-5-{[3-(1-cyano-1-methyl ethyl)benzoyl]amino)-*N-*(6-hydroxypyridin-3-yl)benzamide | 10.56 (s, 1H), 10.34 (s, H), 8.06 (s, 1H), 7.89-8.02 (m, 4H), 7.77 (d, 1H), 7.64 (d, 1H), 7.52 - 7.61 (m, 2H), 6.40 (d, 1H), 1.75 (s, 6H) | 437 | Method 214 and Method 223 |
| **250** | *N-*(6-Amino-5-chloropyridin-3-yl)-5-{[3-(1-cyano-1-methyl ethyl)-5-fluorobenzoyl] amino}-2-methylbenzamide | 10.55 (s, 1H), 10.49 (s, 1H), 8.38 (d, 1H), 8.17 (d, 1H), 7.96 (s, 1H), 7.88 (s, 1H), 7.76-7.86 (m, 2H), 7.64 (d, 1H), 7.33 (d, 1H), 2.36 (s, 3H), 1.76 (s, 6H) | 468 | Method 229 and Method 81 |
| **251** | 5-{[3-(1-Cyano-1-methylethyl)-5-fluorobenzoyl]amino}-*N-*(5-isopropoxypyridin -3-yl)-2-methyl benzamide | 10.77 (s, 1H), 10.51 (s, 1H), 8.63 (s, 1H), 8.12 (s, 1H), 7.91 -7.99 (m, 3H), 7.78 - 7.84 (m, 2H), 7.65 (d, 1H), 7.35 (d, 1H), 4.56 - 4.78 (m, 1H), 2.37 (s, 3H), 1.72 (s, 6H), 1.32(d, 6H) | 476 | Method 72 and Method 229 |
| **252** | 2-Chloro-5-{[3-(1-cyano-1-methylethyl)benzoyl]amino}-*N-*(6-methylpyridin-3-yl)benzamide | 10.68 (s, 1H), 10.55 (s, 1H), 8.72 (d, 1H), 7.99 - 8.05 (m, 3H), 7.93 - 7.96 (m, 2H), 7.75-7.78 (m, 1H), 7.56-7.64 (m, 2H), 7.25 (d, 1H), 2.43 (s, 3H), 1.74 (s, 6H) | 433 | 3-Amino-6-picoline and Method 223 |
| **253** | *N-*{5-[(Dimethylamino)sulfonyl]pyridin-3-yl}-2-methyl-5-{[3-(trifluoromethyl) benzoyl]amino} benzamide | 10.95 (s, 1H), 10.58 (s, 1H), 9.14 (d, 1H), 8.57 - 8.64 (m, 2H), 8.23 3 - 8.32 (m, 2H), 7.96 - 7.99 (m, 2H), 7.76-7.86 (m, 2H), 7.35 (d, 1H), 2.70 (s, 6H), 2.38 (s, 3H) | 507 | Method 185 and Method 21 |
| **254** | 2-Chloro-*N-*(5-chloro-6-methylpyridin-3-yl)-5-{[3-(1-cyano-1-methylethyl)benzoyl]amino} benzamide | 10.97 (s, 1H), 10.61 (s, 1H), 8.69 (d, 1H), 8.32 (d, 1H), 8.02 - 8.10 (m, 2H), 7.90-8.01 (m, 2H), 7.78 (d, 1H), 7.57-7.69 (m, 2H), 2.53 (s, 314), 1.75 (s, 6H) | 469 | Method 212 and Method 223 |
| **255** | 5-{[3-(1-Cyano-1-methylethyl) benzoyl]amino}-*N-*(6-{[2-(dimethylamino)ethyl]amino}pyridin-3-yl)-2-methylbenzamide | 10.40 (s, 1H), 10.12 (s, 1H), 8.40 (s, 1H), 8.06 (s, 1H), 7.89-7.98 (m, 2H), 7.72 - 7.79 (m, 2H), 7.52-7.75 (m, 2H), 7.30 (d, 1H), 7.14 (t, 1H), 6.70 - 6.81 (bs, 1H), 6.59 (d, 1H), 3.56 - 3.61 (m, 2H), 3.24 (t, 2H), 2.84 (s, 6H), 2.35 (s, 3H), 1.75 (s, 6H) | 485 | Method 189 and Method 20 |
| **256** | *N-*(3-{[(6-Amino-5-chloropyridin-3-yl)amino]carbonyl}-4-methylphenyl)-2-(1-cyano-1-methyl ethyl) isonicotinamide | 10.67 (s, 1H), 10.37 (s, 1H), 8.82 (s, 1H), 8.30 (s, 1H), 8.09 (s, 1H), 8.02 (s, 1H), 7.84 -7.93 (m, 2H), 7.81 (d, 1H), 7.34 (d, 1N), 2.32 (s, 3H), 1.77 (s, 6H) | 451 | Method 186 and Method 29 |
| **257** | *N-*(6-Amino-5-chloropyridin-3-yl)-5-{[3-(1-cyanoethyl) benzoyl]amino}-2-methylbenzamide | 10.42 (s, 1H), 10.36 (s, 1H), 8.27 (s, 1H), 8.04 (s, 1H), 7.99 (s, 1H), 7.95 (d, 1H), 7.88(s, 1H), 7.81 (d, 1H), 7.49 - 7.72 (m, 3H), 7.31 (d, 1H), 4.07-4.21 (q, 1H), 2.35 (s, 3H), 1.61 (d, 3H) | 436 | Method 81 and 3-(1-cyanoethyl)benzoic acid |
| **258** | *N-*(6-Amino-5-chloropyridin-3-yl)-2-methyl-5-{[3-(2-methyl-1,3-thiazol-5-yl)benzy] amino}benzamide | 10.50 (s, 1H), 10.48 (s, 1H), 8.51 (s, 1H), 8.36 (s, 1H), 8.11-8.20 (m, 2N), 8.08 (s, 1H), 7.95 (s, 1H), 7.90 (d, 1H), 7.83 (d, 1H), 7.65-7.75 (m, 2H), 7.61 (t, 1H), 7.32 (d, 1H), 2.75 (s, 3H), 2.36 (s, 3H) | 480 | Method 81 and 3-(2-methyl-1,3-thiazol-5-yl)benzoic acid |
| **259** | *N-*(6-Amino-5-chloropyridin-3-yl)-5-{[3-(1-cyano-1,2-dimethylpropyl)benzoyl]amino}-2-methylbenzamide | 10.39 (s, 1H), 10.28 (s, 1H), 8.24 (s, 1H), 8.04 (s, 1H), 8.01 (s, 1H), 7.96 (d, 1H), 7.86 (s, 1H), 7.82 (d, 1H), 7.70 (d, 1H), 7.62 (t, 1H), 7.31 (d, 1H), 2.35 (s, 3H), 2.23 - 2.33 (m, 1H), 1.72 (s, 3H), 1.08 (d, 3H), 0.77 (d, 3H) | 478 | Method 81 and Method 203 |
| **260** | *N-*(6-Amino-5-chloropyridin-3-yl)-5-{[3-(1-cyano-1-methylpropyl)benzoyl]amino}-2-methylbenzamide | 10.40 (s, 1H), 10.28 (s, 1H), 8.25 (s, 1H), 8.04 (s, 1H), 8.02 (s, 1H), 7.96 (d, 1H), 7.91 (s, 1H), 7.82 (d, 1H), 7.71 (d, 1H), 7.61 (t, 1H), 7.31 (d, 1H), 2.35 (s, 3H), 2.04 (q, 2H), 1.73 (s, 3H), 0.88 (t, 3H) | 464 | Method 81 and Method 204 |
| **261** | *N-*(6-Amino-5-chloropyridin-3-yl)-5-{[3-(1-cyano-2-hydroxy-1-methylethyl)benzoyl]amino}-2-methylbenzamide | 10.43 (s, 1H), 10.39 (s, 1H), 8.29 (s, 1H), 8.09 (s, 1H), 8.00 (s, 1H), 7.96 (d, 1H), 7.93 (s, 1H), 7.81 (dd, 1H), 7.72 (d, 1H), 7.60 (t, 1H), 7.31 (d, 1H), 4.14 (d, 2H), 2.36 (s, 3H), 1.70 (s, 3H) | 466 | Method 81 and Method 205 |
| **262** | *N-*(6-Amino-5-methylpyridin-3-y])-2-chloro-5-{[3-(1-cyano-1-methylethyl)benzoyl]amino}benzamide | 10.76 (s, 1H), 10.60 (s, 1H), 8.42 (s, 1H), 8.02-8.09 (m, 2H), 7.96 (d, 1H), 7.89 (dd, 1H), 7.83 (s, 1H), 7.77 (d, 1H), 7.56-7.68 (m, 3H), 2.20 (s, 3H), 1.75 (s, 6H) | 450 | Method 192 and Method 223 |
| **263** | *N-*(5-)Bromo-6-chloropyridin-3-yl)-2-chloro-5-{[3-(trifluoromethyl) benzoy]amino} benzamide | 11.17 (s, 1H), 10.79 (s, 1H), 8.69 (dd, 2H), 8.19 - 8.47 (m, 2H), 7.91 8.20 (m, 3H), 7.84 (t, 1H), 7.65 (d, 1H) | 534 | 5-bromo-6-chloropyridin-3-amine and Method 224 |
| **264** | 2-Chloro-*N-*(2,6-dimethylpyridin-3-yl)-5-{[3-(trifluoromethyl)benzoyl]amino)benzamide | 10.61 (s, 1H), 10.57 (s, 1H), 8.50 (d, 1H), 8.30 - 8.39 (m, 1H), 7.80 - 8.11 (m, 5H), 7.64 (d, 1H), 7.10 (s, 1H), 2.51 (s, 3H), 2.48 (s, 3H) | 448 | 2,6-dimethylpyridin-3-amine and Method 224 |
| **265** | *N-*(6-Amino-4-methylpyridin-3-yl)-2-chloro-5-{[3-(trifluoromethyl) benzoyl]amino} benzamide | 10.78 (s, 1H), 10.54 (s, 1H), 8.40-8.49 (m, 2H), 7.83-7.77 (m, 5H), 7.65-7.71 (m, 2H), 7.10 (s, 1H), 2.81 (s, 3H) | 449 | 4-methylpyridin-2,5-diamine and Method 224 |
| **266** | 2-Chloro-*N*-(4-methylpyridin-3-yl)-5-{[3-(trifluoromethyl)benzoyl]amino}benzamide | 10.65 (s, 1H), 10.46 (s, 1H), 8.42-8.54 (m,3H), 7.41- 7.79 (m, 5H), 7.12-7.31 (m, 2H), 7.10 (s, 1H), 2.84 (s,3H) | 434 | 4-methylpyridin-5-diamine and Method 224 |
| **267** | 2-Chloro-*N*-(2-fluoropyridin-3-yl)-5-{[3-(trifluoromethyl)benzoyl]amino benzamide | 10.73(s, 1H), 10.61-10.69 (m, 1H), 8.21 - 8.45 (m, 3H), 7.90 - 8.11 (m, 4H), 7.82 (t, 1H), 7.59 (d, 1H), 7.37-7.50 (m, 1H) | 438 | 6-fluoropyridin-5-diamine and Method 224 |
| **268** | (R)-2-Chloro-*N*-{6-[(2,3-dihydroxypropyl) amino]pyridin-3-yl}-5-{[3-(trifluoromethyl)benzoyl]amino}benzamide | 10.01 (s, 2H), 9.72 (s, 1H), 8.43 (s, 114), 7.64.- 8.01 (m, 6H), 7.21 - 7.47 (m, 3H), 3.88 (m, 1H), 3.79 - 3.85 (m, 4H) | 509 | Method 246 and Method 224 |
| **269** | 2-Chloro-*N*-(6-{[2-hydroxy-1-(hydroxymethyl) ethyl]amino}pyrid in-3-yl)-5-{[3-(trifluoromethyl) benzoyl]amino}benzamide | 9.98 (s, 2H), 9.64 (s, 1H), 8.50 (s, 1H), 7.84-8.01 (m, 4H), 7.62 - 7.89 (m, 3H), 7.56 (t, 1H), 7.41 (d, 1H), 3.88 (1H,m) 3.54-3.87 (4H, m) | 509 | Method 247 and Method 224 |
| **270** | *N*-[6-(Acetylamino)-4-methylpyridin-3-yl]-2-chloro-5-{[3-(trifluoromethyl)benzoyl]amino} benzamide | 10.01 (s, 2H), 9.70 (s, 1H), 9.32 (s, 1H), 8.55 (s, 1H), 7.67 - 8.00 (m, 6H), 7.31-7.49 (m,2H), 2.83 (s, 3H), 2.01 (s, 6H) | 490 | *N*-(5-amino-4-methylpyridin-2-yl)acetamide and Method 224 |
| **271** | 2-Chloro-*N*-(2-fluoro-6-methylpyridin-3-yl)-5-{[3-(trifluoromethyl) benzoyl]amino} benzamide | 1H NMR (300 MHz) 10.79 (s, 1H), 10.58 (s, 1H), 8.55 (d, 1H), 8.31 - 8.41 (m, 1H), 7.83 - 8.16 (m, 5H), 7.65 (d, 1H), 7.10 (s, 1H), 2.87 (s, 3H) | 451 | 2-fluoro-6-methylpyridin-3-amine and Method 224 |
| **272** | 2- Chloro-*N*-[6-(4-methylpiperazin-1-yl)pyridin-3-yl]-5-{[3-(trifluoromethyl) benzoyl]amino} benzamide | 10.50 (s, 2H), 9.41 (s, 1H), 8.59 (s, 1H), 7.91 - 8.17 (m, 4H), 7.70 - 7.82 (m, 2H), 7.58 (t, 1H), 7.11 - 7.32 (m, 2H), 3.76 - 3.88 (m, 4H), 3.11 - 3.30 (m, 4H), 2.22 (s, 3H), 1.76 (s, 6H) | 517 | Method 190 and Method 224 |
| **273** | *N*-[5-(Hydroxymethyl) pyridin-3-yl]-2-methyl-5-{[3-(trifluoromethyl)benzoyl]amino} benzamide | | 430 | Method 21 and Method 242 |
| **274** | 2-Chloro-*N*-[6-(1H-pyrazol-5-ylamino)pyridin-3-yl]-5-{[3-(trifluoromethyl) benzoyl]amino} benzamide | 10.85 (s, 1H), 10.50 (s, 1H), 8.52 (s, 1H), 8.24 (s, 1H), 8.13 (s, 1H), 7.96 - 8.10 (m, 3H), 7.77 - 7.91 (m, 3H), 7.60 - 7.73 (m, 2H), 7.40 (d, 1H), | 501 | Method 191 and Method 224 |
| **275** | *N*-{6-[(4-Aminobutyl)amino]pyridin-3-yl}-2-chloro-5-{[3-(trifluoromethyl)benzoyl]amino} benzamide | 10.70 (s, 1H), 10.60 (s, 1H), 8.48 (s, 1H), 8.00 - 8.10 (m, 2H), 7.86 - 7.97 (m, 3H), 7.77 (d, 1H), 7.50 - 7.67 (m, 2H), 7.01 (d, 1H), 3.27 - 3.56 (m, 2H), 3.10 - 3.21 (m, 2H), 1.74 - 2.04 (m, 4H), 1.76 (s, 6H) | 505 | Method 248 and Method 224 |
| **276** | 5-{[3-(1-Cyano-1-methylethyl)benzoyl]amino}-2-methyl-*N-*(6-piperazin-1-ylpyridin-3-yl)benzamide | 10.52 (s, 2H), 9.42 (s, 1H), 8.60 (s, 1H), 7.91 - 8.22 (m, 4H), 7.70 - 7.85 (m, 2H), 7.61 (t, 1H), 7.09 - 7.47 (m, 2H), 3.77 - 3.88 (m, 4H), 3.11 - 3.34 (m, 4H), 2.36 (s, 3H), 1.76 (s, 6H) | 483 | Method 249 and Method 20 |
| **277** | *N*-[6-(Acetylamino)pyridin-3-yl]-2-methyl-5-{[3-(trifluoromethyl)benzoyl]amino} benzamide | 10.49 (d, 3H), 8.64 (d, 1H), 8.17 - 8.30 (m, 2H), 7.88-8.09 (m, 3H), 7.85 (d, 1H), 7.68 -.7.81 (m,2H), 7.26 (d, 1H), 2.30 (s, 3H), 2.03 (s, 3H) | 457 | *N*-(5-aminopyridin-2-yl)acetamide and Method 21 |
| **278** | *N*-[6-(Acetylamino) pyridin-3-yl]-2-chloro-5-{[3-(1-cyano-1-methylethyl)benzoyl]amino}benzamide | 10.72 (s, 1H), 10.58 (s, 1H), 10.53 (s, 1H), 8.67 (s, 1H), 8.06 (d,3H), 8.02 (d, 1H), 7.92 - 7.99 (m, 2H), 7.77 (d, 1H), 7.56 - 7.67 (m, 2H), 2.07 - 2.11 (m, 3H), 1.76 (s, 6H) | 476 | *N*-(5-aminopyridin-2-yl)acetamide and Method 223 |
| **279** | 2-Chloro-*N*-[5-(1*H*-pyrrol-1-yl)pyridin-3-yl]-5-{[3-(trifluoromethyl)benzoyl]amino}benzamide | 11.00 (s, 1H), 10.76 (s, 1H), 8.26 - 8.39 (m, 3H), 8.08 (d, 1H), 7.94 - 8.03 (m, 2H), 7.82 (t, 1H), 7.63 (d, 1H), 7.39 - 7.42 (m, 2H), 6.35 (t, 2H) | 485 | Method 202 and Method 224 |
| **280** | *N*-(5,6-Diaminopyridin-3-yl)-2-methyl-5-{[3-(trifluoromethyl) benzoyl]amino}benzamide | 10.59 (s, 1H), 10.47 (s, 1H), 8.24 - 8.35 (m, 2H), 8.00 (d, 1H), 7.88 (s, 2H), 7.74 - 7.85(m, 2H), 7.47 (s, 2H), 7.33 (d, 1H), 7.29 (s, 1H), 2.34 (s, 3H) | 430 | Method 196 and Method 21 |
| **281** | *N*-(6-Amino-5-chloropyridin-3-yl)-2-chloro-5-{[3-(trifluoromethyl) benzoyl]amino} benzamide | 10.44 (s, 1H), 10.41 (s, 1H), 8.33 (s, 1H), 8.11 (s, 1H), 8.05 (s, 1H), 7.96 (d, 1H), 7.90 (s, 1H), 7.74 - 7.82 (m, 2H), 7.61 (t, 1H), 7.31 (d, 1H), | 469 | Method 186 and Method 224 |
| **282** | *N*-(6-Amino-5-bromopyridin-3-yl)-2-chloro-5-{[3-(trifluoromethyl) benzoyl]amino} benzamide | 10.74 (s,1H), 10.51 (s, 1H), 8.25 - 8.33 (m, 3H), 8.16 (s, 1H), 7.91 - 8.03 (m, 3H), 7.81 (t, 1H), 7.59 (d, 1H) | 515 | Method 187 and Method 224 |
| **283** | 2-Chloro-*N*-(5-chloropyridin-3-yl)-5-{[3-(1-cyano-1-methyl ethyl)benzoyl] amino}benzamide | 11.06 (s, 1H), 10.60 (s, 1H), 8.77 (s, 1H), 8.42 (s, 1H), 8.36 (s, 1H), 8.03 - 8.07 (m, 2H), 7.94-8.01 (m, 2H), 7.78 (d, 1H), 7.56 - 7.68 (m, 2H), 1.76 (s, 6H) | 455 | Method 215 Method 223 |
| **284** | 5-{[3-(1-Cyano-1-methylethyl) benzoyl]amino}-*N*-(5,6-dimethylpyridin-3-yl)-2-methylbenzamide | 11.00 (s, 1H), 10.47 (s 1H), 8.96 (s, 1H), 8.30 (s, 1H), 8.06 (s, 1H), 8.02 (s, 1H), 7.96 (d, 1H), 7.74 - 7.82 (m, 2H), 7.62 (t, 1H), 7.34 (d, 1H), 2.58 (s, 6H), 2.39 (s, 3H), 2.36 (s, 3H), 1.76 (s, 6H) | 427 | Method 211 Method 20 |
| **285** | 2-Chloro-5-{[3-(1-cyano-1-methyl ethyl)benzoyl] amino}-*N*-(5-methoxypyridin-3-yl)benzamide | 11.00 (s, 1H), 10.60 (s, 1H), 8.59 (s, 1H), 8.23 (s, 1H), 7.84-8.09 (m, 5H), 7.78 (d, 1H), 7.58 - 7.67 (m, 2H), 3.89 (s, 3H), 1.74 (s, 6H) | 451 | Method 118 and Method 223 |
| **286** | *N*-(6-Amino-5-chloropyridin-3-yl)-5-{[3-(1-cyano-1-methylethyl)benzoyl] amino}-2-methyl benzamide | 10.44 (s, 1H), 10.40 (s, 1H), 8.34 (s, 1H), 8.11 (s, 1H), 8.06 (s, 1H), 7.96 (d, 1H), 7.90 (s, 1H), 7.74 - 7.84 (m, 2H), 7.61 (t, 1H), 7.32 (d, 1H), 2.34 (s, 3H), 1.72 (s, 6H) | 450 | Method 186 and Method 20 |
| **287** | 2-Chloro-*N-*(5-chloropyridin-3-yl)-5-{[3-(trifluoromethyl)benzoyl]amino} benzamide | 11.07 (s, 1H), 10.76 (s, 1H), 8.78 (s, 1H), 8.23 - 8.50 (m, 4H), 8.06 (s, 1H), 7.93 - 8.02 (m, 2H), 7.81 (t, 1H), 7.63 (d, 1H) | 456 | Method 215 and Method 224 |
| **288** | 5-{[3-(1-Cyano-1-methylethyl)benzo yl]amino}-*N*-(6-hydroxy-5-methylpyridin-3-yl)-2-methylbenzamide | 10.39 (s, 1H), 10.04 (s, 1H), 8.06 (s, 114), 7.95 (d, 1H), 7.73 - 7.87 (m, 4H), 7.61 (t, 1H), 7.48 (d, 1H), 7.29 (d, 1H), 2.34 (s, 3H), 1.99 (s, 3H), 1.76 (s, 6H) | 429 | Method 216 and Method 20 |
| **289** | 2-Chloro-*N-*[5-methoxy-6-(methylamino)pyridin-3-yl]-5-{[3-(trifluoromethyl) benzoyl]amino}benzamide | 10.76 (s, 1H), 10.67 (s, 1H), 8.30 (s, 1H), 8.27 (d, 1H), 8.06-8.13 (m, 2H), 8.01 (d, 1H), 7.90 (d, 1H), 7.81 (t, 1H), 7.61 (d, 1H), 7.41 (s, 1H), 3.92 (s, 3H), 2.95 (s, 3H) | 481 | Method 195 and Method 224 . |
| **290** | 2-Chloro-*N*-[5-chloro-6-(methylamino) pyridin-3-yl]-5-{[3-(trifluoromethyl) benzoyl]amino} benzamide | 10.73 (s, 1H), 10.49 (s, 1H), 8.24 - 8.36 (m, 3H), 7.89-8.06 (m, 4H), 7.80 (t, 1H), 7.58 (d, 1H), 2.83 (s, 3H) | 485 | Method 194 and Method 224 |
| **291** | *N*-[6- (Acetylamino)-5-chloropyridin-3-yl]-2-chloro-5-[{3-(trifluoromethyl)benzoyl]amino} benzamide | 10.70 (s, 1H), 8.21 - 8.33 (m, 2H), 7.97 - 8.02 (m, 2H), 7.67 - 7.89 (m, 6H), 7.44 (d, 1H), 2.08 (s, 3H) | 513 | Method 209 and Method 224 |
| **292** | *N*-[6-(Acetylamino)-5-bromopyridin-3-yl]-2-methyl-5-{[3-(trifluoromethyl)benzoyl]amino} benzamide | 10.66 (s, 1H), 8.22 - 8.33 (m, 2H), 7.97 - 8.00 (m, 2H), 7.67 - 7.89 (m, 6H), 7.43 (d, 114), 2.16 (s, 3H), 2.07 (s, 3H) | 536 | Method 208 and Method 21 |
| **293** | 2-Chloro-*N*-[5-chloro-6-(methylamino) pyridin-3-yl]-5-{[3-(1-cyano-1-methylethyl)benzoyl]amino} benzamide | 10.87 (s, 1H), 10.58 (s, 1H), 8.23 (s, 1H), 8.04 (s, 1H), 7.92 - 8.02 (m, 3H), 7.75 - 7.85 (m, 2H), 7.52 - 7.65 (m, 3H), 2.09 (s, 3H), 1.75 (s, 6H) | 484 | Method 194 and Method 223 |
| **294** | 2-Chloro-5-{[3-(1-cyano-1-methyl ethyl)benzoyl]amino}-*N*-{6-[(3-pyrrolidin-1-ylpropyl)amino]pyridin-3-yl]benzamide | 10.75 (s, 1H), 10.63 (s, 1H), 9.75 (bs, 1H), 8.49 (s, 1H), 8.03 - 8.10 (m, 2H), 7.86-7.99 (m, 3H), 7.78 (d, 1H), 7.52 - 7.67 (m, 2H), 7.01 (d, 1H), 3.28 - 3.52 (m, 4H), 3.08 - 3.28 (m, 4H), 1.84-2.06 (m, 6H), 1.76 (s, 6H) | 547 | Method 250 and Method 223 |
| **295** | 2-Chloro-5-{[3-(1-cyano-1-methyl ethyl)benzoyl]amino}-*N*-[5-(hydroxymethyl)pyridin-3-yl]benzamide | 10.95 (s 1H), 10.60 (s, 1H), 8.81 (s, 1H), 8.35 (s, 1H), 8.25 (s, 1H), 8.03 - 8.09 (m, 2H), 7.91 - 7.98 (m, 2H), 7.79 (d, 1H), 7.55 - 7.67 (m,2H), 4.60 (s,2H), 1.75 (s, 6H) | 451 | Method 242 and Method 223 |
| **296** | *N*-[3-({[6-(Acetylamino)pyridin-3-yl]amino}carbonyl)-4-chlorophenyl]-2-(1-cyano-1-methyl ethyl) isonicotinamide | 10.84 (s, 1H), 10.72 (s, 1H), 10.50 (s, 1H), 8.84 (d, 1H), 8.66 (s, 1H), 7.98 - 8.10 (m, 4H), 7.95 (d, 1 H), 7.88 (d, 1H), 7.60 (d, 1H), 2.01 (s, 3H), 1.78 (s, 6H) | 479 | *N*-(5-aminopyridin-2-yl)acetamide and Method 29 |
| **297** | *N-*[6-(Acetylamino)-5-methoxypyridin-3-yl]-2-chloro-5-{[3-(trifluoromethyl) benzoyl]amino}benzamide | 10.86 (s, 1H), 10.76 (s, 1H), 9.75 (bs, 1H), 8.23 - 8.35 (m, 3H), 7.90 8.09 (m, 4H), 7.82 (t, 1H), 7.63 (d, 1H), 3.83 (s, 3H), 2.04 (s, 3H) | 509 | Method 210 and Method 224 |
| **298** | 2-Chloro-5-{[3-(1-cyano-1-methyl ethyl)benzoyl]amino}-*N-*(6-ethylpyridin-3-yl)benzamide | 11.16 (s, 1H), 10.67 (s, 1 H), 9.02 (s, 1H), 8.34 (d, 1H), 8.11 (s, 1H), 8.06 (s, 1H), 7.96 (dd, 2H), 7.78 (d, 1H), 7.57 - 7.72 (m, 3H), 2.88 (q, 2H), 1.76 (s, 6H), 1.27 (t, 3H) | 449 | Method 213 and Method 223 |
| **299** | 5-{[3-(1-Cyano-1-methylethyl) benzoyl]amino}-*N-*[5-(hydroxymethyl)pyridin-3-yl]-2-methylbenzamide | | 457 | Method 20 and Method 242 |
| **300** | 5-{[3-(1-Cyano-1-methylethyl) benzoyl]amino}-*N*-(5-fluoropyridin-3-yl)-2-methylbenzamide | 10.87 (s, 1H), 10.43 (s, 1H), 8.70 (s, 1H), 8.34 (s, 1H), 8.20 (d, 1H), 8.05 (s, 1H), 7.90 - 8.00 (m, 2H), 7.86 (d, 1H), 7.76 (d, 1H), 7.62 (t, 1H), 7.35 (d, 1H), 2.35 (s, 3H), 1.76 (s, 6H) | 417 | Method 252 and Method 20 |
| **301** | 5-{[3-(1-Cyano-1-inethylethyl)benzo yl]amino}-*N-*(6-{[3-(dimethylamino) propyl]amino}pyridin-3-yl)-2-methylbenzamide | 10.42 (s, 1H), 10.01 (s, 1H), 8.24 (s, 1H), 8.07 (s, 1H), 7.96 (d, 1H), 7.66 - 7.87 (m, 4H), 7.62 (t, 1H), 7.28 (d, 1H), 6.48 (d, 1H), 6.39 (bs, 1H), 3.17 - 3.25 (m, 2H), 2.34 (s, 3H), 2.27 (t, 2H), 2.13 (s, 6H), 1.75 (s, 6H), 1.68 (m, 2H) | 500 | Method 193 and Method 20 |
| **302** | 5-[(2-Chloro-5-{[3-(1-cyano-1-methylethyl)benzoyl]amino}benzoyl)amino]nicotinamide | 11.02 (s, 1H), 10.59 (s, 1H), 8.93 (s, 1H), 8.80 (s, 1H), 8.61 (s, 1H), 8.22 (s, 1H), 8.05 - 8.06 (m, 2H), 7.93 - 7.99 (m, 2H), 7.79 (d, 1H), 7.52 - 7.70 (m, 3H), 1.73 (s, 6H) | 464 | Method 134 and Method 223 |
| **303** | *N-*(6-Chloro pyridin-3-yl)-5-{[3-(1-cyano-1-methylethyl)benzoyl]amino}-2-methylbenzamide | 10.75 (s, 1H), 10.44 (s, 1H), 8.77 (s, 1H), 8.23 (d, 1H), 8.03 (s, 1H), 7.96 (d, 1H), 7.95 (s, 1H), 7.85 (d, 1H), 7.77 (d, 1H), 7.61 (t, 1H), 7.54 (d, 1H), 7.34 (d, 1H), 2.37 (s, 3H), 1.84 (s, 6H) | 435 | 2-chloropyridine-5-amine and Method 20 |
| **304** | 2-Chloro-*N-*[5-(hydroxymethyl)pyridin-3-yl]-5-{[3-(trifluoromethyl) benzoyl]amino} benzamide | 10.81 (s, 1H) 10.73 (s, 1H) 8.70 (d, 1H) 8.24 - 8.33 (m, 3H) 8.18 (s, 1 H) 7.92 - 8.03 (m, 3 H) 7.80 (t, 1H) 7.60 (d, 1H) 5.39 (t, 1 H) 4.54 (d, 2 H) | 451 | Method 242 and Method 224 |
| **305** | Methyl5-[(5-{[3-(1-cyano-1-methyl ethyl)benzoyl]amlno}-2-methylbenzoyl)amino]pyridine-2-carboxylate | | 457 | Method 20 and Method 90 |
| **306** | *N*-{5-[2(Dimethylamino)ethoxylpyridin-3-yl}-2-methyl-5-{[3-(trifluoromethyl) benzoyl]amino}benzamide | 10.73 (s, 1H), 10.61 (s, 1H), 9.69 (s, 1H), 8.48 (s, 1H), 8:14 (s, 1H), 8.10 (s, 1H), 7.93 - 8.03 (m, 2H), 7.76 - 7.85 (m, 2H), 7.35 (d, 1H), 4.43 (t, 2H), 3.41 - 3.52 (m, 2H), 2.79 - 2.95 (m, 6H), 2.37 (s, 3H), 2.17 (s 6H) | 488 | Method 221 and Method 21 |
| **307** | *N*-(6-Amino-5- chloropyridin-3-yl)-2-methyl-5-{[3-(trifluoromethyl) benzoyl]amino}benzamide | 10.59 (s, 1H), 10.41 (s, 1 H), 8.22 - 8.46 (m, 3H), 8.13 (s, 1H), 7.99 (d, 1H), 7.90 (s 1H), 7.74 - 7.86 (m, 2H), 7.34 (d, 1H), 2.25 (s 3H) | 451 | Method 186 and Method 21 |
| **308** | 5-{[3-(1-Cyano-1-methylethyl) benzoyl]amino}-*N-*(5-methoxypyridin-3-yl)-2-methylbenzamide | 10.84 (s, 1H), 10.46 (s, 1H), 8.68 (s, 1H), 8.15 (s, 1H), 8.06 (s, 1H), 8.02 (s, 1H), 7.92 - 7.99 (m, 2H), 7.83 (d, 1H), 7.77 (d, 1H), 7.63 (t, 1H), 7.35 (d, 1H), 3.89 (s, 3H), 2.38 (s, 3H), 1.75 (s, 6H) | 430 | Method 118 and Method 20 |
| **309** | 5-{[3-(1-Cyano-1-methylethyl) behzoyl]amino}-*N-*(5-isopropoxypyridin-3-yl)-2-methylbenzamide | 10.56 (s, 1H), 10.43 (s, 1H), 8.47 (s, 1H), 8.04 (s, 1H), 8.02 (d, 1H), 7.96 (d, 1H), 7.92 (s, 1H), 7.80 - 7.88 (m, 2H), 7.76 (d, 1H), 7.61 (t, 1 H), 7.33 (d, 1H), 4.56 - 4.76 (m, 1H), 2.37 (s, 3H), 1.76 (s, 6H), 1.31 (d, 6H) | 458 | Method 219 and Method 20 |
| **310** | 3-(1-Cyano-1-methylethyl)-2-fluoro-*N-*(3-{[(5-methoxypyridin-3-yl)amino] carbonyl}-4-methylphenyl) benzamide | 10.89 (s, 1H), 10.68 (s, 1H), 8.72 (s, 1H), 8.26 (s, 1H), 8.04 (s, 1H), 7.88 (s, 1H), 7.73 (d, 1H), 7.60 - 7.70 (m, 2H), 7.35- 7.41 (m, 2H), 3.90 (s, 3H), 2.37 (s, 3H), 1.79 (s, 6H) | 448 | Method 69 and Method 114 |
| **311** | 5-{[3-(1-Cyano-1-methylethyl)behzo yl]amino}-*N*-{5-[3-(dimethylamino) propoxy]pyridin-3-yl)-2-methylbenzamide | 10.79 (s, 1H), 10.49 (s, 1H), 9.94 - 10.13 (bs, 1H), 8.55 (s, 1H), 8.16 (s, 1H), 8.04 - 8.11 (m, 2H), 7.93 - 8.01 (m, 2H), 7.82 (d, 1H), 7.77 (d, 1H), 7.67 - 7.72 (d, 1H), 7.61 (t, 1H), 7.34 (d, 1H), 4.19 (t, 2H), 3.18 - 3.27 (m, 2H), 2.80 (d, 3H), 2.37 (s, 3H), 2.13 - 2.22 (m, 2H), 1.72 (s, 6H) | 501 | Method 222 and Method 20 |

| **Ex** | **Compound** | **M/z** | **SM** |
|---|---|---|---|
| **312** | *N-*(5-Amino-6-methylpyridin-3-yl)-2-chloro-5-{[3-(1-cyano-1-methylethyl)benzoyl]amino}benzamide | 449 | Method 74 and Method 223 |
| **313** | 2-Chloro-5-{[3-(1-cyano-1-methylethyl)benzoyl]amino}-*N-*(5-cyanopyridin-3-yl)benzamide | 444 | Method 223 and Method 232 |
| **314** | tert-Butyl 2-[({5-[(5-{[3-(1-cyano-1-methylethyl)benzoyl]amino}-2-methylbenzoyl)amino]pyridine-2-yl}amino)methyl]pyrrolidine-1-carboxylate | 598 | Method 82 and Method 20 |
| **315** | tert-Butyl 2-[({5-[(5-{[3-(1-cyano-1-methylethyl)benzoyl]amino}-2-methylbenzoyl)amino]pyridin-3-yl}oxy)methyl]pyrrolidine-1-carboxylate | 599 | Method 220 and Method 20 |
| **316** | *N-*(6-{[(Benzyloxy)acetyl]amino}-5-methylpyridin-3-yl)-5-{[3-(1-cyano-1-methylethyl)benzoyl]amino}-2-methylbenzamide | 576 | Method 233 and Method 20 |
| **317** | 2-Chloro-*N-*{6-amino-pyridin-3-yl)-5-{[3-(trifluoromethyl)benzoyl]amino}benzamide | 435 | pyridine-2,5-diamine and Method 224 |

### Example 318

### N-{6-[(Aminocarbonyl)amino]pyridin-3-yl}-5-{[3-(1-cyano-1-methylethyl)benzoy]amino}-2-methylbenzamide

To a solution of *N-*(6-aminopyridin-3-yl)-5-{[3-(1-cyano-1-methylethyl)benzoyl] amino}-2-methylbenzamide (Example 202; 314 mg, 0.760 mmol) in THF (5ml), sodium hydride (145 mg, 3.04 mmol) and trichloroacetyl isocyanate (0.14 ml, 1.14 mmol) were added and the reaction mixture was stirred overnight at 25°C. The mixture was partitioned between H₂O and EtOAc, and the organic layer was washed with brine and dried with MgSO₄. Evaporation of the solvent gave a white solid that was dissolved in MeOH (10 ml) and purged with ammonia gas. Evaporation of the solvent gave a tan solid, which was purified by reverse phase HPLC (5-55% MeCN/H₂O, 15 min). The title compound (65 mg) was collected by evaporation as a white solid. NMR (300 MHz): 10.41 (s, 2H), 9.20 (s, 1H), 8.58 (s, 1H), 7.99 - 8.08 (m, 2H), 7.96 (d, 1H), 7.89 (s, 1H), 7.83 (dd, 1H), 7.75 (d, 1H), 7.61 (t, 1H), 7.48 (d, 1H), 7.32 (d, 1H), 6.85 (s, 1H), 2.36 (s, 3H), 1.76 (s, 6H); m/z 457.

### Exampe 319

### 2-Chloro-5-{[3-(1-cyano-1-methyl)benzoyl]amino}-N-[5-(dimethylamino)-6-methylpyridin-3-yl]benzamide

To a solution of *N-*(5-amino-6-methylpyridin-3-yl)-2-chloro-5-{[3-(1-cyano-1-methylethyl)benzoyl]amino}benzamide (Example 312; 145 mg) in DMF (2 ml) was added at 0°C Cs₂CO₃ (317 mg) and the mixture was stirred at this temperature for 30 minutes. Mel (0.81 ml) was added and the mixture was stirred at room temperature for 4 hours. The mixture was cooled with an ice bath and H₂O was added. The mixture was partitioned between EtOAc and H₂O. The organics were washed with NaCl_{(Sat}) and then dried with Na₂SO₄₍ₛ₎. Evaporation of the solvent gave a solid, which was purified by reverse phase HPLC (5-95% MeCN/H₂O, 15 min using hexyl benzene column). The title compound (3.4 mg) was collected by evaporation. NMR (300 MHz): 10.79 (s, 1H), 10.23 (s, 1H), 8.04 (s, 1H), 8.01 (d, 1H), 7.98-7.95 (m, 2H), 7.84 (d, 1H), 7.78 (d, 1H), 7.52 - 7.66 (m, 3H), 2.83(s, 6H) 2.41 (s, 3H), 1.65 (s, 6H); m/z 478.

### Example 320

### 5-[(5-{[3-(1-Cyano-1-methylethyl)benzoy]amino}-2-methylbenzoyl)amino]-N-methylpyridine-2-carboxamide

A solution of methyl 5-[(5-{[3-(1-cyano-1-methylethyl)benzoyl]amino}-2-methylbenzoyl)amino]pyridine-2-carboxylate (Example 305; 100 mg) in THF (1 ml) was cooled to -78°C and treated with a solution of Me₃Al-MeNH₂ (the solution was made by adding slowly Me₃Al (0.35 ml, M in THF) to a solution of MeNH₂ (0.35 ml, 1M in THF) at -78°C and allowing the solution to stir for 30 minutes at this temperature). The reaction mixture was stirred overnight at 25 °C. The mixture was partitioned between EtOAc and H₂O. The organics were washed with NaCl₍ₛₐₜ₎ and then dried with Na₂SO₄₍ₛ₎. Evaporation of the solvent gave a solid, which was purified by reverse phase HPLC (5-95% MeCN/H₂O, 15 min). The title compound (2 mg) was collected by evaporation. NMR (300 MHz): 10.85 (s, 1H), 10.44 (s, 1H), 8.95 (s, 1H), 8.20 - 8.50 (m, 2H), 7.70 - 8.18 (m, 6H), 7.51 - 7.66 (m, 1H), 7.19 - 7.51 (m, 1H), 2.87 (s, 3H), 2.30 (s, 3H) 1.70 (s, 6H); m/z 456

### Example 321

### 5-[3-(Cyano-dimethyl-methyl)-benzoylamino]-N-(6-cyano-pyridin-3-yl)-2-methyl-benzamide

A solution of 5-[3-(cyanodimethyl-methyl)-benzoylamino]-2-methyl-benzoic acid (Method 20; 300 mg, 0.928 mmol) in SOCl₂ (5 ml) was refluxed at 80 °C for 2 h. Then a solution of 5-aminopyridine-2-carbonitrile (221 mg, 1.86 mmol) in THF (5 ml) was treated with NaH (50% in mineral oil) (111 mg, 2.32 mmol). This reaction was stirred at 25 °C for 1 h. After removing the SOCl₂ from the first reaction, the resulting product was dissolved in THF and added to the second reaction and stirred at 25 °C for 30 min. The reaction was then quenched with H₂O (10 ml), extracted with EtOAc (25 ml), washed with brine (50 ml) and dried with MgSO₄. The solvents were removed under reduced pressure to give an orange solid (637 mg) which was purified by Gilson reverse phase preparatory HPLC (5-95% MeCN/H₂O, 20 min)to yield a yellow solid (10 mg). NMR (300 MHz): 11.07 (s, 1H), 10.61 (s, 1H), 9.02 (d, 1H), 8.42 (dd, 1H), 8.22-8.36 (m, 2H), 7.95-8.08 (m, 3H), 7.73-7.92 (m, 2H), 7.36 (d, 1H), 2.48-2.51 (m, 6H), 2.38 (s, 3H); *m*/*z* 424.

### Example 322

### 5-{[3-(1-Cyano-1-methylethyl)benzoyl]amino}-2-methyl-N-[5-(1H-pyrazol-4-yl)pyridin-3-yl]benzamide

*N-*(5-Bromopyridin-3-yl)-5-{[3-(1-cyano-1-methylethyl)benzoyl]amino}-2-methylbenzamide (Example 192; 256mg, 0.54mmol), 1*H*-pyrazol-4-ylboronic acid (125mg, 0.64mmol), Cs₂CO₃ (352mg 1.08mmol) and Pd(PPh₃)₄ (62mg, 0.054mmol) were put in a microwave tube, dioxane (4ml) and water (1ml) were added. The tube was heated in microwave (Smith, Personal Chemistry^{™}) and heated at 180°C for 2000 seconds. The solution was filtered, and separated between EtOAc and water. Organic layer was dried and evaporated under reduced pressure. The crude product was purified by reverse phase HPLC (5-75% MeCN/H₂O, 15 min) and the title compound (75.6 mg, 30%) was collected by evaporation. NMR (300 MHz) 10.88 (s, 1H), 10.52 (s, 1H), 8.54 (s, 1H), 8.26 (s, 1H), 8.13 (s, 1H), 7.96 - 8.09 (m, 3H), 7.80 - 7.93 (m, 3H), 7.63 - 7.73 (m, 2H), 7.42 (d, 1H), 2.20 (s 3H), 1.82 (s, 6H); m/z 466.

### Examples 323-324

The following compounds were prepared by the procedure of Example 322, using the appropriate starting material

| **Ex** | **Compound** | **NMR** | **M/z** | **SM** |
|---|---|---|---|---|
| **323** | 5-{[3-(1-Cyano-1-methylethyl)benzoyl] amino}-2-methyl-*N-*[5-(1H-pyrrol-2-□yridinedin-3-yl]benzamide | 11.70 (s, 1H), 10.87 (s, 1H), 10.47 (s, 1H), 8.79 (s, 1H), 8.72 (s, 1H), 8.55 (s, 1H), 8.06 (s, 1H), 8.00 (s, 1H), 7.95 (d, 1H), 7.81 (d, 1H), 7.75 (d, 1H), 7.63 (t, 1H), 7.35 (d, 1H), 7.02 (s, 1H), 6.68 (s, 1H), 6.22 (s, 1H), 2.40 (s, 3H), 1.75 (s, 6H) | 465 | 1 H-pyrrol-2-ylboronic acid |
| **324** | tert-Butyl2-{5-[(5-([3-(1-cyano-1-methylethyl)benzoyl] amino)-2-methylbenzoyl)amino] pyridin-3-yl} pyrrolidine-1-carboxylate | | 569 | 1-(tert-butoxycarbon yl)pyrrolidin-2-yl]boronic acid |

### example 325

### 2-Methyl-N-[5-(4-methylpiperazin-1-yl)pyridin-3-yl]-5-{[3-(trifluoromethyl)benzoyl]amino} benzamide

To a 10 ml, round bottom flask equipped with a magnetic stirring bar was added Pd₂(dba)₃ (34 mg, 0.038 mmol), BINAP (47 mg, 0.075 mmol), and sodium tert-butoxide (72 mg, 0.752 mmol). Toluene (2 ml) was added followed by 1-methylpiperazine (56 mg, 0.564 mmol) and the reaction was allowed to stir at room temperature for 10 min before the addition of *-N*-(5-bromopyridin-3-yl)-2-methyl-5-{[3-(trifluoromethyl)benzoyl]amino}benzamide (Example 193; 180 mg, 0.376 mmol). The resulting reaction mixture was warmed to 80 °C and was allowed to stir for 12 h before being cooled and diluted with EtOAc (∼ 100 ml). The organic phase was poured into a separator funnel and washed with saturated aqueous NaHCO₃ (∼ 100 ml). The organic extract was dried with MgSO₄, filtered, and concentrated *in vacuo* to yield the crude product, which was purified on a 40 g SiO₂ column using MeOH/EtOAc (1:10) as eluent giving 144 mg (77%) of the title compound as a white solid. NMR (300 MHz): 10.91 (s, 1H), 10.65 (s, 1H), 8.57 (s, 1H), 8.32-8.26 (m, 3H), 8.10 (s, 1H), 8.02 (d, 1H), 7.99 (d, 1H), 7.82-7.78 (m, 2H), 7.35 (d, 1H), 3.99-3.93 (m, 2H), 3.55-3.50 (m, 2H), 3.32-3.12 (m, 4H), 2.82 (s, 3H), 2.37 (s, 3H); *m*/*z* 499.

### Examples 326-336

The following compounds were prepared by the procedure in example 325 using *N-*(5-bromopyridin-3-yl)-2-methyl-5-{[3-(trifluoromethyl)benzoyl]amino}benzamide (Example 193), *N-*(5-bromopyridin-3-yl)-5-{[3-(1-cyano-1-methylethyl)benzoyl]amino}-2-methylbenzamide (Example 192), *N-*(5-bromopyridin-3-yl)-2-chloro-5-[(3,5-dimethylbenzoyl)amino]benzamide (Example 219) and the appropriate SM. In some cases, further purification was required (supercritical fluid, Gilson reverse phase preparatory HPLC or column chromatography utilizing an ISCO system).

| **Ex** | **Compound** | **NMR** | **M/z** | **SM** |
|---|---|---|---|---|
| **326** | 2-Methyl-*N-*(5-morpholin-4-ylpyridin-3-yl)-5-{[3-(trifluoromethyl)benzoyl] amino} benzamide | 11.06 (s, 1H), 10.67 (s, 1H), 8.66 (s, 1H), 8.33-8.27 (m, 3H), 8.10 (s, 1H), 8.04 (d, 1H), 7.97 (d, 1H), 7.82-7.77 (m, 2H), 7.36 (d, 1H), 3.77 (t, 4H), 3.30 (t, 4H), 2.37 (s, 3H) | 485 | Morpholine |
| **327** | *N-*{5-[[3- (Dimethylamino)propyl](methyl)amino]pyridin-3-yl}-2-methyl-5-{[3-(trifluoromethyl)benzoyl] amino}benzamide | 11.01 (s, 1H), 10.66 (s, 1H), 8.54 (s, 1H), 8.32-8.27 (m, 2H), 8.14 (d, 1H), 8.05 (d, 1H), 7.98 (d, 1H), 7.89 (s, 1H), 7.82-7.77 (m, 2H), 7.36 (d, 1H), 3.56-3.51 (m, 2H), 3.10-3.03 (m, 2H), 3.02 (s, 3H), 2.74 (s,3H), 2.72 (s, 3H), 2.37 (s, 3H), 1.97-1.89 (m, 2H) | 514 | N,N,N'-trimethylpropan e-1,3-diamine |
| **328** | *N-*[5-(Dimethylamino)pyridin-3-yl]-2-methyl-5-{[3-(trifluoromethyl) benzoyl]amino}benzamide | 11.02 (s, 1H), 10.65 (s, 1H), 8.58 (s, 1H), 8.31-8.27 (m, 2H), 8.04 (dd, 2H), 7.98 (d, 1H), 7.83-7.77 (m, 3H), 7.35 (d, 1H), 3.04 (s, 6H), 2.37 (s, 3H) | 443 | *N,N-*dimethylamine |
| **329** | *N-*(5-{[3-(Dimethylamino)propyl]amino}pyridin-3-yl)-2-methyl-5-{[3-(trifluoromethyl)benzoyl]amino}benzamide | 11.06 (s, 1H), 10.69 (s, 1H), 8.41 (s, 1H), 8.31-8.28 (m, 2H), 8.02-7.92 (m, 4H), 7.82 (t, 2H), 7.33 (d, 1H), 3.29-3.20 (m, 2H), 3.18-3.05 (m, 2H), 2.74 (s, 3H), 2.73 (s, 3H), 2.36 (s, 3H), 1.96-1.89 (m, 2H) | 501 | *N,N-*dimethylpropan e-1,3-diamine |
| **330** | *N-*{5-[(2-Methoxyethyl)(methyl)amino]pyridin-3-yl}-2-methyl-5-{[3-(trifluoromethyl)benzoyl]amino}benzamide | 11.02 (s, 1H), 10.67 (s, 1H), 8.58 (s, 1H), 8.31-8.28 (m, 2H), 8.09 (d, 1H), 8.04-7.96 (m, 2H), 7.84-7.77 (m, 3H), 7.35 (d, 1H), 3.65-3.59 (m, 2H), 3.55-3.49 (m, 2H), 3.24 (s, 3H), 3.04 (s, 3H), 2.37 (s, 3H) | 487 | 2-methoxy-*N-*methylethanamine |
| **331** | *N-*(5-{[(2,2-Dimethyl-1,3-dioxolan-4-yl)methyl]amino}pyridin-3-yl)-2-methyl-5-{[3-(trifluoromethyl) benzoyl]amino}benzamide | 11.04 (s, 1H), 10.66 (s, 1H), 8.60 (s, 1H), 8.32-8.29 (m,2H), 8.02 (dd, 2H), 7.99 (d, 1H), 7.83-7.78 (m, 3H), 7.34 (d, 1H), 4.26-4.22 (m, 1H), 3.79-3.55 (m, 2H), 3.12-3.05 (m, 2H), 2.37 (s, 3H), 1.41 (s, 314), 1.33 (s, 3H) | 530 | 1-(2,2-dimethyl-1,3-dioxolan-4-yl)methanamine |
| **332** | *N-*(5-{C2-(Dimethylamino)ethyl]amino}pyridin-3-yl)-2-methyl-5-{[3-(trifluoromethyl)benzoyl]amino}benzamide | 10.72 (s, 1H), 10.59 (s, 1H), 8.30-8.24 (m, 3H), 7.99-7.96 (m, 2H), 7.87-7.76 (m, 4H), 7.34 (d, 1H), 3.49 (t,2H), 3.29-3.26 (m, 2H), 2.84 (s,6H), 2.36 (s, 3H) | 486 | *N,N-*dimethylethane-1,2-diamine |
| **333** | *N-*{5-[[2-(Dimethylamino)ethyl](methyl)amino]pyridin-3-yl}-2-methyl-5-{[3-(trifluoromethyl)benzoyl] amino]benzamide | 10.72 (s, 1H), 10.59 (s, 1H), 8.30-8.24 (m, 3H), 7.99-7.96 (tri, 2H), 7.87-7.76 (m, 4H), 7.34 (d, 1H), 3.49 (t,2H), 3.29-3.26 (m, 2H), 2.84 (s, 6H), 2.36 (s, 3H) | 501 | N,N,N'-trimethylethane -1,2-diamine |
| **334** | 5-{[3-(1-Cyano-1-methylethyl)benzoyl] amino}-2-methyl-*N-*[5-(4-methylpiperazin-1-yl)pyridin-3-yl]benzamide | 11.06 (s, 1H), 10.77 (s, 1H), 8.55 (s, 1H), 8.34-8.29 (m,3H), 8.12 (s, 1H), 8.03 (d, 1H), 7.98 (d, 1H), 7.84-7.78 (m, 2H), 7.34 (d, 1 H), 4.00-3.93 (m, 2H), 3.56-3.50 (m, 2H), 3.33-3.11 (m, 4H), 2.82 (s,3H), 2.37 (s, 3H), 1.59 (s, 6H) | 498 | 1-methylpiperazine |
| **335** | 2-Chloro-5-[(3,5-dimethylbenzoyl)amino]-*N-*(5-morpholin-4-ylpyridin-3-yl)benzamide | 10.99 (s, 1H), 10.45 (s, 1H), 8.50 (d, 1H), 8.23 (d, 1H), 8.10 (d, 1H), 7.94-7.88 (m, 2H), 7.59 (s, 1H), 7.56 (s,2H), 7.24 (s, 1H), 3.76 (t, 4H), 3.25 (t, 4H), 2.35 (s, 6H) | 466 | Morpholine |
| **336** | 2-Chloro-*N-*{5-[[2-(dimethylamino)ethyl](methyl)amino]pyridin-3-yl}-5-[(3,5-dimethylbenzoyl)amino]benzamide | 10.88 (s, 1H), 10.48 (s, 1H), 8.35 (s, 1H), 8.11 (d, 1H), 8.08 (s, 1H), 7.87 (dd, 1H), 7.73 (s, 1H), 7.58 (s, 1H), 7.56 (s, 2H), 7.24 (s, 1H), 3.76 (t, 2H), 3.29-3.26 (m, 2H), 2.98 (s, 3H), 2.85 (s, 3H), 2.83 (s, 3H), 2.35 (s, 6H) | 481 | N,N,N'-trimethylethane -1,2-diamine |

### Example 337

### N-[5-(3-Aminoprop-1-yn-1-yl)pyridin-3-yl]-2-methyl-5-{[3-(trifluoromethyl)benzoyl]amino} benzamide

To a 10 ml round bottom flask equipped with a magnetic stirring bar was added'*N-*(5-bromopyridin-3-yl)-2-methyl-5-{[3-(trifluoromethyl)benzoyl]amino}benzamide (Example 193; 200 mg, 0.418 mmol) and MeCN (1.75 ml). Et₃N (0.293 ml, 2.10 mmol) was added followed by prop-2-yn-1-amine (58 mg, 1.05 mmol), Cul (25 mg, 0.125 mmol), and Pd(PPh₃)₄ (96 mg, 0.084 mmol). The reaction was warmed to 50 °C with stirring for 12 h before being cooled to room temperature, diluted with 50 ml of EtOAc, and filtered through a pad of SiO₂. The SiO₂ was rinsed with an additional 50 ml of EtOAc and the combined filtrate was concentrated *in vacuo* to yield the crude product, which was purified on a 40 g SiO₂ column using MeOH/EtOAc (1:10) as eluent giving 102 mg (55%) of the title compound as an off-white solid. NMR (300 MHz): 10.82 (s, 1H), 10.62 (s, 1H), 8.46-8.40 (m, 2H), 8.31-8.27 (m, 3H), 7.98-7.95 (m, 2H), 7.84-7.77 (m, 2H), 7.34 (d, 114), 4.08-4.02 (m, 2H), 2.36 (s, 3H); m/z 453.

### Examples 338-340

The following compounds were prepared by the procedure in LD29 using *N-*(5-bromopyridin-3-yl)-2-methyl-5-{[3-(trifluoromethyl)benzoyl]amino}benzamide (Example 193) and the appropriate SM. In some cases, further purification was required (supercritical fluid, Gilson reverse phase preparatory HPLC or column chromatography utilizing an ISCO system).

| **Ex** | **Compound** | **NMR** | **M/z** | **SM** |
|---|---|---|---|---|
| **338** | *N-*[5-(3-Hydroxyprop-1-yn-1-yl)pyridin-3-yl]-2-methyl-5-{[3-(trifluoromethyl)benzoyl]amino}benzamide | 10.73 (s, 1H), 10.59 (s, 1H), 8.83 (s, 1H), 8.34-8.26 (m, 4H), 7.97 (d, 1H), 7.92 (d, 1H), 7.87-7.79 (m, 2H), 7.33 (d, 1H), 4.33 (s, 2H), 2.36 (s, 3H) | 454 | prop-2-yn-1-ol |
| **339** | 2-Methyl-*N-*{5-[3-(methylamino)prop-1-yn-1-yl]pyridin-3-yl}-5-{[3-(trifluoromethyl)benzoyl]amino}benzamide | 10.83 (s, 1H), 10.64 (s, 1H), 8.46 (s, 1H), 8.31-8.26 (m, 3H), 7.98-7.95 (m, 3H), 7.85-7.77 (m, 2H), 7.33 (d, 1H), 4.19 (t, 2H), 3.05 (t, 2H), 2.36 (s, 3H) | 467 | *N-*methylprop-2-yn-1-amine |
| **340** | *N*-{5-[3- (Dimethylamino)prop-1-yn-1-yl]pyridin-3-yl}-2-methyl-5-{[3-(trifluoromethyl)benzoyl]amino}benzamide | 10.82 (s, 1H), 10.63 (s, 1H), 8.48,(s, 1H), 8.31-8.26 (m, 3H), 7.99-7.96 (m, 2H), 7.85-7.77 (m, 3H), 7.34 (d, 1H), 4.39 (s, 2H), 2.88 (s, 6H), 2.36 (s, 3H) | 481 | *N,N-*dimethylprop-2-yn-1-amine |

### Example 341

### N-[5-(3-Hydroxypropyl)pyridin-3-yl]-2-methyl-5-{[3-(trifluoromethyl)benzoyl]amino} benzamide

To a 25 ml round bottom charged with a magnetic stirring bar and *N-*[5-(3-hydroxyprop-1-yn-1-yl)pyridin-3-yl]-2-methyl-5-{[3-(trifluoromethyl)benzoyl]amino} benzamide (Example 338; 45 mg, 0.100 mmol) was added MeOH (10 nil). 10% Pd/C (15 mg) was carefully added and the reaction was placed under 1 atm of H₂ using a balloon. The reaction was allowed to stir for 12 h at room temperature before being purged with argon, diluted with ∼15 ml of EtOAc, and filtered through a pad of SiO₂. The SiO₂ pad was rinsed with an additional 25 ml of EtOAc and the combined filtrate was concentrated *in vacuo* to yield the crude product, which was purified on a 40 g SiO₂ column using EtOAc as eluent giving 44 mg (98%) of the title compound as an off-white solid. NMR (300 MHz): 10.85 (s, 1H), 10.60 (s, 1H), 8.32-8.26 (m, 5H), 7.99-7.97 (m, 2H), 7.84-7.76 (m, 2H), 7.34 (d, 1H), 3.43 (t, 2H), 2.75-2.69 (m, 2H), 2.37 (s, 314),1.78-1.71 (m, 2H); m/z 459.

### Examples 342-343

The following compounds were prepared by the procedure in Example 341 and the appropriate SM. In some cases, further purification was required (supercritical fluid, Gilson reverse phase preparatory HPLC or column chromatography utilizing an ISCO system).

| **Ex** | **Compound** | **NMR** | **M/z** | **SM** |
|---|---|---|---|---|
| **342** | 2-Methyl-*N-*{5-[3-(methylamino)propyl]pyridin-3-yl}-5-{[3-(trifluoromethyl)benzoyl] amino}benzamide | 10.89 (s, 1H), 10.64.(s, 1H), 8.34-8.25 (m,5H), 8.00-7.97 (m, 2H), 7.85-7.76 (m, 2H), 7.33 (d, 1H), 2.64-2.59 (m, 2H); 2.49 (s, 3H), 2.37 (s, 3H), 2.30-2.26 (m, 2H), 1.78-1.75 (m, 2H) | 472 | Example 339 |
| **343** | *N*-{5-[3- (Dimethylamino)propyl]pyridin-3-yl}-2-methyl-5-{[3-(trifluoromethyl) benzoyl]amino}benzamide | 10.67 (s, 1H), 10.60 (s, 1H), 8.30-8.22 (m, 5H), 8.05-7.97 (m, 2H), 7.80 (t, 2H), 7.34 (d, 1H), 3.15-3.09 (m, 2H), 2.79 (s, 3H), 2.77 (s, 3H), 2.65-2.59 (m, 2H), 2.36 (s, 3H), 1.98-1.88 (m, 2H) | 486 | Example 340 |

### Example 344

### 2-Chloro-N-{5-[(4-methylpiperazin-1-yl)methyl]pyridin-3-yl}-5-{[3-(trifluoromethyl)benzoyl]amino}benzamide

To a 10 ml round bottom flask charged with a magnetic stir bar and 2-chloro-*N-*[5-(hydroxymethyl)pyridin-3-yl]-5-{[3-(trifluoromethyl)benzoyl]amino}benzamide (Example 304; 0.200 g, 0.444 mmol) was added Et₃N (0.19 ml, 1.34 mmol). The reaction was cooled to 0 °C in an ice bath and methanesulfonyl chloride (0.05 ml, 0.578 mmol) was added dropwise via syringe. The reaction was allowed to stir at this temperature for 15 min before 1-methylpiperazine (0.500 ml, 4.44 mmol) was added via syringe. The reaction was allowed to stir to room temperature over 3h before being poured over ∼50 ml of saturated aqueous NaHCO₃. The resulting mixture was poured into a separator funnel and extracted with ∼50 ml of EtOAc. The combined organic extract was dried with MgSO₄, filtered, and concentrated *in vacuo* to yield the crude product, which was purified by semi-preparative reverse phase HPLC giving 0.190 g (80%) of the title compound as a pale yellow solid NMR (300 MHz): 10.95 (s, 1H), 10.78 (s, 1H), 8.75 (d, 1H), 8.33-8.26 (m, 4H), 8.10 (d, 1H), 7.99 (d, 1H), 7.88 (dd, 1H), 7.80 (t, 1H), 7.60 (d, 1H), 3.74 (s, 2H), 3.42-3.32 (m, 4H), 3.11-3.00 (m, 4H), 2.78 (s, 3H), *m*/*z* 533.

### Examples 345-351

The following compounds were prepared by the procedure in Example 344 using the appropriate SMs. In some cases, further purification was required (supercritical fluid, Gilson reverse phase preparatory HPLC or column chromatography utilizing an ISCO system).

| **Ex** | **Compound** | **NMR** | **M/z** | **SM** |
|---|---|---|---|---|
| **345** | 2-Methyl-*N-*[5-(morpholin-4-ylmethyl)pyridin-3-yl]-5-{[3-(trifluoromethyl) benzoyl]amino} benzamide | 10.80 (s, 1H), 10.59 (s, 1H), 8.78 (d, 1H), 8.60 (s, 1H), 8.42 (d, 1H), 8.30- 8.24 (m, 2H), 8.01-7.97 (m, 2H), 7.82-7.75 (m, 2H), 7.34 (d, 1H), 3.76 (t, 4H), 3.25 (t, 4H), 3.22 (s, 2H), 2.36 (s, 3H) | 500 | Morpholine and Example 273 |
| **346** | 2-Chloro-*N-*{5-[(dimethylamino)methyl]pyridin-3-yl}-5-{[3-(trifluoromethyl) benzoyl]amino}benzamide | 11.04 (s, 1H), 10.79 (s, 1H), 8.77 (d, 1H), 8.55 (s, 1H), 8.44 (s, 1H), 8.31-8.26 (m, 2H), 8.11 (d, 1H), 8.00 (d, 1H), 7.92 (dd, 1H), 7.80 (t, 1H), 7.62 (d, 1H), 4.39 (s,2H), 2.77 (s, 6H) | 477 | N, *N-*dimethylamine and Example 304 |
| **347** | 2-Chloro-*N-*{5-[(cyclopropylamino)methyl]pyridin-3-yl}-5-[3-(trifluoromethyl) benzoyl]amino}benzamide | 11.00 (s, 1H), 10.77 (s, 1H), 8.71 (s, 1H), 8.55 (s, 1H), 8.45 (s, 1H), 8.31-8.26 (m, 2H), 8.10 (d, 1H), 8.00 (d, 1H), 7.91 (dd, 1H), 7.81 (t, 1H), 7.62 (d, 1H), 4.34 (s, 2H), 2.80-2.75 (m, 1H), 0.85-0.76 (m,4H) | 490 | Cyclopropyl amine and Example 304 |
| **348** | 2-Chloro-*N-*[5-(piperidin-1-ylmethyl)pyridin-3-yl]-5-{[3-(trifluoromethyl)benzoyl]amino} benzamide | 11.03 (s, 1H), 10.78 (s, 1H), 8.76 (d, 1H), 8.55 (s, 1H), 8.44 (d, 114), 8.30-8.26 (m, 2H), 8.12 (d, 1H), 8.00 (d, 1H), 7.90 (dd, 1H), 7.81 (t, 1H), 7.61 (d, 1H), 4.39 (d,2H), 3.36 (d, 2H), 2.95-2.90 (m, 2H), 1.85-1.62 (m, 6H) | 518 | Piperdine and Examples 304 |
| **349** | 2-Chloro-*N-*(5-{[(2-methoxyethyl)(methyl)amino]methyl}pyrindin-3-yl)-5-{[3-(trifluoromethyl)benzoyl]amino} benzamide | 11.03 (s, 1H), 10.78 (s, 1H), 8.77 (d, 1H), 8.57 (s, 1H), 8.45 (s, 1H), 8.30-8.26 (m, 2H), 8.11 (d, 1H), 8.00 (d, 1H), 7.89 (dd, 1H), 7.81 (t, 1H), 7.61 (d, 1H), 4.50-4.30 (m, 2H), 3.68 (s,2H), 3.32-3.25 (m, 5H), 2.75 (s, 3H) | 522 | 2-methoxy-N -methylethan amine and Example 304 |
| **350** | 2-Chloro-*N-*(5-{[[2-(dimethylamino)ethyl] (methyl)amino]methyl}pyridin-3-yl)-5-{[3-(trifluoromethyl) benzoyl]amino} benzamide | 11.01 (s, 1H), 10.80 (s, 1H), 8.77 (d, 1H), 8.44 (s, 2H), 8.30-8.26 (m, 2H), 8.11 (d, 1H), 8.00 (d, 1H), 7.88 (dd, 1H), 7.80 (t, 1H), 7.62 (d, 1H), 3.38 (s, 2H), 2.82 (s, 6H), 2.54-2.40 (m, 4H), 2.44 (s, 3H) | 534 | N,N,N'-trimethylethane-1,2-diamine and example 304 |
| **351** | 2-Chloro-*N-*{5-[(methylamino)methyl]pyridin-3-yl}-5-{C3-(trifluoromethyl) benzoyl]amino} benzamide | 11.01 (s, 1H), 10.80 (s, 1H), 8.77 (d, 1H), 8.44 (s, 2H), 8.30-8.26 (m, 2H), 8.11 (d,1H), 8.00 (d, 1H), 7.88 (dd, 1H), 7.80 (t, 1H), 7.62 (d, 1H), 3.38 (s, 2H), 2.82 (s, 6H), 2.54-2.40 (m, 4H), 2.44 (s, 3H) | 463 | Methylamine and Example 304 |

### Example 352

### 2-Methyl-N-{5-[(pyrrolidin-3-ylmethyl)amino]pyridin-3-yl}-5-{[3-(trifluoromethyl)benzoyl]amino}benzamide

To a 25 ml round bottom flask charged with a magnetic stir bar and *tert*-butyl 3-formylpyrrolidine-1-carboxylate (0.121 g, 0.603 mmol) was added MeOH (3 ml) and glacial acetic acid (0.3 ml). To the reaction mixture was added *N-*(5-aminopyridin-3-yl)-2-methyl-5-{[3-(trifluoromethyl)benzoyl]amino}benzamide (Example 244; 0.25 g, 0.603 mmol) and the redaction was allowed to stir at room temperature for 10 min. To the resulting mixture was added a 1M solution of NaBH₃CN in THF (1.21 ml), and the reaction was allowed to stir for 4 h at room temperature before being quenched with ∼ 5 ml of saturated aqueous NaHCO₃. The resulting mixture was poured into a separator funnel and extracted with ∼50 ml of EtOAc. The combined organic extract was dried with MgSO₄, filtered, and concentrated *in vacuo* to yield the crude product, which was dissolved in MeOH (5 ml). A 4N solution of HCl in dioxane was added until a pH of 3 (~ 0.5 ml) was achieved. The reaction was allowed to stir at room temperature for 12 h before being concentrated *in vacuo* which was purified by semi-preparative reverse phase HPLC giving 0.09 g (30%) of the title compound as a white solid. NMR (300 MHz): 10.97 (s, 1H), 10.67 (s, 1H), 8.42 (s, 1H), 8.33 (s, 1H), 8.29 (d, 1H), 7.91 - 8.05 (m, 4H), 7.76 - 7.89 (m, 2H), 7.36 (d, 1H), 3.27 - 3.38 (m, 2H), 3.20 (d, 2H), 2.86 - 2.99 (m, 3H), 2.48 - 2.64 (m, 2H), 2.05 - 2.18 (m, 2H), 1.61 - 1.76 (m, 1H); *m*/*z* 499.

### Examples 353-356

The following compounds were prepared by the procedure in Example 352, using *N-*(5-aminopyridin-3-yl)-2-methyl-5-{[3-(trifluoromethyl)benzoyl]amino}benzamide (Example 244), and the appropriate SM. In some cases, further purification was required (supercritical fluid, Gilson reverse phase preparatory HPLC or column chromatography utilizing an ISCO system).

| **Ex** | **Compound** | **NMR** | **M/z** | **SM** |
|---|---|---|---|---|
| **353** | 2-Methyl-*N-*{5-[(piperidin-4-ylmethyl)amino]pyridin-3-yl}-5-{[3-(trifluoromethyl)benzoyl]amino} benzamide | 10.82 (s, 1H), 10.61 (s, 1H), 8.58 (s, 1H), 8.31 (d, 2H), 8.27 (d, 1H), 7.95 - 8.03 (m, 2H), 7.76 - 7.89 (m, 314), 7.34 (d, 1H), 3.30-3.25 (m, 214), 3.03 (d, 2H), 2.86 (d, 2H), 2.36 (s, 3H), 1.90-1.86 (m, 4H), 1.36-1.30 (m, 1H) | 512 | tert-butyl4-formylpiperidine-1-carboxylate |
| **354** | *N*-{5-[(1H-Imidazol-2-ylmethyl)amino]pyridin-3-yl}-2-methyl-5-{[3-(trifluoromethyl) benzoyl]amino} benzamide | 10.78 (s, 1H), 10.60 (s, 1H), 8.40 (d, 1 H), 8.30 (s, 1H), 8.27 (d, 1H), 7.89 - 8.00 (m, 314), 7.74 - 7.83 (m, 3H), 7.63 (s, 2H), 7.33 (d, 1H), 4.76 (s, 2H), 2.34 (s, 3H) | 496 | 1H-imidazole-2-carbaldehyde |
| **355** | *N*-{5-[(2-Hydroxyethyl)amino]pyridin-3-yl}-2-methyl-5-{[3-(trifluoromethyl)benzoyl]amino}benzamide | 10.92 (s, 1 H), 10.61 (s, 1H), 8.41 (d, 1H), 8.31 (s, 1H), 8.27 (d, 1H), 7.88 - 8.00 (m, 3H), 7.76 - 7.84 (m, 3H), 7.35 (d, 1H), 3.59 (t, 2H), 3.19 (t, 2H), 2.36 (s, 3H) | 459 | {[tert-butyl(dimethyl)silyl]oxy}acetaldehyde |
| **356** | *N*-{5-[(2-Aminoethyl)amino]pyridin-3-yl}-2-methyl-5-{[3-(trifluoromethyl)benzoyl]amino}benzamide | 10.91 (s, 1H), 10.65 (s, 1H), 8.40 (d, 1H), 8.30 (s, 1H), 8.27 (d, 1H), 7.88 - 8.01 (m, 3H), 7.76 - 7.84 (m, 3H), 7.35 (d, 1H), 3.26 (t, 2H), 3.19 (t, 2H), 2.36 (s, 3H) | 458 | tert-butyl (2-oxoethyl)carbamate |

### Example 357

### 2-Chloro-N-{5-[(methylsulfonyl)umino]pyridin-3-yl}-5-{[3-(trifluoromethyl)benzoyl]amino} benzamide

To a 10 ml round bottom flask equipped with a magnetic stir bar was added *N*-(5-aminopyridin-3-yl)-2-chloro-5-{[3-(trifluoromethyl)benzoyl]amino}benzamide (Example 243; 80 mg, 0.184 mmol). Pyridine (2.0 ml) was added followed by methanesulfonyl chloride (0.018 ml, 0.23 mmol). The reaction was warmed to 50 °C and allowed to stir to for 12 h before being poured over ∼50 ml of saturated aqueous NaHCO₃. The resulting mixture was poured into a separator funnel and extracted with ∼50 ml of EtOAc. The combined organic extract was dried with MgSO₄, filtered, and concentrated in *vacuo* to yield the crude product, which was purified on a 40 g SiO₂ column using EtOAc as eluent giving 50 mg (53%) of the title compound as an off-white solid. NMR (300 MHz): 10.90 (s, 1H), 10.74 (s, 1H), 10.14 (s, 1H), 8.66 (s, 1H), 8.26 (s, 3H), 8.15 (s, 1H), 7.98 (s, 3H), 7.81 (s, 1H), 7.60 (s, 1H), 3.08 (s, 3H); *m*/*z* 514.

### Example 358

The following compound was prepared by the procedure in Example 357, using *N*-(6-aminopyridin-3-yl)-5-{[3-(1-cyano-1-methylethyl)benzoyl]amino}-2-methylbenzamide (Example 202) and the appropriate SM. Further purification was required (column chromatography utilizing an ISCO system).

| **Ex** | **Compound** | **NMR** | **M/z** | **SM** |
|---|---|---|---|---|
| **358** | 5-{[3-(1-Cyano-1-methylethyl)benzoyl] amino}-2-methyl-*N*-{6-[(methylsulfonyl)amino]pyridin-3-yl}benzamide | 10.46 (d, 2H), 8.60 (s, 1H), 8.06 (s, 2H), 7.94 (s, 2H), 7.85 (s, 1H), 7.76 (s, 2H), 7.61 (d, 1H), 7.31 (d, 1H), 7.00 (d, 1H), 3.27 (s, 3H), 2.35 (s, 3H), 1.74 (s, 6H) | 493 | Methanesulfonylchloride |

### Example 359

### N-[5-(Acetylamino)pyridin-3-yl]-2-methyl-5-{[3-(trifluoromethyl)benzoyl]amino}benzamide

To a 10 ml round bottom flask equipped with a magnetic stir bar was added *N*-(5-aminopyridin-3-yl)-2-methyl-5-{[3-(trifluoromethyl)benzoyl]amino}benzamide (Example 244; 200 mg, 0.482 mmol). Pyridine (5.0 ml) was added followed acetyl chloride (0.044 ml, 0.603 mmol). The reaction was allowed to stir to for 12 h at room temperature before being poured over ∼50 ml of saturated aqueous NaHCO₃. The resulting mixture was poured into a separator funnel and extracted with ∼50 ml of EtOAc. The combined organic extract was dried with MgSO₄, filtered, and concentrated *in vacuo* to yield the crude product, which was purified by on a 40 g SiO₂ column using EtOAc as eluent giving 160 mg (73%) of the title compound as an off-white solid. NMR (300 MHz): 10.74 (s, 1H), 10.57 (s, 1H), 10.34 (s, 1H), 8.63 (d, 2H), 8.59 (s, 1H), 8.31 (s, 1H), 8.27 (d, 1H), 7.90 - 8.00 (m, 2H), 7.76 - 7.86 (m, 2H), 7.33 (d, 1H), 2.36 (s, 3H), 2.09 (s, 3H); *m*/*z* 457.

### Examples 360-361

The following compound was prepared by the procedure in Example 359, using *N*-(6-aminopyridin-3-yl)-5-{[3-(1-cyano-1-methylethyl)benzoyl]amino}-2-methylbenzamide (Example 202) and the appropriate SM. In some cases, further purification was required (supercritical fluid, Gilson reverse phase preparatory HPLC or column chromatography utilizing an ISCO system).

| **Ex** | **Compound** | **NMR** | **M/z** | **SM** |
|---|---|---|---|---|
| **360** | 5-{[3-(1-Cyano-1-methylethyl)benzoyl] amino}-*N*-{6-[(2,2-dimethylpropanoyl)amino]pyridin-3-yl}-2-methylbenzamide | 10.50 (s, 1H), 10.41 (s, 1H), 9.79 (s, 1H), 8.72 (d, 1H), 8.00 - 8.09 (m, 3H), 7.88 - 7.97 (m, 2H), 7.83 (dd, 1H), 7.75 (d, 1H), 7.60 (t, 1H), 7.31 (d, 1H), 2.36 (s, 3H), 1.74 (s, 6H), 1.10 (m, 9H) | 499 | 2,2-dimethylpropanoyl chloride |
| **361** | 5-{[3-(1-Cyano-1-methylethyl)benzoyl] amino}-*N-*{6-[(cyclobutylcarbonyl)amino]pyridin-3-y}-2-methylbenzamide | 10.48 (s, 1H), 10.40 (s, 1H), 10.29 (s, 1H), 8.67 (d, 1H), 8.02 - 8.13 (m, 3H), 7.94 (d, 1H), 7.80 - 7.90 (m; 2H), 7.73 - 7.78 (m, 1H), 7.60 (t, 1H), 7.31 (d, 1H), 2.36 (s, 3H), 2.21 (d, 3H), 2.03 - 2.19 (m, 3H), 1.86 - 1.96 (m, 1H), 1.81 (s, 1H), 1.74 (s, 6H) | 496 | cyclobutanecarbonyl chloride |

### Example 362

### N-{5-[(Aminocarbonyl)amino]pyridin-3-yl}-2-methyl-5-{[3-(trifluoromethyl)benzoyl]amino} benzamide

To a 25 ml round bottom flask equipped with a magnetic stir bar was added *N*-(5-aminopyridin-3-yl)-2-methyl-5-{[3-(trifluoromethyl)benzoyl]amino}benzamide(Example 244; 0.25 g, 0.603 mmol). Anhydrous THF (3.0 ml) was added followed trichloroacetyl isocyanate (0.286 ml, 2.40 mmol). The reaction was allowed to stir to for 1 h at room temperature before concentrated *in vacuo.* The resulting residue was dissolved in a 2N solution of NH₃ in MeOH (10 ml) and allowed to stir for 3 h at room temperature. The reaction mixture was concentrated *in vacuo* to yield the crude product, which was purified by semi-preparative reverse phase HPLC giving 0.132 g (49%) of the title compound as a white solid NMR (300 MHz): 10.75 (s, 1H), 10.57 (s, 1H), 9.10 (s, 1H), 8.56 (s, 2H), 8.43 (s, 1H), 8.25 - 8.32 (m, 2H), 7.92 - 8.00 (m, 2H), 7.76 - 7.85 (m, 2H), 7.33 (d, 1H), 6.13 (s, 2H), 2.36 (s, 3H); *m*/*z* 458.

### Example 363

### N-[5-(Formylamino)pyridin-3-yl]-2-methyl-5-{[3-(trifluoromethyl)benzoyl]amino}benzamide

To a 25 ml round bottom flask equipped with a magnetic stir bar was added *N*-(5-aminopyridin-3-yl)-2-methyl-5-{[3-(trifluoromethyl)benzoyl]amino}benzamide (Example 244; 0.30 g, 0.723 mmol). Anhydrous DCM (3.0 ml) was added followed Et₃N (0.507 ml, 3.62 mmol), and acetic-formic anhydride (0.159 g, 1.80 mmol). The reaction was allowed to stir to for 4 h at room temperatures before being poured over ∼50 ml of saturated aqueous NaHCO₃. The resulting mixture was poured into a separator funnel and extracted with ∼50 ml of EtOAc. The combined organic extract was dried with MgSO₄, filtered, and concentrated *in vacuo* to yield the crude product, which was purified on a 40 g SiO₂ column using EtOAc as eluent giving 171 mg (53%) of the title compound as a white solid. NMR (300 MHz): 10.73 (s, 1H), 10.57 (s, 2H), 8.58-8.69 (m, 3H), 8.33 (d, 2H), 8.27 (d, 1H), 7.90 - 8.00 (m, 2H), 7.76 - 7.87 (m, 2H), 7.33 (d, 1H), 2.36 (s, 3H); *m*/*z* 443.

### Example 364

### 2-Methyl-N-[5-(methylamino)pyridin-3-yl]-5-{[3-(trifluoromethyl)benzoyl]amino}benzamide

To a 25 ml round bottom flask equipped with a magnetic stir bar was added *N*-[5-(formylamino)pyridin-3-yl]-2-methyl-5-{[3-(trifluoromethyl)benzoyl]amino}benzamide (Example 363; 0.12 g, 0.271 mmol). Anhydrous THF (3 ml) was added and the reaction mixture was cooled to 0°C with an ice bath. Lithium aluminium hydride (15 mg, 0.394 mmol) was added and the reaction was allowed to warm to room temperature with stirring over 3 h. The reaction was carefully quenched with ∼ 10 ml of H₂O and the resulting mixture was poured into a separator funnel and extracted with ∼50 ml of EtOAc. The combined organic extract was dried with MgSO₄, filtered, and concentrated *in vacuo* to yield the crude product, which was purified on a 40 g SiO₂ column using BtOAc as eluent giving 68 mg (59%) of the title compound as a white solid. NMR (300 MHz): 10.86 (s, 1H), 10.59 (s, 1H), 8.38 (d, 1H), 8.24 - 8.34 (m, 2H), 7.95 - 8.01 (m, 2H), 7.73 - 7.86 (m, 4H), 7.34 (d, 1H), 2.76 (s, 3H), 2.36 (s, 3H); *m*/*z* 430.

### Example 365

### N-{5-[(2,3-Dihydroxypropyl)aminolpyridin-3-yl}-2-methyl-5-{[3-(trifluoromethyl)benzol] amino}benzamide

To a 25 ml round bottom flask charged with a magnetic stir bar and *N*-(5-{[(2,2-dimethyl-1,3-dioxolan-4-yl)methyl]amino}pyridin-3-yl)-2-methyl-5-{[3-(trifluoromethyl) benzoyl]amino}benzamide (Example 331; 0.50 mg, 0.094 mmol) was added 3 ml of a 2 N HCl solution in Et₂O. Water (0.05 ml was added and the reaction was allowed to stir for 2 h at room temperature before being concentrated *in vacuo* to yield the crude product which was purified by semi-preparative reverse phase HPLC giving 0.037 g (80%) of the title compound as a white solid. MMR (300 MHz): 11.00 (s, 1H), 10.65 (s, 1H), 8.59 (s, 1H), 8.32-8.29 (m, 2H), 8.02 (dd, 2H), 7.99 (d, 1H), 7.83- 7.78 (m, 3H), 7.34 (d, 1H), 4.26-4.22 (m, 1H), 3.79-3.54 (m, 2H), 3.12-3.05 (m, 2H), 2.37 (s, 3H); *m*/*z* 489.

### Example 366

### 2-Chloro-N-{6-[(3-methoxypropanoyl)amino]pyridin-3-yl}-5-{[3-(trifluoromethyl) benzoyl]amino}benzamide

To a solution of 2-chloro-*N*-{6-amino-pyridin-3-yl)-5-{[3-(trifluoromethyl) benzoyl]amino}benzamide (Example 317; 40mg) in DCM (0.5 ml) at 0°C was added 3-methoxy propanoyl chloride (0.100 ml) followed by Et₃N (0.80 ml) and the resulting mixture was stirred at room temperature for 3 hours. The mixture was partitioned between H₂O and EtOAc, and the organic layer was washed with brine and dried with MgSO₄. Evaporation of the solvent gave a solid, which was purified by reverse phase HPLC (5-95% MeCN/H₂O, 15 min). The title compound (4 mg) was collected by evaporation. NMR (300 MHz): 10.64 (s, 1H), 10.02 (s, 1H), 8.18-8.47 (m, 3H), 7.82 - 7.96 (m, 3H), 7.43-7.66 (m, 3H), 7.07 (d, 1H), 3.69 - 3.72 (m, 2H), 3.33 (s, 3H), 2.11-2.21 (m, 2H); m/z 520.

### Example 367

### 5-({3-[2-(Dimethylamino)-1,1-dimethyl-2-oxoethyl]benzoyl}amino)-2-methyl-N-(6-methylpyridin-3-yl)benzamide

A solution of 5-({3-[2-(dimethylamino)-1,1-dimethyl-2-oxoethyl]benzoyl}amino)-2-methylbenzoic acid (Method 54, 71 mg, 0.193 mmol), 3-amino-6-picoline (21 mg, 0.193 mmol) and DIEA (0.10 ml, 0.58 mmol, 3.0 equiv) in DMF (2 ml) was treated with HATU (88 mg, 0.232 mmol, 1.2 equiv). The reaction mixture was stirred for 12 h at 40 °C. The reaction was quenched with 10% NaOH and extracted with EtOAc. The organics were dried with NaCl₍ₛₐₜ₎ and then Na₂SO₄₍ₛ₎ and removed under reduced pressure. The residue was purified directly by column chromatography (5% MeOH in EtOAc) to give 70 mg of product (73%). NMR: 11.16 (s, 1H), 10.46 (s, 1H), 9.15 (m, 1H), 8.48 (d, 1H), 8.02 (s, 1H), 7.84 (m, 4H), 7.51 (t, 1H), 7.34 (m, 2H), 2.78 (bs, 3H), 2.68 (s, 1H), 2.46 (s, 3H), 2.37 (s, 3H), 1.48 (s, 6H); *m*/*z* 459.

### Example 368

### 5-[(Dimethylamino)sulfonyl]-N-(4-methyl-3-{[(6-methylpyridin-3-yl)amino]carbonyl}phenyl)nicotinamide

A solution of 5-[({5-[(dimethylamino)sulfonyl]pyridin-3-yl}carbonyl)amino]-2-methylbenzoic acid (Method 55; 77 mg, 0.212 mmol), 3-amino-6-picoline (23 mg, 0.212 mmol) and DIEA (0.12 ml, 0.64 mmol, 3.0 equiv) in DMF (2 ml) was treated with HATU (97 mg, 0.254 mmol, 1.2 equiv). The reaction mixture was stirred for 12 h at 40 °C. The reaction, was quenched with 10% NaOH and extracted with EtOAc. The organics were dried with NaCl₍ₛₐₜ₎ and then Na₂SO₄₍ₛ₎ and removed under reduced pressure. The residue was purified directly by Gilson reverse phase preparatory HPLC (5-95% MeCN/H₂O) to give 18 mg of product (13%). NMR: 10.89 (s, 2H), 10.80 (s, 1H), 9.39 (d, 1H), 9.11 (d, 1H), 9.02 (s, 1H), 8.64 (t, 1H), 8.29 (dd, 1H), 7.96 (d, 1H), 7.82 (dd, 1H), 7.62 (d, 1H), 7.37 (d, 1H), 2.71 (s, 6H), 2.57 (s, 3H), 2.38 (s, 3H); *m*/*z* 454.

### Example 369

### N-{6-[(2-Aminoethyl)amino]pyridin-1-yl}-5-{[3-cyano-1-methylethyl)benzoyl]amino}-2-methylbenzamide

Tert-butyl {2-[(5-aminopyridin-2-yl)amino]ethyl}carbamate (Method 73; 331 mg, 1.24 mmol) was combined with 5-[3-(cyanodimethyl-methyl)-benzoylamino]-2-methyl-benzoic acid (Method 20; 400 mg, 1.24 mmol), HATU (708 mg, 1.86 mmol) and DIEA (0.65 ml, 3.72 mmol) in 2.48 ml of DMF and the reaction mixture was stirred overnight at 25°C. LC/MS confirmed the formation of tert-butyl [2-({5-[(5-{[3-(1-cyano-1-methylethyl)benzoyl]amino}-2-methylbenzoyl)amino]pyridin-2-yl}amino)ethyl]carbamate, and the reaction was quenched with H₂O. the mixture was partitioned between H₂O and EtOAc, and the organic layer was washed with brine and dried with MgSO₄. Evaporation of the solvent gave a light brown solid (*m*/*z* 557), which was redissolved in 10 ml of HCl in 1,4-dioxane and stirred overnight at 25°C. Evaporation of the solvent gave a brown gummy solid, which was purified by reverse phase HPLC (5-95% MeCN/H₂O, 20 min). The title compound (44 mg) was collected by evaporation as a brown solid. NMR, (300 MHz): 10.41 (s, 1H), 10.18 (s, 1H), 10.00 (s, 1H), 8.28 - 8.50 (m, 1H), 8.01 - 8.12 (m, 1H), 7.88 - 8.00 (m, 2H), 7.70 - 7.87 (m, 4H), 7.61 (t, 1H), 7.32 (d, 1H), 6.65 (s, 2H), 2.94 - 3.13 (m, 1H), 2.85 - 2.96 (m, 1H), 2.68 - 2.80 (m, 1H), 2.35 (s, 3H), 2.23 - 2.33 (m, 1H), 1.76 (s, 6H); *m*/*z* 457.

### Example 370

### 5-{[3-(1-Cyano-1-methylethyl)benzoyl]aminol-N-[5-(isopropylamino)pyridin-3-yl]-2-methylbenzamide

*N*-(5-Aminopyridin-3-yl)-5-{[3-(1-cyano-1-methylethyt)benzoyl]amino}-2-methylbenzamide (Example 205; 164 mg, 0.398 mmol) was combined with acetone and sodium triacetoxyborohydride (337 mg, 1.59 mmol) in 0.80 ml of THF and heated at 30°C in a sealed tube overnight. LC/MS confirmed the formation of the product and the mixture was partitioned between H₂O and EtOAc. The organic layer was washed with brine and dried with MgSO₄ Evaporation of the solvent gave a yellow solid, which was purified by reverse phase HPLC (5-95% MeCN/H₂O, 20 min). The title compound (40 mg) was collected by evaporation as a white solid. NMR (300 MHz): 10.91 (s, 1H), 10.47 (s, 1H), 8.39 (s, 1H), 8.06 (t, 1H), 7.93 - 8.01 (m, 2H), 7.74 - 7.85 (m, 5H), 7.63 (t, 1H), 7.36 (d, 1H), 2.37 (s, 3H), 1.76 (s, 6H), 1.18 (m, 7H); m/z 456.

### Example 371

### 5-{[3-(1-Cyano-1-methylethyl)benzol]amino}-2-methyl-N-(5-pyrrolidin-2-ylpyridin-3-yl)benzamide

*tert-*Butyl 2-{5-[(5-{[3-(1-cyano-1-methylethyl)benzoyl]amino}-2-methylbenzoyl)amino]pyridin-3-yl}pyrolidine-1-carboxylate (Example 324; 50 mg) was dissolved in MeOH (3 ml) and 4N HCl in 1,4-dioxane (2.5 ml) was added at 0°C and the resulting mixture was stirred for 2 hours at room temperature. Evaporation of the solvents afforded the title compound as an off-white solid (32 mg). NMR (300 MHz): 10.72 (s, 1H), 10.46 (s, 1H), 8.85 (s, 1H), 8.43 - 8.54 (m, 1H), 8.25 (s, 1H), 8.06 (s, 1H), 8.01 (t, 1H), 7.96 (d, 1H), 7.-3 - 7.81 (m, 2H), 7.61 (t, 1H), 7.34 (d, 1H), 4.23 (m, 1H), 3.73 (m, 2H), 2.27 (s, 3H), 1.93-2.22 (m, 4H), 1.78 (s, 6H), m/z 469.

### Examples 372-373

The following examples were made by the procedure of Example 371 using the appropriate starting materials.

| **Ex** | **Compound** | **NMR** | **M/z** | **SM** |
|---|---|---|---|---|
| **372** | 5-{[3-(1-Cyano-1-methylethyl)benzoyl ]amino}-2-methyl-*N*-{6-[(pyrrolidin-2-ylmethyl)amino]pyridin-3-yl}benzamide | 10.69 (s, 1H), 10.54 (s, 1H), 9.42 (bs, 1H), 9.32 (bs, 1H), 8.58 (s, 1H), 8.-2 - 8.15 (m, 2H), 7.-1 - 8.01 (m, 2H), 7.84 (d, 1H), 7.75 (d, 1H), 7.61 (t, 1H), 7.31 (d, 1H), 7.23 (d, 1H), 3.-5 - 3.85 (m, 4H), 3-3-3.26 (m, 1H), 2.36 (s, 3H), 2.-9 - 2.22 (m, 2H), 1.-3 - 2.01 (m, 2H), 1.76 (s, 6H) | 498 | Example 314 |
| **373** | 5-{[3-(1-Cyano-1-methylethyl)benzoyl ]amino}-2-methyl-*N*-[5-(pyrrolidin-2-ylmethoxy)pyridin-3-yl]benzamide | 10.35 (s, 1H), 8.04 (s, 1H), 7.-8-8.00 (m, 2H), 7.76 (d, 1H), 7.-5 - 7.73 (m, 2H), 7.-5 - 7.65 (m, 2H), 7.27 (d, 1H), 7.21 (s, 1H), 4.-0 - 4.56 (m, 1H), 4.33 (t, 1H), 3.-9 - 3.24 (m, 2H), 2.15 (s, 3H), 1.-6 - 2.04 (m, 4H), 1.75 (s, 6H) | 499 | Example 315 |

### Preparation of Starting Materials

### Method 1

### 3-Cyanomethyl-benzoic acid methyl ester

A suspension of methyl-3-(bromomethyl)benzoate (13.5 g, 58.9 mmol) and sodium cyanide (4.33 g, 88.4 mmol) in DMF (25 ml) and water (1 ml) was stirred at 75 °C for 5 h. The reaction mixture was quenched with water (50 ml) and extracted with EtOAc (100 ml × 3). The combined organics were dried and concentrated under reduced pressure. The resulting residue was purified by column chromatography utilizing an ISCO system (hexane-EtOAc) to give 7.2 g (70%) of colourless oil. NMR: 7.90 (s, 1H), 7.86 (d, 1H), 7.60 (d, 1H), 7.50 (m, 1H), 4.10 (s, 2H), 3.80 (s, 3H); *mlz* 175.

### Methods 2-7

The following compounds were prepared by the procedure of Method 1, using the appropriate starting material.

| **Meth** | **Compound** | **m/z** | **SM** |
|---|---|---|---|
| **2** | Methyl 4-chloro-3-(cyanomethyl)benzoate | 210 | Method 136 |
| **3** | [4-({[tert-Butyl(diphenyl)silyl]oxy}methyl)-2-thienyl]acetonitrile | 393 | Method 106 |
| **4** | Methyl 3-(cyanomethyl)-5-methylbenzoate | 189 | Method 137 |
| **5** | Methyl 5-(cyanomethyl)nicotinate | 177 | Method 140 |
| **6** | (2-Fluoro-3-methylphenyl)acetonitrile | 150 | 1-(Bromomethyl)-2-fluoro-3-methylbenzene |
| **7** | Methyl 5-(cyanomethyl)-2-fluorobenzoate | 195 | Method 141 |

### Method 8

### 3-(1-Cyano-1-methylethyl)benzoic acid methyl ester

A solution of 3-cyanomethyl-benzoic acid methyl ester (Method 1; 7.2 g, 41.1 mmol) in anhydrous DMSO (80 ml) was treated with NaH (60% in mineral oil, 4.9 g, 123.3 mmol). Methyl iodide was added dropwise at 0 °C. The reaction mixture was stirred at 25 °C for 12 h. The reaction mixture was quenched with water (200 ml) and extracted with EtOAc. The combined organics were dried and concentrated under reduced pressure. The crude product was purified by column chromatography utilizing an ISCO system (hexane-EtOAc) to give 5.5 g (66%) of a colourless oil. NMR: 8.05 (s, 1H), 7.90 (d, 1H), 7.75 (d, 1H), 7.55 (m, 1H), 3.80 (s, 3H), 1.62 (s, 6H); *m*/*z* 203.

### Methods 9-18

The following compounds were prepared by the procedure of Method 8, using the appropriate starting material.

| **Meth** | **Compound** | **m/z** | **SM** |
|---|---|---|---|
| **9** | 2-Methyl-2-(2-thienyl)propanenitrile | 152 | 2-Thienyl acetonitrile |
| **10** | Methyl 4-chloro-3-(1-cyano-1-methylethyl)benzoate | 238 | Method 2 |
| **11** | 2-[4-({[tert-Butyl(diphenyl)silyl]oxy}methyl)-2-thienyl]-2-methylpropanenitrile | 421 | Method 3 |
| **12** | Methyl 3-(1-cyano-1-methylethyl)-5-methylbenzoate | 217 | Method 4 |
| **13** | Methyl 5-(1-cyano-1-methylethyl)nicotinate | 205 | Method 5 |
| **14** | Methyl 5-(1-cyano-1-methylethyl)-2-fluorobenzoate | 222 | Method 7 |
| **15** | 2-(2-Fluoro-3-methylphenyl)-2-methylpropanenitrile | 178 | Method 6 |
| **16** | Methyl 2-(3-bromophenyl)-2-methylpropanoate | 258 | Method 89 |
| **17** | 2-(3-Bromophenyl)-2-methylpropyl methyl ether | 244 | Method 179 |
| **18** | 2-(3-Bromo-5-fluorophenyl)-2-methylpropanenitrile | 298 | Method 199 and Methyl iodide |

### Method 19

### 3-(1-cyano-1-methylethyl)benzoic acid

A solution of 3-(1-cyano-1-methylethyl)benzoic acid methyl ester (Method 8; 5.5 g, 27.1 mmol) in 100 ml of THF/MeOH/H₂O (3:1:1) was treated with lithium hydroxide (1.95 g) in 20 ml water. The mixture was stirred at 25 °C for 12 h. The solvent was removed under reduced pressure and the resulting solution was diluted with water, then acidified with 10% HCl to pH = 1-3. The resulting white solid (4.83 g, 94%) was filtered, washed with water and dried. NMR: 13.00 (s, 1H), 7.95 (s, 1H), 7.80 (d, 1H), 7.65 (d, 1H), 7.45 (m, 1H), 1.60 (s, 6H); *mlz* 189.

### Methods 20-55

The following compounds were prepared by the procedure of Method 19, using the appropriate starting material.

| **Meth** | **Compound** | **m/z** | **SM** |
|---|---|---|---|
| **20** | 5-[3-(Cyanodimethyl-methyl)-benzoylamino]-2-methyl-benzoic acid | 336 | Method 96 |
| **21** | 2-Methyl-5-{[3-(trifluoromethyl)benzoyl]-amino}benzoic acid | 323 | Method 97 |
| **22** | 4-[(4-Ethylpiperazin-1-yl)methyl]-3-(trifluoromethyl)benzoic acid | 316 | Method 143 |
| **23** | 4-[(Dimethylamino)methyl]-3-(trifluoromethyl)benzoic acid | 247 | Method 144 |
| **24** | 3-(3,3-Dimethylbut-1-yn-1-yl)benzoic acid | 203 | Method 115 |
| **25** | 3-(3-Hydroxy-3-methylbut-1-yn-1-yl)benzoic acid | 204 | Method 116 |
| **26** | 4-chloro-3-(1-cyano-1-methylethyl)benzoic acid | 224 | Method 10 |
| **27** | 3-(1-Cyano-1-methylethyl)-5-[(4-methylpiperazin-1-yl)methyl]benzoic acid | 301 | Method 145 |
| **28** | 3-(1-cyano-1-methylethyl)-5-[(dimethylamino)methyl] benzoic acid | 246 | Method 146 |
| **29** | 5-{[2-(1-Cyano-1-methylethyl)isonicotinoyl]amino}-2methylbenzoic acid | 323 | Method 98 |
| **30** | 5-Piperidin-1-ylnicotinic acid | 207 | Method 234 |
| **31** | 5-Morpholin-4-ylnicotinic acid | 209 | Method 235 |
| **32** | 5-(Diethylamino)nicotinic acid | 195 | Method 236 |
| **33** | 5-(1-Cyano-1-methylethyl)nicotinic acid | 191 | Method 13 |
| **34** | 3-Cyclopropylbenzoic acid | 163 | Method 237 |
| **35** | 4-[(4-Methylpiperazin-1-yl)methyl]-3-(trifluoromethyl) benzoic acid | 303 | Method 147 |
| **36** | 2-[(Dimethylamino)methyl]-5-(trifluoromethyl)benzoic acid | 248 | Method 148 |
| **37** | 3-(1-Cyano-1-methylethyl)-5-[(methylthio)methyl]benzoic acid | 250 | Method 253 |
| **38** | Sodium [3-carboxy-5-(1-cyano-1-methylethyl)phenyl] methanesulfonate | 283 | Method 254 |
| **39** | 1,3,3-Trimethyl-2-oxoindoline-5-carboxylic acid | 219 | Method 218 |
| **40** | 3-{1-[(Dimethylamino)sulfonyl]-1-methylethyl}benzoic acid | 271 | Method 160 |
| **41** | 3-(1,1-Dimethylprop-2-yn-1-yl)benzoic acid | 188 | Method 161 |
| **42** | 3-(1,1-Dimethylbut-2-yn-1-yl)benzoic acid | 202 | Method 164 |
| **43** | 3-(2,3-Dihydroxy-1,1-dimethylpropyl)benzoic acid | 224 | Method 166 |
| **44** | 3-(1-Cyclopropyl-1-methylethyl)benzoic acid | 205 | Method 238 |
| **45** | 3-{[(Dimethylamino)carbonyl]amino}benzoic acid | 209 | Method 168 |
| **46** | 3-(1-cyclopropyl-1-hydroxyethyl)benzoic acid | 206 | Method 170 |
| **47** | 3-(1,1-Difluoroethyl)benzoic acid | 186 | Method 171 |
| **48** | 3-[Cyclopropyl(hydroxy)methyl]benzoic acid | 193 | Method 172 |
| **49** | 3-(1-Methylcyclopropyl)benzoic acid | 177 | Method 239 |
| **50** | 3-(1-Cyano-1-methylethyl)-5-{[methoxy(methyl)amino] methyl}benzoic acid | 262 | Method 174 |
| **51** | 3-(1,3-Thiazol-2-yl)benzoic acid | 205 | Method 176 |
| **52** | 2-(3-Bromophenyl)-2-methylpropanoic acid | 244 | Method 16 |
| **53** | 5-(1-Cyano-1-methylethyl)-2-fluorobenzoic acid | 208 | Method 14 |
| **54** | 5-({3-[2-(Dimethylamino)-1,1-dimethyl-2-oxoethyl]benzoyl}amino)-2-methylbenzoic acid | 369 | Method 182 |
| **55** | 5-[({5-[(Dimethylamino)sulfonyl]pyridin-3-yl}carbonyl)amino]-2-methylbenzoic acid | 364 | Method 183 |

### Method 56

### 4-Methyl-3-trifluoromethyl-benzoic acid methyl ester

A solution of KOH (84 mg, 1.5 mmol) in DMSO (5 ml) was stirred for 30 min at 25 °C. The above slurry was then treated with 4-methyl-3-trifluoromethyl-benzoic acid (306 mg, 1.5 mmol) in DMSO (5 ml) and the resulting mixture was stirred for 15 min, and iodomethane (426 mg, 3 mmol) was then added to the mixture. The reaction was stirred for 2 h at 25 °C and then quenched with water. The resulting solution was extracted with EtOAc. The organic layer was washed with NaCl(ₛₐₜ) and dried with Na₂SO₄₍ₛ₎. The organics were removed under reduced pressure to give the title compound as an oil 327 mg (100%). NMR: 8.10 (m, 2H), 7.60 (s, 1H), 3.86 (s, 3H), 2.45 (s, 3H); *m*/*z* 218.

### Method 57

### 2-Methyl-5-nitro-N-pyridin-3-ylbenzamide

A solution of 2-methyl-5-nitrobenzoic acid (1.4 grams, 7.7 mmol) and 3-aminoaniline (0.73 g, 7.7 mmol) in DMF (10 ml) was treated with HATU (2.2 grams, 7.7 mmol) and pyridine (5 equiv). The resulting solution was stirred at 25°C for 48 h. The solvent was evaporated under reduced pressure and the residue was purified by column chromatography utilizing an ISCO system to afford 1.65 grams (84% yield) of the title compound as a white, crystalline solid. NMR (300 MHz): 10.82 (s, 1H), 8.91 (d, 1H), 8.35-8.39 (m, 2H), 8.26-8.29 (m, 1H), 8.20(d, 1H), 7.95 (s, 1H), 7.64 (d, 1H), 7.43-7.48 (m, 1H), 2.68 (s, 3H); *m*/*z* 258.

### Methods 58-67

The following compound was prepared by the procedure of Method 57, using the appropriate starting material.

| **Meth** | **Compound** | **m/z** | **SM** |
|---|---|---|---|
| **58** | 2-Fluoro-5-nitro-*N-*pyridin-3-ylbenzamide | 261 | 2-Fluoro-5-nitrobenzoic acid |
| **59** | 2-Chloro-5-nitro-*N-*pyridin-3-ylbenzamide | 278 | 2-Chloro-5-nitrobenzoic acid |
| **60** | 2-Methyl-5-nitro-*N-*(6-amino-5-chloropyridin-3-yl)benzamide | 308 | 2-Methyl-5-nitrobenzoic acid and Method 186 |
| **61** | *N-*(5-Methoxypyridin-3-yl)-2-methyl-5-nitrobenzamide | 288 | 2-Methyl-5-nitrobenzoic acid and Method 118 |
| **62** | 2-Chloro-*N-*(5-methoxypyridin-3-yl)-5-nitrobenzamide | 309 | 2-Chloro-5-nitrobenzoic acid and Method 118 |
| **63** | *N-*[6-(Acetylamino)pyridin-3-yl]-2-methyl-5-nitrobenzamide | 315 | 2-Methyl-5-nitrobenzoic acid and *N-*(5-amino pyridin-2-yl)acetamide |
| **64** | 2-Methoxy-5-nitro-*N-*pyridin-3-ylbenzamide | 273 | 2-Methoxy-5-nitrobenzoic acid and 3-aminopyridine |
| **65** | 2-Chloro-*N-*(5-fluoropyridin-3-yl)-5-nitrobenzamide | 296 | 2-Chloro-5-nitrobenzoic acid and Method 252 |
| **66** | *N-*(5-Bromopyridin-3-yl)-2-chloro-5-nitrobenzamide | 357 | 2-Chloro-5-nitrobenzoic acid and Method 119 |
| **67** | *N-*(5-Isopropoxypyridin-3-yl)-2-methyl-5-nitrobenzamide | 316 | Method 219 and 2-methyl-5-nitrobenzoic acid |

### Method 68

### 5-Amino-2-methyl-N-pyridin-3-ylbenzamide

A solution of 2-methyl-5-nitro-*N-*pyridin-3-ylbenzamide (Method 57; 1.65 grams, 6.4 mmol) in MeOH was treated with 10% Pd/C and hydrogenated for 45 min at 10 psi using a Parr Hydrogenator. The catalyst was removed by filtration and the solvent evaporated. The crude product was purified by column chromatography utilizing an ISCO system to provide 975 mgs (67% yield) of white solid. NMR (300 MHz): 10.25 (s, 1H), 8.71-8.72 (m, 1H), 8.11-8.14 (m, 1H), 8.01 (d, 1H), 8.18-8.23 (m, 1H), 6.79 (d, 1H), 6.63-6.64 (m, 1H), 6.42-6.46 (m, 1H), 4.96 (bs, 2H), 2.04 (s, 3H); *m*/*z* 228.

### Methods 69-74

The following compounds were prepared by the procedure of Method 68, using the appropriate starting material.

| **Method** | **Compound** | **M/z** | **SM** |
|---|---|---|---|
| **69** | 5-Amino-*N-*(5-methoxypyridin-3-yl)-2-methylbenzamide | 258 | Method 61 |
| **70** | *N-*[6-(Acetylamino)pyrdin-3-yl]-5-amino-2-methylbenzamide | 285 | Method 63 |
| **71** | 5-Amino-2-methoxy-*N-*pyridin-3-ylbenzamide | 244 | Method 64 |
| **72** | 5-Amino-*N-*(5-isopropoxypyridin-3-yl)-2-methylbenzamide | 286 | Method 67 |
| **73** | Tert-Butyl {2-[(5-aminopyridin-2-yl)amino]ethyl}carbamate | 253 | Method 207 |
| **74** | 2-Methylpyridine-3,5-diamine | 124 | Method 200 |

### Method 75

### 5-Amino-2-fluoro-N-pyridin-3-ylbenzamide

A solution of 2-fluoro-5-nitro-*N-*pyridin-3-ylbenzamide (Method 58; 127 mg, 0.487 mmol) in MeOH (2 ml) was treated with an excess of zinc metal and acetic acid (150 µl, 2.5 mmol). The reaction was heated to reflux for 30 min then cooled to 25 °C. The solids were removed by filtration through a pad of diatomaceous earth and the solvents were removed under reduced pressure. The crude product was purified by column chromatography utilizing an ISCO system to provide the title compound (35 mgs, 31% yield); *m*/*z* 231.

### Methods 76-82

The following compounds were prepared by the procedure of Method 75, using the appropriate starting material.

| **Meth** | **Compound** | **m/z** | **SM** |
|---|---|---|---|
| **76** | 5-Amino-2-chloro-*N-*pyridin-3-ylbenzamide | 248 | Method 59 |
| **77** | 3-Bromopyridine-2,5-diamine | 188 | 3-Bromo-5-nitro pyridin-2-amine |
| **78** | 5-Amino-2-chloro-*N-*(5-methoxypyridin-3-yl)benzamide | 278 | Method 62 |
| **79** | 5-Amino-2-chloro-*N-*(5-fluoropyridin-3-yl)benzamide | 267 | Method 65 |
| **80** | 5-Amino-*N-*(5-bromopyridin-3-yl)-2-chlorobenzamide | 327 | Method 66 |
| **81** | 5-Amino-*N-*(6-amino-5-chloropyridin-3-yl)-2-methyl benzamide | 278 | Method 60 |
| **82** | *tert*-Butyl 2-{[(5-aminopyridin-2-yl)amino]methyl} pyrrolidine-1-carboxylate | 294 | Method 157 |

### Method 83

### 4-(5-Nitropyridin-2-yl)morpholine

A solution of 2-chloro-5-nitropyridine (400 mg, 1.52 mmol) and DIEA (440 µL, 1.52 mmol) in EtOH (10 ml) was treated with morpholine (660 µL, 4.56 mmol). The solution was heated up at 70°C for 12 h. The solvents were removed under reduced pressure. M/z 211.

### Method 84

### 6-Morpholin-4-ylpyridin-3-amine

A solution of 4-(5-nitropyridin-2-yl)morpholine (Method 83; 418 mg, 2 mmol) in MeOH (5 ml) was treated with a solution of ammonium chloride (540 mg, 10 mmol) and iron powder in water (5 ml). The resulting solution was heated to 78 °C for 2 h. The solution was then filtered at 50 °C and the solvents were removed under reduced pressure. The crude product was dissolved in acetone and filtered to remove any inorganic salts. The organic phase was then concentrated in vacuo to give the title compound material. M/z 179.

### Method 85

The following compound was prepared by the procedure of Method 84, using the appropriate starting material.

| **Meth** | **Compound** | **m/z** | **SM** |
|---|---|---|---|
| **85** | 6-Chloro-5-methylpyridin-3-amine | 337 | 2-Chloro-3-methyl-5-nitropyridine |

### Method 86

### Methyl 2-methyl-5-nitrobenzoate

A solution of 2-methyl-5-nitrobenzoic acid (3.9 g, 21.5 mmol) in MeOH (20 ml) was treated with HCl(g) for 10 min. The reaction was then refluxed in a sealed tube at 65°C for 24 h. The solvent was evaporated giving a cream coloured solid (4.8 g) which was dissolved in EtOAc (200 ml), washed with H₂O (200 ml) and brine (200 ml), and dried with MgSO₄. The solvents were removed under reduced pressure to give a white solid (3.4 g). *M*/*z:* 179.

### Methods 87-91

The following compounds were prepared by the procedure of Method 86, using the appropriate starting material.

| **Ex** | **Compound** | **M/z** | **SM** |
|---|---|---|---|
| **87** | Methyl 3-acetylbenzoate | 179 | 3-acetylbenzoic acid |
| **88** | Methyl 2-fluoro-5-methylbenzoate | 169 | 2-Fluoro-5-methyl benzoic acid |
| **89** | Methyl (3-bromophenyl)acetate | 230 | (3-Bromophenyl)-acetic acid |
| **90** | Methyl 5-aminopyridine-2-carboxylate | 153 | 5-aminopyridine-2-carboxylic acid |
| **91** | Methyl 2-chloro-5-nitrobenzoate | 216 | 2-chloro-5-nitrobenzoic acid |

### Method 92

### Methyl 5-amino-2-methylbenzoate

A solution of methyl 2-methyl-5-nitrobenzoate (Method 86; 3.4 g) and 10% palladium on carbon (672 mg) in MeOH (20 ml) was treated with H₂ for 48 h. The reaction mixture was then filtered through diatomaceous earth and washed with MeOH (20 ml) and HtOAc (10 ml). The solvents were removed under reduced pressure to give a brown oil (2.7 g). M/z 165.

### Methods 93-95

The following compounds were prepared by the procedure of Method 92, using the appropriate starting material.

| **Meth** | **Compound** | **m/z** | **SM** |
|---|---|---|---|
| **93** | N²-Methylpyridine-2,5-diamine | 123 | Method 132 |
| **94** | 3-Methylpyridine-2,5-diamine | 123 | 3-Methyl-5-nitropyridin-2-amine |
| **95** | Pyridine-3,5-diamine | 109 | 2-Chloro-3,5-dinitropyridine |

### Method 96

### Methyl 5-{[3-(1-cyano-1-methylethyl)benzoyl]amino}-2-methylbenzoate

A solution of methyl 5-amino-2-methylbenzoate (Method 92; 2:7 g, 16.4 mmol), 3-(cyano-dimethyl-methyl)-benzoic acid (3.133 g, 16.6 mmol) and DIEA (8.67 ml, 49.8 mmol) in DMF (33 ml) at 0 °C was treated with HATU (9.466 g, 24.9 mmol). The reaction was stirred at 25 °C for 24 h. The reaction mixture was quenched with H₂O (30 ml) and then extracted with EtOAc (100 ml). The organics were washed with NaCl₍ₛₐₜ₎ (200 ml) and dried with MgSO₄. The solvents were removed under reduced pressure to give a reddish brown oil (5.58 g) of the title compound. *M*/*z* 336.

### Methods 97-99

The following compounds were prepared by the procedure of Method 96, using the appropriate starting material.

| **Meth** | **Compound** | **m/z** | **SM** |
|---|---|---|---|
| **97** | Methyl 2-methyl-5-{[3-(trifluoromethyl)benzoyl) amino}benzoate | 337 | 3-(Trifluoromethyl)-benzoic acid |
| **98** | Methyl 5-{[2-(1-cyano-1-methylethyl) isonicotinoyl]amino}-2-methylbenzoate | 337 | Method 112 |
| **99** | *N-*{6-[(2-{[tert-Butyl(dimethyl)silyl]oxy} ethyl)amino]pyridine-3-yl}-2-methyl-5-{[3-(trifluoromethyl)benzoyl]amino}benzamide | 573 | Method 188 and Method 21 |

### Method 100

### 5-Methylpyridin-3-amine

A solution of 6-chloro-5-methylpyridin-3-amine (Method 85; 2 mmol) and 10% Pd on carbon (20% w/w) in MeOH (10 ml) was treated with H₂. The solution was stirred at 25°C for 12 h. The reaction mixture was filtered through a pad of diatomaceous earth and the solvents were removed under reduced pressure to yield the title compound which was used without further purification. *M*/*z* 108.

### Method 101

### tert-Butyl(diphenyl)(3-thienylmethoxy)silane

To 3-thienylmethanol (5.0 g, 43.8 mmol) was added 86 ml of DMF followed by imidazole (8.94 g, 131.4 mmol). The reaction mixture was cooled to 0 °C and treated with *tert*-butylchlorodiphenylsilane (15.0 g, 54.7 mmol) and was allowed to stir 6 h to 25 °C before being quenched by the addition of 250 ml saturated aqueous NH₄Cl. The resulting mixture was extracted with EtOAc (3 x 125 ml). The combined organic phase was washed 1x with brine (100 ml), dried with MgSO₄, and concentrated *in vacuo.* The crude reaction product was purified on 120 g SiO₂ using hexanes/EtOAc 10:1 as eluent giving 14.8 g of the title compound as a colourless oil (96 %). *M*/*z* 353.

### Method 102

The following compound was prepared by the procedure of Method 101, using the appropriate starting material.

| **Meth** | **Compound** | **m/z** | **SM** |
|---|---|---|---|
| 102 | 3-Bromo-5-({[*tert*-butyl(diphenyl)silyl]oxy}methyl)pyridine | 427 | Method 240 |

### Method 103

### 2-(5-Formyl-2-thienyl)-2-methylpropanenitrile

THF (5.8 ml) was added to 2-methyl-2-(2-thienyl)prypanenitrile (Method 9; 0.260 g, 1.71 mmol) and the reaction mixture was cooled to -78°C. To the cooled reaction was added 1.26 ml of *tert*-butyl lithium (1.7 M solution in pentanes) dropwise via syringe. The resulting bright yellow mixture was allowed to stir for 1 h before DMF (0.330 ml, 4.27 mmol) was added via syringe. The reaction was stirred for 6 h at -78°C before being quenched by the addition of 25 ml of saturated aqueous NH₄Cl. The resulting mixture was extracted with EtOAc (3 × 25 ml). The combined organic phase was washed 1 × with brine (50 ml), dried with MgSO₄, and concentrated *in vacuo* giving 0.271 g of the title compounds (88 %) as a colourless oil. *M*/*z* 180.

### Method 104

The following compound was prepared by the procedure of Method 103, using the appropriate starting material.

| **Meth** | **Compound** | **m/z** | **SM** |
|---|---|---|---|
| 104 | 4-({[*tert*-Butyl(diphenyl)silyl]oxy}methyl)thiophene-2-carbaldehyde | 381 | Method 101 |

### Method 105

### [4-({[tert-Butyl(diphenyl)silyl]oxy}methyl)-2-thienyl]methanol

4-({[*tert*-Butyl(diphenyl)silyl]oxy}methyl)thiophene-2-carbaldehyde (Method 104; 3.99 g, 10.48 mmol) was dissolved in MeOH (50 ml). With stirring, NaBH₄ (0.792 g, 20.96 mmol) was added in one portion. After 1h, the reaction was carefully quenched with a solution of NH₄Cl₍ₛₐₜ₎ (∼250 ml). The resulting mixture was extracted with EtOAc (3 x 125 ml). The combined organic phase was washed with brine (250 ml), dried with MgSO₄ and concentrated *in vacuo* giving the crude reaction product which was purified on 120 g SiO₂ using hexanes/EtOAc 5:2 as eluent gibing 3.99 g of the title compound as a colourless oil (98 %) m/z 384.

### Method 106

### {[5-(Brothomethyl)-3-thienyl]methoxy}(tert-butyl)diphenylsilane

Anhydrous THF (45 ml) was added to [4-({[*tert*-butyl(diphenyl)silyl]oxy}methyl)-2-thienyl]methanol (Method 105; 4.2 g, 10.98 mmol). Phosphorous tribromide (3.56 g, 13.17 mmol) was added dropwise via syringe and the reaction was allowed to stir for 1 h. at 25°C before being quenched by NaHCO₃₍ₛₐₜ₎ (250 ml). The reaction mixture was extracted with EtOAc (2 × 250 ml) and the combined organic phase was dried with MgSO₄ and concentrated *in vacuo* to yield the crude reaction product which was purified on 120 g SiO₂ using hexanes/EtOAc 10:1 as eluent giving 3.70 g of the title compound as a yellow oil (76 %) m/z 447.

### Method 107

### 2-[4-(Hydroxymethyl)-2-thienyl]-2-methylpropanenitrile

Anhydrous THF (25 ml) was added to 2-[4-({[*tert*-butyl(diphenyl)silyl]oxy}methyl)-2-thienyl]-2-methylpropanenitrile (Method 11; 0.880g, 2.10 mmol). A 1 M solution of tetrabutylammonium fluoride in THF (5.25 mmol) was added dropwise via syringe and the reaction was allowed to stir for 12 h at 25 °C before being quenched with NH₄Cl₍ₛₐₜ₎ (50 ml). The reaction mixture was extracted with EtOAc (2 x 50 ml) and the combined organic phase was dried with MgSO₄ and concentrated *in vacuo* to yield the crude reaction product which was purified on 40 g SiO₂ using hexanes/EtOAc 2:1 as eluent giving 0.270 g of the title compound as a colourless oil (71 %) m/z 182.

### Method 108

### 2-(4-Formyl-2-thienyl)-2-methylpropanenitrile

To DMSO (0.277 g, 3.55 mmol) was added 10 ml of anhydrous DCM. The reaction was cooled to -78 °C and oxalyl chloride (0.225 g, 1.78 mmol) was added dropwise via syringe and the reaction was allowed to stir for 30 min. at this temperature. A 1 M solution of 2-[4-(hydroxymethyl)-2-thienyl)-2-methylpropanenitrile (Method 107; 0.270 g, 1.48 mmol) in DCM was then added dropwise via syringe and the reaction was allowed to stir for 30 min. at this temperature. Triethylamine (0.718 g, 7.40 mmol) was then added and the reaction was allowed to warm to 25 °C with stirring over 1 h before being quenched with NaHCO_{3 (sat)} (250 ml). The reaction mixture was then extracted with EtOAc (2 x 50 ml) and the combined organic phase was dried with MgSO₄ and concentrated *in vacuo* to yield the crude reaction product which was purified on 40 g SiO₂ using hexanes/EtOAc 10:1 as eluent giving 0.262 g of the title compound as a colourless oil (99 %) m/z 180.

### Method 109

### 5-(1-cyano-1-methylethyl)thiophene-2-carboxylic acid

To 2-(5-formyl-2-thienyl)-2-methylpropanenitrile (Method 103; 0.271 g, 1.51 mmol) was added 7.5 ml of tertiary butyl alcohol and 4.5 ml of 2-methyl-2-butene. The reaction mixture was treated dropwise with an aqueous pre-mixed solution of NaClO₂ (1.22 g, 13.60 mmol) and NaH₂PO₄ (1.45 g, 10.57 mmol) in 7 ml of H₂O. The reaction mixture was stirred for 30 min. at 25 °C before the volatiles were removed on a rotary evaporator. The resulting crude product was washed with NaHCO₃₍ₛₐₜ₎ (1 x 50 ml) and extracted was extracted with EtOAc (3 x 25 ml). The combined organic phase was washed 1 × with brine (50 ml), dried with MgSO₄, and conc. *in vacuo* giving 0.265 g of the title compound (90 %) as a white solid. *Mlz* 196*.*

### Method 110

The following compound was prepared by the procedure of Method 109, using the appropriate starting material.

| **Meth** | **Compound** | **m/z** | **SM.** |
|---|---|---|---|
| **110** | 5-(1-Cyano-1-methylethyl)thiophene-3-carboxylic acid | 196 | Method 108 |

### Method 111

### 2-Methyl-2-(4-methylpyridin-2-yl)propanenitrile

2-Fluoro-4-methylpyridine (1.00 g, 9.00 mmol), 2-methylpropanenitrile (2.48g, 36 mmol), and anhydrous toluene (30 ml) were stirred. Potassium hexamethyldisilazide (13.5 mmol) was added and the reaction was refluxed for 1 h. before being cooled to 25 °C. The reaction was quenched with NH₄Cl₍ₛₐₜ₎ (50 ml) and the mixture was extracted with EtOAc (2 x 50 ml). The combined organic phase was dried with MgSO₄ and concentrated in *vacuo* to yield the crude reaction product which was purified on 40 g SiO₂ using hexanes/EtOAc 5:1 as eluent giving 0.870, g of the title compound as a colourless oil (60 %). M/z 161.

### Method 112

### 2-(1-Cyano-1-methylethyl)isonicotinic acid

Water (15 ml) was added to 2-methyl-2-(4-methylpyridin-2-yl)propanenitrile (Method 111; 0.870 g, 5.43 mmol). The reaction mixture was heated to 60 °C and KMnO₄ (4.3 g, 27 mmol) was added. The reaction was heated to reflux for 2 h, and was then filtered through a bed of diatomaceous earth. The pH was adjusted to 4 by the careful addition of 1 N HCl and the aqueous phase was extracted with EtOAc (4 x 25 ml): The organic phase was dried with MgSO₄ and concentrated in *vacuo* to yield the crude reaction product which was purified on 40 g SiO₂ using EtOAc/MeON 10:1 as eluent giving 0.700 g of the title compound as a white solid (68 %); *m*/*z* 191.

### Methods 113-114

The following compounds were prepared by the procedure of Method 112, using the appropriate starting material.

| **Meth** | **Compound** | **m/z** | **SM** |
|---|---|---|---|
| **113** | 3-tert-Butylbenzoic acid | 179 | 1-tert-Butyl-3-methylbenzene |
| **114** | 3-(1-Cyano-1-methylethyl)-2-fluorobenzoic acid | 208 | Method 15 |

### Method 115

### Ethyl 3-(3,3-dimethylbut-1-yn-1-yl)benzoate

MeCN (8.70 ml) was added to ethyl 3-bromobenzoate (0.500 g, 2.18 mmol). Triethylamine (1.53 ml, 10.9 mmol) was added followed by 3,3-dimethylbut-1-yne (0.27 g, 3.27 mmol). With stirring Pd(PPh₃)₄ (0.25 g, 0.21 mmol) and Cul .(0.083 g, 0.436 mmol) were added and the reaction was warmed to 60 °C for 4 h. The reaction was then diluted with EtOAc (~50 ml) and filtered through a pad of SiO₂, and concentrated *in vacuo.* The crude product was purified on 40 g SiO₂ using hexanes/P-tOAc 10:1 as eluent giving 0.45 g of the title compound as a colourless oil (91 %); m/z 231.

### Method 116

The following compound was prepared by the procedure of Method 115, using the appropriate starting material.

| **Meth** | **Compound** | **m/z** | **SM** |
|---|---|---|---|
| **116** | Methyl 3-(3-hydroxy-3-methylbut-1-yn-1-yl)benzoate | 218 | Methyl 3-bromobenzoate and 2-methylbut-3-yn-2-ol |

### Method 117

### 5-Methoxynicotinic acid

A solution of methyl 5-hydroxynicotinate (633 mg, 4.1 mmol) in DMSO (7 ml) was treated with potassium hydroxide (918 mg, 16:4 mmol). The solution was stirred at 25 °C for 1 h. Methyl iodide (2.0 M in TMF, 2.25 ml, 4.5 mmol) was then added. The solution was stirred an additional 1 h. The reaction was quenched with H₂O and extracted with EtOAc. The water layer was concentrated under reduced pressure to give 494 mg of crude product, 78.8%. *M*/*z* 153.

### Method 118

### 5-Methoxypyridin-3-amine hydrochloride:

A solution of 5-methoxynicotinic acid (Method 117; 494 mg, 3.24 mmol) and DIEA (1.1 ml, 6.5 mmol) in tert-BuOH (16 ml) was treated with diphenylphosphoryl azide (1.4 ml, 6.5 mmol). The resulting solution was heated at 85 °C for 5 h. The solution was then concentrated under reduced pressure and the crude product was dissolved in 4.0 M HCl in dioxane (20 ml). The solution was stirred at 25 °C for 12 h. The solvents were removed under reduced pressure to give 392 mg, 75.4% of the title compound as its hydrochloride salt. M/z 124.

### Method 119

The following compounds were prepared by the procedure of Method 118, using the appropriate starting material.

| **Meth** | **Compound** | **m/z** | **SM** |
|---|---|---|---|
| **119** | 5-Bromopyridin-3-amine | 173 | 5-Bromonicotinic acid |

### Method 120

### 3-[(Dimethylamino)sulfonyl]benzoic acid

A solution of 3-(chlorosulfonyl) benzoic acid (2.60 g, 12 mmol) in DCM (20 ml) was treated with dimethylamine (2.0 M in THF, 20 ml, 40 mmol, 3.3 equiv). After 30 min, the reaction was quenched with 10% HCl and extracted with EtOAc. The organics were washed with NaCl₍ₛₐₜ₎ and then dried with Na₂SO₄₍ₛ₎. The organics were then removed under reduced pressure to give 1.80 g, 65%; *m*/*z* 229.

### Methods 121-131

The following compounds were prepared by the procedure of Method 120, using the appropriate starting material.

| **Meth** | **Compound** | **m/z** | **SM** |
|---|---|---|---|
| **121** | 3-(Azetidin-1-ylsulfonyl)-benzoic acid | 241 | Azetidine |
| **122** | 3-[(Cyclopropylamino)sulfonyl]benzoic acid | 241 | Cyclopropylamine |
| **123** | 3-{[3-(Hydroxymethyl)piperidin-1-yl]sulfonyl}benzoic acid | 300 | Piperidin-3-ylmethanol , |
| **124** | 3-{[2-(Hydroxymethyl)piperidin-1-yl]sulfonyl} benzoic acid | 300 | Piperidin-2-ylmethanol |
| **125** | 3-{[Methoxy(methyl)amino]sulfonyl}benzoic acid | 246 | (Methoxyamino)-methane |
| **126** | 3-{[(2,3-Dihydroxypropyl)(methyl)amino] sulfonyl}benzoic acid | 304 | 3-(Methylamino) propane-1,2-diol |
| 127 | 3-{[Tetrahydrofuran-2-ylmethyl)amino]sulfonyl} benzoic acid | 286 | (Tetrahydrofuran-2-ylmethyl)amine |
| 128 | 3-(Morpholin-4-ylsulfonyl)benzoic acid | 272 | Morpholine |
| 129 | *N,N*-Dimethylpyridine-3-sulfonamide | 187 | 3-Pyridine sulfonyl chloride hydrochloride and dimethylamine |
| 130 | 3-(Aminosulfonyl)benzoic acid | 202 | Ammonia |
| 131 | 3-{[4-(Hydroxymethyl)piperidin-1-yl]sulfonyl}benzoic acid | 300 | Piperidin-4-ylmethanol |

### Method 132

### N-Methyl-5-nitropyridin-2-amine

A solution of 2-chloro-5-nitropyridine (400 mg, 1.52 mmol) was dissolved in 2.0 M methyl amine in MeOH (5 ml). The solution was heated up at 70 °C for 12 h. The solvents were removed under reduced pressure; *m*/*z* 153.

### Method 133

### 5-Amino-N-methylnicotinamide

A solution of 5-aminonicotinic acid (414 mg, 3 mmol), DIEA (1.57 ml, 9 mmol) and methyl amine (2.0 M in THF, 4.5 ml, 9 mmol) in DMF (10 ml) was treated with HATU (1.71 g, 4.5 mmol). The reaction was stirred for 5 h and then quenched with H₂O (30 ml). The reaction mixture was extracted with EtOAc (50 ml), washed with NaCl₍ₛₐₜ₎ (20 ml) and dried with MgSO₄. The organics were removed under reduced pressure; *m*/*z* 151.

### Method 134

The following intermediate were prepared according to the procedure of Method 133 using the appropriate starting materials.

| **Method** | **Compound** | **m/z** | **SM** |
|---|---|---|---|
| 134 | 5-Aminonicotinamide | 138 | ammonia |

### Method 135

### 4-Bromomethyl-3-trifluoromethyl-benzoic acid methyl ester

A suspension of 4-methyl-3-trifluoromethyl-benzoic acid methyl ester (Method 56; 327 mg, 1.5 mmol), NBS (267 mg, 1.5 mmol) and catalytic amount of benzoyl peroxide in CCl₄ (10 ml) was heated to reflux for 3 h. The reaction mixture was cooled to 25 °C, filtered through a pad of silica gel, and washed with DCM. The organics were removed under reduced pressure and the crude product was purified by column chromatography utilizing an ISCO system (hexane-EtOAc) to give 252 mg (56.5%). NMR: 7.70-8.25 (m, 3H), 4.85 (s, 2H), 3.91 (s, 3H); *m*/*z* 297.

### Methods 136-142

The following compounds were prepared by the procedure of Method 135, using the appropriate starting material.

| **Meth** | **Compound** | **m/z** | **SM.** |
|---|---|---|---|
| 136 | Methyl 3-(bromomethyl)-4-chlorobenzoate | 264 | Methyl 4-chloro-3-methylbenzoate |
| 137 | Methyl 3-(bromomethyl)-5-methylbenzoate | 243 | Methyl 3,5-dimethylbenzoate |
| 138 | Methyl 3-(bromomethyl)-5-(1-cyano-1-methylethyl)benzoate | 296 | Method 12 |
| 139 | Methyl 2-(bromomethyl)-5-(trifluoromethyl)benzoate | 298 | Methyl 2-methyl-5-(trifluoromethyl)benzoate |
| 140 | Methyl 5-(bromomethyl)nicotinate | 231 | Methyl 5-methylnicotinate |
| 141 | Methyl 5-(bromomethyl)-2-fluorobenzoate | 248 | Method 88 |
| 142 | Methyl4-(bromomethyl)-3-(trifluoromethyl)benzoate | 298 | Methyl 4-methyl-3-(trifluoromethyl)benzoate |

### Method 143

### Methyl 4-[(4-ethylpiperazin-1-yl)methyl]-3-(trifluoromethyl)benzoate

A mixture of 4-bromomethyl-3-trifluoromethyl-benzoic acid methyl ester (Method 135; 252 mg, 0.85 mmol), *N-*ethyl piperazine (193 mg, 1.70 mmol) and potassium carbonate (235 mg, 1.70 mmol) in MeCN (10 ml) was stirred at 80 °C for 4 h. The reaction mixture was then loaded on silica gel and purified by column chromatography utilizing an ISCO system (hexane-EtOAc) to give 172 mg, (61.5%). NMR: 8.20 (d, 1H), 8.15 (s, 1H), 7.94 (d, 1H), 3.89 (s, 3H), 3.68 (s, 2H), 2.40 (bs, 8H), 2.30 (q, 2H), 0.98 (t, 3H); *m*/*z* 330.

### Methods 144-145

The following compounds were prepared by the procedure of Method 143, using the appropriate starting material.

| **Meth** | **Compound** | **m/z** | **SM** |
|---|---|---|---|
| 144 | Methyl 4-[(dimethylamino)methyl]-3-(trifluoromethyl)benzoate | 261 | Dimethylamine and Method 135 |
| 145 | Methyl 3-(1-cyano-1-methylethyl)-5-[(4-methylpiperazin-1-yl)methyl]benzoate | 315 | Methyl piperazine and Method 138 |
| 146 | Methyl 3-(1-cyano-1-methylethyl)-5-[(dimethylamino)methyl]benzoate | 260 | Dimethylamine and Method 138 |
| 147 | Methyl 4-[(4-methylpiperazin-1-yl)methyl]- 3-(trifluoromethyl)benzoate | 317 | Methyl piperazine and Method 142 |
| 148 | Methyl 2-[(dimethylamino)methyl]-5-(trifluoromethyl)benzoate | 262 | Dimethylamine and Method 139 |

### Method 149

### N-Cyclopropyl-5-nitropyridin-2-amine

To a 100 ml round bottom flask charged with a magnetic stir bar and 2-bromo-5-nitropyridine (1.00 g, 4.92 mmol) was added EtOH (20 ml). Et₃N (2.80 ml, 20 mmol) was added followed by cyclopropyl amine (0.86 g, 15 mmol) and the reaction was warmed to 70 °C with stirring for 4 h before being cooled to room temperature. The crude reaction mixture was then quenched with ~150 ml of saturated aqueous NaHCO₃. The resulting mixture was poured into a separator funnel and extracted with ~150 ml of EtOAc. The combined organic extract was dried with MgSO₄, filtered, and concentrated *in vacuo* to yield the title compound as a yellow solid 0.850 g (96%), which was used without further purification; *m*/*z* 180.

### Methods 150-158

The following compound were prepared by the procedure of Method 149, using the appropriate starting material.

| **Meth** | **Compound** | **m/z** | **SM** |
|---|---|---|---|
| 150 | *N-*Isopropyl-5-nitropyridin-2-amine | 182 | isopropylamine |
| 151 | (2*R*)-3-[(5-Nitropyridin-2-yl)amino]propane-1,2-diol | 214 | (2R)-3-aminopropane-1,2-diol |
| 152 | 2-[(5-Nitropyridin-2-yl)amino]propane-1,3-diol | 214 | 2-aminopropane-1,3-diol |
| 153 | 1-(5-Nitropyridin-2-yl)piperazine | 209 | piperazine |
| 154 | 5-Nitro-*N-*(3-pyrrolidin-1-ylpropyl)pyridin-2-amine | 251 | (3-pyrrolidin-1-ylpropyl)amine |
| 155 | *N-*(5-Nitropyridin-2-yl)butane-1,4-diamine | 211 | butane-1,4-diamine |
| 156 | *N-*(2-{[tert-Butyl(dimethyl)silyl] oxy}ethyl)-5-nitropyridin-2-amine | 298 | 2-((tert-butyl(dimethyl)silyl] oxy)ethyl amine |
| 157 | *tert*-Butyl 2-{[(5-nitropyridin-2-yl) amino]methyl}pyrrolidine-1-carboxylate | 323 | tert-butyl 2-(aminomethyl) pyrrolidine-1-carboxylate |
| 158 | *N-*Cyclobutyl-5-nitropyridin-2-amine | 194 | cyclobutylamine |

### Method 159

### Methyl 3-{[(dimethylamino)sulfonyl]methyl}benzoate

To a solution of sodium [3-(methoxycarbonyl)phenyl]methanesulfonate (Method 225; 3.3 g, 13.0 mmol) in DCM at -40 °C was added PCl₅. The reaction was allowed to warm to room temperature with stirring over 4 hours. The crude reaction was filtered, and concentrated *in vacuo* to yield the crude product that was subjected to ISCO purification using EtOAc/hexanes (3:1) as the eluent produced 372 mg of the sulfonyl chloride intermediate. The intermediate was then diluted in a solution of k₂CO₃, HNMe₂ and DCM. After allowing the reaction mixture to stir for 10 minutes, the mixture was concentrated *in vacuo* to yield the crude product that was purified on an ISCO using EtOAc/hexanes (1:4) which yielded 165 mg (5 %) of the title compound as a yellow solid. ¹H MMR (300 MHz): 7.95 - 8.03 (m, 2H), 7.58 (d, 1H), 7.42 (t, 1H), 4.21 (s, 2H), 3.87 (s, 3H), 2.71 (s, 6H).

### Method 160

### Methyl 3-{1-[(dimethylamino)sulfonyl]-1-methylethyl}benzoate

A solution of methyl 3-{[(dimethylamino)sulfonyl]methyl}benzoate (Method 159; 165 mg, 0.642 mmol) in anhydrous THF (5 ml) was treated with NaHMDS (1.4 ml, 1.41 mmol) at -78 °C. Immediately, iodomethane (0.1 ml, 1.61 mmol) was added and the reaction mixture was allowed to gradually warm to room temperature while stirring for an additional 2 h. The reaction mixture was then quenched with aqueous NH₄Cl and extracted with EtOAc. The combined organic extracts were dried with MgSO₄ and concentrated *in vacuo* to yield the crude product. The crude product was purified by column chromatography utilizing an ISCO system EtOAc/hexanes (1:5) to give 85 mg (47%) of title compound. ¹H NMR (300 MHz): 8.26 (s, 1H), 7.97 - 8.09 (m, 1H), 7.89 (d, 1H), 7.47 (t, 1H), 3.95 (s, 3H), 2.55 - 2.67 (m, 6H), 1.86(s,6H).

### Method 161

The following compounds were prepared by the procedure of Method 160, using the appropriate starting material.

| **Meth** | **Compound** | **M/z** | **SM** |
|---|---|---|---|
| **161** | Methyl 3-[1,1-dimethyl-3-(trimethylsilyl)prop-2-yn-1-yl]benzoate | 276 | Method 162 |

### Method 162

### Methyl3-[3-(trimethylsilyl)prop-2-yn-1-yl]benzoate

Trimethylsilyl acetylene (2.4 ml, 17.0 mmol) was added to a solution of methyl 3-(bromomethyl)benzoate (3.0 g, 13.1 mmol), Pd₂dba₃ (300 mg, 0.3 mmol), triphenylphosphine (343 mg, 1.3 mmol), Cs₂CO₃ (6.0 g, 18.3 mmol), and Cul (187 mg, 1.0 mmol) in THF (50 ml). The reaction mixture was stirred overnight at 50 °C. After allowing the mixture to cool to room temperature, it was then diluted with EtOAc (~ 100 ml) and washed with brine. The mixture was then filtered through a pad of diatomaceous earth, dried over Na₂SO₄ and concentrated *in vacuo.* The crude product was purified on SiO₂ using hexanes/EtOAc 4:1 as eluent giving 2.2 g (67 %) of the title compound. ¹H NMR (300 MHz): 8.03 (s, 1H), 7.92 (d, 1H), 7.57 (d, 1H), 7.40 (t, 1H), 3.93 (s, 3H), 3.71 (s, 2H), 0.21 (s, 9H).

### Method 163

### Methyl 3-5(1,1-dimethylprop-2-yn-1-yl)benzoate

To a solution of methyl 3-[1,1-dimethyl-3-(trimethylsilyl)prop-2-yn-1-yl]benzoate (Method. 161; 170 mg, 0.62 mmol) in anhydrous MeOH (5 ml) was added potassium carbonate (1.2 g, 9.3 mmol). The reaction was stirred overnight at room temperature, filtered, and concentrated *in vacuo.* The crude product was subjected to ISCO purification using EtOAc/Hexanes (1:5) as eluent to yield 180 mg (70 %) of the title compound as a colourless oil. ¹H NMR (300 MHz): 8.20 (s, 1H), 7.91 (d, 1H), 7.79 (d, 1H), 7.39 (t, 1H); 3.91 (s, 3H), 2.37 (s, 1H), 1.61 (s, 6H).

### Method 164

### Methyl 3-(1,1-dimethylbut-2-yn-1-yl)benzoate

A solution of methyl 3-(1,1-dimethylprop-2-yn-1-yl)benzoate (Method 163; 100 mg, 0.495 mmol) in 1 ml of THF was cooled to -78 °C. A 1M solution of LiHMDS in THF (0.55 ml, 0.55 mmol) was added, followed by methyl iodide (46 µL, 0.743 mmol). The reaction mixture was then allowed to warm to room temperature over 2 h and was then quenched with brine. The mixture was extracted with EtOAc, and the combined organic extract was died over MgSO₄, filtered, and concentrated in vacuo to yield 100 mg (93%) of the title compound which was used without further purification. ¹H NMR (300 MHz): 8.19 (s, 1H), 7.89 (d, 1H), 7.79 (d, 1H), 7.39 (t, 1H), 3.91 (s, 3H), 1.83 - 1.88 (m, 3H), 1.56 (s, 6H).

### Method 165

### Methyl 3-(1,1-dimethylpro-2-en-1-yl)benzoate

To a solution of methyl 3-[1,1-dimethyl-3-(trimethylsilyl)prop-2-yn-1-yl]benzoate (Method 161; 300 mg, 1.49 mmol) and quinoline (4 drops) in anhydrous toluene (5 ml) was added Lindlar's catalyst (50 mg) under an atmosphere of H₂. The reaction mixture was allowed to stir for 5 h at room temperature before being filtered through diatomaceous earth. The filtrate was concentrated *in vacuo* which yielded 170 mg (56 %) of the title compound which was used without further purification. ¹H NMR (300 MHz): 8.03 (s, 1H), 7.86 (d, 2H), 7.52 (d, 2H), 7.36 (t, 2H), 6.01 (dd, 1H), 5.08 (s, 1H), 5.03 (d, 1H), 3.90 (s, 3H), 1.41 (s, 6H).

### Method 166

### Methyl 3-(2,3-dihydroxy-1,1-dimethylpropyl)benzoate

A solution of methyl 3-(1,1-dimethylprop-2-en-1-yl)benzoate (Method 165;.0.273 g, 1.33 mmol) in 2 ml of *tert*-BuOH and 2 ml of H₂O was added NMO and K₂OsO₄(H₂O)₂. The reaction mixture was allowed to stir at room temperature overnight before being quenched with 30 mg of Na₂SO₃. The mixture was extracted with EtOAc and the combined extract was washed with brine, dried over Na₂SO₄, and concentrated *in vacuo* to yield 240 mg .(75 %) of the title compound crude was used without further purification. ¹H NMR (300 MHz): 8.06 (s, 1H), 7.90 (d, 1H), 7.59 (d, 1H), 7.40 (t, 1H), 3.82 (d, 1H), 3.53 (d, 1H), 3.37 (t, 1H), 1.38 (d, 6H).

### Method 167

### 3-(1,1-Dimethylpropyl)benzoic acid

A solution of 3-(1,1-dimethylprop-2-yn-1-yl)benzoic acid (Method 41; 170 mg, 0.90 mmol) in anhydrous MeOH (5 ml) was treated with Pd/C (17 mg). The reaction mixture was placed under an atmosphere of 142 with a balloon and allowed to stir for 12 h at room temperature. The reaction mixture was then filtered through pad of diatomaceous earth, and the filtrate was concentrated *in vacuo* which yielded 150 mg (86%) of the title compound which was used without further purification. *M*/*z* 192.

### Method 168

### Methyl- 3-{[(dimethylamino)carbonyl]amino}benzoate

To a solution of methyl 3-isocyanatobenzoate (1.0 g, 5.64 mmol) in dichloroethane was added triethylamine (2.36 ml, 16.9 mmol) and dimethylamine hydrochloride (550 mg, 6.74 mmol). After allowing the reaction mixture to stir for 10 minutes, the solvent was removed *in vacuo.* The crude product was subjected to ISCO purification using EtOAc/hexanes (1:1) to yield 670 mg (54 %) of the title compound as a white crystalline solid. *M*/*z* 222.

### Method 169

### Ethyl 3-(cyclopropylcarbonyl)benzoate

To a solution of ethyl 3-iodobenzoate (1.8 ml, 10.0 mmol) in The (40 ml) at -78 °C was added isopropyl magnesium chloride (2.0M, 7.0 ml, 14.0 mmol). After 30 min. of stirring, CuCN (1.1 g, 12.0 mmol) and LiCl (1.0 g, 24.0 mmol) were added simultaneously. After 20 minutes when reaction mixture colour changed to a orange colour, cyclopropane carbonyl chloride (3.0 ml, 33.0 mmol) was added, and then the reaction mixture was allowed to reach to room temperature over 1 hour with stirring. The mixture was diluted with EtOAc and added to aqueous solution of NH₄Cl. The organic extract was dried over Na₂SO₄, filtered, and concentrated *in vacuo* to yield the crude product which was chromatographed with a ISCO system using EtOAc/hexanes (1:1) as eluent to yield 1.2 g (50%) of the title compound. ¹H NMR (300 MHz): 8.66 (s, 1H), 8.22 (d, 1H), 8.17 (d, 1H), 7.55 (t, 1H), 4.40 (q, 2H), 2.76 - 2.67 (m, 1H), 1.40 (t, 3H), 1.29 - 1.21 (m, 2H), 1.12 - 1.01 (m, 2H).

### Method 170

### Ethyl 3-(1-cyclopropyl-1-hydroxyethyl)benzoate

A solution of ethyl 3-(cyclopropylcarbonyl)benzoate (Method 169; 363 mg, 1.66 mmol) in the (6 ml) was cooled to -78 °C. To the reaction mixture was added methyl magnesium bromide (3.0M, 0.73 ml, 2.16 mmol). The reaction was allowed to stir for 3 h at this temperature before being quenched with saturated aqueous NH₄Cl. The mixture was diluted with EtOAc and poured into a separator funnel. The organic extract was collected, dried over Na₂SO₄, and concentrated *in vacuo* to yield the crude product which was purified with an ISCO system using EtOAc/hexanes (1:1) as eluent to yield 1.2 g (50%) of the title compound. ¹H NMR (300 MHz): 8.19 (s,1H), 7.92 (d, 1H), 7.72 (d, 1H), 7.40 (t, 1H), 4.37 (q, 2H), 1.78 (s, 1H), 1.51 (s, 3H), 1.38 (t, 3H),1.32 - 1.21 (m, 1H), 0.46-0.3.7 (m, 4H).

### Method 171

### Methyl 3-(1,1-difluoroethyl)benzoate

A solution of methyl 3-acetylbenzoate (Method 87; 700 mg, 3.9 mmol) in 5 ml of DeoxoFluor^{™} was stirred overnight at 85 °C. The reaction was allowed to cool to room temperature and quenched with brine. The mixture was poured into a separator funnel and extracted with EtOAc. The organic extract was dried over Na₂SO₄, filtered, and concentrated *in vacuo* to yield the crude product. The crude oil was then subjected to ISCO purification using P-tOAc/hexanes (1:4) as eluent to yield 396 mg (50%) of the title compound as a colourless oil. ¹H NMR (300 MHz): 7.96 (s, 1H), 7.86 (d, 1 H), 7.50 (d, 1H), 7.31 - 7.22 (m, 114), 3.73 (s, 3H), 1.74 (t, 314).

### Method 172

### Ethyl 3-[cyclopropyl(hydroxy)methyl]benzoate

To a solution of ethyl 3-(cyclopropylcarbonyl)benzoate (Method 169; 363 mg, 1.66 mmol) in anhydrous EtOH (5 ml) was added sodium borohydride (70 mg, 1.86 mmol). The reaction was allowed to stir for 3 h at room temperature before being quenched by the addition of saturated aqueous NH4cl. The mixture was diluted with EtOAc and poured into a separator funnel. The organic extract was dried over Na₂SO₄, filtered, and concentrated *in vacuo.* The crude product was purified with an ISCO system using EtOAc/hexanes (1:1) a eluent which yielded 210 mg (77 %) of the title compound. ¹H MMR (300 MHz): 8.07 (s, 1H), 7.95 (d, 1H), 7.61 (d, 1H), 7.41 (t, 1H), 4.36 (q, 2H), 4.04 (d, 1H), 2.16 (s, 1H), 1.38 (t, 3H), 1.27 - 1.15 (m,1H), 0.66 - 0.54 (m, 2H), 0.52.- 0.36 (m, 2H).

### Method 173

### Methyl 3-isoprobenylbenzoate

A solution of methyl triphenylphosphonium iodide in THF (5 ml) was cooled to 0 °C. To the cooled mixture was added n-BuLi (2.5 M, 1.0 ml, 2.52 mmol) and the reaction was allowed to stir for 30 min at this temperature. To the mixture was added a solution of methyl 3-acetylbenzoate (Method 87; 375 mg, 2.10 mmol) in THF (15 ml). After stirring at 0 °C for one hour, the reaction was allowed to warm to room temperature and was stirred for an additional 2 h. The mixture was then quenched with water and diluted with EtOAc and poured into a separator funnel. The organic extract was dried over Na₂SO₄, filtered and concentrated *in vacuo* giving the crude product which was purified using an ISCO system with EtOAc/hexanes (1:9) as eluent to yield 108 mg (29%) of the title compound as a colourless oil. ¹HNMR(300 MHz): 8.15 (s, 1H), 7.96 (d, 1H), 7.67 (d, 1H), 7.41 (t, 1H), 5.45 (s, 1H), 5.16 (s, 1H), 3.94 (s; 3H), 2.19 (s, 3H).

### Method 174

### Methyl 3-(1-cyano-1-methylethyl)-5-{[methoxy(methyl)amino]methyl}benzoate

To a solution of methyl 3-(bromomethyl)-5-(1-cyano-1-methylethyl)benzoate (Method 138; 176 mg, 0.595 mmol) in 5 ml of DMF was added K₂CO₃ (206 mg, 1.49 mmol) and NHMeOMe·HCl (65 mg, 0.666 mmol). The reaction was warmed to 85 °C. and stirred at this temperature for 12 h. The reaction was then cooled to room temperature and quenched with water. The mixture was poured into a separator funnel and extracted with EtOAc. The organic extract were dried over Na₂SO₄, filtered, and concentrated *in vacuo.* The crude residue was purified on an ISCO using EtOAc/hexanes (1:1) as the eluent giving 88 mg (54%) of the title compound as a colourless oil. ¹H NMR (300 MHz): 8.02 (s, 1H), 7.98 (s, 1H), 7.74 (s, 1H), 3.93 (s,3H), 3.82 (s,2H), 3.35 (s, 3H), 2.65 (s, 3H), 1.77 (s,6H).

### Method 175

### 3-(1-Cyano-1-methylethyl)-5-[(methylsulfonyl)methyl]benzoic acid

To a solution of 3-(1-cyano-1-methylethyl)-5-[(methylthio)methyl]benzoic acid (Method 37; 58 mg, 0.23 mmol) in MeCN (5 ml) and H₂O (3 ml) was added potassium peroxymonosulfate (358 mg, 0.582 mmol). The resulting reaction mixture was allowed to stir at room temperature overnight before being quenched with water. The mixture was poured into a separator funnel and extracted with EtOAc. The organic extract was dried over Na₂SO₄, filtered, and concentrated *in vacuo* which yielded 60 mg (92%) of the title compound as a colourless oil which was used without further purification. *M*/*z* 281.

### Method 176

### Methyl 3-(1,3-thiazol-2-yl)benzoate

To a round bottom flask equipped with a magnetic stir bat was added a solution of thiazole (461 µl, 6.51 mmol) in 15 ml of THF. The resulting mixture was cooled to -78°C. and n-BuLi ml, 6.0 mmol) was added to the solution. The reaction was allowed to stir at this temperature for 10 min. and ZnCl₂ (1.9 g, 13.95 mmol) was then added. The resulting mixture was allowed to stir for an additional 30 min. at this temperature followed by the addition of Pd(PPh₃)₄(269 mg, 0.233 mmol) and methyl 3-broniobenzoate (1.0 g, 4.65 mmol). The reaction was allowed to warm to room temperature, was fitted with a reflux condense, and was heated to 80 °C for 2 h. The mixture was then cooled to room temperature and quenched with saturated aqueous NH₄Cl. The reaction was poured into a separator funnel and with EtOAc. The organic extract was dried over Na₂SO₄, filtered, and concentrated *in vacuo* which provided 282 mg (28%) of the title compound as a colourless oil which was used without further purification. *Mlz* 219.

### Method 177

### 2-(Trifluoromethyl)pyrimidine-4-carboxylic acid

A solution of methyl 2-(trifluoromethyl)pyrimidine-4-carboxylic acid (1.00 g, 4.85 mmol) in THF/H₂O (3:1, 8 ml) was treated with 2 N LiOH (2.5 ml, 4.85 mmol). After stirring the solution for 12 h, the solvents were removed under reduced pressure to give the desired product; *m*/*z* 193.

### Method 178

### 3-(2-Methoxy-1,1-dimethylethyl)benzoic acid

2-(3-Bromophenyl)-2-methylpropyl methyl ether (Method 17; 281 mg, 1.16 mmol) in THF (10ml) at-78°C under Ar was treated with *tert*-BuLi (1.7 M in pentane, 1.4 ml, 2.31 mmol), 2.0 equiv). The reaction stirred for 15 min and then CO_{2(g)} was bubbled through the reaction mixture. After 10 min, the reaction was quenched with 10% NaOH and extracted with EtOAc. The aqueous layer was acidified with 10% HCl and extracted with EtOAc. The organics were dried with NaCl₍ₛₐₜ₎ and Na₂SO₄₍ₛ₎ and then removed under reduced pressure; *m*/*z* 209.

### Method 179

### 2-(3-Bromophenyl)-2-methylpropan-1-ol

A solution of 2-(3-bromophenyl)-2-methylpropanoic acid (Method 52, 300 mg, 1.23 mmol) in anhydrous THF (10 ml) was treated with BH₃ (1.0 M in THF, 2.49 ml, 2.49 mmol, 1.5 equiv) drop wise under argon at 0 °C. The ice bath was removed and the reaction was stirred for 12 h. The reaction was quenched with 10% HCl and extracted with EtOAc. The organics were washed with NaCl₍ₛₐₜ₎ and the dried with Na₂SO₄₍ₛ₎. The solvents were removed under reduced pressure to give 265 mg (94%); *m*/*z* 230.

### Method 180

### 2-(3-Bromophenyl)-2-methylpropanoyl chloride

A solution of 2-(3-bromophenyl)-2-methylpropanoic acid (Method 52, 564 mg, 2.32 mmol) in DCM (5 ml) was treated with oxalyl chloride (0.31 ml, 3.48 mmol, 1.5 equiv) and DMF (0.10 ml), under argon at 25 °C. The reaction stirred for 4 h, and then the solvents were removed under reduced pressure to give 265 mg (94%); *m*/*z* 262.

### Method 181

### 2-(3-Bromophenyl)-N,N,2-tritimethylpropanamide

2-(3-Bromophenyl)-2-methylpropanoyl chloride (Method 180, 607 mg, 2.32 mmol) was treated with dimethylamine (2.0 M in THF, 5 ml, 10 mmol, 4.3 equiv). The reaction stirred for 30 min. The reaction was quenched with 10% HCl and extracted with EtOAc. The organics were washed with NaCl₍ₛₐₜ₎ and the dried with Na₂SO₄₍ₛ₎. The solvents were removed under reduced pressure to give 539 mg (86%); *m*/*z* 271.

### Method 182

### Methyl 5-({3-[2-(dimethylamino)-1,1-dimethyl-2-oxoethyl]benzoyl}amino)-2-methylbenzoate

2-(3-Bromophenyl)-*N,N*,2-trimethylpropanamide (Method 181; 200 mg, 0.740 mmol), methyl 5-amino-2-methylbenzoate (Method 92; 122 mg, 0.740 mmol, 1.0 equiv), Pd(OAc)₂ (17 mg, 0.075 mmol, 10 mol%), Mo(CO)₆ (293 mg, 1.11 mmol), 1.5 equiv), Cs₂CO₃ (362 mg, 1.11 mmol, 1.5 equiv) and BINAP (46 mg, 0.074 mmol, 10 mol%) in toluehe-MeCM 1:1 (2 ml) was heated at 90 °C under Ar for 12 h. The reaction was quenched with 10% NaOH and extracted with EtOAc. The organics were dried with NaCl₍ₛₐₜ₎ and Na₂SO₄₍ₛ₎ and then removed under reduced pressure. The residue was then purified by column chromatography utilizing an ISCO system (EtOAc-hexane) to give 75 mg (27%) of the desired product; *m*/*z* 383.

### Method 183

The following compound was prepared by the procedure of Method 182, using the appropriate starting material.

| **Meth** | **Compound** | ***m*/*z*** | **SM** |
|---|---|---|---|
| **183** | Methyl5-[({5-[(dimethylamino)sulfonyl]pyridin-3-yl}carbonyl)amino]-2-methylbenzoate | 378 | Method 230 and Method 92 |

### Method 184

### 5-[(Diphenylmethylene)amino]-N,N-dimethylpyridine-3-sulfonamide

A stirred mixture of 5-bromo-*N,N*-dimethylpyridine-3-sulfonamide (Method 230; 170 mg, 0.64) mmol), benzophenone imine (0.13 ml, 0.769 mmol, 1.2 equiv), Cs₂CO₃ (313 mg, 0.962 mmol, 1.5 equiv), BINAP (40 mg, 0.064 mmol, 10 mol%) in dioxane (3 ml) was treated with Pd₂(dba)₃ (30 mg, 0.032 mmol, 5 mol%). The reaction mixture was heated to 100 °C for 12 hours. The reaction was then quenched with 10% NaOH(aq) and extracted with EtOAc. The organics were dried with NaCl(ₛₐₜ) and then Na₂SO₄(ₛ) and removed under reduces pressure. The resulting solid was purified by column chromatography utilizing an ISCO system (hexanes-EtOAc) to give 234 mg (99%); *m*/*z* 366.

### Method 185

### 5-Amino-N,N-dimethylpyridine-3-sulfonamide

A solution of 5-[(diphenylmethylene)amino]-*N*,*N-*dimethylpyridine-3-sulfonamide (Method 184; 234 mg, 0.640 mmol) in THF (3 ml) was treated with 10% HCl (5 ml). The reaction mixture was stirred for 10 min. The reaction was quenched with 10% HCl(aq) and extracted with EtOAc. The aqueous layer was saturated with K₂CO₃ and extracted with EtOAc and the solvents were removed under reduced pressure to give 129 mg (99%); *m*/*z* 202.

### Method 186

### 3-Chloropyridine-2,5-diamine

Into a 300ml three-neck flask, 13.9g(0.1mol) of 2-amino-5-nitropyridine, (14.7g, 0.11mol) of *N*-chlorosuccinimide and 150ml of toluene were added. The resulting reaction mixture was stirred at 80 °C for one hour. During the reaction, formation of 2-chloroamino-5-nitropyridine was confirmed. After completion of the reaction by LCMS, the reaction mixture was cooled to room temperature and 100 ml of H₂O were added. The solution was filtered and the isolated solid was washed with H₂O and toluene (50ml each). 2-amino-3-chloro-5-nitropyridine was obtained (10.8 g, 62%) and it was used in the next step without further purification. *M*/*z* 175.

Into a 500ml three-neck flask, 2-amino-3-chloro-5-nitropyridine (8.72g, 50mmol) was dissolved in MeON (125 ml). A solution of ammonium chloride (13.5 g, 250mmol) in H₂O (125 ml) were added, followed by 14g of iron powder. The solution was stirred with mechanic stirring at 78 °C for 2 hours. The solution was filtered at 50 °C and the isolated solid was washed by hot MeOH. The solvent was evaporated by vacuum and the crude product was extracted by acetone and filtered. The acetone solution was evaporated and dissolved in 50ml MeOH whereupon 50ml of a solution of 4N hydrogen chloride in dioxane was added slowly. The resulting solution was sonicated for 10 minutes, and then filtered. The solid was washed by acetone and dried in a vacuum oven. 3-chloropyridine-2, 5-diamine (7.5 g, HCl salt) was isolated as an off-white powder. *M*/*z* 145.

### Method 187

The following compound was prepared by the procedure of Method 186, using the appropriate starting material.

| **Method** | **Compound** | **m/z** | **SM** |
|---|---|---|---|
| **187** | 3-Bromo-pyridine-2,5-diamine | 189 | 2-amino-5-nitropyridine and NBS |

### Method 188

### N²-(2-{[tert-butyl(dimethyl)silyl]oxy}ethyl)pyridine-2,5-diamine

To a solution of 2-chloro-5-nitro pyridine (400 mg, 2.5 mmol) in EtOH (5ml) was added 2-((tett-butyl(dimethy])silyl]oxy)ethyl amine (3 ml) and the resulting mixture was heated to reflux for 3 hours. Evaporation of the solvent under reduced pressure afforded 5-nitro-2-[2-hydroxy)ethylamino]pyridine (680 mg) that was used in the next step without any further purification.

*N*-(2-{[tert-butyl(dimethyl)silyl]oxy}ethyl)-5-nitropyridin-2-amine (Method 156; 1.29 g, 4.34 mmol) was dissolved in 20 ml of MeOH and 260 mg of palladium (10 wt. % on activated carbon-Degussa®) was added. The reaction was subjected to 1 atmosphere of hydrogen overnight. LC/MS confirmed the formation of the product. The reaction mixture was filtered through diatomaceous earth and washed with MeOH and EtOAc. The title compound (970 mg) was collected by evaporation as a thick red oil. *M*/*z* 268.

### Methods 189-199

The following compounds were prepared by the procedure of Method 188, using the appropriate starting material.

| **Meth** | **Compound** | **m/z** | **SM** |
|---|---|---|---|
| **189** | *N*-2-[2-(Dimethylamino)ethyl]pyridine-2,5-diamine | 181 | 2,2-dimethylamino-ethylamine |
| **190** | 6-(4-Methylpiperazin-1-yl)pyridin-3-amine | 193 | 4-methyl piperazine |
| **191** | *N*-2-1H-Pyrazol-5-ylpyridine-2,5-diamine | 176 | 3-amino-1H-pyrazole |
| **192** | 3-Methylpyridine-2,5-diamine | 124 | 2-chloro-3-methyl-5-nitropyridine |
| **193** | *N*-2-[3-(Dimethylamino)propyl] pyridine-2,5-diamine | 195 | 2,2-dimethylamino-propylamine |
| **194** | *N*-2-Methylamine-3-chloro-pyridine-5-amine | 158 | 2,3-dichloro-5-nitro amine and methylamine |
| **195** | *N*-2-Methylamine-3-methoxy-pyridine-5-amine | 154 | 2-chloro-3-methoxy-5-nitro pyridine |
| **196** | Pyridine 2,3,5-triamine | 125 | 2-chloro-3,5-dinitropyridine And ammonia |

### Method 197

### (3-Bromo-5-fluorophenyl)methanol

A solution of 3-bromo-5-fluorobenzoic acid (1.14 g, 5.21 mmol) in anhydrous THF (10 ml) was treated with BH₃ (1.0 M in THF, 8.0 ml, 8.0 mmol, 1.5 equiv) dropwise under nitrogen at 0 °C. The mixture was stirred at 0 °C for 30 min then allowed to warm to 25 °C and stirred for 12 h. The reaction was quenched with 10% HCl and extracted with EtOAc. The organic layer was washed with 10% NaOH and then dried with NaCl(ₛₐₜ) and Na₂SO₄(ₛ). The solvents were removed under reduced pressure. The resulting product was carried directly to the next step; *m*/*z* 284.

### Method 198

### 3-Bromo-5-fluorobenzyl methanesulfonate

A solution of (3-bromo-5-fluorophenyl)methanol (Method 197; 1.07 g, 5.22 mmol) in anhydrous DCM (20 ml) was cooled to 0 °C. To this solution, DIEA (1.4 ml, 7.83 mmol, 1.5 equiv) and methane sulfonyl chloride (0.5 ml, 6.26 mmol), 1.2 equiv) were added respectively. The mixture was stirred at 25 °C for 2 h. The reaction was quenched with 10% HCl and extracted with EtOAc. The organic layer was washed with NaHCO₃(ₛₐₜ) and then dried with NaCl(ₛₐₜ) and Na₂SO₄(ₛ). The solvents were removed under reduced pressure. The resulting product was carried directly to the next step; *mlz* 208.

### Method 199

### (3-Bromo-5-fluorophenyl)acetonitrile

A suspension of 3-bromo-5-fluorobenzyl methanesulfonate (Method 198; 1.27 g, 4.49 mmol) and sodium cyanide (0.264 g, 5.38 mmol, 1.2 equiv) in DMF (9 ml) and water (1 ml) was stirred at 40 °C for 5 h. The reaction mixture was quenched with water and extracted with EtOAc. The organic layer was dried with NaCl₍ₛₐₜ₎ and Na₂SO₄₍ₛ₎. The solvents were removed under reduced pressure. The resulting product was purified by chromatography using 5% EtOAc in Hexane to give the desired product; *m*/*z* 215.

### Method 200

### 2-Methyl-3,5-dinitropyridine

Diethyl malonate (1.52mL ,10mmol) was dissolved in 50mL THF, sodium hydride (400 mg, 10 mmol) was added to the solution in portions. The solution was stirred until gas evolution ceased. 2-chloro-3,5-dinitropyridine (2.03g, 10mmol) was added and the resulting solution was stirred an additional 10 minutes. The solvent was evaporated *in vacuo.* Sulfuric acid (20 mL, 6 N) was added to the crude intermediate and the resulting solution was heated to 100°C over night. The solution was cooled to room temperature and neutralized by the addition of 2N sodium hydroxide_{(aq)}. The solution was extracted with ethyl acetate and the combined organic layer was washed with water and dried with sodium sulfate. The solution was filtered and concentrated *in vacuo,* yielding 680 mg of the title compound that was used without further purification (37.2%).

### Method 201

### 3-(4-Methyl-1H-imidazol-1-yl)-5-(trifluoromethyl)benzoic acid

To a solution of 3-(4-methyl-1*H*-imidazol-1-yl)-5-(trifluoromethyl)benzonitrile (Method 206; 180 mg, 0.717 mmol) in 5 ml of dioxane was added 7 ml of a 1M NaOH solution. The reaction mixture was allowed to stir overnight at 100 °C. The reaction mixture was cooled to room temperature and quenched by the careful addition of concentrated HCl until a of pH 3 was obtained. The aqueous phase was extracted with EtOAc, dried over Na₂SO₄, filtered and concentrated *in vacuo* to yield 816 mg (74 %) of the title compound as a yellow solid which was used without further purification. *M*/*z* 271.

### Method 202

### 5-(1H-Pyrrol-3-yl)pyridin-3-amine

To a solution of 5-(1*H*-pyrrol-1-yl)nicotinic acid (3.0 g, 15.9 mmol) in 80 ml of t-butanol, DPPA (6.89 ml, 31.9 mmol) and DIEA (5.56 ml, 31.9 mmol) were added and the reaction mixture was heated at 80°C in a sealed tube overnight. The mixture was partitioned between H₂O and EtOAc, and the organic layer was washed with brine and sodium bicarbonate and dried with MgSO₄. Evaporation of the solvent gave a brown solid *mlz* 260. This material was dissolved in MeOH (30 ml) and 4N HCl in 1,4-dioxane (25 ml) was added at 0°C and the resulting mixture stirred for 2 hours at room temperature. Ether was then added to the mixture and 5-(1*H*-pyrrol-1-yl)pyridin-3-amine (3.356g), a mustard yellow precipitate, was collected by filtration. *M*/*z* 160.

### Method 203

### 3-(1-Cyano-1,2-dimethylpropyl)benzoic acid

To a solution of 3-(1-cyanoethyl)benzoic acid (500 mg) in THF (50ml) at -78°C was added slowly a solution of LiHMDS (60ml, 1.0M in THF) and the resulting red solution was allowed to stir at his temperature for 30 minutes. Isopropyl iodide (10 ml) was added slowly and the resulting mixture was warmed up to room temperature and stirred for 10 hours. The mixture was partitioned between EtoAc and H₂O and the aqueous layer was acidified with 1N HCl until pH 3. The aqueous layer was extracted with EtOAc (3x) and dried with MgSO₄. Evaporation of the solvent under pressure afforded the desired compound as a white solid (300 mg). 1H NMR (300 MHz) 8.03 (s, 1H), 7.92 (d, 2H), 7.71 (d,2H), 7.56 (t, 1H), 2.20 (h, 1H), 1.67 (s, 3H), 1.05 (d, 3H), 0.72 (d, 3H).

### Methods 204-205

The following compounds were prepared by the procedure of Method 203, using the appropriate starting material.

| **Method** | **Compound** | **m/z** | **SM** |
|---|---|---|---|
| **204** | 3-(1-Cyano-1-methylpropyl)benzoic acid | 1H NMR (300 MHz) 8.07 (s, 1H), 7.91 (d,1H), 7.73 (d,1H), 7.57 (t, 1H),1.98 (q,2H), 1.69 (s, 3H), 0.82 (t, 3H) | iodoethane |
| **205** | 3-(1-Cyano-2-hydroxy-1-methylethyl)benzoic acid | 1H NMR (300 MHz) 8.01 (s, 1H), 7.90 (d, 1H), 7.72 (d, 1H), 7.54 (t,1H), 4.20 (d, 1H), 4.01 (d, 1H), 1.69 (s, 3H), 0.82 (t, 3H) | paraformaldehyde |

### Method 206

### 3-(4-Methyl-1H-imidazol-1-yl)-5-(trifluoromethyl)benzonitrile

To a solution of 3-fluoro-5-(trifluoromethyl)benzonitrile (5.0 g, 26.4 mmol) in 25 ml of DMA was added 2-methyl imidazole (6.5 g, 79.3 mmol). The reaction mixture was stirred at 145 °C overnight. The reaction mixture was allowed to cool to room temperature and was quenched with ~50 ml of brine and then extracted three times with EtOAc. The combined organic extracts were dried over Na₂SO₄, filtered and concentrated *in vacuo* to yield the crude product. The crude sample was subjected to ISCO purification using EtOAc as eluent to yield 4.0 g (61%) of the title compound as a white solid. *M*/*z* 251.

### Method 207

### tert-Butyl {2-(5-nitropyridin-2-yl)amino]ethyl}carbamate

To a solution of *N*-(5-nitropyridin-2-yl)ethane-1,2-diamine (1.0 g, 5.49 mmol) in THF (20 ml) at 0°C, sodium hydride (395 mg, 8.22 mmol) and di-tert-butyl dicarbonate (1.20g, 5.49 mmol) were added. The reaction mixture was stirred overnight at 25°C. LC/MS confirmed the formation of the product so the reaction was quenched with H₂O. The mixture was partitioned between H₂O and EtOAc, and the organic layer was washed with brine and dried with MgSO₄. The title compound (1.11g) was collected by evaporation as a yellow oily solid. *M*/*z* 284

### Method 208

### N-(5-Amino-3-bromopyridin-2-yl)acetamide

To a solution of 2-amino-3-bromo-5-nitro pyridine (3.0g, 13.8 mmol) in pyridine (20 ml) at 0°C was added slowly acetyl chloride (2.5 ml) and the resulting mixture was stirred for 1 hour at ambient temperature. The mixture was partitioned between H₂O and EtOAc, and the organic layer was washed with brine and dried with MgSO₄. Evaporation under reduced pressure afforded the acetyl derivative, which was used in the next step without further purification.

Into a three-neck flask, 2-acetylamino-3-bromo-5-nitropyridine (2.0g) was dissolved in MeOH (25 ml). A solution of ammonium chloride (1.3 g, 250mmol) in H₂O (25 ml) was added, followed by 1.4g of iron powder. The solution was stirred by at 78 °C for 2 hours. The solution was filtered at 50 °C and the isolated solid was washed by hot MeOH. The solvent was evaporated under reduced pressure and the product was extracted by acetone and filtered. The acetone solution was evaporated to give the title compounds (900 mg) as solid. *Mlz* 231.

### Methods 209-210

The following compounds were prepared by the procedure of Method 208, using the appropriate starting material.

| **Method** | **Compound** | **m/z** | **SM** |
|---|---|---|---|
| **209** | *N*-(5-Amino-3-chloropyridin-2-yl)acetamide | 186 | Method 186(1^{st} part) |
| **210** | *N*-(5-Amino-3-methoxypyridin-2-yl)acetamide | 182 | 2-amino-3-methoxy-5-nitro pyridine |

### Method 211

### 2,3-Dimethyl-pyridine-5-amine

To a solution of NaH (80 mg, 60% w/w) in ether (4 ml) at 0°C was added slowly diethyl malonate (0.3 ml). After the evolution of gas had ceased, 2-bromo-3-methyl-5-nitro pyridine (434 mg) was added portion-wise. After 30 min, the reaction was quenched with 10% HCl and extracted with EtOAc. The organics were washed with NaCl₍ₛₐₜ₎ and then dried with Na₂SO₄₍ₛ₎. The organics were then removed under reduced pressure to give the malonate adduct. The crude product was dissolved in 30 ml of 6N H₂SO₄ and heated to 110°C for 5 hours. The mixture was cooled in an ice bath and a solution of 2N NaOH was added until pH neutral. The desired product was collected by filtration (100 mg). *M*/*z* 154.

The 2,3-dimethyl-5-nitro-pyridine (100 mg) was dissolved in 10 ml of MeOH and 100 mg of palladium (10 wt. % on activated carbon-Degussa®) was added. The reaction mixture was subjected to 1 atmosphere of hydrogen overnight. LC/MS confirmed the formation of the product and the reaction mixture was filtered through diatomaceous earth and washed with MeOH and EtOAc. The product (60 mg) was collected by evaporation. *M*/*z* 123.

### Methods 212-213

The following compounds were prepared by the procedure of Method 211, using the appropriate starting material.

| **Method** | **Compound** | **m/z** | **SM** |
|---|---|---|---|
| **212** | 2-Methyl-3-chloro-pyridine-5-amine | 143 | Method 227 |
| | 6-Ethylpyridin-3-amine | 123 | 2-chloro-5-nitro-pyridine and diethyl methyl malonate |

### Method 214

### 2-Hydroxypyridine-5-amine

5-Nitropyrid-2-one (420 mg, 3 mmol) was dissolved in MeOH (7 ml) and a solution of ammonium chloride (810 mg) in H₂O (7 ml) was added, followed by 840 mg of iron powder. The solution was stirred at 78 °C for 2 hours. The solution was filtered at 50 °C and the isolated solid was washed by hot MeOH. The solvent was evaporated under reduced pressure and the desired product was isolated by filtration (170 mg). *M*/*z* 111.

### Methods 215-217

The following compounds were prepared by the procedure of Method 214, using the appropriate starting material.

| **Method** | **Compound** | **m/z** | **SM** |
|---|---|---|---|
| **215** | 3-Chloropyridine-5-amine | 129 | 3-chloro-5-nitro pyridine |
| **216** | 2-Hydroxy-picoline-5-amine | 125 | 2-hydroxy-5-nitro-3-picoline |
| **217** | Methyl 2-chloro-5-amino benzoate | 186 | Method 91 |

### Method 218

### Methyl 1,3,3-trimethyl-2-oxoindoline-5-carboxylate

To a solution of methyl 2-oxoindoline-5-carboxylate (400 mg, 2.09 mmol) in THF (10 ml) at -78 °C was added LiHMDS (1.0 M, 16.7 ml, 16.75 mmol) and Mel (1.3 ml, 20.9 mmol). The reaction was allowed to stir overnight to room temperature. The reaction was then quenched with ~25 ml aqueous NH₄Cl. After dilution with EtOAc, the mixture was washed with brine, and then dried over Na₂SO₄ and concentrated *in vacuo* to yield the crude product that was purified on SiO₂ using EtOAc/hexanes as eluent which yielded 81 mg (17%) the title compound as a yellow solid. *Mlz* 233.

### Method 219

### 5-Isopropoxypyridin-3-amine

5-Bromopyridin-3-ol (1g, 5.75mmol) was dissolved in 20ml DMF, isopropyl bromide (1.65ml, 17.2mmol) and Cs₂CO₃ (8.6gram, 25.9mmol) were added. The solution was stirred at room temperature overnight. The solution was filtered and separated between EtOAc and water. The organic layer was dried and evaporated under reduced pressure to afford the title compound (1.2 g).

### Methods 220-222

The following compounds were prepared by the procedure of Method 219, using the appropriate starting material.

| **Method** | **Compound** | **m/z** | **SM** |
|---|---|---|---|
| **220** | tert-Butyl2-{[(5-aminopyridin-3-yl)oxy]methyl}pyrrolidine-1-carboxylate | 293 | tert-butyl2-bromomethyl)pyrrolidine-1-carboxylate |
| **221** | 5-[2-(Dimethylamino) ethoxy]pyridin-3-amine | 181 | (2-chloroethyl) dimethylamine |
| **222** | 5-[3-(Dimethylamino) propoxy]pyridin-3-amine | 196 | (3-chloropropyl) dimethylpropylamine |

### Method 223

### 5-{[3-(1-Cyano-1-methylethyl)benzoyl]amino}-2-chlorobenzoic acid

3-(1-Cyano-1-methylethyl)benzoic acid (Method 19, 150 mg) was combined with methyl 5-amino-2-chlorobenzoate (Method 217, 120 mg)), HATU (400 mg) and DIEA (0.6 ml) in DMF (3 ml) and stirred overnight at 25°C. LC/MS confirmed the formation of the product, methyl 5-{[3-(1-cyano-1-methylethyl)benzoyl]amino]-2-chlorobenzoate. The mixture was partitioned between H₂O and EtOAc, and the organic layer was washed with brine and dried with MgSO₄. Methyl 5-{[3-(1-cyano-1-methylethyl)benzoyl]amino}-2-chlorobenzoate was collected by evaporation as a reddish-brown oil.

Methyl 5-{[3-(1-cyano-1-methylethyl)benzoyl]amino}-2-chlorobenzoate (120 mg) was dissolved in a 3:1:1 (v/v/v) solution of THF/MeOH/H₂O and lithium hydroxide (50 mg) was added slowly. The reaction was then stirred overnight at 25°C. LC/MS confirmed the formation of the product. The organic solvents were evaporated and the remaining material was separated between EtOAc and H₂O. The aqueous layer was collected and acidified to a pH of between 3 and 4 with 2N HCl. The mixture was partitioned between H₂O and EtOAc, and the organic layer was washed with brine and dried with MgSO₄. The title compound (85 mg) was collected by evaporation as a white solid.

### Method 224

The following compound was prepared by the procedure of Method 223, using the appropriate starting material.

| **Method** | **Compound** | **M/z** | **SM** |
|---|---|---|---|
| **224** | 2-Chloro-5-{[3-(trifluoromethyl)benzoyl] amino}benzoic acid | 344 | Methyl 2-chloro-5-amino benzoate (Method 217) and 3-trifluorobenzoic acid |

### Method 225

### Sodium [3-(methoxycarbonyl)phenyl]methanesulfonate

To a solution of methyl 3-(bromomethyl)benzoate (2.5 g, 19.0 mmol) in 25 ml of acetone and 25 ml of H₂O, was added sodium sulfite (1.5 g, 12.0 mmol) and tertabutylammonium iodide (4.4 g, 12.0 mmol). The reaction warmed to 75 °C and was allowed to stir for 12 h at this temperature. The reaction mixture was cooled to room temperature and concentrated *in vacuo* to yield the title compound as a yellow solid 2.5 g (99%). *M*/*z* 230.

### Method 226

### 3-Cyclopropyl-5-fluorobenzoic acid

To a 50 ml round bottom flask charged with a magnetic stir bar was added 3-bromo-5-fluorobenzoic acid (0.500 g, 4.56 mmol) and cyclopropylboronic acid (0.590 g, 6.84 mmol). Toluene (15 ml) and H₂O(0.75 ml) were added followed by K₃PO₄ (3.86 g, 18.24 mmol) and Pd(PPh₃)₄ (1.05 g, 0.912 mmol). The resulting reaction mixture was heated to 100 °C for 12 h, was cooled to room temperature, and quenched with 10% aqueous NaOH (~100 ml). The reaction mixture was poured into a separator funnel and extracted with EtOAc (~100 ml). The resulting aqueous phase was isolated and brought to a pH of ~2 by the careful addition of 3N HCl at which time the desired product precipitated. The precipitate was collected via vacuum filtration, washed with an additional portion of H₂O (~100 ml), collected, and dried under vacuum for 24 h which yielded 0.310 g of the title compound (37%) as an off white solid. *M*/*z* 181.

### Method 227

### 2,3-Dichloro-5-nitro-pyridine

To a solution of 5-nitro-2-hydroxypyridine (700 mg, 5 mmol) in concentrated HCl (3.3ml)) was heated to 50°C to facilitate dissolution. A solution of KClO₃ (214 mg) in H₂O (3ml) was added drop-wise to the above solution in such a way the internal temperature did not exceed 60°C. The resulting mixture was stirred at ambient temperatures for 15 minutes, cooled to 0 °C and the product 3-chloro-5-nitro-2-hydroxypyridine was collected by filtration (600 mg, 68%). The pyridine (600 mg) was dissolved in quinoline (0.4 ml) and POCl₃ (0.35 ml). The resulting mixture was heated to 120°C for 2 hours. The mixture was partitioned between EtOAc and H₂O. The organics were washed with NaCl₍ₛₐₜ₎ and then dried with Na₂SO₄₍ₛ₎. The organics were then removed under reduced pressure to give the title compound (358 mg, 55%) as a brown solid. *M*/*z* 194.

### Method 228

### 3-[2-Cyano-1H-pyrrol-1-yl)methyl]benzoic acid

To a solution of methyl 3-(bromomethyl)benzoate (500 mg) in DMF (10 ml) was added a solution of 2-cyano pyrrole (350 mg) in DMF (5 ml) which had been previously treated with NaH (100 mg). The resulting mixture was stirred at ambient temperature for 3 hours. The mixture was partitioned between EtOAc and H₂O. The organics were washed with NaCl₍ₛₐₜ₎ and then dried with Na₂SO₄₍ₛ₎. Evaporation of the solvent gave methyl 3-[(2-cyano-1*H*-pyrrol-1-yl)methyl]benzoate (300 mg). *M*/*z* 241.

Methyl 3-[(2-cyano-1*H*-pyrrol-1-yl)methyl]benzoate (300 mg) was dissolved in MeOH/H₂O (10 ml, 3:1 v/v) and LiOH (60 mg) was added. The resulting mixture was stirred at room temperature for 10 hours. The aqueous phase was filtered and washed with EtOA_{C}. The basic aqueous layer was acidified by the addition of 1N HCl until pH 3. The aqueous layer was extracted with EtOAc (3x) and then dried with Na₂SO₄₍ₛ₎. Evaporation of the solvent afforded the title compound as a white solid (210 mg); *m*/*z* 226.

### Method 229

### 3-(1-Cyano-1-methylethyl)-5-fluorobenzoic acid

2-(3-Bromo-5-fluorophenyl)-2-methylpropanenitrile (Method 18; 258 mg, 1.07 mmol) in THF (10 ml) at-78 °C under Ar was treated with /BuLi (1.7 M in pentane, 2.13 mmol, 2.0 equiv). The reaction was stirred for 15 min and then CO_{2(g)} was bubbled through the reaction mixture. After 10 min, the reaction was quenched with 10% NaOH and extracted with EtOAc. The aqueous layer was acidified with 10% HCl and extracted with EtOAc. The organics were dried with NaCl₍ₛₐₜ₎ and Na₂SO₄₍ₛ₎ and then removed under reduced pressure; *mlz* 208.

### Method 230

### 5-Bromo-N,N-dimethylpyridine-3-sulfonamide

A solution of *N*,*N*-dimethylpyridine-3-sulfonamide (Method 129, 1.30 g, 6.98 mmol) and NaOAc (1.72 g, 20.9 mmol, 3.0 equiv) in HOAc (10 ml) was treated with Br₂ (0.72 ml, 14.0 mmol, 2.0 equiv). The reaction was stirred for 12 h at 50 °C. The reaction was quenched with H₂O and extracted with EtOAc. The organics were dried with NaCl₍ₛₐₜ₎ and Na₂SO₄₍ₛ₎ and then removed under reduced pressure. The residue was then purified by column chromatography utilizing an ISCO system (EtOAc-hexane) to give 340 mg (18%) of the desired product; *m*/*z* 266.

### Method 231

### 5-Fluoronicotinic acid

To a 1L round bottom flask charged with a magnetic stir bar and 2,6-dichloro-5-fluoronicotinic acid (5.00 g, 23.8 mmol) was added MeOH (240 ml) and DIEA (8.30 ml, 48.0 mmol). To this solution was carefully added 500 mg of palladium on carbon (10 wt %). The resulting reaction mixture was purged with H₂ and placed under an atmosphere of H₂ (1atm) using a balloon. The reaction was allowed to stir at ambient temperature for 12 h before being filtered through a bed of diatomaceous earth. The filtrate was concentrated *in vacuo* to a volume of ~ 25 ml and diluted with -250 ml of Et₂O. A precipitate formed (DIPEA/HCl salt) which was filtered off using a Buchner funnel. The filtrate was concentrated *in vacuo* to yield 3.2 g (98%) the title compound as a yellow oil which was used without further purification; *m*/*z* 141.

### Method 232

### 5-Aminonicotinonitrile

5-Bromonicotinonitrile (1.26g, 6.8mmol), Cs₂CO₃ (3.74g, 11.5mmol), BINAP (716mg, 1.15mmol), Pd₂(dba)₃ (526mg, 0.575mmol) were put in a round bottle, toluene 25mL and 1,1-diphenylmethanimine (1.43ml, 8.6mmoL) were added and the mixture was degassed for 5 minutes. The solution was heated at 100 °C over night. The crude product was filtered and evaporated, and dissolved in dioxane (10ml) followed by the addition of 4N HCl in dioxane 1.0ml. The solution was stirred at room temperature for 30 minutes. The residue left after evaporation was separated between water and ethyl acetate. Evaporation of the solvent gave the title compound (800 mg). *M*/*z* 120.

### Method 233

### N-(5-Amino-3-methylpyridin-2-yl-2-(benzyloxy)acetamide

To a solution of 2-amino-3-methyl-5-nitropyridine (1.0g) in pyridine (20 ml) at 0°C was added slowly (benzyloxy)acetyl chloride (2.5 ml) and the resulting mixture was stirred for 1 hour at ambient temperature. The mixture was partitioned between H₂O and EtOAc, and the organic layer was washed with brine and dried with MgSO₄. Evaporation under reduced pressure afforded the acetyl derivative which was used in the next step without further purification. (m/z 302)

Into a three-neck flask, the residue (500 mg) was dissolved in MeOH (25 ml). A solution of ammonium chloride (1.3 g, 250mmol) in H₂O (25 ml) was added, followed by 1.4g of iron powder. The solution was stirred by at 78°C for 2 hours. The solution was filtered at 50°C and the isolated solid was washed by hot methanol. The solvent was evaporated under reduced pressure and the product was extracted by acetone and filtered. The acetone solution was evaporated to give the title compound (100 mg) as solid. *M*/*z* 272.

### Method 234

### Methyl 5-piperidin-1-ylnicotinate

A 25 ml round bottom flask was charged with a magnetic stir bar, methyl 5-bromonicotinate (0.500 g, 2.31 mmol), piperidine (0.305 g, 3.46 mmol), and toluene (5 ml). Caesium carbonate (2.25 g, 6.93 mmol), palladium (II) acetate (52 mg, 0.23 mmol), and BINAP (0.287 g, 0.46 mmol) were then added. The reaction was heated to 80 °C for 8 h before being diluted with EtOAc (~ 50 ml), filtered through a pad of SiO₂, and concentrated *in vacuo.* The crude product was purified on 40 g SiO₂ using EtOAc as eluent giving 0.376 g of the title compound as a colourless oil (74 %). M/z 221.

### Methods 235-236

The following compounds were prepared by the procedure of Method 234, using the appropriate starting material.

| **Meth** | **Compound** | **m/z** | **SM** |
|---|---|---|---|
| **235** | Methyl 5-morpholin-4-ylnicotinate | 223 | morpholine |
| **236** | Methyl 5-(diethylamino)nicotinate | 209 | diethylamine |

### Method 237

### Methyl 3-cyclopropylbenzoate

To a' 100 ml found bottom flask charged with a magnetic stir bar and DCM (20 ml) was added 12.3 ml of diethyl zinc (1M in hexanes). The reaction mixture was cooled to 0 °C and trifluoroacetic acid (1.40 g, 12.3 mmol) was added dropwise via syringe. The reaction was stirred at this temperature for 20 mins. followed by the addition of CH₂l₂ (3.30 g, 12.3 mmol). The reaction mixture was stirred for 20 mins. before Methyl 3-vinylbenzoate (1.00 g, 6.16 mmol) was added. The reaction was then allowed to warm to room temperature with stirring for 3 h. before being quenched by the addition of ~50 ml of saturated aqueous NH₄Cl. The mixture was poured into a separator funnel and the aqueous phase was further extracted with DCM (3 x 50 ml). The combined organic extract was dried with MgSO₄ and concentrated *in vacuo* to yield the crude reaction product which was purified on 120 g SiO₂ using hexanes/EtOAc 10:1 as eluent giving 1.01 g of the title compound as a colourless oil (94 %) *mlz* 177.

### Methods 238-239

The following compounds were prepared by the procedure of Method 237, using the appropriate starting material.

| **Meth** | **Compound** | **m/z** | **SM** |
|---|---|---|---|
| **238** | Methyl 3-(1-cyclopropyl-1-methylethyl)benzoate | 219 | Method 165 |
| **239** | Methyl 3-(1-methylcyclopropyl)benzoate | 191 | Method 173 |

### Method 240

### (5-Bromopyridin-3-yl)methanol

To a 500 ml three neck flask fitted with a reflux condenser was added with a magnetic stir bar and MeOH (65 ml). NaBH₄ (12.2 g, 324 mmol) was carefully added followed by methyl 5-bromonicotinate (7.0 g, 32.4 mmol). The reaction mixture was heated to reflux with stirring for 5 h before being cooled to room temperature and the reaction mixture was quenched by the slow addition of ~ 250 ml of H₂O. The MeOH was removed under reduced pressure and the resulting aqueous phase was extracted with ~250 ml of EtOAc. The organic extract was dried with MgSO₄, filtered, and concentrated *in vacuo* to yield the crude product, which was purified on a 120 g SiO₂ column using MeOH/EtOAc (1:10) as eluent giving 2.3 g (37%) of the title compound as a white solid m/z 189.

### Method 241

### 5-({[tert-Butyl(diphenyl)silyl]oxy}methyl)-N-(diphenylmethylene)pyridin-3-amine

To a 200 ml round bottom flask charged with a magnetic stir bar was added 3-bromo-5-({[*tert*-butyl(diphenyl)silyl]oxy}methyl)pyridine (Method 102; 5.2 g, 12.2 mmol). Toluene (60 ml) was added followed by benzophenone imine (2.55 ml, 15.25 mmol), sodium *tert-*butoxide (2.00 g, 21.35 mmol), Pd₂(dba)₃ (0.558 g, 0.61 mmol), and BINAP (1.13 g, 1.83 mmol). The reaction was heated to 80 °C with stirring for 5 h before being cooled to room temperature and quenched with ~200 ml saturated aqueous NaHCO₃. The mixture was poured into a separator funnel and extracted with ~200 ml of EtOAc. The combined organic extract was dried with MgSO₄, filtered, and concentrated *in vacuo* to yield the crude product, which was purified on a 120 g SiO₂ column using hexanes/EtOAc (9:1) as eluent giving 5.26 g (82%) of the title compound as a white solid *m*/*z* 527.

### Method 242

### (5-Aminopyridin-3-yl)methanol hydrochloride

To a 500 ml round bottom flask charged with a magnetic stir bar was added *5-*({[*tert-*butyl(diphenyl)silyl]oxy]methyl)-*N*-(diphenylmethylene)pyridin-3-amine (Method 241; 5.2 g, 9.87 mmol). MeOH (200 ml) was added followed by the addition of 50 ml of 3N HCl with stirring. The reaction was allowed to stir for 12 h at room temperature before being concentrated *in vacuo.* The crude oil diluted with EtOAc (~100 ml) and a precipitate formed. The solid was collected on a Buchner funnel to provide 1.24 g (79 %) of the title compound as an off white solid *m*/*z* 125.

### Method 243

### N²-Cyclopropylpyridine-2,5-diamine

To a 100 ml round bottom flask charged with a magnetic stir bar and *N*-cyclopropyl-5-nitropyridin-2-amine (Method 149; 0.800 g, 4.46 mmol) was added ETOH (15 ml). Tin (II) chloride dihydrate (4.03 g, 17.84 mmol) was added and the reaction was warmed to 70 °C with stirring for 4 h before being cooled to room temperature. The crude reaction mixture was then quenched with ~150 ml of saturated aqueous NaHCO₃. The resulting mixture was poured into a separator funnel and extracted with ~150 ml of EtOAc. The combined organic extract was dried with MgSO₄, filtered, and concentrated *in vacuo* to yield the title compound as as yellow solid 0.551 g (83%), which was used without further purification; *m*/*z* 150.

### Methods 244-250

The following compounds were prepared by the procedure of Method 243, using the appropriate starting material.

| **Meth** | **compound** | **m/z** | **SM** |
|---|---|---|---|
| **244** | *N*²-Cyclobutylpyridine-2,5-diamine | 164 | Method 158 |
| **245** | *N*²-Isopropylpyridine-2,5-diamine | 152 | Method 150 |
| **246** | (2*R*)-3-[(5-Aminopyridin-2-yl)amino]propane-1,2-diol | 184 | Method 151 |
| **247** | 2-[(5-Aminopyridin-2-yl)amino]propane-1,3-diol | 184 | Method 152 |
| **248** | *N*²-(4-Aminobutyl)pyridine-2,5-diamine | 180 | Method 155 |
| **249** | 6-Piperazin-1-ylpyridin-3-amine | 178 | Method 153 |
| **250** | *N*²-(3-Pyrrolidin-1-ylpropyl)pyridine-2,5-diamine | 220 | Method 154 |

### Method 251

### tert-Butyl (5-fluoropyridin-3-yl)carbamate

To a 100 ml round bottom flask charged with a magnetic stir bar was added 5-fluoronicotinic acid (Method 231; 1.00 g, 7.09 mmol). *Tert*-butyl alcohol was added (20 ml) followed by DIEA (3.08 ml, 17.7 mmol). Diphenyl phosphoryl azide (3.83 ml, 17.72 mmol) was added and the flask was fitted with a reflux condenser. The reaction was heated to reflux for 12 h, cooled, and the crude reaction mixture was concentrated *in vacuo. The* resulting oil was dissolved in EtOAc (~ 200 ml) and the organic phase was washed 3 x 200 ml saturated aqueous NaHCO₃. The organic phase was dried with MgSO₄, filtered, and concentrated *in vacuo* yielding the crude product which was purified on SiO₂ (120 g) using EtOAc/hexanes (1:5) was eluent to yield 0.83g (55%) of the title compound; *m*/*z* 213.

### Method 252

### 5-Fluoropyridin-3-amine hydrochloride

To a 100 nil round bottom flask charged with a magnetic stir bar and *tert-*butyl (5-fluoropyridin-3-yl)carbamate (Method 251; 0.800 g, 3.77 mmol) was added MeOH (100 ml). A 4N solution of HCl in dioxane was then added until the reaction mixture reached a pH of ~ 3. The reaction was allowed to stir for 12 h before being concentrated *in vacuo*. The crude product was suspended in ~250 ml of H₂O. A white precipitate formed which was collected via vacuum filtration. The filter cake was washed with an additional 250 ml of Et₂O and dried on a vacuum to yield 0.510 g (91%) of the title compound as a white solid; *m*/*z* 113.

### Method 253

### Methyl 3-(1-cyano-1-methylethyl)-5-[(methylthio)methyl]benzoate

A solution of methyl 3-(bromomethyl)-5-(1-cyano-1-methylethyl)benzoate (Method 138; 80 mg, 0.27 mmol) in 1 ml of EtOH was added sodium thiomethoxide. The mixture was stirred over open atmosphere at reflux. Evaporation the residual solvents yielded the product which was used without any further purification *m*/*z* 263.

### Method 254

### Sodium [3-(1-cyano-1-methylethyl)-5-(methoxycarbonyl)phenyl]methanesulfonate

To a solution of methyl 3-(bromomethyl)-5-(1-cyano-1-methylethyl)benzoate (Method 138; 230 mg, 0.777 mmol) in 5 ml of acetone and 5 ml of water was added sodium sulfite. The mixture was stirred over open atmosphere at reflux. The reaction was cooled to room temperature and the solvents were removed *in vacuo* to yield the title compound which was used without any further purification. *M*/*z* 297.

## Claims

1. A compound of formula **(I):** 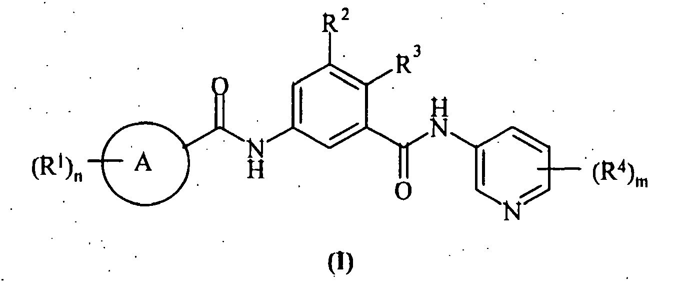 wherein:
**Ring A** is phenyl or a monocyclic 5 or 6 membered fully-unsaturated heterocyclic ring; wherein said phenyl or heterocyclic ring may be optionally fused to a five or six membered carbocyclyl or heterocyclyl forming a bicyclic ring; and wherein if said heterocyclyl or heterocyclic ring contains an -NH- moiety that nitrogen may be optionally substituted by a group selected from R⁵;
**R¹** is a substituent on carbon and is selected from halo, nitro, cyano, hydroxy, trifluoromethoxy, amino, carboxy, carbamoyl, mercapto, sulphamoyl, C₁₋₆alkyl, C₂₋₆alkenyl, C₂₋₆alkynyl, C₁-₆alkoxy, C₁₋₆alkanoyl, C₁₋₆alkanoyloxy, *N*-(C₁₋₆alkyl)amino, *N*,*N*-(C₁₋₆alkyl)₂amino, C₁₋₆alkanoylamino, *N*-(C₁₋₆alkyl)carbamoyl, *N*,*N-*(C₁₋₆alkyl)₂carbamoyl, C₁₋₆alkylS(O)ₐ wherein a is 0 to 2, C₁₋₆alkoxycarbonyl, C₁₋₆alkoxycarbonylamino, *N*-(C₁₋₆alkyl)sulphamoyl, *N*,*N-*(C₁₋₆alkyl)₂sulphamoyl, *N*-(C₁₋₆alkyl)-*N*-(C₁₋₆alkoxy)sulphamoyl, *N*,*N'*-(C₁₋₆alkyl)₂ureido, *N*,*N'*-(C₁₋₆alkyl)₂ureido, *N*-(C₁₋₆alkyl)-*N'*,*N'*-(C₁₋₆alkyl)₂ureido, C₁₋₆alkylsulphonylamino, carbocyclyl-R⁶- or heterocyclyl-R⁷-; wherein R¹ may be optionally substituted on carbon by one or more R⁸; and wherein if said heterocyclyl contains an -NH- moiety that nitrogen may be optionally substituted by a group selected from R⁹;
**n** is selected from 0-4; wherein the values of R¹ may be the same or different;
**R²** is selected from hydrogen, halo, nitro, cyano, hydroxy, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N*,*N*-(C₁₋₆alkyl)₂amino, C₁₋₆alkanoylamino, *N*-(C₁₋₆alkyl)carbamoyl, *N*,*N*-(C₁₋₆alkyl)₂carbamoyl, C₁₋₆alkylS(O)ₐ wherein a is 0 to 2, C₁₋₆alkoxycarbonyl, *N*-(C₁₋₆alkyl)sulphamoyl, *N*,*N-*(C₁₋₆alkyl)₂sulphamoyl, C₁₋₆alkylsulphonylamino, carbocyclyl-R¹⁰- or heterocyclyl-R¹¹-; wherein **R²** may be optionally substituted on carbon by one or more R¹²; and wherein if said heterocyclyl contains an -NH- moiety that nitrogen may be optionally substituted by a group selected from R¹³**;**
**R³** is selected from halo, hydroxy, cyano, methyl, methoxy or hydroxymethyl;
**R⁴** is selected from halo, nitro, cyano, hydroxy, trifluoromethoxy, amino, carboxy, carbamoyl, mercapto, sulphamoyl, ureido, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N*,*N-*(C₁₋₆alkyl)₂amino, C₁₋₆alkanoylamino, *N*-(C₁₋₆alkyl)carbamoyl, *N*,*N*-(C₁₋₆alkyl)₂carbamoyl, C₁₋₆alkylS(O)ₐ wherein a is 0 to 2, C₁₋₆alkoxycarbonyl, *N*-(C₁₋₆alkyl)sulphamoyl, *N*,*N*-(C₁₋₆alkyl)₂sulphamoyl, C₁₋₆alkylsulphonylamino, carbocyclyl-R¹⁴- or heterocyclyl-R¹⁵-; wherein R⁴ may be optionally substituted on carbon by one or more R¹⁶; and wherein if said heterocyclyl contains an -NH- moiety that nitrogen may be optionally substituted by a group selected from R¹⁷;
**m** is selected from 0-4; wherein the values of R⁴ may be the same or different;
**R⁸** and **R¹²** are independently selected from halo, nitro, cyano, hydroxy, trifluoromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N*,*N*-(C₁₋₆-alkyl)₂amino, *N*-(C₁₋₆alkyl)-*N*-(C₁₋₆alkoxy)amino, C₁₋₆alkanoylamino, *N*-(C₁₋₆alkyl)carbamoyl, *N*,*N*-(C₁₋₆alkyl)₂carbamoyl, C₁₋₆alkylS(O)ₐ wherein a is 0 to 2, C₁₋₆alkoxycarbonyl, *N*-(C₁₋₆alkyl)sulphamoyl, *N*,*N-*(C₁₋₆alkyl)₂sulphamoyl, C₁₋₆alkylsulphonylamino, carbocyclyl-R¹⁸- or heterocyclyl-R¹⁹-; wherein R⁸ and R¹² independently of each other may be optionally substituted on carbon by one or more R²⁰; and wherein if said heterocyclyl contains an -NH- moiety that nitrogen may be optionally substituted by a group selected from R²¹;
**R¹⁶** is selected from halo, nitro, cyano, hydroxy, trifluromethoxy, amino, carboxy, carbamoyl, mercapto, sulphamoyl, C₁₋₆alkyl, C₂₋₆alkenyl, C₂₋₆alkynyl, C₁₋₆alkoxy, C₁₋₆alkanoyl, C₁₋₆alkanoyloxy, *N*-(C₁₋₆alkyl)amino, *N*,*N*-(C₁₋₆alkyl)₂amino, C₁₋₆alkanoylamino, *N*-(C₁₋₆alkyl)carbamoyl, *N*,*N*-(C₁₋₆alkyl)₂carbamoyl, C₁₋₆alkylS(O)ₐ wherein a is 0 to 2, C₁₋₆alkoxycarbonyl, *N*-(C₁₋₆alkyl)sulphamoyl, *N,N*-(C₁₋₆alkyl)₂sulphamoyl, C₁₋₆alkylsulphonylamino, carbocyclyl-R²²- or heterocyclyl wherein R¹⁶ may be optionally substituted on carbon by one or more R²⁴; and wherein if said heterocyclyl contains an -NH- moiety that nitrogen, may be optionally substituted by a group selected from R²⁵;
**R⁶, R⁷,R¹⁰, R¹¹, R¹⁴, R¹⁵, R¹⁸, R¹⁹, R²²** and **R²³** are independently selected from a direct bond, -O-, -N(R²⁶ )-, -C(O)-,-N(R²⁷)C(O)-, -C(O)N(R²⁸)-, -S(O)_{S}-, -SO₂N(R²⁹)-or -N(R³⁰)SO₂-; wherein **R²⁶, R²⁷, R²⁸, R²⁹** and **R³⁰** are independently selected from hydrogen or C₁-₆alkyl and s is 0-2;
**R⁵, R⁹, R¹³, R¹⁷, R²¹** and **R²⁵** are independently selected from C₁₋₆alkyl, C₁₋₆alkanoyl, C₁₋₆alkylsulphonyl, C₁₋₆atkoxycatbonyl, carbamoyl, *N*-(C₁₋₆alkyl)carbamoyl, *N,N*-(C₁₋₆alkyl)carbamoyl, benzyl, benzyloxycarbonyl, benzoyl and phenylsulphonyl;
**R²⁰** and **R²⁴** are independently selected from halo, nitro, cyano, hydroxy, trifluoromethoxy, trifluoromethyl, amino, carboxy, carbamoyl, mercapto, sulphamoyl, methyl, ethyl, methoxy, ethoxy, acetyl, acetoxy, methylamino, ethylamino, dimethylamino, diethylamino, *N*-methyl-*N*-ethylamino, acetylamino, *N-m*ethylcarbamoyl, *N*-ethylcarbamoyl, *N*,*N*-dimethylcarbamoyl, *N*,*N*-diethylcarbamoyl, *N*-methyl-*N*-ethylcarbamoyl, phenyl, methylthio, ethylthio, methylsulphinyl, ethylsulphinyl, mesyl, ethylsulphonyl, methoxycarbonyl, ethoxycarbonyl, *N*-methylsulphamoyl, *N*-ethylsulphamoyl, *N,N*-dimethylsulphamoyl, *N,N*-diethylsulphamoyl or *N*-methyl-N-ethylsulphamoyl;
or a pharmaceutically acceptable salt thereof;
with the proviso that said compound is not:
*N*-[4-chloro-3-({[6-(4-methylpiperazin-1-yl)pyridin-3-yl]amino}carbonyl)phenyl]-2-morpholin-4-ylisonicotinamide;
*N*-[4-chloro-3-({[6-(4-ethylpipetazin-1-yl)pyridin-3-yl]amino}carbonyl)phenyl]-2-morpholin-4-ylisonicotinamide;
*N*-{4-chloro-3-[({6-[[3-(dimethylamino)propyl](methyl)amino]pyridin-3-yl}amino)carbonyl] phenyl}-2-morpholin-4-ylisonicotinamide;
*N*-{4-chloro-3-[({6-[[2-(dimethylamino)ethyl](methyl)amino]pyridin-3-yl}amino)carbonyl] phenyl}-2-morpholin-4-ylisonicotinamide; or
*N*-[4-chloro-3-({[6-(4-methyl-1,4-diazepan-1-yl)pyridin-3-yl]amino}carbonyl)phenyl]-2-morpholin-4-ylisonicotinamide.

2. A compound of formula **(I)**, or a pharmaceutically acceptable salt thereof, as claimed in claim 1 wherein Ring A is phenyl, pyridyl, pyrazolyl, thienyl, indolyl, 2,3-dihydrobenzofuranyl, imidazo[1,2-a]pyridinyl,isoxazolyl, benzimidazolyl, 2-oxoindolinyl, furanyl, 1,3-thiazolyl, pyrimidinyl and pyrrolyl; wherein said pyrazolyl, indolyl, pyrrolyl may be optionally substituted on nitrogen by a group selected from R⁵; wherein R⁵ is selected from C₁₋₆alkyl.

3. A compound of formula **(I)**, or a pharmaceutically acceptable salt thereof, as claimed in either claim 1 or claim 2 wherein R¹ is a substituent on carbon and is selected from halo, cyano, hydroxy, sulphamoyl, C₁₋₆alkyl, C₂-₆alkynyl, C₁₋₆alkoxy, *N,N*-(C₁₋₆alkyl)₂amino, C₁₋₆alkylS(O)ₐ wherein a is 0 to 2, C₁₋₆alkoxycarbonylamino, *N*-(C₁₋₆alkyl)sulphamoyl, *N,N*-(C₁₋₆alkyl)₂sulphamoyl, *N*-(C₁₋₆alkyl)-*N*-(C₁₋₆atkoxyl)sulphamoyl, *N',N*'-(C₁₋₆alkyl)₂ureido, C₁₋₆alkylsulphonylamino, carbocyclyl-R⁶- or heterocyclyl-R⁷-; wherein R¹ may be optionally substituted on carbon by one or more R⁸; wherein
R⁸ is selected from halo, cyano, hydroxy, C₁₋₆yalkyl, C₁₋₆alkoxy, *N,N*-(C₁₋₆alkyl)₂amino, *N,N*-(C₁₋₆alkyl)₂carbamoyl, C₁₋₆alkylS(O)ₐ wherein a is 0 to 2, *N,N*-(C₁₋₆alkyl)₂sulphamoyl, *N*-(C₁₋₆alkyl)-*N*-(C₁₋₆alkoxy)amino, carbocyclyl-R¹⁸-or heterocyclyl-R¹⁹-; wherein R⁸ may be optionally substituted on carbon by one or more R²⁰; and wherein if said heterocyclyl contains an -NH- moiety that nitrogen may be optionally substituted by a group selected from R²¹;
R⁶, R⁷, R¹⁸ and R¹⁹ are independently selected from a direct bond, -O-, -S(O)ₛ- or -N(R³⁰)SO₂-; wherein R³⁰ is selected from hydrogen and s is 2;
R²¹ is selected from C₁₋₆alkyl; and
R²⁰ is selected from cyano or hydroxy.

4. A compound of formula **(I),** or a pharmaceutically acceptable salt thereof, as claimed in any one of claims 1-3 wherein n is selected from 0-2; wherein the values of R¹ may be the same or different.

5. A compound of formula **(I),** or a pharmaceutically acceptable salt thereof, as claimed in any one of claims 1-4 wherein R² is hydrogen.

6. A compound of formula **(I),** or a pharmaceutically acceptable salt thereof, as claimed in any one of claims 1-5 wherein R³ is selected from halo, methyl or methoxy.

7. A compound of formula **(I),** or a pharmaceutically acceptable salt thereof, as claimed in any one of claims 1-6 wherein R⁴ is selected from halo, cyano, hydroxy, amino, carbamoyl, ureido, C₁₋₆alkyl, C₂₋₆alkynyl, C₁₋₆alkoxy, C₁₋₆alkanoyl, *N*-(C₁₋₆alkyl)amino, *N,N*-(C₁₋₆alkyl)amino, C₁₋₆alkanoylamino, *N*-(C₁₋₆alkyl)carbamoyl, C₁₋₆alkoxycarbonyl, *N,N*-(C₁₋₆alkyl)₂sulphamoyl, C₁₋₆alkylsulphonylamino, carbocyclyl-R¹⁴- or heterocyclyl-R¹⁵-; Wherein R⁴ may be optionally substituted on carbon by one or more R¹⁶; and wherein if said heterocyclyl contains an -NH- moiety that nitrogen may be optionally substituted by a group selected from R¹⁷; wherein
R¹⁶ is selected from halo, hydroxy, amino, C₁₋₆alkoxy, *N*-(C₁₋₆alkyl)amino, *N,N*-(C₁₋₆alkyl)₂amino, carbocyclyl-R²²- or heterocyclyl-R²³-; wherein R¹⁶ may be optionally substituted on carbon by one or more R²⁴; and wherein if said heterocyclyl contains an -NH-moiety that nitrogen may be optionally substituted by a group selected from R²⁵;
R¹⁴, R¹⁵, R²² and R²³ are independently selected from a direct bond, -N(R²⁶)- or -C(O)N(R²⁸)-; wherein R²⁶ and R²⁸ are hydrogen;
R¹⁷ and R²⁵ are independently selected from C₁₋₆alkyl and C₁₋₆alkoxycarbonyl; and
R²⁴ is methyl or phenyl.

8. A compound of formula **(I),** or a pharmaceutically acceptable salt thereof, as claimed in any one of claims 1-7 wherein m is selected from 0-2; wherein the values of R⁴ may be the same or different.

9. A compound of formula **(I)**: 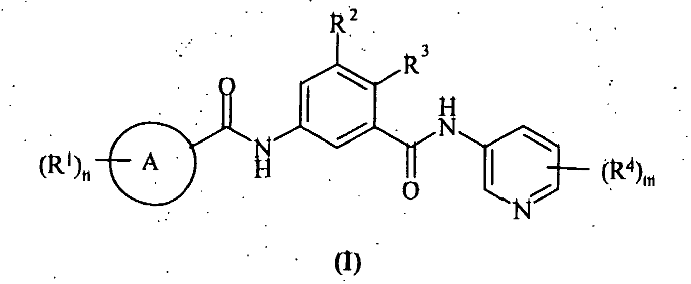 wherein:
Ring A is phenyl, pyrid-2-yl, pyrid-3-yl, pyrid-4-yl, 1-methylpyrazol-5-yl, 1-*t-*butylpyrazol-5-yl, thien-2-yl, thien-3-yl, indol-2-yl, 1-methylindol-2-yl, indol-4-yl, indol-5-yl, indol-6-yl, indol-7-yl, 2,3-dihydrobenzofuran-7-yl, imidxzo[1,2-a]pyridin-2-yl, isoxazol-3-yl, pyrrol-2-yl, benzimidnzol-6-yl,1-metltyl-2-oxoindolin-5-yl, buran-2-yl, 1,3-thiazol-5-yl, pyrimidin-4-yl or 1-methylpyrrol-2-yl);
R¹ is a substituent on carbon and is selected from fluoro, chloro, bromo, iodo, cyano, hydroxy, sulphamoyl, methyl, trifluoromethyl, 1-cyano-1-methylethyl, methoxy, ethoxy, propoxy, isopropoxy, isobutoxy, *N,N*-dimethylamino, difluoromethylthio, *N,N*-dimethylsulphamoyl, *t-*butyl, mesyl, cyclopropylaminosulphonyl, azetidin-1-ylsulphonyl, tetrahydrofuran-2-ylmethylaminosulphonyl, *N*-methyl-*N*-(2,3-dihydroxypropyl)sulphamoyl, mesylamino, morpholinosulphonyl, 1-methylpiperazin-4-ylmethyl, 1-ethylpiperazin-4-ylmethyl, 3,3-dimethylbut-1-yn-1-yl, morpholino, *N,N*-dimethylaminomethyl, 3-methyl-3-hydroxybut-1-yn-1-yl, methylthiomethyl, mesylmethyl, *N*-(methyl)-N-(methoxy)sulphamoyl, 2-hydroxymethylpiperidin-1-ylsulphonyl, 3-hydroxymethylpiperidin-1-ylsulphonyl, 4-hydroxymethylpiperidin-1-ylsulphonyl, 1,1-difluoroethyl, piperidin-1-yl, *N,N*-diethylamino, *N*',*N*'-dimethylureido, cyclopropyl, *t*-butoxycarbonylamino, pyrid-2-yl, phenoxy, 2-methoxy-1,1-dimethylethyl, mesylmethyl, 1,3-thiazol-2-yl, 2-methyl-1,3-thiazol-5-yl, 1-methylcyclopropyl, 1,1-dimethylprop-2-yn-1-yl, 1-(*N,N*-dimethylsulphamoyl)-1-methylethyl, 1,1-dimethylbut-2-yn-1-yl, *N*-(methyl)-*N*-(methoxy)aminomethyl, 1-(*N,N*-dimethylcarbamoyl)-1-methylethyl, 4-methylimidazol-1-yl, 1-(cyclopropyl)-1-methylethyl, 2-methyl-3,4-dihydroxybut-2-yl, 2-methylbut-2-yl, 1-hydroxy-1-cyclopropylethyl, 1-cyanoethyl, 2-cyano-3-methylbut-2-yl, 2-cyanobut-2-yl, 1-hydroxy-2-cyanoprop-2-yl and 2-cyanopyrrol-1-ylmethyl;
n is selected from 0-2; wherein the values of R¹ may be the same or different;
R² is hydrogen;
R³ is selected from fluoro, chloro, methyl or methoxy;
R⁴ is selected from fluoro, chloro, bromo, cyano, hydroxy, amino, carbamoyl, ureido, methyl, ethyl, methoxy, methylamino, isopropylamino, morpholino, 2-(dimethylamino)ethylamino, 2-(hydroxy)ethylamino, 2-(amino)ethylamino, 3-(pyrrolidin-1-yl)propylamino, *N*-methylcarbamoyl, acetylamino, 2-hydroxyacetylamino, trifluoromethyl, mesylamino, 2,2-dimethylpropanoylamino, 3-methoxypropanoylamino, cyclobutylcarbonylamino, cyclopropylamino, 2,3-dihydroxypropylamino, 1,3-dihydroxyprop-2-ylamino, 1-methylpiperazin-4-yl, 1-methylpiperazin-4-ylmethyl, acetyl, *N*-methyl-*N*-(3-dimethylaminopropyl)amino, *N*-methyl-N-(2-methoxyethyl)amino, dimethylamino, hydroxymethyl, 1,2-dihydroxyethyl, pyrazol-5-ylamino, 3-aminoprop-1-yn-1-yl, 3-hydroxyprop-1-yn-1-yl, 3-methylaminoprop-1-yn-1-yl, 3-dimethylaminoprop-1-yn-1-yl, 4-aminobutylamino, pyrrolidin-2-ylamino, 3-methylaminopropyl, 3-dimethylaminopropyl, 3-hydroxypropyl, 3-dimethylaminopropylamino, aminomethyl, piperazin-1-yl, 1-methylpiperazin-4-yl, 2,2-dimethyl-1,3-dioxolan-4-ylmethylamino, pyrrolidin-3-ylmethylamino, piperidin-4-ylmethylamino, imidazol-2-ylmethylamino, methoxymethyl, *N,N*-dimethylsulphamoyl, formylamino, morpholinomethyl, aminomethyl, 2-(dimethylamino)ethylamino, pyrrol-1-yl, pyrrol-2-yl, pyrrolidin-2-yl, imidazol-4-yl, cyclobutylamino, *N*-methyl-*N*-(2-dimethylaminoethyl)amino, 2-dimethylaminoethoxy, dimethylaminomethyl, cyclopropylaminomethyl, piperidin-1-ylmethyl, methylaminomethyl, pyrrolidin-2-ylmethoxy, 3-dimethylaminopropoxy, methoxycarbonyl, 1-(*t-*butoxycarbonyl)pyrrolidin-2-ylmethylamino, 1-(*t-*butoxycarbonyl)pyrrolidin-2-ylmethoxy, 2-phenoxyacetylamino and 1-(*t-*butoxycarbonyl)pyrrolidin-2-yl;
m is selected from 0-2; wherein the values of R⁴ may be the same or different;
or a pharmaceutically acceptable salt thereof;
with the proviso that said compound is not:
*N*-[4-chloro-3-({[6-(4-methylpiperazin-1-yl)pyridin-3-yl]amino}carbonyl)phenyl]-2-morpholin-4-ylisonicotinamide;
*N*-{4-chloro-3-[({6-[3-(dimethylamino)propyl](methyl)amino]pyridin-3-yl}amino)carbonyl] phenyl}-2-morpholin-4-ylisonicotinamide; or
*N*-{4-chloro-3-[({6-[[2-(dimethylamino)ethyl](methyl)amino]pyridin-3-yl}amino)carbonyl phenyl}-2-morpholin-4-ylisonicotinatnide.

10. A compound of formula (1): 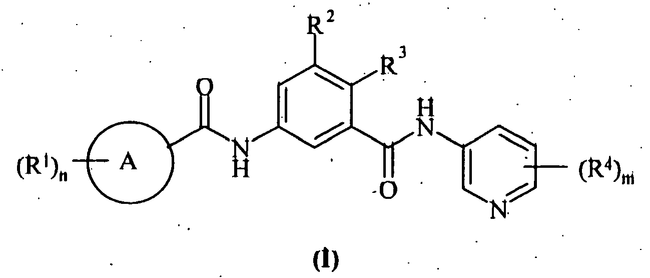 selected from:
*N*-(6-amino-5-chloropyridin-3-yl)-5-{[3-(1-cyano-1-methylethyl)benzoyl]amino}-2-methylbenzamide;
*N*-(6-amino-5-chloropyridin-3-yl)-5-{[3-(1-cyano-1-methylethyl)-5-fluorobenzoyl]amino)-2-methylbenzamide;
5-{[3-(1-cyano-1-methylethyl)benzoyl]amino}-*N*-(5-methoxypyridin-3-yl)-2-methyl benzamide;
*N*-(6-amino-5-chloropyridin-3-yl)-2-methyl-5-{[3-(trifluoromethyl)benzoyl]amino} benzamide;
5-{[3-(1-cyano-1-methylethyl)benzoyl]amino}-*N*-(5,6-dimethylpyridin-3-yl)-2-methylbenzamide;
*N-*(3-{[(6-amino-5-chloropyridin-3-yl)amino]carbonyl}-4-methylphenyl)-2-(1-cyano-1-methylethyl)isonicotinamide;
*N*-(6-amino-5-chloropyridin-3-yl)-2-chloro-5-{[3-(trifluoromethyl)benzoyl]amino} benzamide;
*N*-(6-acetylamino-pyridin-3-yl)-5-[3-(cyano-dimethyl-methyl)-benzoylamino]-2-methyl-benzamide;
*N*-[6-(acetylamino)pyridin-3-yl]-2-chloro-5-{[3-(trifluoromethyl)benzoyl]amino}benzamide; and
*N*-(6-amino-5-methylpyridin-3-yl)-2-chloro-5-{[3-(1-cyano-1-methylethyl)benzoyl]amino} benzamide;
or a pharmaceutically acceptable salt thereof.

11. A process for preparing a compound of formula **(I)** or a pharmaceutically acceptable salt thereof which process, wherein variable groups are, unless otherwise specified, as defined in claim 1, comprises of:
*Process a)* reacting an amine of the formula **(II)** 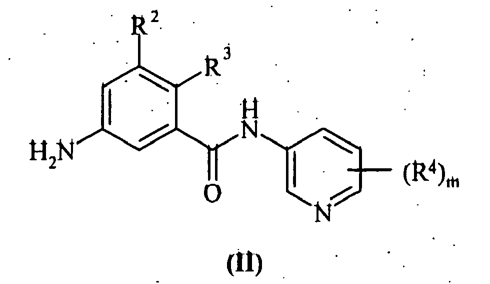 with an acid of formula **(III):** 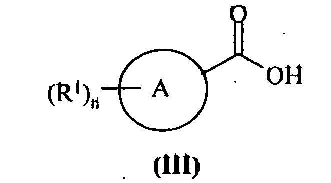 or an activated acid derivative thereof;
*Process b)* reacting an acid of formula **(IV):** 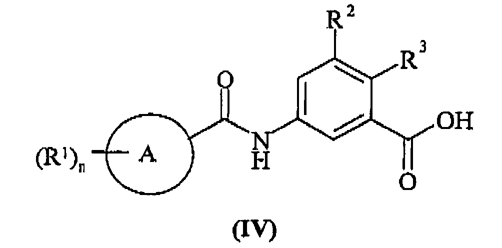 with an amine of formula **(V)**: 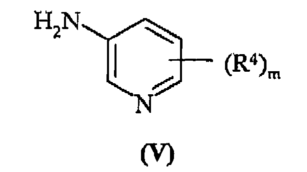
or an activated acid derivative hereof;
and thereafter if necessary:
i) converting a compound of the formula **(I)** into another compound of the formula **(I)**;
ii) removing any protecting groups;
iii) forming a pharmaceutically acceptable salt.

12. A pharmaceutical composition which comprises a compound of the formula **(I)**, or a pharmaceutically acceptable salt thereof, as claimed in any one of claims 1-10, in association with a pharmaceutically-acceptable diluent or carrier.

13. A compound of the formula **(I),** or a pharmaceutically acceptable salt thereof, as claimed in any one of claims 1-10 for use as a medicament.

14. The use of a compound of the formula **(I)**, or a pharmaceutically acceptable salt thereof, as claimed in any one of claims 1-10, in the manufacture of a medicament for the production of a B-Raf inhibitory effect in a warm-blooded animal such as man.

15. The use of a compound of the formula **(I)**, or a pharmaceutically acceptable salt thereof, as claimed in any one of claims 1-10, in the manufacture of a medicament for the production of an anti-cancer effect in a warm-blooded animal such as man.

16. The use of a compound of the formula **(I)**, or a pharmaceutically acceptable salt thereof, as claimed in any one of claims 1-10, in the manufacture of a medicament for the treatment of melanoma, papillary thyroid tumours, cholangiocarcinomas, colon cancer, ovarian cancer, lung cancer, leukaemias, lymphoid malignancies, carcinomas and sarcomas in the liver, kidney, bladder, prostate, breast and pancreas, and primary and recurrent solid tumours of the skin, colon, thyroid, lungs and ovaries.

## Patentansprüche

1. Verbindung der Formel **(I):** 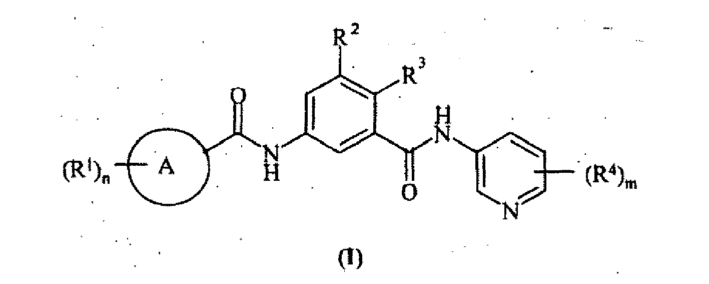 worin:
**Ring A** für Phenyl oder einen monocyclischen 5- oder 6-gliedrigen vollständig ungesättigten heterocyclischen Ring steht, wobei das Phenyl bzw. der heterocyclische Ring gegebenenfalls unter Bildung eines bicyclischen Rings an ein fünf- oder sechsgliedriges Carbocyclyl oder Heterocyclyl ankondensiert sein kann und worin dann, wenn das Heterocyclyl bzw. der heterocyclische Ring eine -NH-Gruppierung enthält, dieser Stickstoff gegebenenfalls durch eine unter R⁵ ausgewählte Gruppe substituiert sein kann;
**R¹** für einen Substituenten an Kohlenstoff steht und unter Halogen, Nitro, Cyano, Hydroxy, Trifluormethoxy, Amino, Carboxy, Carbamoyl, Mercapto, Sulfamoyl, C₁₋₆-Alkyl, C₂₋₆-Alkenyl, C₂₋₆-Alkinyl, C₁₋₆-Alkoxy, C₁₋₆-Alkanoyl, C₁₋₆-Alkanoyloxy, *N*-(C₁₋₆-Alkyl) amino, *N,N-*(C₁₋₆-Alkyl)₂amino, C₁₋₆-Alkanoylamino, *N*-(C₁₋₆-Alkyl) carbamoyl, *N,N-*(C₁₋₆-Alkyl)₂carbamoyl, C₁₋₆-AlkylS(O)ₐ, worin a für 0 bis 2 steht, C₁₋₆-Alkoxycarbonyl, C₁₋₆-Alkoxycarbonylamino, *N*-(C₁₋₆-Alkyl) sulfamoyl, *N,N-*(C₁₋₆-Alkyl)₂-sulfamoyl, *N*-(C₁₋₆-Alkyl)-*N*-(C₁₋₆-alkoxy) sulfamoyl, *N,N'-*(C₁₋₆-Alkyl)₂ureido, *N',N'-*(C₁₋₆-Alkyl)₂ureido, *N*-(C₁₋₆-Alkyl) *-N',N'-*(C₁₋₆-alkyl)₂ureido, C₁₋₆-Alkylsulfonylamino, Carbocyclyl-R⁶- oder Heterocyclyl-R⁷- ausgewählt ist; wobei R¹ gegebenenfalls an Kohlenstoff durch eine oder mehrere Gruppen R⁸ substituiert sein kann und dann, wenn das Heterocyclyl eine -NH-Gruppierung enthält, dieser Stickstoff gegebenenfalls durch eine unter R⁹ ausgewählte Gruppe substituiert sein kann;
**n** unter 0-4 ausgewählt ist; wobei die Werte von R¹ gleich oder verschieden sein können;
**R²** unter Wasserstoff, Halogen, Nitro, Cyano, Hydroxy, Trifluormethoxy, Amino, Carboxy, Carbamoyl, Mercapto, Sulfamoyl, C₁₋₆-Alkyl, C₂₋₆-Alkenyl, C₂₋₆-Alkinyl, C₁₋₆-Alkoxy, C₁₋₆-Alkanoyl, C₁₋₆-Alkanoyloxy, *N*-(C₁₋₆-Alkyl) amino, *N,N-*(C₁₋₆-Alkyl)₂amino, C₁₋₆-Alkanoylamino, *N*-(C₁₋₆-Alkyl) carbamoyl, *N,N-*(C₁₋₆-Alkyl)₂carbamoyl, C₁₋₆-AlkylS(O)ₐ, worin a für 0 bis 2 steht, C₁₋₆-Alkoxycarbonyl, *N*-(C₁₋₆-Alkyl) sulfamoyl, *N,N-*(C₁₋₆-Alkyl)₂sulfamoyl, C₁₋₆-Alkylsulfonylamino, Carbocyclyl-R¹⁰- oder Heterocyclyl-R¹¹- ausgewählt ist; wobei R² gegebenenfalls an Kohlenstoff durch eine oder mehrere Gruppen R¹² substituiert sein kann und dann, wenn das Heterocyclyl eine -NH-Gruppierung enthält, dieser Stickstoff gegebenenfalls durch eine unter R¹³ ausgewählte Gruppe substituiert sein kann;
**R³** unter Halogen, Hydroxy, Cyano, Methyl, Methoxy oder Hydroxymethyl ausgewählt ist;
**R⁴** unter Halogen, Nitro, Cyano, Hydroxy, Trifluormethoxy, Amino, Carboxy, Carbamoyl, Mercapto, Sulfamoyl, Ureido, C₁₋₆-Alkyl, C₂₋₆-Alkenyl, C₂₋₆-Alkinyl, C₁₋₆-Alkoxy, C₁₋₆-Alkanoyl, C₁₋₆-Alkanoyloxy, *N*-(C₁₋₆-Alkyl) amino, *N,N-*(C₁₋₆-Alkyl)₂amino, C₁₋₆-Alkanoylamino, *N*-(C₁₋₆-Alkyl) carbamoyl, *N,N-*(C₁₋₆-Alkyl)₂carbamoyl, C₁₋₆-AlkylS(O)ₐ, worin a für 0 bis 2 steht, C₁₋₆-Alkoxycarbonyl, *N*-(C₁₋₆-Alkyl)-sulfamoyl, *N,N-*(C₁₋₆-Alkyl)₂sulfamoyl, C₁₋₆-Alkylsulfonylamino, Carbocyclyl-R¹⁴- oder Heterocyclyl-R¹⁵- ausgewählt ist; wobei R⁴ gegebenenfalls an Kohlenstoff durch eine oder mehrere Gruppen R¹⁶ substituiert sein kann und dann, wenn das Heterocyclyl eine -NH-Gruppierung enthält, dieser Stickstoff gegebenenfalls durch eine unter R¹⁷ ausgewählte Gruppe substituiert sein kann;
**m** unter 0-4 ausgewählt ist; wobei die Werte von R⁴ gleich oder verschieden sein können;
**R⁸** und **R¹²** unabhängig voneinander unter Halogen, Nitro, Cyano, Hydroxy, Trifluormethoxy, Amino, Carboxy, Carbamoyl, Mercapto, Sulfamoyl, C₁₋₆-Alkyl, C₂₋₆-Alkenyl, C₂₋₆-Alkinyl, C₁₋₆-Alkoxy, C₁₋₆-Alkanoyl, C₁₋₆-Alkanoyloxy, *N*-(C₁₋₆-Alkyl) amino, *N,N-*(C₁₋₆-Alkyl) ₂amino, *N*-(C₁₋₆-Alkyl) *-N-*(C₁₋₆-alkoxy) amino, C₁₋₆-Alkanoylamino, *N*-(C₁₋₆-Alkyl) - carbamoyl, *N,N-*(C₁₋₆-Alkyl) ₂carbamoyl, C₁₋₆-AlkylS(O)ₐ, worin a für 0 bis 2 steht, C₁₋₆-Alkoxycarbonyl, *N*-(C₁₋₆-Alkyl)sulfamoyl, *N,N*-(C₁₋₆-Alkyl)₂-sulfamoyl, C₁₋₆-Alkylsulfonylamino, Carbocyclyl-R¹⁸-oder Heterocyclyl-R¹⁹- ausgewählt sind; wobei R⁸ und R¹² unabhängig voneinander gegebenenfalls an Kohlenstoff durch eine oder mehrere Gruppen R²⁰ substituiert sein können und dann, wenn das Heterocyclyl eine -NH-Gruppierung enthält, dieser Stickstoff gegebenenfalls durch eine unter R²¹ ausgewählte Gruppe substituiert sein kann;
**R¹⁶** unter Halogen, Nitro, Cyano, Hydroxy, Trifluormethoxy, Amino, Carboxy, Carbamoyl, Mercapto, Sulfamoyl, C₁₋₆-Alkyl, C₂₋₆-Alkenyl, C₂₋₆-Alkinyl, C₁₋₆-Alkoxy, C₁₋₆-Alkanoyl, C₁₋₆-Alkanoyloxy, *N*-(C₁₋₆-Alkyl) amino, *N,N-*(C₁₋₆-Alkyl)₂amino, C₁₋₆-Alkanoylamino, *N*-(C₁₋₆-Alkyl)carbamoyl, *N,N-*(C₁₋₆-Alkyl)₂carbamoyl, C₁₋₆-AlkylS(O)ₐ, worin a für 0 bis 2 steht, C₁₋₆-Alkoxycarbonyl, *N*- (C₁₋₆-Alkyl) - sulfamoyl, *N,N-* (C₁₋₆-Alkyl) ₂sulfamoyl, C₁₋₆-Alkylsulfonylamino, Carbocyclyl-R²²- oder Heterocyclyl-R²³- ausgewählt ist; wobei R¹⁶ gegebenenfalls an Kohlenstoff durch eine oder mehrere Gruppen R²⁴ substituiert sein kann und dann, wenn das Heterocyclyl eine -NH-Gruppierung enthält, dieser Stickstoff gegebenenfalls durch eine unter R²⁵ ausgewählte Gruppe substituiert sein kann;
**R⁶ , R⁷ , R¹⁰ , R¹¹, R¹⁴ , R¹⁵, R¹⁸, R¹⁹, R²²** und **R²³** unabhängig voneinander unter einer direkten Bindung, -O-, -N(R²⁶)-, -C(O)-, -N(R²⁷)C(O)-, -C(O)N(R²⁸ )-, -S(O)ₛ-, -SO₂N(R²⁹)- oder -N(R³⁰)SO₂-ausgewählt sind, wobei **R²⁶, R²⁷, R²⁸, R²⁹** und **R³⁰** unabhängig voneinander unter Wasserstoff oder C₁₋₆-Alkyl ausgewählt sind und s für 0-2 steht;
**R⁵, R⁹, R¹³, R¹⁷, R²¹** und **R²⁵** unabhängig voneinander unter C₁₋₆-Alkyl, C₁₋₆-Alkanoyl, C₁₋₆-Alkylsulfonyl, C₁₋₆-Alkoxycarbonyl, Carbamoyl, *N*-(C₁₋₆-Alkyl)-carbamoyl, *N,N-*(C₁₋₆-Alkyl)₂carbamoyl*,* Benzyl, Benzyloxycarbonyl, Benzoyl und Phenylsulfonyl ausgewählt sind;
**R²⁰** und **R²⁴** unabhängig voneinander unter Halogen, Nitro, Cyano, Hydroxy, Trifluormethoxy, Trifluormethyl, Amino, Carboxy, Carbamoyl, Mercapto, Sulfamoyl, Methyl, Ethyl, Methoxy, Ethoxy, Acetyl, Acetoxy, Methylamino, Ethylamino, Dimethylamino, Diethylamino, *N*-Methyl-N-ethylamino, Acetylamino, *N*-Methylcarbamoyl, *N*-Ethylcarbamoyl, *N,N*-Dimethylcarbamoyl, *N,N*-Diethylcarbamoyl, *N*-Methyl-N-ethylcarbamoyl, Phenyl, Methylthio, Ethylthio, Methylsulfinyl, Ethylsulfinyl, Mesyl, Ethylsulfonyl, Methoxycarbonyl, Ethoxycarbonyl, *N*-Methylsulfamoyl, *N*-Ethylsulfamoyl, *N,N*-Dimethylsulfamoyl, *N,N*-Diethylsulfamoyl oder *N*-Methyl-*N-*ethylsulfamoyl ausgewählt sind;
oder ein pharmazeutisch annehmbares Salz davon;
mit der Maßgabe, daß es sich bei der Verbindung nicht um:
*N*-[4-Chlor-3-({[6-(4-methylpiperazin-1-yl)pyridin-3-yl]amino}carbonyl)phenyl]-2-morpholin-4-ylisonicotinamid;
*N*-[4-Chlor-3-({[6-(4-ethylpiperazin-1-yl)pyridin-3-yl]amino}carbonyl)phenyl]-2-morpholin-4-ylisonicotinamid;
*N*-{4-Chlor-3-[({6-[[3-(dimethylamino)propyl]-(methyl)amino]pyridin-3-yl}amino)carbonyl]phenyl}-2-morpholin-4-ylisonicotinamid;
*N*-{4-Chlor-3-[({6-[[2-(dimethylamino)ethyl]-(methyl)amino]pyridin-3-yl}amino)carbonyl]phenyl}-2-morpholin-4-ylisonicotinamid oder
*N*-[4-Chlor-3-({[6-(4-methyl-1,4-diazepan-1-yl)-pyridin-3-yl]amino}carbonyl)phenyl]-2-morpholin-4-ylisonicotinamid
handelt.

2. Verbindung der Formel **(I)** oder ein pharmazeutisch annehmbares Salz davon nach Anspruch 1, worin Ring A für Phenyl, Pyridyl, Pyrazolyl, Thienyl, Indolyl, 2,3-Dihydrobenzofuranyl, Imidazo[1,2-a]-pyridinyl, Isoxazolyl, Benzimidazolyl, 2-Oxoindolyl, Furanyl, 1,3-Thiazolyl, Pyrimidinyl und Pyrrolyl steht; wobei das Pyrazolyl, Indolyl, Pyrrolyl gegebenenfalls an Stickstoff durch eine unter R⁵ ausgewählte Gruppe substituiert sein kann, wobei R⁵ unter C₁₋₆-Alkyl ausgewählt ist.

3. Verbindung der Formel **(I)** oder ein pharmazeutisch annehmbares Salz davon nach Anspruch 1 oder Anspruch 2, worin R¹ für einen Substituenten an Kohlenstoff steht und unter Halogen, Cyano, Hydroxy, Sulfamoyl, C₁₋₆-Alkyl, C₂₋₆-Alkinyl, C₁₋₆-Alkoxy, *N,N-*(C₁₋₆-Alkyl)₂amino, C₁₋₆-AlkylS(O)ₐ, worin a für 0 bis 2 steht, C₁₋₆-Alkoxycarbonylamino, *N*-(C₁₋₆-Alkyl) sulfamoyl, *N,N*-(C₁₋₆-Alkyl)₂-sulfamoyl, *N*-(C₁₋₆-Alkyl)-*N*-(C₁₋₆-alkoxy)sulfamoyl, *N',N'-*(C₁₋₆-Alkyl)₂ureido, C₁₋₆-Alkylsulfonylamino, Carbocyclyl-R⁶- oder Heterocyclyl-R⁷- ausgewählt ist; wobei R¹ gegebenenfalls an Kohlenstoff durch eine oder mehrere Gruppen R⁸ substituiert sein kann, wobei
R⁸ unter Halogen, Cyano, Hydroxy, C₁₋₆-Alkyl, C₁₋₆-Alkoxy, *N,N-* (C₁₋₆-Alkyl) ₂amino, *N,N-* (C₁₋₆-Alkyl)₂-carbamoyl, C₁₋₆-AlkylS(O)ₐ, worin a für 0 bis 2 steht, *N,N*- (C₁₋₆-Alkyl) ₂sulfamoyl, *N*-(C₁₋₆₋Alkyl)-*N-*(C₁₋₆-alkoxy) amino, Carbocyclyl-R¹⁸- oder Heterocyclyl-R¹⁹- ausgewählt ist; wobei R⁸ gegebenenfalls an Kohlenstoff durch eine oder mehrere Gruppen R²⁰ substituiert sein kann;
und dann, wenn das Heterocyclyl eine -NH-Gruppierung enthält, dieser Stickstoff gegebenenfalls durch eine unter R²¹ ausgewählte Gruppe substituiert sein kann;
R⁶, R⁷, R¹⁸ und R¹⁹ unabhängig voneinander unter einer direkten Bindung, -O-, -S(O)s- oder - N (R³⁰) SO₂- ausgewählt sind; wobei R³⁰ unter Wasserstoff ausgewählt ist und s für 2 steht;
R²¹ unter C₁₋₆-Alkyl ausgewählt ist und
R²⁰ unter Cyano oder Hydroxy ausgewählt ist.

4. Verbindung der Formel **(I)** oder ein pharmazeutisch annehmbares Salz davon nach einem der Ansprüche 1-3, worin n unter 0-2 ausgewählt ist; wobei die Werte von R¹ gleich oder verschieden sein können.

5. Verbindung der Formel **(I)** oder ein pharmazeutisch annehmbares Salz davon nach einem der Ansprüche 1-4, worin R² für Wasserstoff steht.

6. Verbindung der Formel **(I)** oder ein pharmazeutisch annehmbares Salz davon nach einem der Ansprüche 1-5, worin R³ unter Halogen, Methyl oder Methoxy ausgewählt ist.

7. Verbindung der Formel **(I)** oder ein pharmazeutisch annehmbares Salz davon nach einem der Ansprüche 1-6, worin R⁴ unter Halogen, Cyano, Hydroxy, Amino, Carbamoyl, Ureido, C₁₋₆-Alkyl, C₂₋₆-Alkinyl, C₁₋₆-Alkoxy, C₁₋₆-Alkanoyl, *N*-(C₁₋₆-Alkyl) amino, *N,N-*(C₁₋₆-Alkyl) ₂amino, C₁₋₆-Alkanoylamino, *N*- (C₁₋₆-Alkyl) carbamoyl, C₁₋₆-Alkoxycarbonyl, *N,N-* (C₁₋₆-Alkyl)₂sulfamoyl, C₁₋₆-Alkylsulfonylamino, Carbocyclyl-R¹⁴- oder Heterocyclyl-R¹⁵- ausgewählt ist; wobei R⁴ gegebenenfalls an Kohlenstoff durch eine oder mehrere Gruppen R¹⁶ substituiert sein kann und dann, wenn das Heterocyclyl eine -NH-Gruppierung enthält, dieser Stickstoff gegebenenfalls durch eine unter R¹⁷ ausgewählte Gruppe substituiert sein kann; wobei
R¹⁶ unter Halogen, Hydroxy, Amino, C₁₋₆-Alkoxy, N-(C₁₋₆-Alkyl) amino, ***N,N-*** (C₁₋₆-Alkyl) ₂amino, Carbocyclyl-R²²- oder Heterocyclyl-R²³- ausgewählt ist; wobei R¹⁶ gegebenenfalls an Kohlenstoff durch eine oder mehrere Gruppen R²⁴ substituiert sein kann und dann, wenn das Heterocyclyl eine -NH-Gruppierung enthält, dieser Stickstoff gegebenenfalls durch eine unter R²⁵ ausgewählte Gruppe substituiert sein kann;
R¹⁴, R¹⁵, R²² und R²³ unabhängig voneinander unter einer direkten Bindung, -N (R²⁶) - oder -C (O) N (R²⁸) - ausgewählt sind; wobei R²⁶ und R²⁸ für Wasserstoff stehen;
R¹⁷ und R²⁵ unabhängig voneinander unter C₁₋₆-Alkyl und C₁₋₆-Alkoxycarbonyl ausgewählt sind und
R²⁴ für Methyl oder Phenyl steht.

8. Verbindung der Formel **(I)** oder ein pharmazeutisch annehmbares Salz davon nach einem der Ansprüche 1-7, worin m unter 0-2 ausgewählt ist; wobei die Werte von R⁴ gleich oder verschieden sein können.

9. Verbindung der Formel **(I):** 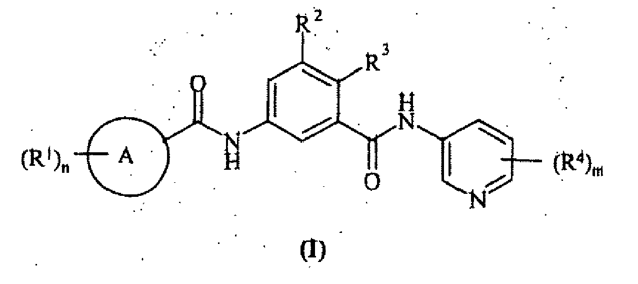 worin:
Ring A für Phenyl, Pyrid-2-yl, Pyrid-3-yl, Pyrid-4-yl, 1-Methylpyrazol-5-yl, 1-*t*-Butylpyrazol-5-yl, Thien-2-yl, Thien-3-yl, Indol-2-yl, 1-Methylindol-2-yl, Indol-4-yl, Indol-5-yl, Indol-6-yl, Indol-7-yl, 2,3-Dihydrobenzofuran-7-yl, Imidazo[1,2-a]-pyridin-2-yl, Isoxazol-3-yl, Pyrrol-2-yl, Benzimidazol-6-yl, 1-Methyl-2-oxoindolin-5-yl, Furan-2-yl, 1,3-Thiazol-5-yl, Pyrimidin-4-yl oder 1-Methylpyrrol-2-yl steht;
R¹ für einen Substituenten an Kohlenstoff steht und unter Fluor, Chlor, Brom, Iod, Cyano, Hydroxy, Sulfamoyl, Methyl, Trifluormethyl, 1-Cyano-1-methylethyl, Methoxy, Ethoxy, Propoxy, Isopropoxy, Isobutoxy, *N,N*-Dimethylamino, Difluormethylthio, *N,N*-Dimethylsulfamoyl, *t-*Butyl, Mesyl, Cyclopropylaminosulfonyl, Azetidin-1-ylsulfonyl, Tetrahydrofuran-2-ylmethylaminosulfonyl, *N-*Methyl-*N-*(2,3-dihydroxypropyl)sulfamoyl, Mesylamino, Morpholinosulfonyl, 1-Methylpiperazin-4-ylmethyl, 1-Ethylpiperazin-4-ylmethyl, 3,3-Dimethylbut-1-in-1-yl, Morpholino, *N,N*-Dimethylaminomethyl, 3-Methyl-3-hydroxybut-1-in-1-yl, Methylthiomethyl, Mesylmethyl, *N-*(Methyl)-*N-*(methoxy)sulfamoyl, 2-Hydroxymethylpiperidin-1-ylsulfonyl, 3-Hydroxymethylpiperidin-1-ylsulfonyl, 4-Hydroxymethylpiperidin-1-ylsulfonyl, 1,1-Difluorethyl, Piperidin-1-yl, *N,N*-Diethylamino, *N',N'*-Dimethylureido, Cyclopropyl, *t*-Butoxycarbonylamino, Pyrid-2-yl, Phenoxy, 2-Methoxy-1,1-dimethylethyl, Mesylmethyl, 1,3-Thiazol-2-yl, 2-Methyl-1,3-thiazol-5-yl, 1-Methylcyclopropyl, 1,1-Dimethylprop-2-in-1-yl, 1-(*N,N*-Dimethylsulfamoyl)-1-methylethyl, 1,1-Dimethylbut-2-in-1-yl, *N-*(Methyl)-*N-*(methoxy)-aminomethyl, 1-(*N,N*-Dimethylcarbamoyl)-1-methylethyl, 4-Methylimidazol-1-yl, 1-(Cyclopropyl)-1-methylethyl, 2-Methyl-3,4-dihydroxybut-2-yl, 2-Methylbut-2-yl, 1-Hydroxy-1-cyclopropylethyl, 1-Cyanoethyl, 2-Cyano-3-methylbut-2-yl, 2-Cyanobut-2-yl, 1-Hydroxy-2-cyanoprop-2-yl und 2-Cyanopyrrol-1-ylmethyl ausgewählt ist;
n unter 0-2 ausgewählt ist; wobei die Werte von R¹ gleich oder verschieden sein können;
R² für Wasserstoff steht;
R³ unter Fluor, Chlor, Methyl oder Methoxy ausgewählt ist;
R⁴ unter Fluor, Chlor, Brom, Cyano, Hydroxy, Amino, Carbamoyl, Ureido, Methyl, Ethyl, Methoxy, Methylamino, Isopropylamino, Morpholino, 2-(Dimethylamino)ethylamino, 2-(Hydroxy)ethylamino, 2-(Amino)ethylamino, 3-(Pyrrolidin-1-yl)propylamino, *N-*Methylcarbamoyl, Acetylamino, 2-Hydroxyacetylamino, Trifluormethyl, Mesylamino, 2,2-Dimethylpropanoylamino, 3-Methoxypropanoylamino, Cyclobutylcarbonylamino, Cyclopropylamino, 2,3-Dihydroxypropylamino, 1,3-Dihydroxyprop-2-ylamino, 1-Methylpiperazin-4-yl, 1-Methylpiperazin-4-yl-methyl, Acetyl, *N-*Methyl-*N-*(3-dimethylaminopropyl)amino, *N-*Methyl-*N-*(2-methoxyethyl)amino, Dimethylamino, Hydroxymethyl, 1,2-Dihydroxyethyl, Pyrazol-5-ylamino, 3-Aminoprop-1-in-1-yl, 3-Hydroxyprop-1-in-1-yl, 3-Methylaminoprop-1-in-1-yl, 3-Dimethylaminoprop-1-in-1-yl, 4-Aminobutylamino, Pyrrolidin-2-ylamino, 3-Methylaminopropyl, 3-Dimethylaminopropyl, 3-Hydroxypropyl, 3-Dimethylaminopropylamino, Aminomethyl, Piperazin-1-yl, 1-Methylpiperazin-4-yl, 2,2-Dimethyl-1,3-dioxolan-4-ylmethylamino, Pyrrolidin-3-ylmethylamino, Piperidin-4-ylmethylamino, Imidazol-2-yl-methylamino, Methoxymethyl, *N,N*-Dimethylsulfamoyl, Formylamino, Morpholinomethyl, Aminomethyl, 2-(Dimethylamino)ethylamino, Pyrrol-1-yl, Pyrrol-2-yl, Pyrrolidin-2-yl, Imidazol-4-yl, Cyclobutylamino, *N-*Methyl-*N-*(2-dimethylaminoethyl)amino, 2-Dimethylaminoethoxy, Dimethylaminomethyl, Cyclopropylaminomethyl, Piperidin-1-ylmethyl, Methylaminomethyl, Pyrrolidin-2-ylmethoxy, 3-Dimethylaminopropoxy, Methoxycarbonyl, 1-(*t-*Butoxycarbonyl)pyrrolidin-2-ylmethylamino, 1-(*t-*Butoxycarbonyl)pyrrolidin-2-ylmethoxy, 2-Phenoxyacetylamino und 1-(*t-*Butoxycarbonyl)pyrrolidin-2-yl ausgewählt ist;
m unter 0-2 ausgewählt ist; wobei die Werte von R⁴ gleich oder verschieden sein können;
oder ein pharmazeutisch annehmbares Salz davon;
mit der Maßgabe, daß es sich bei der Verbindung nicht um:
*N-*[4-Chlor-3-({[6-(4-methylpiperazin-1-yl)pyridin-3-yl]amino}carbonyl)phenyl]-2-morpholin-4-ylisonicotinamid;
*N-*{4-Chlor-3-[({6-[[3-(dimethylamino)propyl]-(methyl)amino]pyridin-3-yl}amino)carbonyl]phenyl}-2-morpholin-4-ylisonicotinamid oder
*N-*{4-Chlor-3-[({6-[[2-(dimethylamino)ethyl]-(methyl)amino]pyridin-3-yl}amino)carbonyl]phenyl}-2-morpholin-4-ylisonicotinamid
handelt.

10. Verbindung der Formel **(I):** 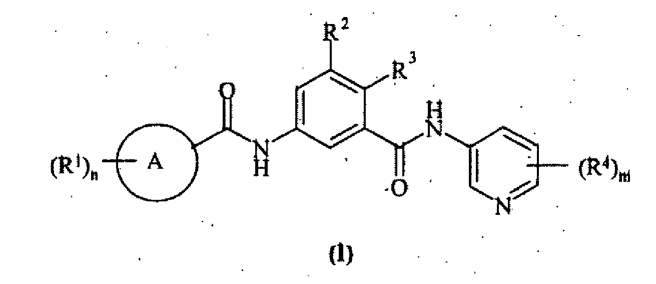 ausgewählt unter:
*N-*(6-Amino-5-chlorpyridin-3-yl)-5-{[3-(1-cyano-1-methylethyl)benzoyl]amino}-2-methylbenzamid;
*N-*(6-Amino-5-chlorpyridin-3-yl)-5-{[3-(1-cyano-1-methylethyl)-5-fluorbenzoyl]amino}-2-methylbenzamid;
5-{[3-(1-Cyano-1-methylethyl)benzoyl]amino}-*N-*(5-methoxypyridin-3-yl)-2-methylbenzamid;
*N-*(6-Amino-5-chlorpyridin-3-yl)-2-methyl-5-{[3-(trifluormethyl)benzoyl]amino}benzamid;
5-{[3-(1-Cyano-1-methylethyl)benzoyl]amino}-*N-*(5,6-dimethylpyridin-3-yl)-2-methylbenzamid;
*N-*(3-{[(6-Amino-5-chlorpyridin-3-yl)amino]-carbonyl}-4-methylphenyl)-2-(1-cyano-1-methylethyl)isonicotinamid;
*N-*(6-Amino-5-chlorpyridin-3-yl)-2-chlor-5-{[3-(trifluormethyl)benzoyl]amino}benzamid;
*N-*(6-Acetylaminopyridin-3-yl)-5-[3-(cyanodimethylmethyl)benzoylamino]-2-methylbenzamid;
*N-*[6-(Acetylamino)pyridin-3-yl]-2-chlor-5-{[3-(trifluormethyl)benzoyl]amino}benzamid und
*N-*(6-Amino-5-methylpyridin-3-yl)-2-chlor-5-{[3-(1-cyano-1-methylethyl)benzoyl]amino}benzamid;
oder ein pharmazeutisch annehmbares Salz davon.

11. Verfahren zur Herstellung einer Verbindung der Formel **(I)** oder eines pharmazeutisch annehmbaren Salzes davon, wobei variable Gruppen wie in Anspruch 1 definiert sind, sofern nicht anders angegeben, bei dem man:
*Verfahren a)* ein Amin der Formel **(II)** 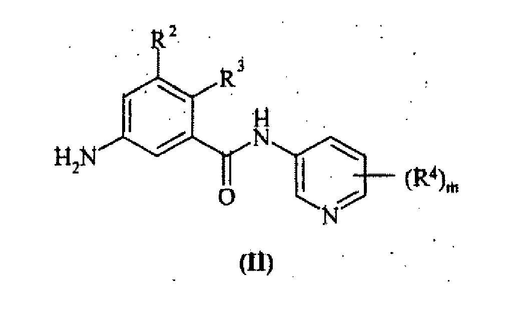 mit einer Säure der Formel **(III):** 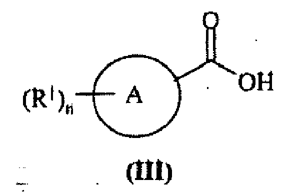 oder einem aktivierten Säurederivat davon umsetzt;
*Verfahren b)* eine Säure der Formel **(IV):** 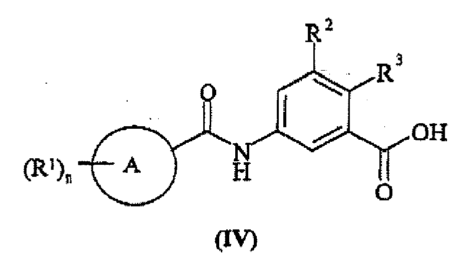 mit einem Amin der Formel **(V):** 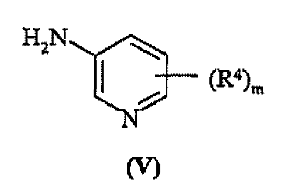
oder einem aktivierten Säurederivat davon umsetzt;
und danach gegebenenfalls:
i) eine Verbindung der Formel **(I)** in eine andere Verbindung der Formel **(I)** umwandelt;
ii) gegebenenfalls vorhandene Schutzgruppen abspaltet;
iii) ein pharmazeutisch annehmbares Salz bildet.

12. Pharmazeutische Zusammensetzung, die eine Verbindung der Formel **(I)** oder ein pharmazeutisch annehmbares Salz davon nach einem der Ansprüche 1-10 zusammen mit einem pharmazeutisch annehmbaren Verdünnungsmittel oder Träger enthält.

13. Verbindung der Formel **(I)** oder ein pharmazeutisch annehmbares Salz davon nach einem der Ansprüche 1-10 zur Verwendung als Arzneimittel.

14. Verwendung einer Verbindung der Formel **(I)** oder eines pharmazeutisch annehmbaren Salzes davon nach einem der Ansprüche 1-10 bei der Herstellung eines Arzneimittels zur Hervorrufung einer B-Rafinhibitorischen Wirkung bei einem Warmblüter wie dem Menschen.

15. Verwendung einer Verbindung der Formel **(I)** oder eines pharmazeutisch annehmbaren Salzes davon nach einem der Ansprüche 1-10 bei der Herstellung eines Arzneimittels zur Hervorrufung einer Antikrebswirkung bei einem Warmblüter wie dem Menschen.

16. Verwendung einer Verbindung der Formel **(I)** oder eines pharmazeutisch annehmbaren Salzes davon nach einem der Ansprüche 1-10 bei der Herstellung eines Arzneimittels zur Behandlung von Melanom, papillären Schilddrüsentumoren, Gallengangskarzinomen, Kolonkrebs, Eierstockkrebs, Lungenkrebs, Leukämien, lymphoiden Malignomen, Karzinomen und Sarkomen in der Leber, den Nieren, der Blase, der Prostata, der Brust und der Bauchspeicheldrüse und primären und rezidivierenden soliden Tumoren der Haut, des Kolons, der Schilddrüse, der Lunge und der Eierstöcke.

## Revendications

1. Composé de formule **(I) :** 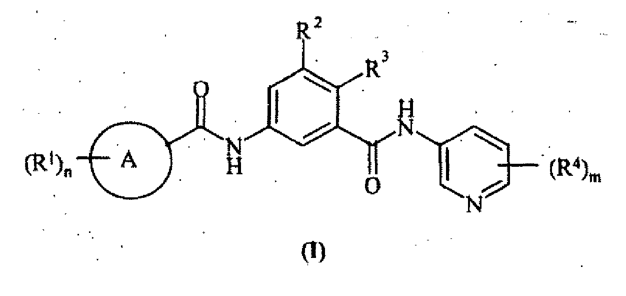 dans laquelle :
**Le noyau A** est un groupe phényle ou un noyau hétérocyclique monocyclique totalement insaturé à 5 ou 6 chaînons ; où ledit groupe phényle ou noyau hétérocyclique peut être éventuellement condensé sur un groupe carbocyclyle ou un groupe hétérocyclyle à cinq ou six chaînons, formant un noyau bicyclique ; et où si ledit groupe hétérocyclyle ou noyau hétérocyclique renferme un fragment -NH-, cet atome d'azote peut être éventuellement substitué par un groupe choisi parmi R⁵ ;
**R¹** est un substituant sur un atome de carbone et est choisi parmi un groupe halogéno, un groupe nitro, un groupe cyano, un groupe hydroxy, un groupe trifluorométhoxy, un groupe amino, un groupe carboxy, un groupe carbamoyle, un groupe mercapto, un groupe sulfamoyle, un groupe alkyle en C₁₋₆, un groupe alcényle en C₂₋₆, un groupe alcynyle en C₂₋₆, un groupe alcoxy en C₁₋₆, un groupe alcanoyle en C₁₋₆, un groupe alcanoyloxy en C₁₋₆, un groupe *N-*(C₁₋₆-alkyl)amino, un groupe *N,N-*(C₁₋₆-alkyl)₂amino, un groupe C₁₋₆-alcanoylamino, un groupe *N-*(C₁₋₆-alkyl) carbamoyle, un groupe *N,N-*(C₁₋₆-alkyl) ₂carbamoyle, un groupe C₁₋₆-alkylS (O) ₐ dans lequel a vaut de 0 à 2, un groupe C₁₋₆-alcoxycarbonyle, un groupe C₁₋₆-alcoxycarbonylamino, un groupe *N*-(C₁₋₆-alkyl) sulfamoyle, un groupe *N,N*- (C₁₋₆-alkyl) ₂sulfamoyle, un groupe *N*-(C₁₋₆-alkyl) -*N-*(C₁₋₆-alcoxy) sulfamoyle, un groupe *N,N*'-(C₁₋₆-alkyl) ₂uréido, un groupe *N',N'*-(C₁₋₆-alkyl)₂uréido, un groupe *N-*(C₁₋₆-alkyl)-*N',N'*-(C₁₋₆-alkyl)₂uréido, un groupe C₁₋₆-alkylsulfonylamino, un groupe carbocyclyl-R⁶- ou un groupe hétérocyclyl-R⁷- ; où R¹ peut être éventuellement substitué sur un atome de carbone par un ou plusieurs R⁸ ; et où si ledit groupe hétérocyclyle renferme un fragment -NH-, cet atome d'azote peut être éventuellement substitué par un groupe choisi parmi R⁹ ;
**n** est choisi parmi de 0 à 4 ; où les valeurs de R¹ peuvent être identiques ou différentes ;
**R²** est choisi parmi un atome d'hydrogène, parmi un groupe halogéno, un groupe nitro, un groupe cyano, un groupe hydroxy, un groupe trifluorométhoxy, un groupe amino, un groupe carboxy, un groupe carbamoyle, un groupe mercapto, un groupe sulfamoyle, un groupe alkyle en C₁₋₆, un groupe alcényle en C₂₋₆, un groupe alcynyle en C₂₋₆, un groupe alcoxy en C₁₋₆, un groupe alcanoyle en C₁₋₆, un groupe alcanoyloxy en C₁₋₆, un groupe *N-*(C₁₋₆-alkyl)amino, un groupe *N,N*-(C₁₋₆-alkyl)₂amino, un groupe C₁₋₆-alcanoylamino, un groupe *N-*(C₁₋₆-alkyl)carbamoyle, un groupe *N,N*-(C₁₋₆-alkyl)₂carbamoyle, un groupe C₁₋₆-alkylS(O)ₐ dans lequel a vaut de 0 à 2, un groupe C₁₋₆-alcoxycarbonyle, un groupe *N-*(C₁₋₆-alkyl)sulfamoyle, un groupe *N,N*- (C₁₋₆-alkyl)₂sulfamoyle, un groupe C₁₋₆-alkylsulfonylamino, un groupe carbocyclyl-R¹⁰- ou un groupe hétérocyclyl-R¹¹- ; où R² peut être éventuellement substitué sur un atome de carbone par un ou plusieurs R¹² ; et où si ledit groupe hétérocyclyle renferme un fragment -NH-, cet atome d'azote peut être éventuellement substitué par un groupe choisi parmi R¹³ ;
**R³** est choisi parmi un groupe halogéno, un groupe hydroxy, un groupe cyano, un groupe méthyle, un groupe méthoxy ou un groupe hydroxyméthyle ;
**R⁴** est choisi parmi un groupe halogéno, un groupe nitro, un groupe cyano, un groupe hydroxy, un groupe trifluorométhoxy, un groupe amino, un groupe carboxy, un groupe carbamoyle, un groupe mercapto, un groupe sulfamoyle, un groupe uréido, un groupe alkyle en C₁₋₆, un groupe alcényle en C₂₋₆, un groupe alcynyle en C₂₋₆, un groupe alcoxy en C₁₋₆, un groupe alcanoyle en C₁₋₆, un groupe alcanoyloxy en C₁₋₆, un groupe *N-*(C₁₋₆-alkyl)amino, un groupe *N,N*-(C₁₋₆-alkyl)₂amino, un groupe C₁₋₆-alcanoylamino, un groupe *N-*(C₁₋₆-alkyl)carbamoyle, un groupe *N,N*-(C₁₋₆-alkyl)₂carbamoyle, un groupe C₁₋₆-alkylS(O)ₐ dans lequel a vaut de 0 à 2, un groupe C₁₋₆-alcoxycarbonyle, un groupe *N-*(C₁₋₆-alkyl)sulfamoyle, un groupe *N,N*-(C₁₋₆-alkyl)₂sulfamoyle, un groupe C₁₋₆-alkylsulfonylamino, un groupe carbocyclyl-R¹⁴- ou un groupe hétérocyclyl-R¹⁵-; où R⁴ peut être éventuellement substitué sur un atome de carbone par un ou plusieurs R¹⁶ ; et où si ledit groupe hétérocyclyle renferme un fragment -NH-, cet atome d'azote peut être éventuellement substitué par un groupe choisi parmi R¹⁷ ;
**m** est choisi parmi de 0 à 4 ; où les valeurs de R⁴ peuvent être identiques ou différentes ;
**R⁸** et **R¹²** sont choisis indépendamment parmi un groupe halogéno, un groupe nitro, un groupe cyano, un groupe hydroxy, un groupe trifluorométhoxy, un groupe amino, un groupe carboxy, un groupe carbamoyle, un groupe mercapto, un groupe sulfamoyle, un groupe alkyle en C₁₋₆, un groupe alcényle en C₂₋₆, un groupe alcynyle en C₂₋₆, un groupe alcoxy en C₁₋₆, un groupe alcanoyle en C₁₋₆, un groupe alcanoyloxy en C₁₋₆, un groupe *N*-(C₁₋₆-alkyl)amino, un groupe *N,N*-(C₁₋₆-alkyl)₂amino, un groupe *N-*(C₁₋₆-alkyl)-*N-*(C₁₋₆-alcoxy)amino, un groupe C₁₋₆-alcanoylamino, un groupe *N-*(C₁₋₆-alkyl)carbamoyle, un groupe *N,N*-(C₁₋₆-alkyl)₂carbamoyle, un groupe C₁₋₆-alkylS(O)ₐ dans lequel a vaut de 0 à 2, un groupe C₁₋₆-alcoxycarbonyle, un groupe *N-*(C₁₋₆-alkyl)sulfamoyle, un groupe *N,N*-(C₁₋₆-alkyl)₂sulfamoyle, un groupe C₁₋₆-alkylsulfonylamino, un groupe carbocyclyl-R¹⁸- ou un groupe hétérocyclyl-R¹⁹- ; où R⁸ et R¹², indépendamment l'un de l'autre, peuvent être éventuellement substitués sur un atome de carbone par un ou plusieurs R²⁰ ; et où si ledit groupe hétérocyclyle renferme un fragment -NH-, cet atome d'azote peut être éventuellement substitué par un groupe choisi parmi R²¹ ;
**R¹⁶** est choisi parmi un groupe halogéno, un groupe nitro, un groupe cyano, un groupe hydroxy, un groupe trifluorométhoxy, un groupe amino, un groupe carboxy, un groupe carbamoyle, un groupe mercapto, un groupe sulfamoyle, un groupe alkyle en C₁₋₆, un groupe alcényle en C₂₋₆, un groupe alcynyle en C₂₋₆, un groupe alcoxy en C₁₋₆, un groupe alcanoyle en C₁₋₆, un groupe alcanoyloxy en C₁₋₆, un groupe *N-*(C₁₋₆-alkyl)amino, un groupe *N,N-*(C₁₋₆-alkyl)₂amino, un groupe C₁₋₆-alcanoylamino, un groupe *N-*(C₁₋₆-alkyl) carbamoyle, un groupe *N,N-*(C₁₋₆-alkyl) ₂carbamoyle, un groupe C₁₋₆-alkylS (O) ₐ dans lequel a vaut de 0 à 2, un groupe C₁₋₆-alcoxycarbonyle, un groupe *N-*(C₁₋₆-alkyl)sulfamoyle, un groupe *N,N*-(C₁₋₆-alkyl)₂sulfamoyle, un groupe C₁₋₆-alkylsulfonylamino, un groupe carbocyclyl-R²²- ou un groupe hétérocyclyl-R²³- ; où R¹⁶ peut être éventuellement substitué sur un atome de carbone par un ou plusieurs R²⁴ ; et où si ledit groupe hétérocyclyle renferme un fragment -NH-, cet atome d'azote peut être éventuellement substitué par un groupe choisi parmi R²⁵ ;
**R⁶, R⁷, R¹⁰, R¹¹, R¹⁴, R¹⁵, R¹⁸, R¹⁹, R²²** et **R²³** sont choisis indépendamment parmi une liaison directe, un groupe -O-, un groupe -N(R²⁶) -, un groupe -C(O) -, un groupe -N(R²⁷)C(O)-, un groupe -C(O)N(R²⁸)-, un groupe -S(O)ₛ-, un groupe -SO₂N(R²⁹)- ou un groupe -N(R³⁰)SO₂- ; dans lesquels **R²⁶, R²⁷, R²⁸, R²⁹** et **R³⁰** sont choisis indépendamment parmi un atome d'hydrogène ou un groupe alkyle en C₁₋₆ et s vaut de 0 à 2 ;
**R⁵, R⁹, R¹³, R¹⁷, R²¹** et **R²⁵** sont choisis indépendamment parmi un groupe alkyle en C₁₋₆, un groupe alcanoyle en C₁₋₆, un groupe alkylsulfonyle en C₁₋₆, un groupe alcoxycarbonyle en C₁₋₆, un groupe carbamoyle, un groupe *N-*(C₁₋₆-alkyl)carbamoyle, un groupe *N,N-*(C₁₋₆-alkyl)₂carbamoyle, un groupe benzyle, un groupe benzyloxycarbonyle, un groupe benzoyle et un groupe phénylsulfonyle ;
**R²⁰** et **R²⁴** sont choisis indépendamment parmi un groupe halogéno, un groupe nitro, un groupe cyano, un groupe hydroxy, un groupe trifluorométhoxy, un groupe trifluorométhyle, un groupe amino, un groupe carboxy, un groupe carbamoyle, un groupe mercapto, un groupe sulfamoyle, un groupe méthyle, un groupe éthyle, un groupe méthoxy, un groupe éthoxy, un groupe acétyle, un groupe acétoxy, un groupe méthylamino, un groupe éthylamino, un groupe diméthylamino, un groupe diéthylamino, un groupe *N-*méthyl-*N-*éthylamino, un groupe acétylamino, un groupe *N-*méthylcarbamoyle, un groupe *N-*éthylcarbamoyle, un groupe *N,N-*diméthylcarbamoyle, un groupe *N,N-*diéthylcarbamoyle, un groupe *N*-méthyl-*N-*éthylcarbamoyle, un groupe phényle, un groupe méthylthio, un groupe éthylthio, un groupe méthylsulfinyle, un groupe éthylsulfinyle, un groupe mésyle, un groupe éthylsulfonyle, un groupe méthoxycarbonyle, un groupe éthoxycarbonyle, un groupe *N-*méthylsulfamoyle, un groupe *N-*éthylsulfamoyle, un groupe *N,N*-diméthylsulfamoyle, un groupe *N,N*-diéthylsulfamoyle ou un groupe *N-*méthyl-*N-*éthylsulfamoyle ;
ou un sel pharmaceutiquement acceptable de celui-ci ;
à condition que ledit composé ne soit pas :
le *N-*[4-chloro-3-({[6-(4-méthylpipérazin-1-yl)pyridin-3-yl]amino}carbonyl)phényl]-2-morpholin-4-ylisonicotinamide ;
le *N-*[4-chloro-3-({[6-(4-éthylpipérazin-1-yl)pyridin-3-yl]amino}carbonyl)phényl]-2-morpholin-4-ylisonicotinamide ;
le *N-*{4-chloro-3-[({6-[[3-(diméthylamino)propyl](méthyl)amino]pyridin-3-yl}amino)carbonyl]phényl}-2-morpholin-4-ylisonicotinamide ;
le *N-*{4-chloro-3-[({6-[[2-(diméthylamino)éthyl](méthyl)amino]pyridin-3-yl}amino)carbonyl]phényl}-2-morpholin-4-ylisonicotinamide ; ou
le *N-*[4-chloro-3-({[6-(4-méthyl-1,4-diazépan-1-yl)pyridin-3-yl]amino}carbonyl)phényl]-2-morpholin-4-ylisonicotinamide.

2. Composé de formule **(I),** ou un sel pharmaceutiquement acceptable de celui-ci, selon la revendication 1, dans laquelle le noyau A est un groupe phényle, un groupe pyridyle, un groupe pyrazolyle, un groupe thiényle, un groupe indolyle, un groupe 2,3-dihydrobenzofuranyle, un groupe imidazo[1,2-a]pyridinyle, un groupe isoxazolyle, un groupe benzimidazolyle, un groupe 2-oxoindolinyle, un groupe furanyle, un groupe 1,3-thiazolyle, un groupe pyrimidinyle et un groupe pyrrolyle ; où ledit groupe pyrazolyle, ledit groupe indolyle et ledit groupe pyrrolyle peuvent être éventuellement substitués sur un atome d'azote par un groupe choisi parmi R⁵ ; où R⁵ est choisi parmi un groupe alkyle en C₁₋₆.

3. Composé de formule **(I),** ou un sel pharmaceutiquement acceptable de celui-ci, selon la revendication 1 ou la revendication 2, dans laquelle R¹ est un substituant sur un atome de carbone et est choisi parmi un groupe halogéno, un groupe cyano, un groupe hydroxy, un groupe sulfamoyle, un groupe alkyle en C₁₋₆, un groupe alcynyle en C₂₋₆, un groupe alcoxy en C₁₋₆, un groupe *N,N-*(C₁₋₆-alkyl) ₂amino, un groupe C₁₋₆-alkylS(O)ₐ dans lequel a vaut de 0 à 2, un groupe C₁₋₆-alcoxycarbonylamino, un groupe *N-*(C₁₋₆-alkyl)sulfamoyle, un groupe *N,N*-(C₁₋₆-alkyl)₂sulfamoyle, un groupe *N-*(C₁₋₆-alkyl)-*N-*(C₁₋₆-alcoxy) sulfamoyle, un groupe *N',N'*-(C₁₋₆-alkyl)₂uréido, un groupe C₁₋₆-alkylsulfonylamino, un groupe carbocyclyl-R⁶- ou un groupe hétérocyclyl-R⁷- ; où R¹ peut être éventuellement substitué sur un atome de carbone par un ou plusieurs R⁸ ; où
R⁸ est choisi parmi un groupe halogéno, un groupe cyano, un groupe hydroxy, un groupe alkyle en C₁₋₆, un groupe alcoxy en C₁₋₆, un groupe *N,N*-(C₁₋₆-alkyl)₂amino, un groupe *N,N*-(C₁₋₆-alkyl)₂carbamoyle, un groupe C₁₋₆-alkylS(O)ₐ dans lequel a vaut de 0 à 2, un groupe *N,N*-(C₁₋₆-alkyl)₂sulfamoyle, un groupe *N-*(C₁₋₆-alkyl)-*N-*(C₁₋₆-alcoxy) amino, un groupe carbocyclyl-R¹⁸- ou un groupe hétérocyclyl-R¹⁹-; où R⁸ peut être éventuellement substitué sur un atome de carbone par un ou plusieurs R²⁰;
et où si ledit groupe hétérocyclyle renferme un fragment -NH-, cet atome d'azote peut être éventuellement substitué par un groupe choisi parmi R²¹ ;
R⁶, R⁷, R¹⁸ et R¹⁹ sont choisis indépendamment parmi une liaison directe, un groupe -O-, un groupe -S(O)ₛ- ou un groupe -N(R³⁰)SO₂- ; dans lesquels R³⁰ est choisi parmi un atome d'hydrogène et s vaut 2 ;
R²¹ est choisi parmi un groupe alkyle en C₁₋₆ ; et
R²⁰ est choisi parmi un groupe cyano ou un groupe hydroxy.

4. Composé de formule **(I),** ou un sel pharmaceutiquement acceptable de celui-ci, selon l'une quelconque des revendications 1 à 3, dans laquelle n est choisi parmi de 0 à 2 ; où les valeurs de R¹ peuvent être identiques ou différentes.

5. Composé de formule **(I),** ou un sel pharmaceutiquement acceptable de celui-ci, selon l'une quelconque des revendications 1 à 4, dans laquelle R² est un atome d'hydrogène.

6. Composé de formule **(I),** ou un sel pharmaceutiquement acceptable de celui-ci, selon l'une quelconque des revendications 1 à 5, dans laquelle R³ est choisi parmi un groupe halogéno, un groupe méthyle ou un groupe méthoxy.

7. Composé de formule **(I),** ou un sel pharmaceutiquement acceptable de celui-ci, selon l'une quelconque des revendications 1 à 6, dans laquelle R⁴ est choisi parmi un groupe halogéno, un groupe cyano, un groupe hydroxy, un groupe amino, un groupe carbamoyle, un groupe uréido, un groupe alkyle en C₁₋₆, un groupe alcynyle en C₂₋₆, un groupe alcoxy en C₁₋₆, un groupe alcanoyle en C₁₋₆, un groupe *N-*(C₁₋₆-alkyl)amino, un groupe *N,N*-(C₁₋₆-alkyl)₂amino, un groupe C₁₋₆-alcanoylamino, un groupe *N-*(C₁₋₆-alkyl)carbamoyle, un groupe C₁₋₆-alcoxycarbonyle, un groupe *N,N*-(C₁₋₆-alkyl)₂sulfamoyle, un groupe C₁₋₆-alkylsulfonylamino, un groupe carbocyclyl-R¹⁴- ou un groupe hétérocyclyl-R¹⁵- ; où R⁴ peut être éventuellement substitué sur un atome de carbone par un ou plusieurs R¹⁶ ; et où si ledit groupe hétérocyclyle renferme un fragment -NH-, cet atome d'azote peut être éventuellement substitué par un groupe choisi parmi R¹⁷ ; où
R¹⁶ est choisi parmi un groupe halogéno, un groupe hydroxy, un groupe amino, un groupe alcoxy en C₁₋₆, un groupe *N-*(C₁₋₆-alkyl)amino, un groupe *N,N*-(C₁₋₆-alkyl)₂amino, un groupe carbocyclyl-R²²- ou un groupe hétérocyclyl-R²³- ; où R¹⁶ peut être éventuellement substitué sur un atome de carbone par un ou plusieurs R²⁴ ; et où si ledit groupe hétérocyclyle renferme un fragment -NH-, cet atome d'azote peut être éventuellement substitué par un groupe choisi parmi R²⁵ ;
R¹⁴, R¹⁵, R²² et R²³ sont choisis indépendamment parmi une liaison directe, un groupe -N(R²⁶)- ou un groupe -C(O)N(R²⁸)-; dans lesquels R²⁶ et R²⁸ sont un atome d'hydrogène ;
R¹⁷ et R²⁵ sont choisis indépendamment parmi un groupe alkyle en C₁₋₆ et un groupe C₁₋₆-alcoxycarbonyle ; et
R²⁴ est un groupe méthyle ou un groupe phényle.

8. Composé de formule **(I),** ou un sel pharmaceutiquement acceptable de celui-ci, selon l'une quelconque des revendications 1 à 7, dans laquelle m est choisi parmi de 0 à 2 ; où les valeurs de R⁴ peuvent être identiques ou différentes.

9. Composé de formule **(I) :** 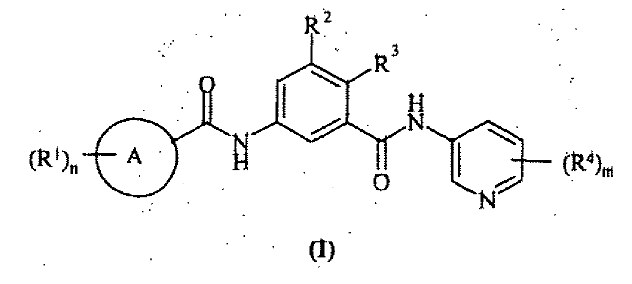 dans laquelle :
Le noyau A est un groupe phényle, un groupe pyrid-2-yle, un groupe pyrid-3-yle, un groupe pyrid-4-yle, un groupe 1-méthylpyrazol-5-yle, un groupe 1-*t-*butylpyrazol-5-yle, un groupe thién-2-yle, un groupe thién-3-yle, un groupe indol-2-yle, un groupe 1-méthylindol-2-yle, un groupe indol-4-yle, un groupe indol-5-yle, un groupe indol-6-yle, un groupe indol-7-yle, un groupe 2,3-dihydrobenzofuran-7-yle, un groupe imidazo[1,2-a]pyridin-2-yle, un groupe isoxazol-3-yle, un groupe pyrrol-2-yle, un groupe benzimidazol-6-yle, un groupe 1-méthyl-2-oxoindolin-5-yle, un groupe furan-2-yle, un groupe 1,3-thiazol-5-yle, un groupe pyrimidin-4-yle ou un groupe 1-méthylpyrrol-2-yle ;
R¹ est un substituant sur un atome de carbone et est choisi parmi un groupe fluoro, un groupe chloro, un groupe bromo, un groupe iodo, un groupe cyano, un groupe hydroxy, un groupe sulfamoyle, un groupe méthyle, un groupe trifluorométhyle, un groupe 1-cyano-1-méthyléthyle, un groupe méthoxy, un groupe éthoxy, un groupe propoxy, un groupe isopropoxy, un groupe isobutoxy, un groupe *N,N-*diméthylamino, un groupe difluorométhylthio, un groupe *N,N*-diméthylsulfamoyle, un groupe *t-*butyle, un groupe mésyle, un groupe cyclopropylaminosulfonyle, un groupe azétidin-1-ylsulfonyle, un groupe tétrahydrofuran-2-ylméthylaminosulfonyle, un groupe *N-*méthyl-*N-*(2,3-dihydroxypropyl)sulfamoyle, un groupe mésylamino, un groupe morpholinosulfonyle, un groupe 1-méthylpipérazin-4-ylméthyle, un groupe 1-éthylpipérazin-4-ylméthyle, un groupe 3,3-diméthylbut-1-yn-1-yle, un groupe morpholino, un groupe *N,N*-diméthylaminométhyle, un groupe 3-méthyl-3-hydroxybut-1-yn-1-yle, un groupe méthylthiométhyle, un groupe mésylméthyle, un groupe *N-*(méthyl)-*N-*(méthoxy)sulfamoyle, un groupe 2-hydroxyméthylpipéridin-1-ylsulfonyle, un groupe 3-hydroxyméthylpipéridin-1-ylsulfonyle, un groupe 4-hydroxyméthylpipéridin-1-ylsulfonyle, un groupe 1,1-difluoroéthyle, un groupe pipéridin-1-yle, un groupe *N,N*-diéthylamino, un groupe *N',N'-*diméthyluréido, un groupe cyclopropyle, un groupe *t-*butoxycarbonylamino, un groupe pyrid-2-yle, un groupe phénoxy, un groupe 2-méthoxy-1,1-diméthyléthyle, un groupe mésylméthyle, un groupe 1,3-thiazol-2-yle, un groupe 2-méthyl-1,3-thiazol-5-yle, un groupe 1-méthylcyclopropyle, un groupe 1,1-diméthylprop-2-yn-1-yle, un groupe 1-(*N,N-*diméthylsulfamoyl)-1-méthyléthyle, un groupe 1,1-diméthylbut-2-yn-1-yle, un groupe *N-*(méthyl)-*N-*(méthoxy)aminométhyle, un groupe 1-(*N,N-*diméthylcarbamoyl)-1-méthyléthyle, un groupe 4-méthylimidazol-1-yle, un groupe 1-(cyclopropyl)-1-méthyléthyle, un groupe 2-méthyl-3,4-dihydroxybut-2-yle, un groupe 2-méthylbut-2-yle, un groupe 1-hydroxy-1-cyclopropyléthyle, un groupe 1-cyanoéthyle, un groupe 2-cyano-3-méthylbut-2-yle, 2-cyanobut-2-yle, un groupe 1-hydroxy-2-cyanoprop-2-yle et un groupe 2-cyanopyrrol-1-ylméthyle ;
n est choisi parmi de 0 à 2 ; où les valeurs de R¹ peuvent être identiques ou différentes ;
R² est un atome d'hydrogène ;
R³ est choisi parmi un groupe fluoro, un groupe chloro, un groupe méthyle ou un groupe méthoxy ;
R⁴ est choisi parmi un groupe fluoro, un groupe chloro, un groupe bromo, un groupe cyano, un groupe hydroxy, un groupe amino, un groupe carbamoyle, un groupe uréido, un groupe méthyle, un groupe éthyle, un groupe méthoxy, un groupe méthylamino, un groupe isopropylamino, un groupe morpholino, un groupe 2-(diméthylamino)éthylamino, un groupe 2-(hydroxy)éthylamino, un groupe 2-(amino)éthylamino, un groupe 3-(pyrrolidin-1-yl)propylamino, un groupe *N-*méthylcarbamoyle, acétylamino, un groupe 2-hydroxyacétylamino, un groupe trifluorométhyle, un groupe mésylamino, un groupe 2,2-diméthylpropanoylamino, un groupe 3-méthoxypropanoylamino, un groupe cyclobutylcarbonylamino, un groupe cyclopropylamino, un groupe 2,3-dihydroxypropylamino, un groupe 1,3-dihydroxyprop-2-ylamino, un groupe 1-méthylpipérazin-4-yle, un groupe 1-méthylpipérazin-4-ylméthyle, un groupe acétyle, un groupe *N-*méthyl-*N-*(3-diméthylaminopropyl)amino, un groupe *N-*méthyl-*N-*(2-méthoxyéthyl)amino, un groupe diméthylamino, un groupe hydroxyméthyle, un groupe 1,2-dihydroxyéthyle, un groupe pyrazol-5-ylamino, un groupe 3-aminoprop-1-yn-1-yle, un groupe 3-hydroxyprop-1-yn-1-yle, un groupe 3-méthylaminoprop-1-yn-1-yle, un groupe 3-diméthylaminoprop-1-yn-1-yle, un groupe 4-aminobutylamino, un groupe pyrrolidin-2-ylamino, un groupe 3-méthylaminopropyle, un groupe 3-diméthylaminopropyle, un groupe 3-hydroxypropyle, un groupe 3-diméthylaminopropylamino, un groupe aminométhyle, un groupe pipérazin-1-yle, un groupe 1-méthylpipérazin-4-yle, un groupe 2,2-diméthyl-1,3-dioxolan-4-ylméthylamino, un groupe pyrrolidin-3-ylméthylamino, un groupe pipéridin-4-ylméthylamino, un groupe imidazol-2-ylméthylamino, un groupe méthoxyméthyle, un groupe *N,N-*diméthylsulfamoyle, un groupe formylamino, un groupe morpholinométhyle, un groupe aminométhyle, un groupe 2-(diméthylamino)éthylamino, un groupe pyrrol-1-yle, un groupe pyrrol-2-yle, un groupe pyrrolidin-2-yle, un groupe imidazol-4-yle, un groupe cyclobutylamino, un groupe *N-*méthyl-*N-*(2-diméthylaminoéthyl)amino, un groupe 2-diméthylaminoéthoxy, un groupe diméthylaminométhyle, un groupe cyclopropylaminométhyle, un groupe pipéridin-1-ylméthyle, un groupe méthylaminométhyle, un groupe pyrrolidin-2-ylméthoxy, un groupe 3-diméthylaminopropoxy, un groupe méthoxycarbonyle, un groupe 1-(*t-*butoxycarbonyl)pyrrolidin-2-ylméthylamino, un groupe 1-(*t-*butoxycarbonyl)pyrrolidin-2-ylméthoxy, un groupe 2-phénoxyacétylamino et un groupe 1-(*t-*butoxycarbonyl)pyrrolidin-2-yle ;
m est choisi parmi de 0 à 2 ; où les valeurs de R⁴ peuvent être identiques ou différentes ;
ou un sel pharmaceutiquement acceptable de celui-ci ;
à condition que ledit composé ne soit pas :
le *N-*[4-chloro-3-({[6-(4-méthylpipérazin-1-yl)pyridin-3-yl]amino}carbonyl)phényl]-2-morpholin-4-ylisonicotinamide ;
le *N-*{4-chloro-3-[({6-[[3-(diméthylamino)propyl](méthyl)amino]pyridin-3-yl}amino)carbonyl]phényl}-2-morpholin-4-ylisonicotinamide ; ou
le *N-*{4-chloro-3-[({6-[[2-(diméthylamino)éthyl](méthyl)amino]pyridin-3-yl}amino)carbonyl]phényl}-2-morpholin-4-ylisonicotinamide.

10. Composé de formule **(I) :** 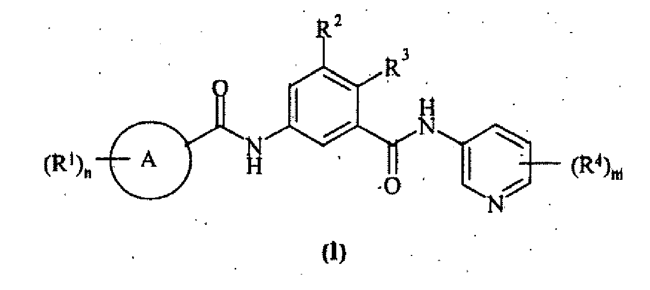 choisi parmi :
le *N-*(6-amino-5-chloropyridin-3-yl)-5-{[3-(1-cyano-1-méthyléthyl)benzoyl]amino}-2-méthylbenzamide ;
le *N-*(6-amino-5-chloropyridin-3-yl)-5-{[3-(1-cyano-1-méthyléthyl)-5-fluorobenzoyl]amino}-2-méthylbenzamide ;
le 5-{[3-(1-cyano-1-méthyléthyl)benzoyl]amino}-*N-*(5-méthoxypyridin-3-yl)-2-méthylbenzamide ;
le *N-*(6-amino-5-chloropyridin-3-yl)-2-méthyl-5-{[3-(trifluorométhyl)benzoyl]amino}benzamide ;
le 5-{[3-(1-cyano-1-méthyléthyl)benzoyl]amino}-*N-*(5,6-diméthylpyridin-3-yl)-2-méthylbenzamide ;
le *N-*(3-{[(6-amino-5-chloropyridin-3-yl)amino]carbonyl}-4-méthylphényl)-2-(1-cyano-1-méthyléthyl)isonicotinamide ;
le *N-*(6-amino-5-chloropyridin-3-yl)-2-chloro-5-{[3-(trifluorométhyl)benzoyl]amino}benzamide ;
le *N-*(6-acétylaminopyridin-3-yl)-5-[3-(cyanodiméthylméthyl)benzoylamino]-2-méthylbenzamide ;
le *N-*[6-(acétylamino)pyridin-3-yl]-2-chloro-5-{[3-(trifluorométhyl)benzoyl]amino}benzamide ; et
le *N-*(6-amino-5-méthylpyridin-3-yl)-2-chloro-5-{[3-(1-cyano-1-méthyléthyl)benzoyl]amino}benzamide ;
ou un sel pharmaceutiquement acceptable de celui-ci .

11. Procédé de préparation d'un composé de formule **(I)** ou d'un sel pharmaceutiquement acceptable de celui-ci, lequel procédé, dans lequel les groupes variables sont, sauf spécification contraire, tels que définis dans la revendication 1, comprend :
*Procédé a)* la réaction d'une amine de formule **(II)** 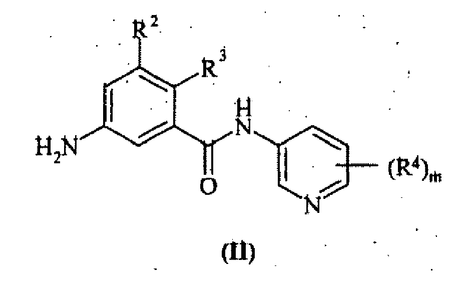 avec un acide de formule **(III) :** 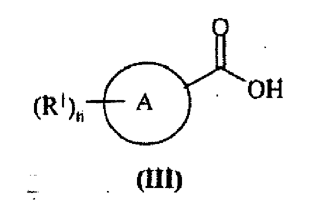 ou un dérivé acide activé de celui-ci ;
*Procédé b)* la réaction d'un acide de formule **(IV) :** 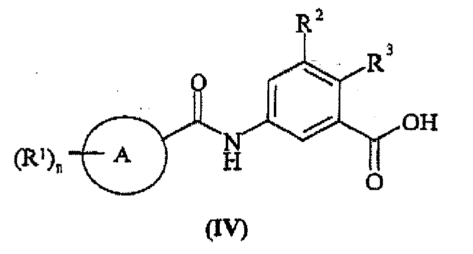 avec une amine de formule **(V) :** 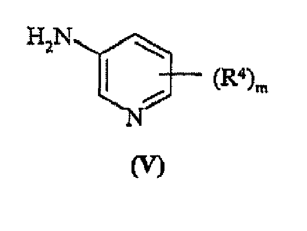
ou un dérivé acide activé de celui-ci ;
et ensuite, le cas échéant :
i) la conversion d'un composé de formule **(I)** en un autre composé de formule **(I) ;**
ii) l'élimination de tout groupe protecteur ;
iii) la formation d'un sel pharmaceutiquement acceptable.

12. Composition pharmaceutique comprenant un composé de formule **(I),** ou un sel pharmaceutiquement acceptable de celui-ci, selon l'une quelconque des revendications 1 à 10, en association avec un diluant ou un support pharmaceutiquement acceptable.

13. Composé de formule **(I),** ou un sel pharmaceutiquement acceptable de celui-ci, selon l'une quelconque des revendications 1 à 10, destiné à être utilisé en tant que médicament.

14. Utilisation d'un composé de formule **(I),** ou d'un sel pharmaceutiquement acceptable de celui-ci, selon l'une quelconque des revendications 1 à 10, pour la fabrication d'un médicament destiné à la production d'un effet inhibiteur de B-Raf chez un animal à sang chaud tel que l'homme.

15. Utilisation d'un composé de formule **(I),** ou d'un sel pharmaceutiquement acceptable de celui-ci, selon l'une quelconque des revendications 1 à 10, pour la fabrication d'un médicament destiné à la production d'un effet anticancéreux chez un animal à sang chaud tel que l'homme.

16. Utilisation d'un composé de formule **(I),** ou d'un sel pharmaceutiquement acceptable de celui-ci, selon l'une quelconque des revendications 1 à 10, pour la fabrication d'un médicament destiné au traitement d'un mélanome, de tumeurs papillaires de la thyroïde, de cholangiocarcinomes, du cancer du colon, du cancer ovarien, du cancer du poumon, de leucémies, de tumeurs malignes lymphoïdes, de carcinomes et de sarcomes dans le foie, le rein, la vessie, la prostate, le sein et le pancréas, et de tumeurs solides primaires et récurrentes de la peau, du colon, de la thyroïde, des poumons et des ovaires.
